# EUROPEAN PATENT APPLICATION

(11) **EP 4 825 442 A2**
(43) Date of publication of application: **21.10.2026**
(21) Application number: 26178499.5
(22) Date of filing: 05.05.2023
(51) Int. Cl.: A61P 21/00

(54) **METHODS FOR TREATING POMPE DISEASE**

(30) Priority: 05.05.2022 US 202263338601 P; 19.08.2022 US 202263399491 P; 19.08.2022 US 202263399498 P; 07.09.2022 US 202263404475 P; 10.10.2022 US 202263414897 P; 30.11.2022 US 202263428970 P; 12.12.2022 US 202263431920 P; 22.12.2022 US 202263434791 P; 21.02.2023 US 202363447222 P
(62) Document number(s) of the earlier application(s) in accordance with Art. 76 EPC: 23728984.8 / 4 518 885
(71) Applicant: Amicus Therapeutics, Inc., Philadelphia, PA 19104 (US)
(72) Inventor: BARTH, Jay, Philadelphia, Pennsylvania, 19104 (US); DAS, Sheela Sitaraman, Philadelphia, Pennsylvania, 19104 (US); CASTELLI, Jeff, Philadelphia, Pennsylvania, 19104 (US); HING, Char., Philadelphia, Pennsylvania, 19104 (US)
(74) Representative: Hoffmann Eitle

(57) **Abstract**

Provided herein are methods for treating Pompe disease by administering to a subject a population of recombinant human acid α-glucosidase molecules or a pharmaceutical composition or formulation thereof, and an enzyme stabilizer.

## Description

### TECHNICAL FIELD

The disclosure relates to methods for treating Pompe disease by administering recombinant human α-glucosidase.

### BACKGROUND

Pompe disease (also known as glycogen storage disease type II (GSD-II) or acid maltase deficiency disease) is an inherited lysosomal storage disease that results from a deficiency in acid α-glucosidase (GAA) activity. A person having Pompe disease lacks or has reduced levels of acid α-glucosidase (GAA), the enzyme which breaks down glycogen to glucose, a main energy source for muscles. This enzyme deficiency causes excess glycogen accumulation in the lysosomes, which are intra-cellular organelles containing enzymes that ordinarily break down glycogen and other cellular debris or waste products. Glycogen accumulation in certain tissues of a subject having Pompe disease, especially muscles, impairs the ability of cells to function normally. In Pompe disease, glycogen is not properly metabolized and progressively accumulates in the lysosomes, especially in skeletal muscle cells and, in the infant onset form of the disease, in cardiac muscle cells. The accumulation of glycogen damages the muscle and nerve cells as well as those in other affected tissues.

Current non-palliative treatment of Pompe disease involves enzyme replacement therapy (ERT) using recombinant alglucosidase alfa products sold under the trademarks LUMIZYME^{®} and MYOZYME^{®} and avalglucosidase alfa products sold under the trademark NEXVIAZYME^{®}. This conventional enzyme replacement therapy seeks to treat Pompe disease by replacing the missing GAA in lysosomes by administering rhGAA thus restoring the ability of cells to break down lysosomal glycogen. LUMIZYME^{®}/MYOZYME^{®} and NEXVIAZYME^{®} are conventional forms of rhGAA produced or marketed as biologics by Sanofi Genzyme and approved by the U.S. Food and Drug Administration and are described by reference to the *Physician's Desk Reference* (2014) (which is hereby incorporated by reference).

However, the currently marketed ERT, at best, offers limited improvement in measures of muscle function, strength and respiratory function for a finite duration followed by slow decline in these parameters (Toscano and Schoser, (2013) J Neurol 260, 951-959; Wyatt et al., (2012) Health Technol Assess 16(39)). There is a need for Pompe disease treatments that offer long term improvement.

### SUMMARY

Provided herein is a method for improving and/or stabilizing motor function and/or pulmonary function for at least 24 months, at least 36 months, or at least 48 months in a subject having Pompe disease, the method comprising administering to the subject a population of recombinant human acid α-glucosidase (rhGAA) molecules, concurrently or sequentially with an enzyme stabilizer; wherein each rhGAA molecule comprises seven potential N-glycosylation sites; wherein 40%-60% of the N-glycans on the rhGAA molecules are complex type N-glycans; wherein the rhGAA molecules comprise at least 0.5 mol bis-mannose-6-phosphate (bis-M6P) per mol of rhGAA at the first potential N-glycosylation site as determined using liquid chromatography tandem mass spectrometry (LC-MS/MS); and wherein the method improves and/or stabilizes motor function, muscle strength, and/or pulmonary function in the subject compared to baseline.

In some embodiments, the subject is an enzyme replacement therapy (ERT)-experienced subject.

In some embodiments of methods for treating an ERT-experienced subject, the motor function is measured by a 6-minute walk test; and the improvement from baseline in 6-minute walk distance (6MWD) is at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 meters at 12 or 24 months after initiation of treatment.

In some embodiments of methods for treating an ERT-experienced subject, the motor function is measured by a 6-minute walk test; and the improvement from baseline in 6-minute walk distance (6MWD) is at least 5, 6, 7, 8, 9, 10, 15, 20, 25, 30, 35, 40, 41, 42, 43, 44, 45, 46, or 47 meters at 36 months after initiation of treatment.

In some embodiments of methods for treating an ERT-experienced subject, the subject has a baseline 6MWD of: (a) at least 300 meters; or (b) less than 300 meters.

In some embodiments of methods for treating an ERT-experienced subject, the muscle strength is measured by a manual muscle test (MMT); and the improvement from baseline in a MMT lower extremity score is at least 1.0, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5 points at 12, 24, 36, 48 months after initiation of treatment.

In some embodiments of methods for treating an ERT-experienced subject, the subject has a baseline MMT lower extremity score of: (a) at least 25; or (b) less than 25.

In some embodiments of methods for treating an ERT-experienced subject, the pulmonary function is measured by a sitting forced vital capacity (FVC) test, and the subject's percent-predicted FVC is stable compared to baseline at 24 months or 36 months after initiation of treatment.

In some embodiments of methods for treating an ERT-experienced subject, the subject has a baseline percent-predicted FVC of: (a) at least 50%; or (b) less than 50%.

In some embodiments, the ERT-experienced subject had been previously treated with alglucosidase alfa. In some embodiments, the ERT-experienced subject had been previously treated with alglucosidase alfa for from about 2 years to about 6 years. In some embodiments, the ERT-experienced subject had been previously treated with alglucosidase alfa for at least about 7 years. In some embodiments, the ERT-experienced subject is non-ambulatory. In some embodiments, the ERT-experienced subject is ambulatory.

In some embodiments, the subject is an ERT-naïve subject.

In some embodiments of methods for treating an ERT-naïve subject, the motor function is measured by a 6-minute walk test; and the improvement from baseline in 6-minute walk distance (6MWD) is at least 40, 41, 42, 43, 44, 45, 46, 47, 48, 49, 50, 51, 52, 53, 54, 55, 56, 57, 58, 59, or 60 meters at 12, 24, 36 or 48 months after initiation of treatment.

In some embodiments of methods for treating an ERT-naïve subject, the motor function is measured by a 6-minute walk test; and the improvement from baseline in 6-minute walk distance (6MWD) is at least 34, 35, 40, 41, 42, 43, 44, or 45 meters at 36 months after initiation of treatment.

In some embodiments of methods for treating an ERT-naïve subject, the subject has a baseline 6MWD of: (a) at least 300 meters; or (b) less than 300 meters.

In some embodiments of methods for treating an ERT-naïve subject, the muscle strength is measured by a manual muscle test (MMT); and the improvement from baseline in a MMT lower extremity score is at least 1.0, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5 points at 12, 24, 36, or 48months after initiation of treatment.

In some embodiments of methods for treating an ERT-naïve subject, the subject has a baseline MMT lower extremity score of: (a) at least 25; or (b) less than 25.

In some embodiments of methods for treating an ERT-naïve subject, the pulmonary function is measured by a sitting forced vital capacity (FVC) test; and the improvement from baseline in the subject's percent-predicted FVC is at least 2.0, 2.5, 3.0, 3.5, 4.0, 4.1, 4.2, 4.3, 4.4, 4.5, 4.6, 4.7, 4.8, 4.9, 5.0, 5.5, 6.0, 6.4, 6.5, 6.6, 6.7 6.8, 7.0, 7.5, 8.0% at 12, 24, 36 or 48 months after initiation of treatment.

In some embodiments of methods for treating an ERT-naïve subject, the pulmonary function is measured by a sitting forced vital capacity (FVC) test; and the improvement from baseline in the subject's percent-predicted FVC is at least 5.7, 5.8, 5.9, 6.0, 6.1, or 6.2% at 36 months after initiation of treatment.

In some embodiments of methods for treating an ERT-naïve subject, the subject has a baseline percent-predicted FVC of a) at least 50%; or (b) less than 50%.

In some embodiments of any of the methods disclosed herein, the method further reduces the levels of at least one marker of muscle damage and/or at least one marker of glycogen accumulation in the subject compared to baseline. In some embodiments, the at least one marker of muscle damage is creatine kinase (CK), and/or the at least one marker of glycogen accumulation is urine hexose tetrasaccharide (Hex4).

In some embodiments, the population of rhGAA molecules is administered at a dose of 5 mg/kg to 20 mg/kg, optionally 20 mg/kg.

In some embodiments, the population of rhGAA molecules is administered bi-weekly. In some embodiments, the population of rhGAA molecules is administered intravenously.

In some embodiments, the enzyme stabilizer is miglustat or a pharmaceutically acceptable salt thereof, wherein further optionally the miglustat or pharmaceutically acceptable salt thereof is administered orally. In some embodiments, the miglustat or pharmaceutically acceptable salt thereof is administered at a dose of 195 mg or 260 mg.

In some embodiments, the miglustat or pharmaceutically acceptable salt thereof is administered prior to administration of the population of rhGAA molecules, optionally one hour prior to administration of the population of rhGAA molecules. In some embodiments, the subject fasts for at least two hours before and at least two hours after the administration of miglustat or a pharmaceutically acceptable salt thereof.

In some embodiments, the rhGAA molecules comprise an amino acid sequence at least 95% identical to SEQ ID NO: 4 or SEQ ID NO: 6.

In some embodiments, the rhGAA molecules comprise the amino acid sequence of SEQ ID NO: 4 or SEQ ID NO: 6.

In some embodiments, at least 30% of the rhGAA molecules comprise one or more N-glycan units bearing one mannose-6-phosphate residue (mono-M6P) or bis-M6P, as determined using LC-MS/MS.

In some embodiments, the rhGAA molecules comprise on average from 0.5 mol to 7.0 mol of mono-M6P or bis-M6P per mol of rhGAA, as determined using LC-MS/MS.

In some embodiments, the rhGAA molecules comprise on average from 2.0 to 8.0 mol of sialic acid per mol of rhGAA, as determined using LC-MS/MS.

In some embodiments, the rhGAA molecules comprise on average at least 2.5 mol M6P per mol of rhGAA and at least 4 mol sialic acid per mol of rhGAA, as determined using LC-MS/MS.

In some embodiments, per mol of rhGAA, the rhGAA molecules comprise on average: (a) 0.4 to 0.6 mol mono-M6P at the second potential N-glycosylation site; (b) 0.4 to 0.6 mol bis-M6P at the fourth potential N-glycosylation site; or (c) 0.3 to 0.4 mol mono-M6P at the fourth potential N-glycosylation site; wherein (a)-(c) are determined using LC-MS/MS.

In some embodiments, per mol of rhGAA, the rhGAA molecules further comprise 4 mol to 7.3 mol sialic acid; and, per mol of rhGAA, the rhGAA molecules comprise on average: (a) 0.9 to 1.2 mol sialic acid at the third potential N-glycosylation site; (b) 0.8 to 0.9 mol sialic acid at the fifth potential N-glycosylation site; or (c) 1.5 to 4.2 mol sialic acid at the sixth potential N-glycosylation site; wherein (a)-(c) are determined using LC-MS/MS.

In some embodiments, the population of rhGAA molecules is formulated in a pharmaceutical composition further comprising at least one pharmaceutically acceptable buffer, excipient, or carrier.

In some embodiments, the pharmaceutical composition further comprises at least one buffer selected from the group consisting of a citrate, a phosphate, and a combination thereof, and at least one excipient selected from the group consisting of mannitol, polysorbate 80, and a combination thereof; wherein the pharmaceutical composition has a pH of 5.0 to 7.0.

In some embodiments, the pharmaceutical composition has a pH of 5.0 to 6.0.

In some embodiments, the pharmaceutical composition further comprises water, an acidifying agent, an alkalizing agent, or a combination thereof.

In some embodiments, in the pharmaceutical composition, the population of rhGAA molecules is present at a concentration of 5-50 mg/mL, the at least one buffer is a sodium citrate buffer present at a concentration of 10-100 mM, the at least one excipient is mannitol present at a concentration of 10-50 mg/mL and polysorbate 80 present at a concentration of 0.1-1 mg/mL, and the pharmaceutical composition further comprises water and optionally comprises an acidifying agent and/or alkalizing agent; wherein the pharmaceutical composition has a pH of 6.0.

In some embodiments, in the pharmaceutical composition, the population of rhGAA molecules is present at a concentration of 15 mg/mL, the sodium citrate buffer is present at a concentration of 25 mM, the mannitol is present at a concentration of 20 mg/mL, and the polysorbate 80 is present at a concentration of 0.5 mg/mL.

In some embodiments, the rhGAA is produced from Chinese hamster ovary cells.

### BRIEF DESCRIPTION OF THE DRAWINGS

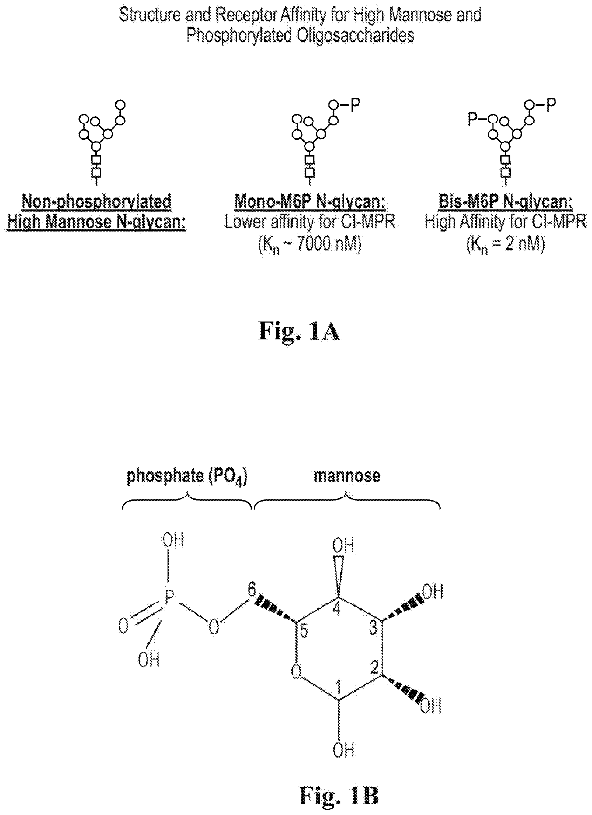

FIG. 1A shows non-phosphorylated high mannose N-glycan, a mono-M6P N-glycan, and a bis-M6P N-glycan. Fig. 1B shows the chemical structure of the M6P group. Each square represents N-acetylglucosamine (GlcNAc), each circle represents mannose, and each P represents phosphate.
FIG. 2A describes productive targeting of rhGAA via N-glycans bearing M6P to target tissues (e.g., muscle tissues of subject with Pompe Disease). FIG. 2B describes non-productive drug clearance to non-target tissues (e.g., liver and spleen) or by binding of non-M6P N-glycans to non-target tissues.
FIG. 3 is a schematic diagram of an exemplary process for the manufacturing, capturing and purification of a recombinant lysosomal protein.
FIG. 4 shows a DNA construct for transforming CHO cells with DNA encoding rhGAA.
FIG. 5 is a graph showing the results of CIMPR affinity chromatography of ATB200 rhGAA with (Embodiment 2) and without (Embodiment 1) capture on an anion exchange (AEX) column.

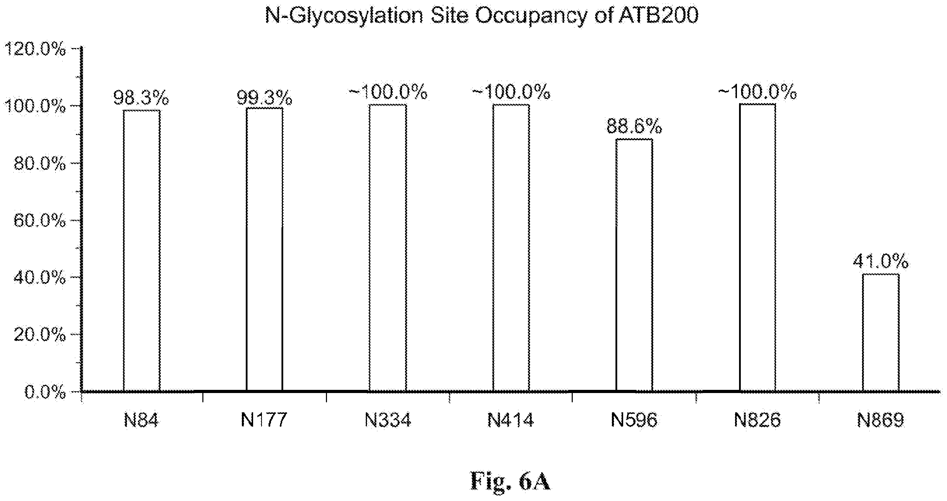

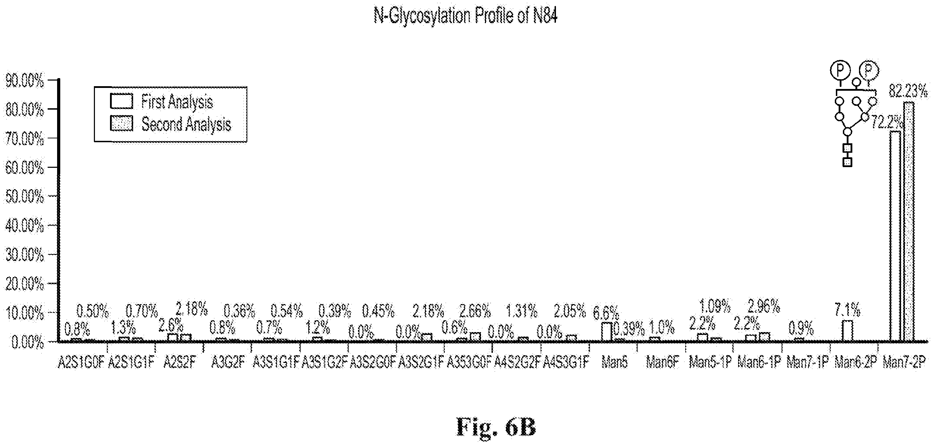

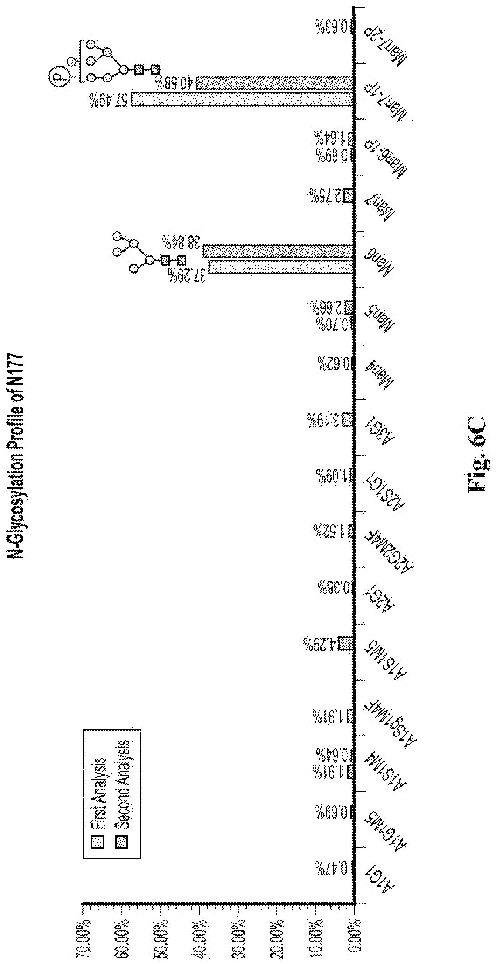

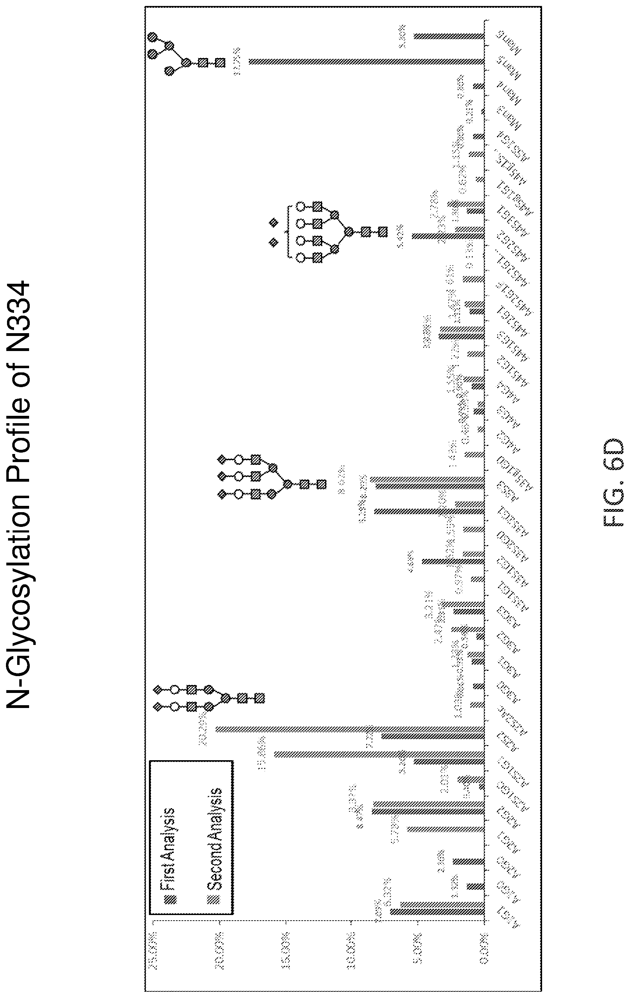

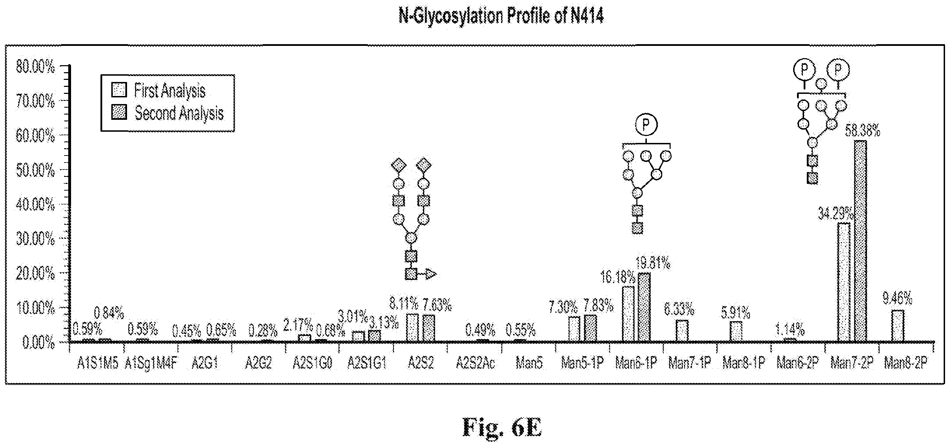

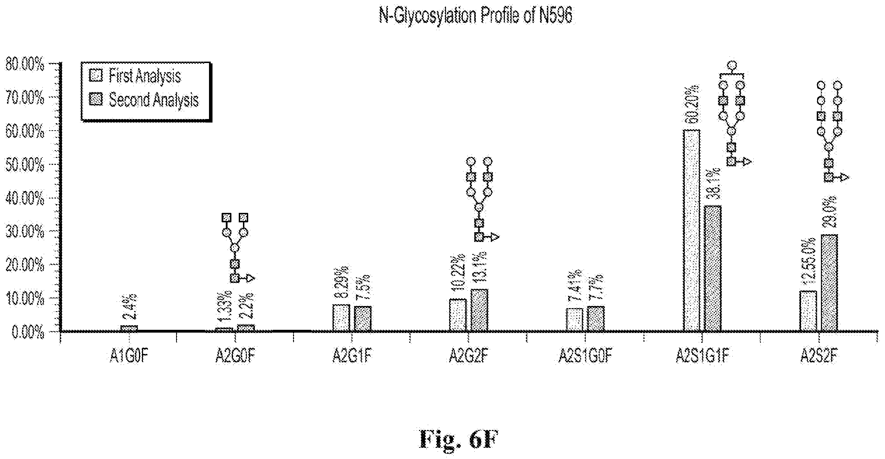

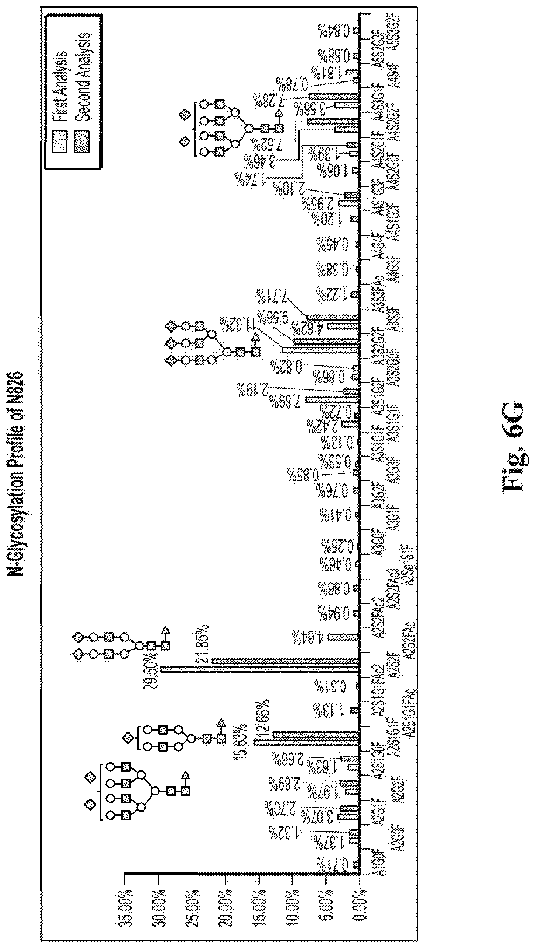

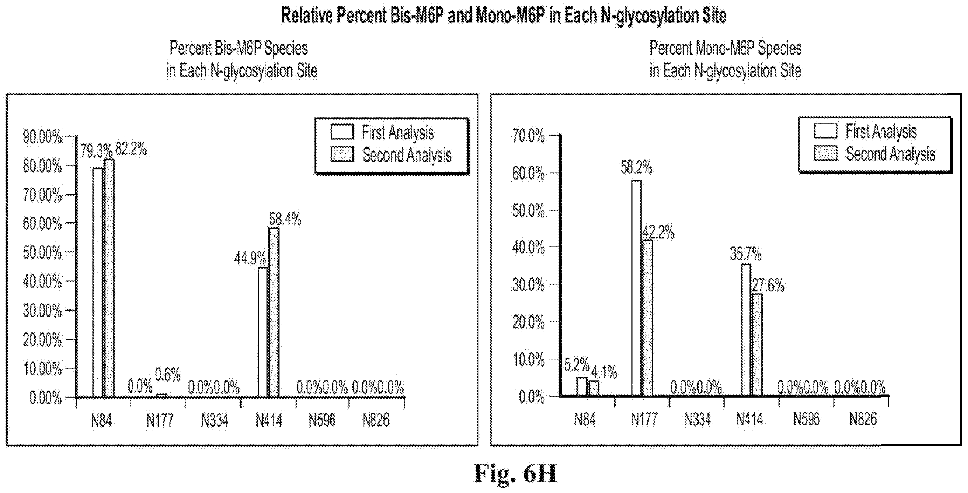

FIG. 6A - FIG. 6H show the results of a site-specific N-glycosylation analysis of ATB200 rhGAA, using two different LC-MS/MS analytical techniques. FIG. 6A shows the site occupancy of the seven potential N-glycosylation sites for ATB200. FIG. 6B shows two analyses of the N-glycosylation profile of the first potential N-glycosylation site for ATB200. FIG. 6C shows two analyses of the N-glycosylation profile of the second potential N-glycosylation site for ATB200. FIG. 6D shows two analyses of the N-glycosylation profile of the third potential N-glycosylation site for ATB200. FIG. 6E shows two analyses of the N-glycosylation profile of the fourth potential N-glycosylation site for ATB200. FIG. 6F shows two analyses of the N-glycosylation profile of the fifth potential N-glycosylation site for ATB200. FIG. 6G shows two analyses of the N-glycosylation profile of the sixth potential N-glycosylation site for ATB200. FIG. 6H summarizes the relative percent mono-phosphorylated and bis-phosphorylated species for the first, second, third, fourth, fifth, and sixth potential N-glycosylation sites.
FIG. 7 is a graph showing Polywax elution profiles of LUMIZYME^{®} (thinner line, eluting to the left) and ATB200 (thicker line, eluting to the right).
FIG. 8 is a table showing a summary of N-glycan structures of LUMIZYME^{®} compared to three different preparations of ATB200 rhGAA, identified as BP-rhGAA, ATB200-1 and ATB200-2.
FIG. 9A and FIG. 9B are graphs showing the results of CIMPR affinity chromatography of LUMIZYME^{®} and MYOZYME^{®}, respectively.
FIG. 10 is a graph comparing the CIMPR binding affinity of ATB200 rhGAA (left trace) with that of LUMIZYME^{®} (right trace).
FIG. 11A is a graph comparing ATB200 rhGAA activity (left trace) with LUMIZYME^{®} rhGAA activity (right trace) inside normal fibroblasts at various GAA concentrations. FIG. 11B is a table comparing ATB200 rhGAA activity (left trace) with LUMIZYME^{®} rhGAA activity (right trace) inside fibroblasts from a subject having Pompe Disease at various GAA concentrations. FIG. 11C is a table comparing Kᵤₚₜₐₖₑ of fibroblasts from normal subjects and subjects with Pompe disease.
FIG. 12 depicts the stability of ATB200 in acidic or neutral pH buffers evaluated in a thermostability assay using SYPRO Orange, as the fluorescence of the dye increases when proteins denature.
FIG. 13 shows tissue glycogen content of WT mice or *Gaa* KO mice treated with a vehicle, alglucosidase alfa, or ATB200/miglustat, determined using amyloglucosidase digestion. Bars represent Mean ± SEM of 7 mice/group. * p<0.05 compared to alglucosidase alfa in multiple comparison using Dunnett's method under one-way ANOVA analysis.
FIG. 14 depicts LAMP1-positive vesicles in muscle fibers of *Gaa* KO mice treated with a vehicle, alglucosidase alfa, or ATB200/miglustat or WT mice. Images were taken from *vastus lateralis* and were representative of 7 mice per group. Magnification = 200x (1,000x in insets).
FIG. 15A shows LC3-positive aggregates in muscle fibers of *Gaa* KO mice treated with a vehicle, alglucosidase alfa, or ATB200/miglustat or WT mice. Images were taken from *vastus lateralis* and were representative of 7 mice per group. Magnification = 400x. FIG. 15B shows a western blot analysis of LC3 II protein. A total of 30 mg protein was loaded in each lane.
FIG. 16 shows Dysferlin expression in muscle fibers of *Gaa* KO mice treated with a vehicle, alglucosidase alfa, or ATB200/miglustat or WT mice. Images were taken from *vastus lateralis* and were representative of 7 mice per group. Magnification = 200x.
FIG. 17 depicts co-immunofluorescent staining of LAMP1 (green) (see for example, "B") and LC3 (red) (see, for example, "A") in single fibers isolated from the white gastrocnemius of *Gaa* KO mice treated with a vehicle, alglucosidase alfa, or ATB200. "C" depicts clearance of autophagic debris and absence of enlarged lysosome. A minimum of 30 fibers were examined from each animal.
FIG. 18 depicts stabilization of ATB200 by miglustat at 17 µM, and 170 µM miglustat, respectively, as compared to ATB200 alone.
FIG. 19A - FIG. 19H show the results of a site-specific N-glycosylation analysis of ATB200 rhGAA, including an N-glycosylation profile for the seventh potential N-glycosylation site, using LC-MS/MS analysis of protease-digested ATB200. FIG. 19A - FIG. 19H provide average data for ten lots of ATB200 produced at different scales.

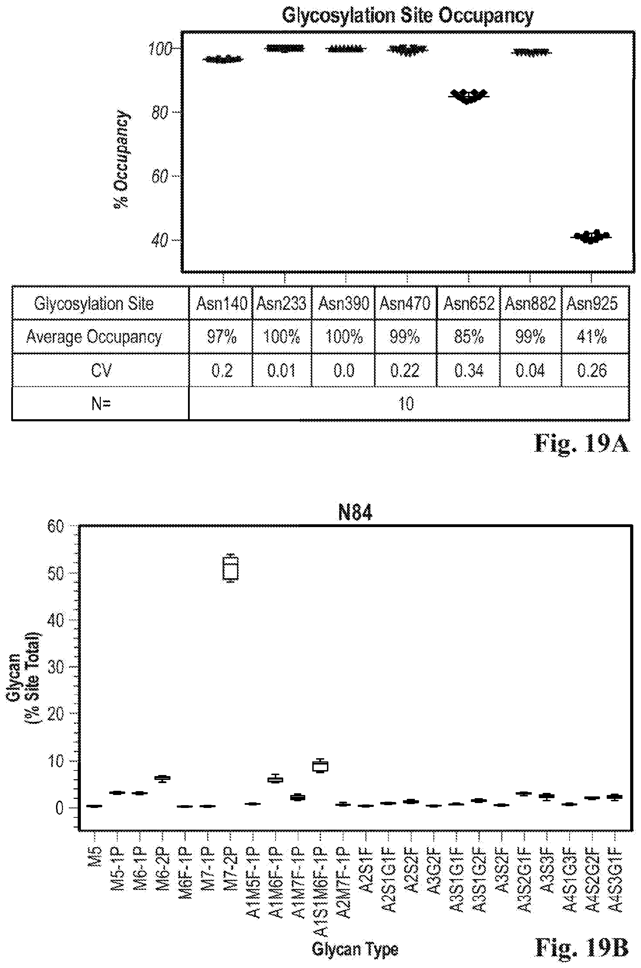

FIG. 19A shows the average site occupancy of the seven potential N-glycosylation sites for ATB200. The N-glycosylation sites are provided according to SEQ ID NO: 1. CV = coefficient of variation.

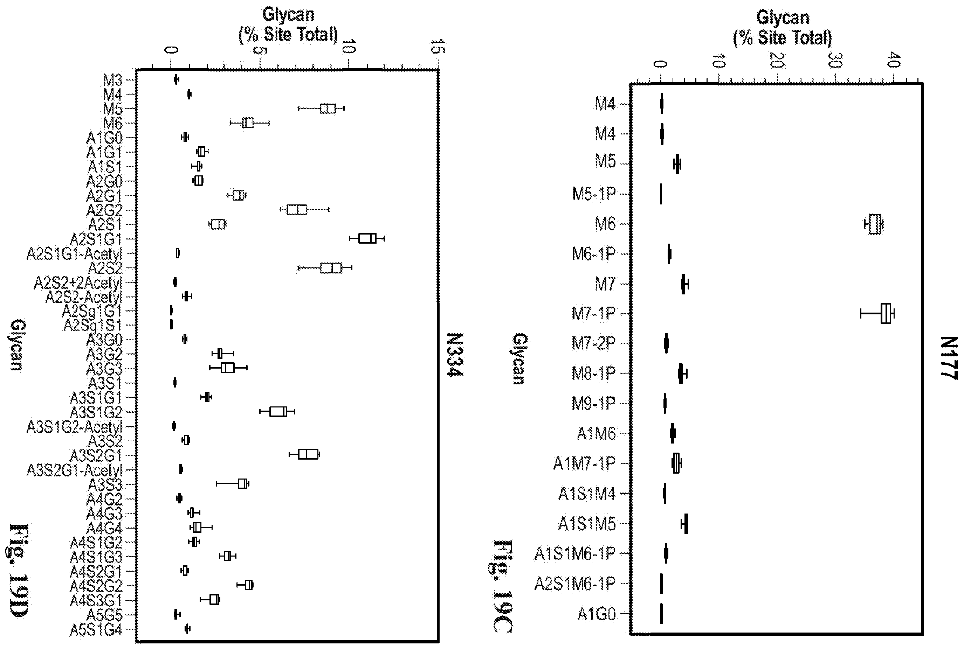

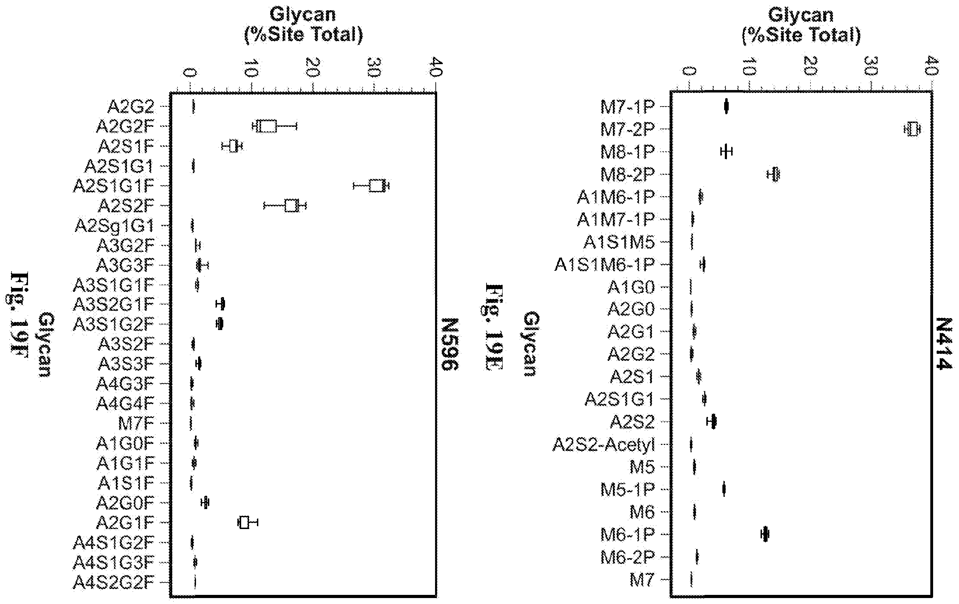

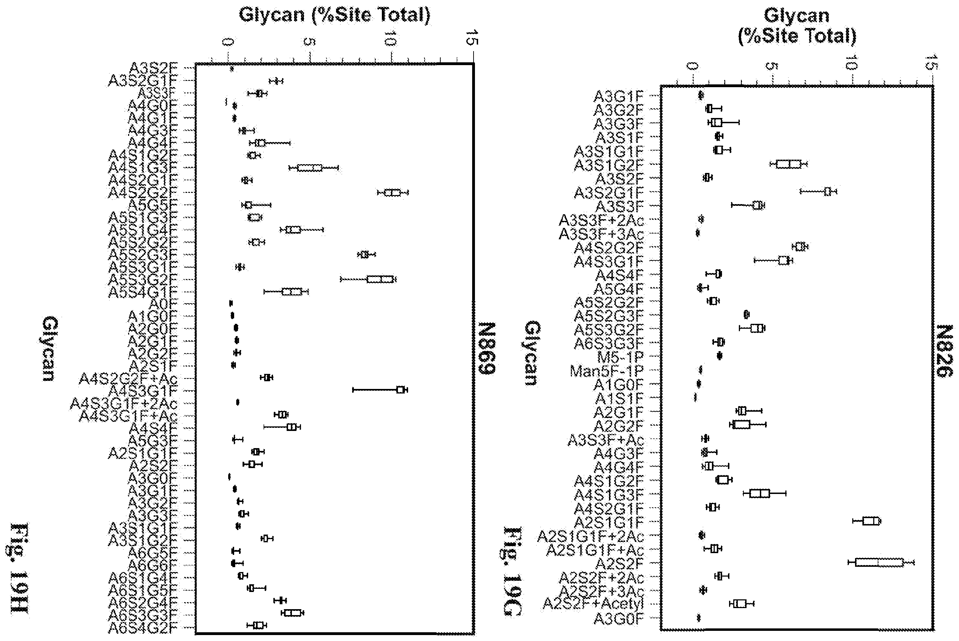

FIG. 19B - FIG. 19H show the site-specific N-glycosylation analyses of all seven potential N-glycosylation sites for ATB200, with site numbers provided according to SEQ ID NO: 5. Bars represent the maximum and minimum percentage of N-glycan species identified as a particular N-glycan group for the ten lots of ATB200 analyzed. FIG. 19B shows the N-glycosylation profile of the first potential N-glycosylation site for ATB200. FIG. 19C shows the N-glycosylation profile of the second potential N-glycosylation site for ATB200. FIG. 19D shows the N-glycosylation profile of the third potential N-glycosylation site for ATB200. FIG. 19E shows the N-glycosylation profile of the fourth potential N-glycosylation site for ATB200. FIG. 19F shows the N-glycosylation profile of the fifth potential N-glycosylation site for ATB200. FIG. 19G shows the N-glycosylation profile of the sixth potential N-glycosylation site for ATB200. FIG. 19H shows the N-glycosylation profile of the seventh potential N-glycosylation site for ATB200.

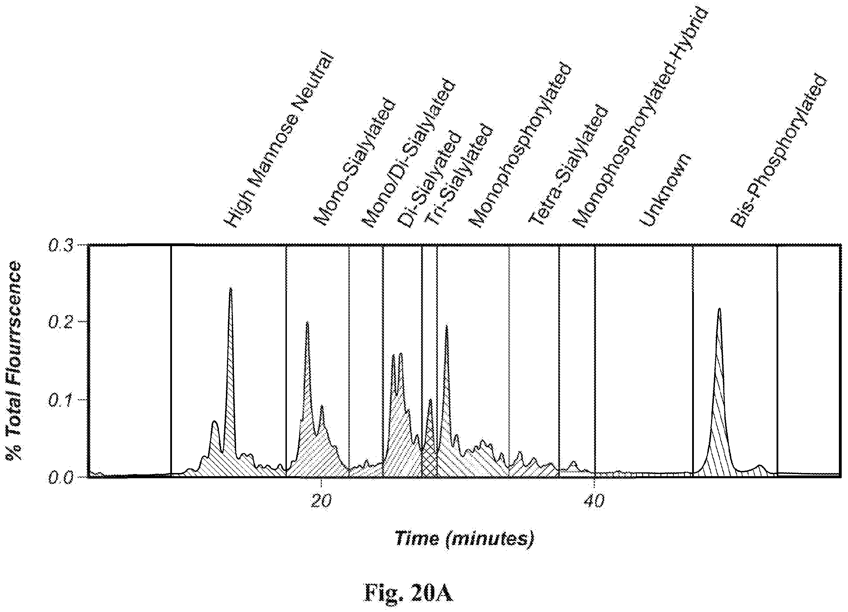

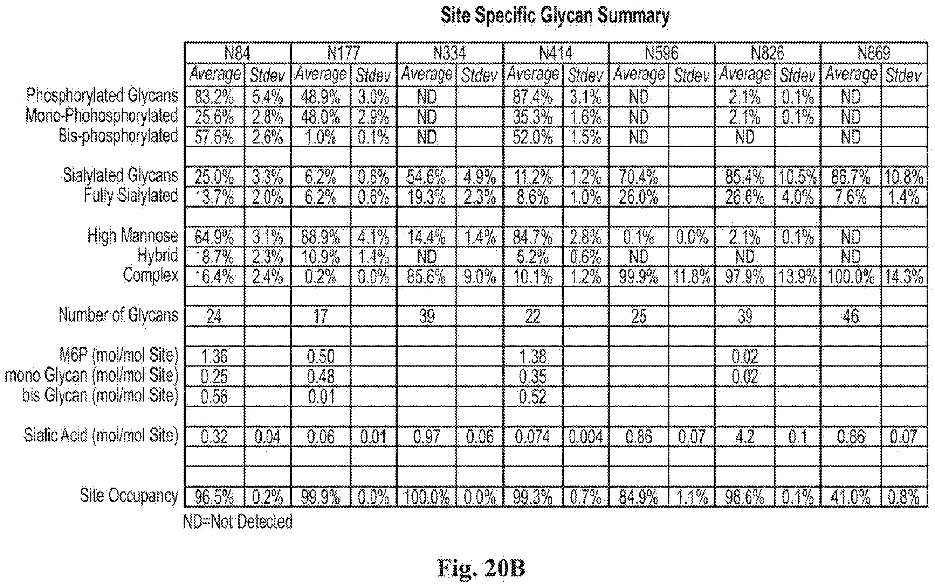

FIG. 20A - FIG. 20B further characterize and summarize the N-glycosylation profile of ATB200, as also shown in Figs. 19A-19H. FIG. 20A shows 2-Anthranilic acid (2-AA) glycan mapping and LC/MS-MS analysis of ATB200 and summarizes the N-glycan species identified in ATB200 as a percentage of total fluorescence. Data from 2-AA glycan mapping and LC-MS/MS analysis are also depicted in Table 6. FIG. 20B summarizes the average site occupancy and average N-glycan profile, including total phosphorylation, mono-phosphorylation, bis-phosphorylation, and sialylation, for all seven potential N-glycosylation sites for ATB200. ND = not detected.

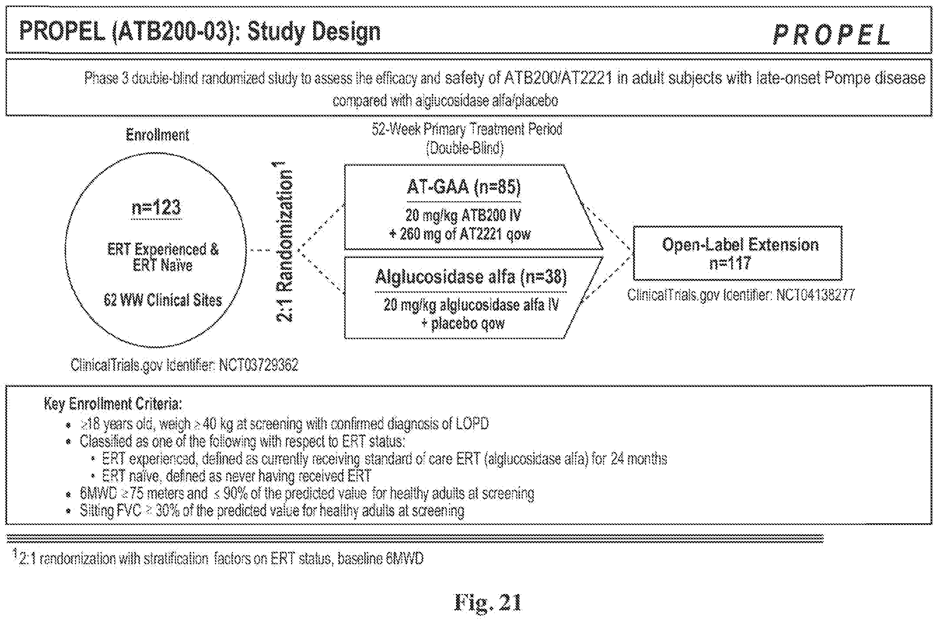

FIG. 21 shows the ATB200-03 study design schematic.

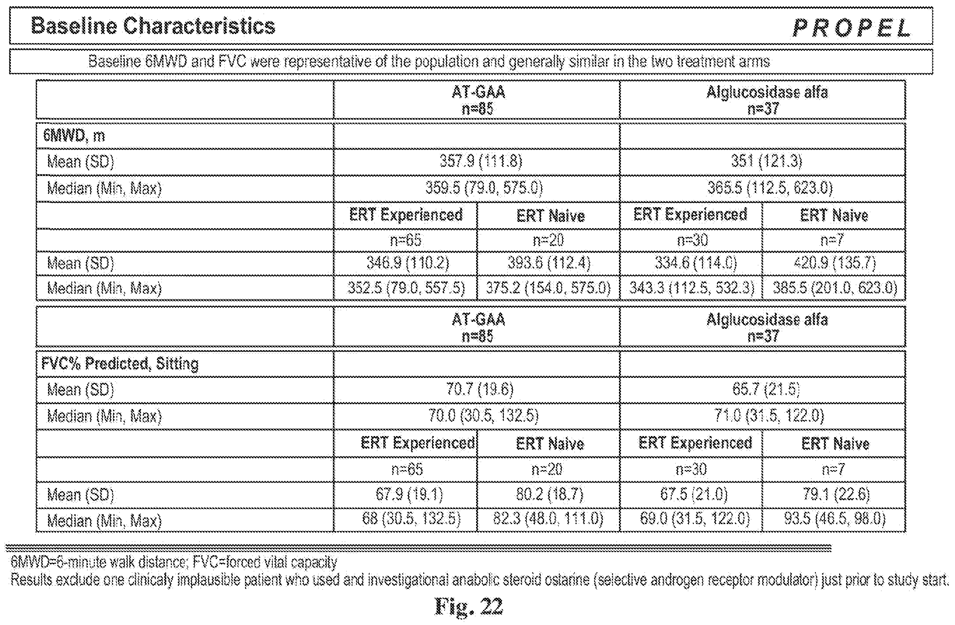

FIG. 22 shows the baseline 6-minute walk distance (6MWD) and sitting forced vital capacity (FVC) characteristics of the 122 subjects who participated in the ATB200-03 study. AT-GAA group: subjects who received the ATB200/miglustat treatment; Alglucosidase alfa group: subjects who received the alglucosidase alfa/placebo treatment.

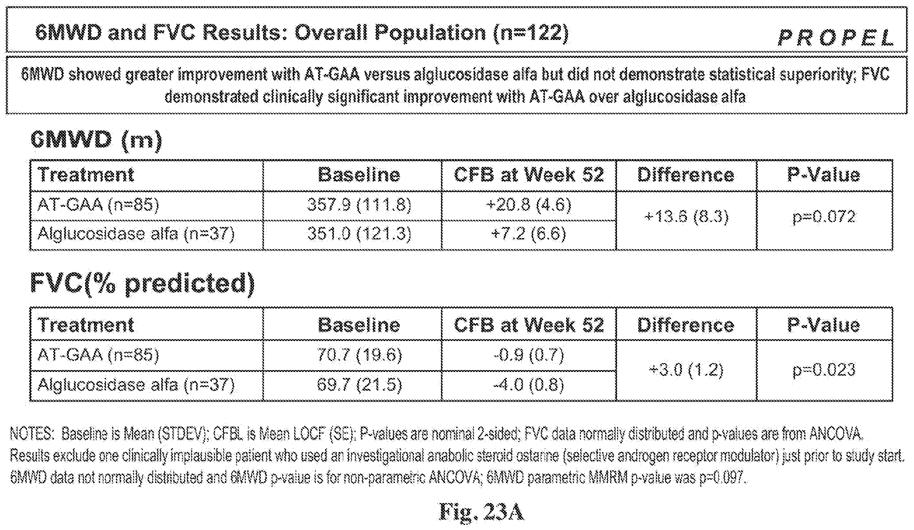

FIG. 23A depicts the 6MWD and FVC data, showing the baseline, change from the baseline ("CFBL") at week 52, difference, and P-value, for the overall population (n=122). AT-GAA group: subjects who received the ATB200/miglustat treatment; Alglucosidase alfa group: subjects who received the alglucosidase alfa/placebo treatment.

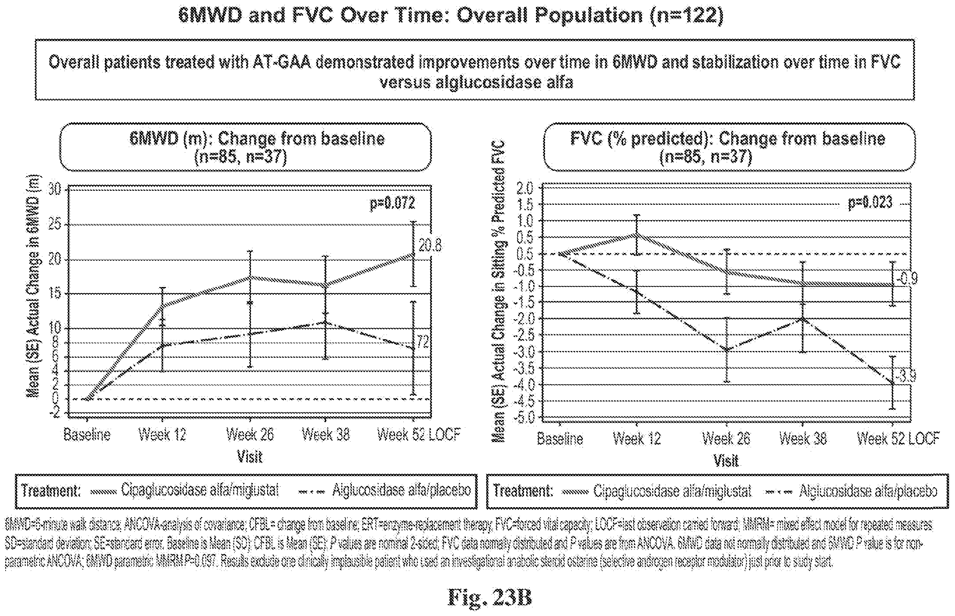

FIG. 23B depicts the 6MWD and FVC data showing change from the baseline over time for the overall population (n=122). Cipaglucosidase alfa/miglustat group: subjects who received the ATB200/miglustat treatment; Alglucosidase alfa/placebo: subjects who received the alglucosidase alfa/placebo treatment.

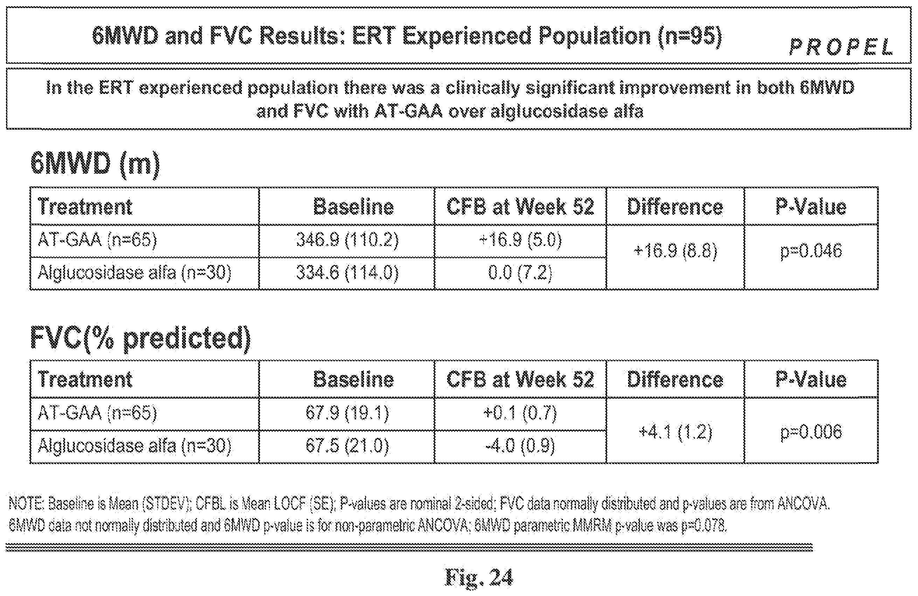

FIG. 24 depicts the 6MWD and FVC data, showing the baseline, CFBL at week 52, difference, and P-value, for the ERT-experienced population (n=95). AT-GAA group: subjects who received the ATB200/miglustat treatment; Alglucosidase alfa group: subjects who received the alglucosidase alfa/placebo treatment.

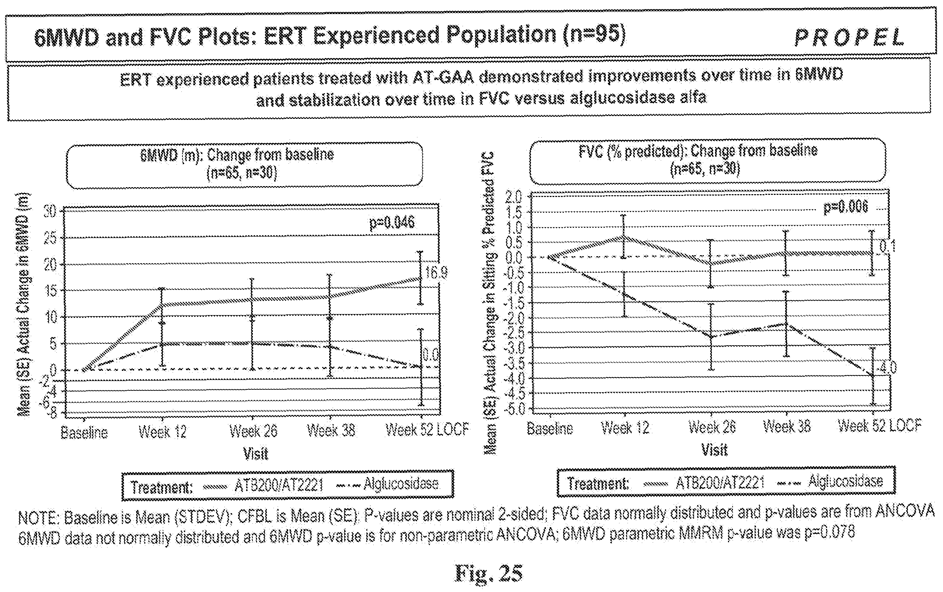

FIG. 25 depicts the 6MWD and FVC changes relative to baseline at week 12, week 26, and week 38, and week 52, for the ERT-experienced population (n=95).

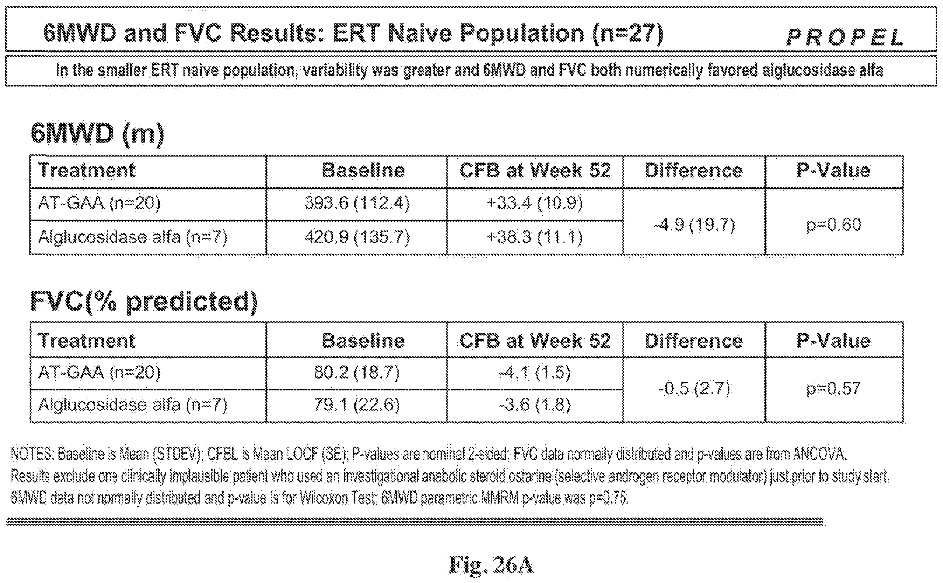

FIG. 26A depicts the 6MWD and FVC data, showing the baseline, CFBL at week 52, difference, and P-value, for the ERT-naïve population (n=27). AT-GAA group: subjects who received the ATB200/miglustat treatment; Alglucosidase alfa group: subjects who received the alglucosidase alfa/placebo treatment.

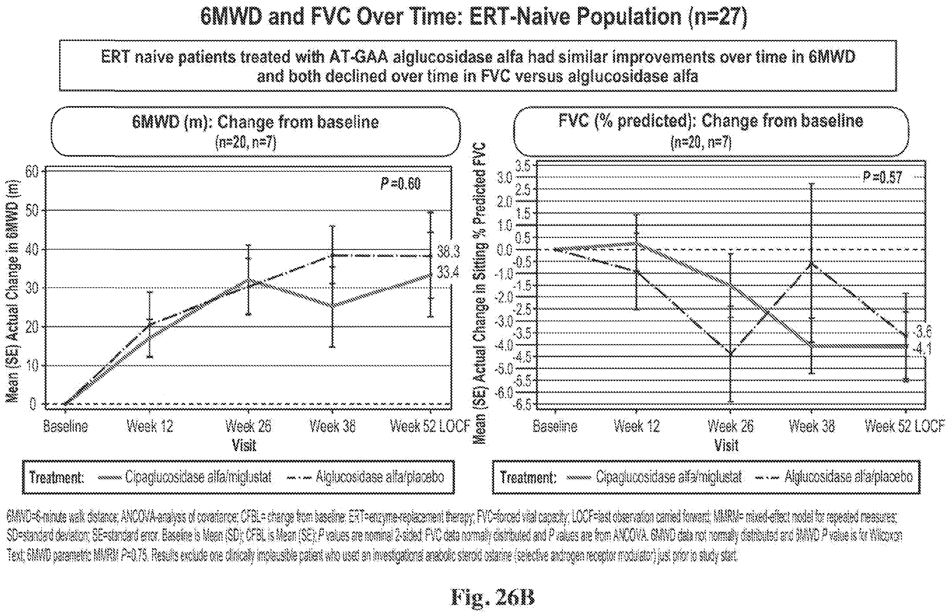

FIG. 26B depicts the 6MWD and FVC data showing change from the baseline over time for the ERT-naïve population (n=27). Cipaglucosidase alfa/miglustat group: subjects who received the ATB200/miglustat treatment; Alglucosidase alfa/placebo: subjects who received the alglucosidase alfa/placebo treatment.

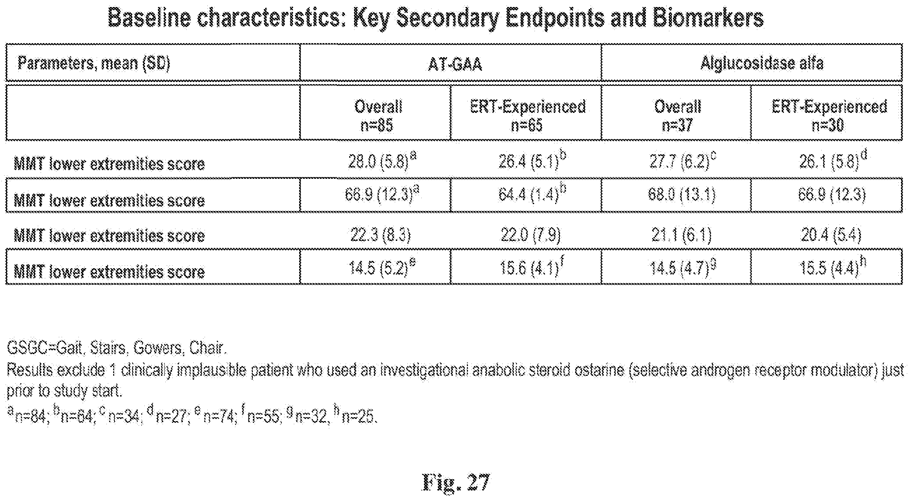

FIG. 27 depicts baseline characteristics on key secondary endpoints and biomarkers for the overall and ERT-experienced populations. AT-GAA group: subjects who received the ATB200/miglustat treatment; Alglucosidase alfa group: subjects who received the alglucosidase alfa/placebo treatment.

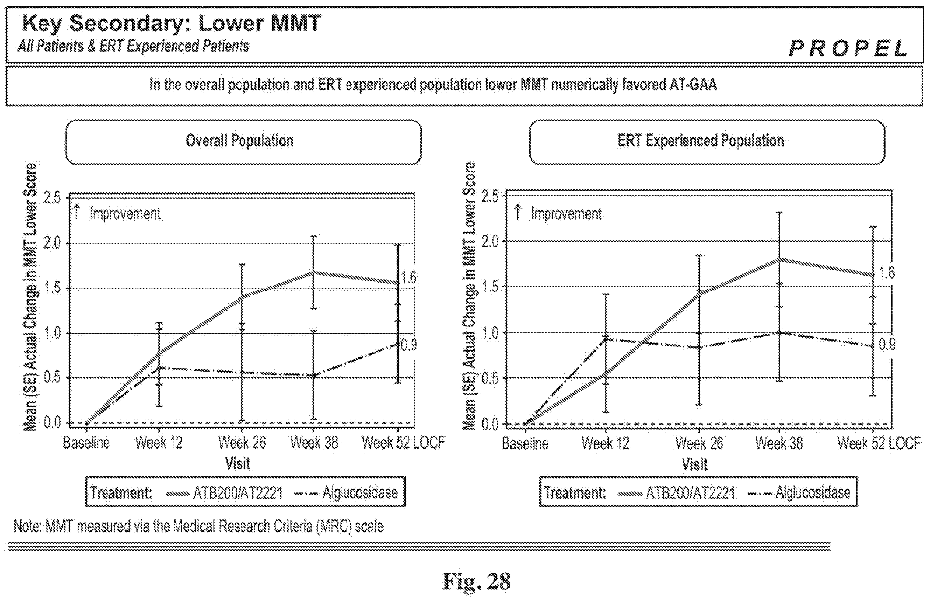

FIG. 28 depicts the lower manual muscle testing (MMT) changes relative to baseline at week 12, week 26, week 38, and week 52, for the overall population (left) and ERT-experienced population (right).

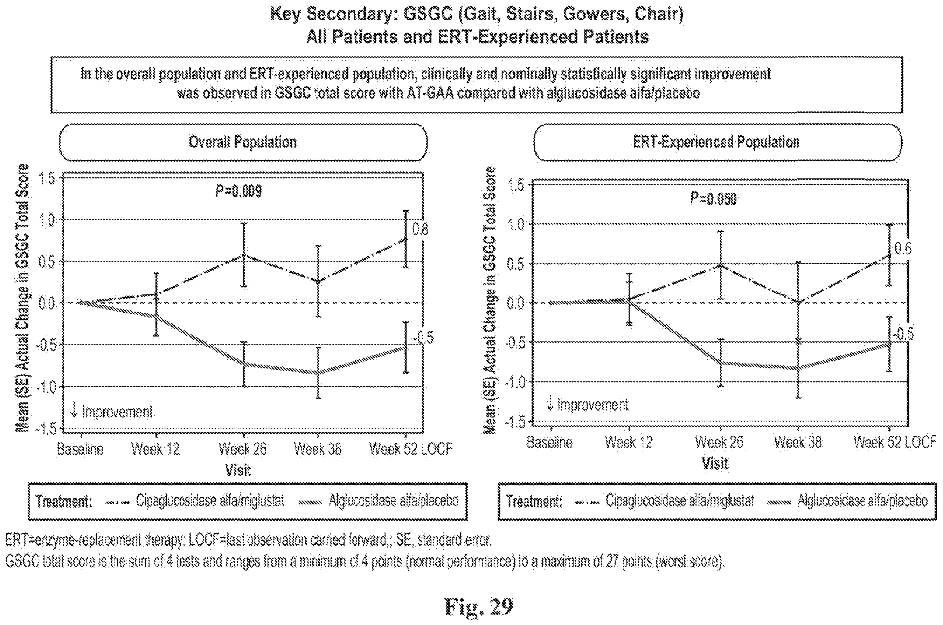

FIG. 29 depicts the gait, stairs, gowers, chair (GSGC) changes relative to baseline at week 12, week 26, week 38, and week 52, for the overall population (left) and ERT-experienced population (right). Cipaglucosidase alfa/miglustat group: subjects who received the ATB200/miglustat treatment; Alglucosidase alfa/placebo: subjects who received the alglucosidase alfa/placebo treatment.

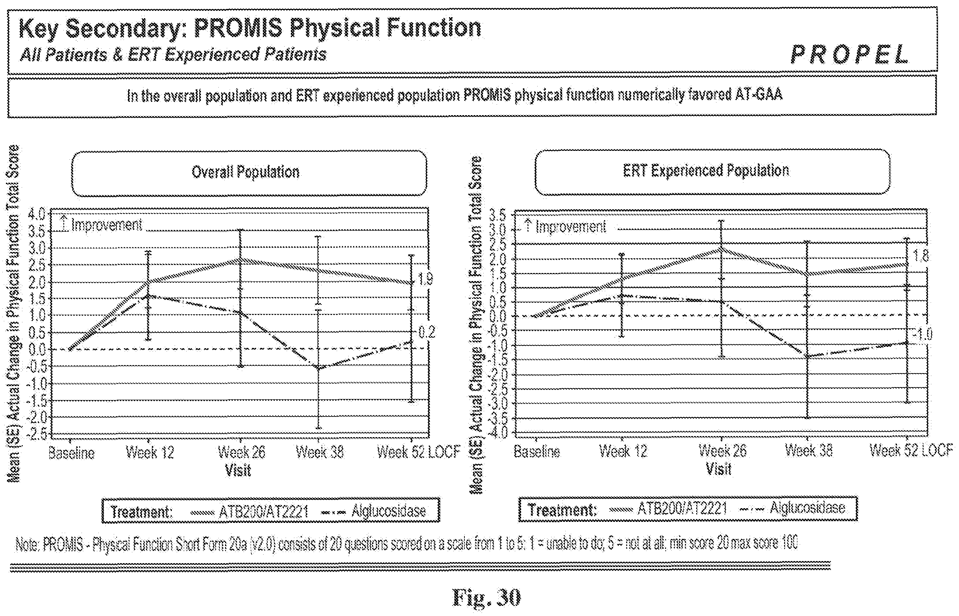

FIG. 30 depicts the patient-reported outcomes measurement information system (PROMIS) for physical function changes relative to baseline at week 12, week 26, week 38, and week 52, for the overall population (left) and ERT-experienced population (right).

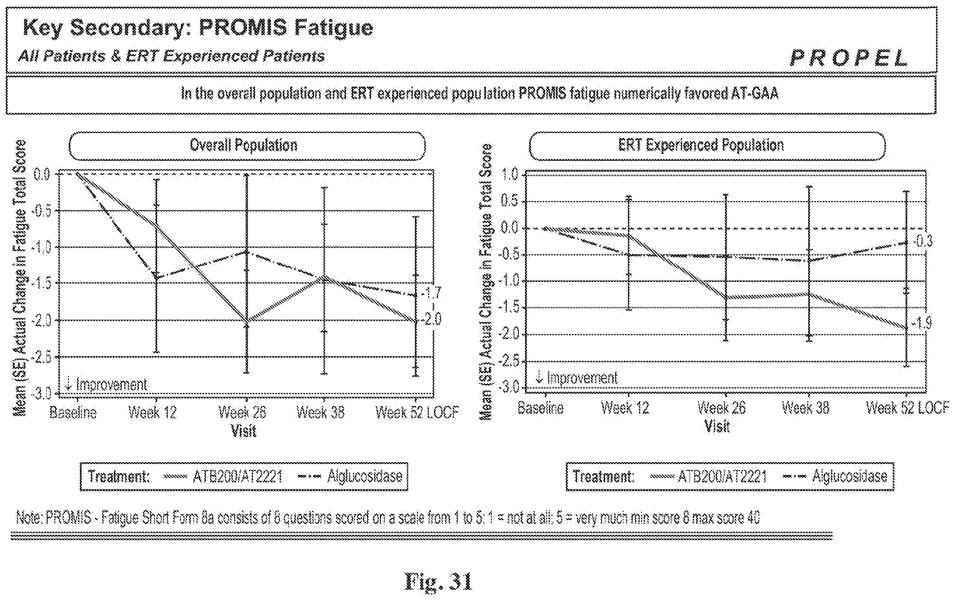

FIG. 31 depicts the PROMIS for fatigue changes relative to baseline at week 12, week 26, week 38, and week 52, for the overall population (left) and ERT-experienced population (right).

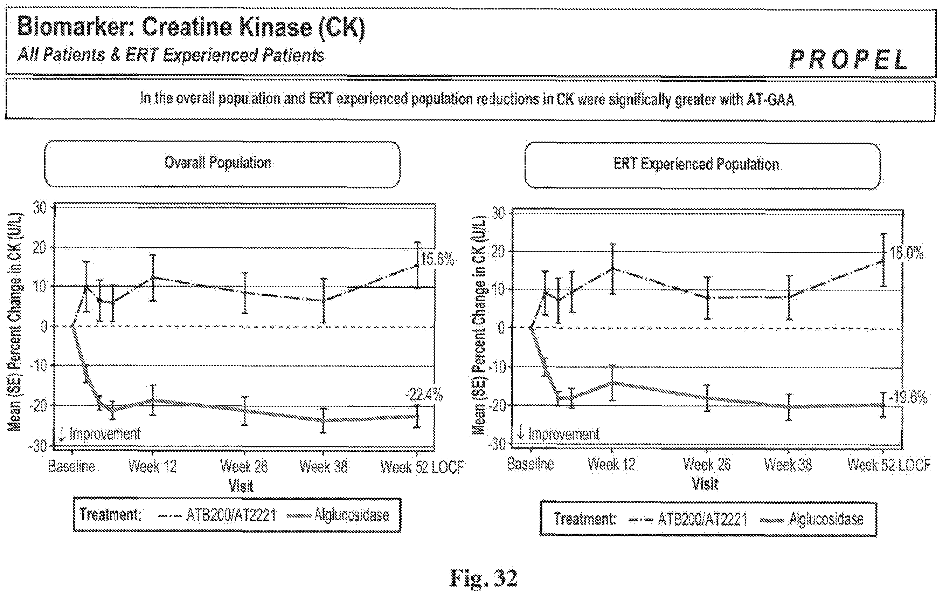

FIG. 32 depicts the creatine kinase (CK) biomarker changes relative to baseline at week 12, week 26, week 38, and week 52, for the overall population (left) and ERT-experienced population (right).

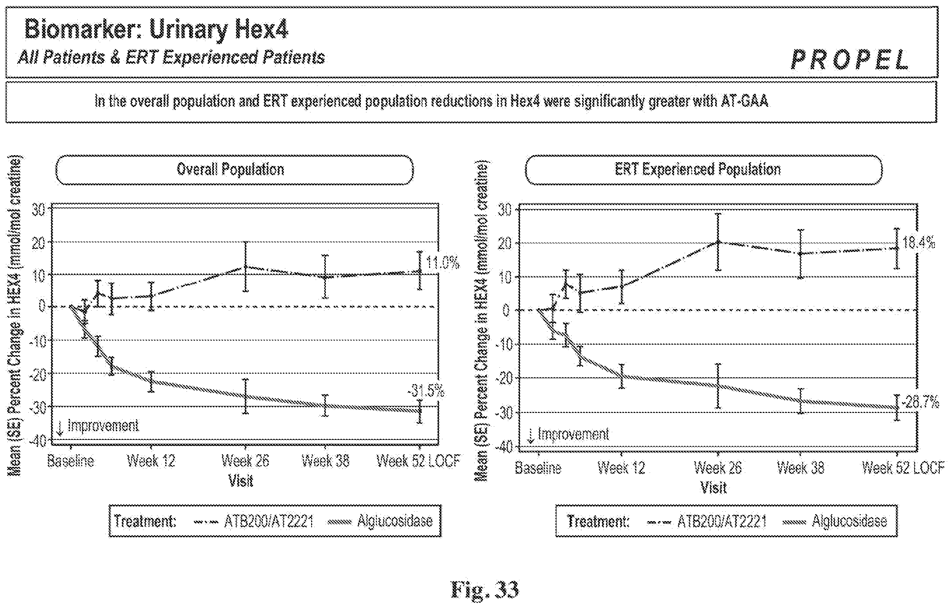

FIG. 33 depicts the urine hexose tetrasaccharide (Hex4) biomarker changes relative to baseline at week 12, week 26, week 38, and week 52, for the overall population (left) and ERT-experienced population (right).

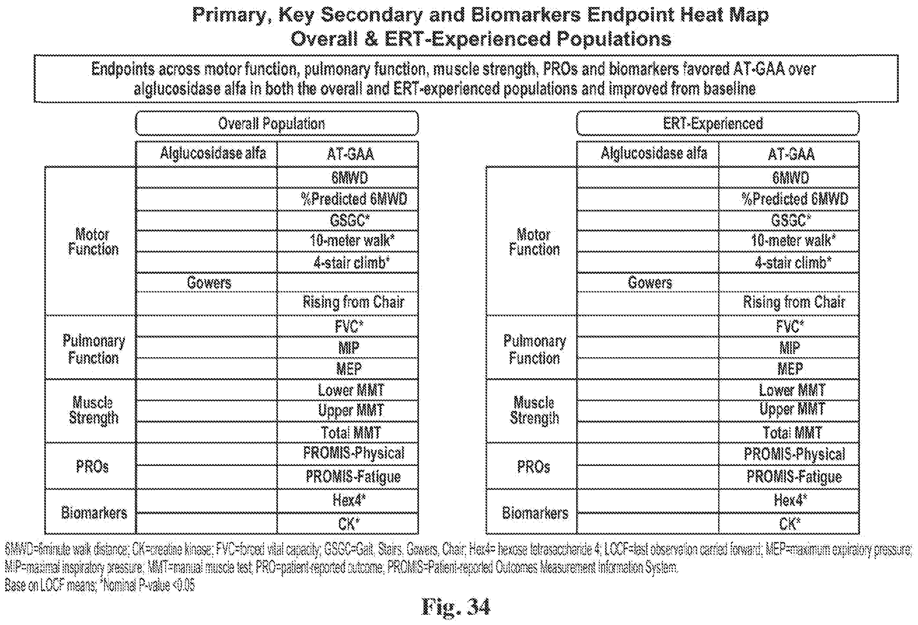

FIG. 34 shows the primary, secondary and biomarker endpoint heat map for the overall population (left) and ERT-experienced population (right). AT-GAA group: subjects who received the ATB200/miglustat treatment; Alglucosidase alfa group: subjects who received the alglucosidase alfa/placebo treatment.

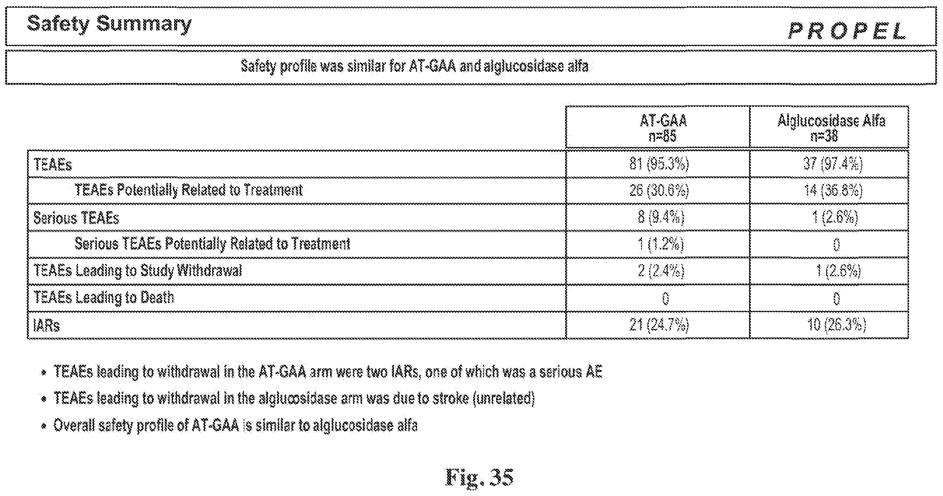

FIG. 35 summarizes the safety data from the ATB200-03 study. AT-GAA group: subjects who received the ATB200/miglustat treatment; Alglucosidase alfa group: subjects who received the alglucosidase alfa/placebo treatment. TEAE: treatment emergent adverse event; IAR: infusion-associated reaction.

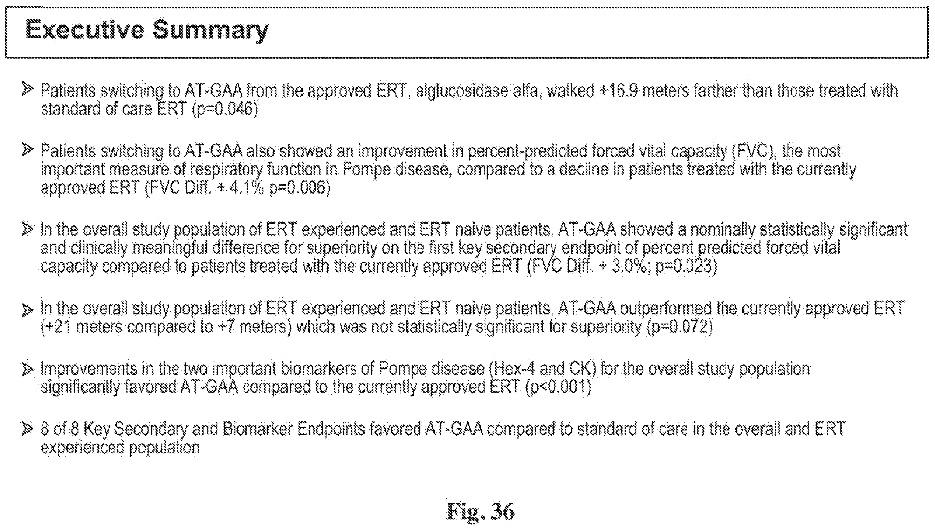

FIG. 36 summarizes results from the ATB200-03 study.

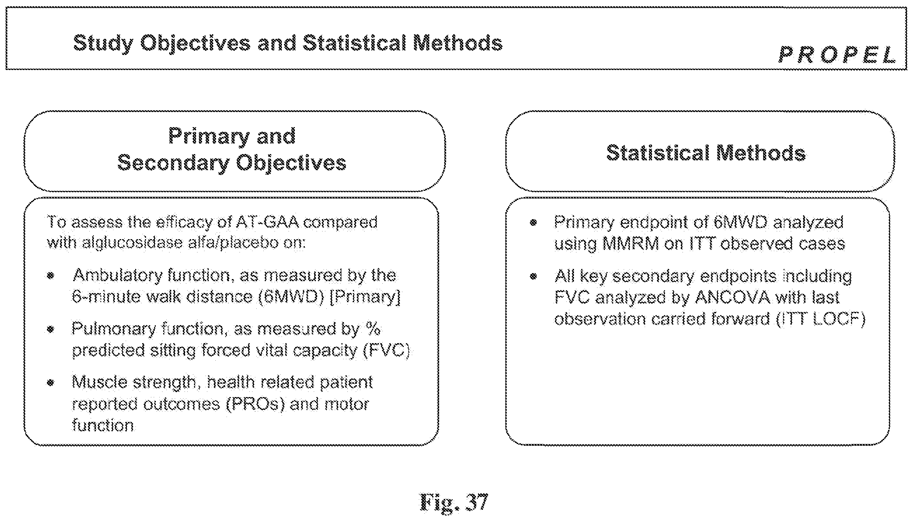

FIG. 37 describes the study objectives and statistical methods of the ATB200-03 study.

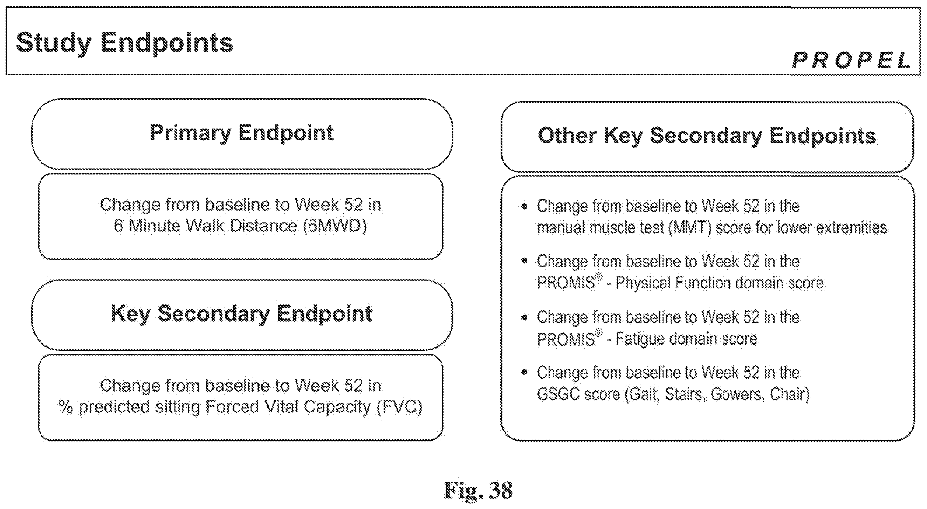

FIG. 38 describes the primary endpoint and secondary endpoints of the ATB200-03 study.

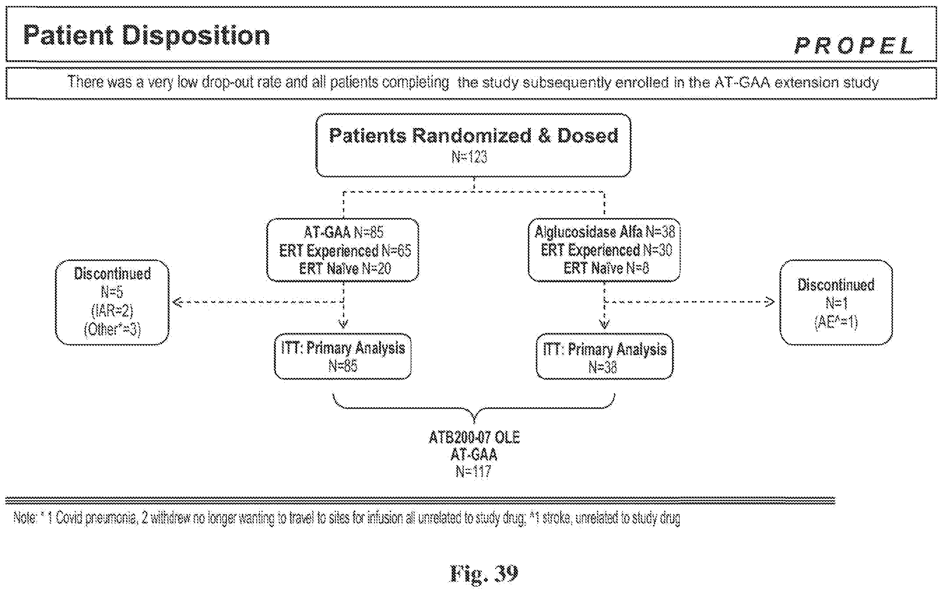

FIG. 39 summarizes the patient disposition of the ATB200-03 study.

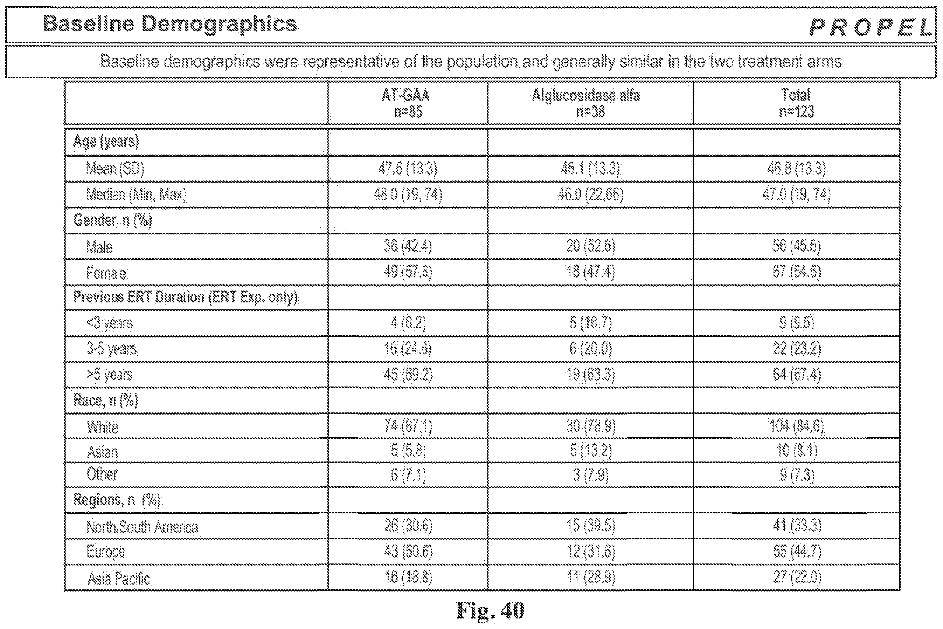

FIG. 40 summarizes the baseline demographics of the ATB200-03 study.

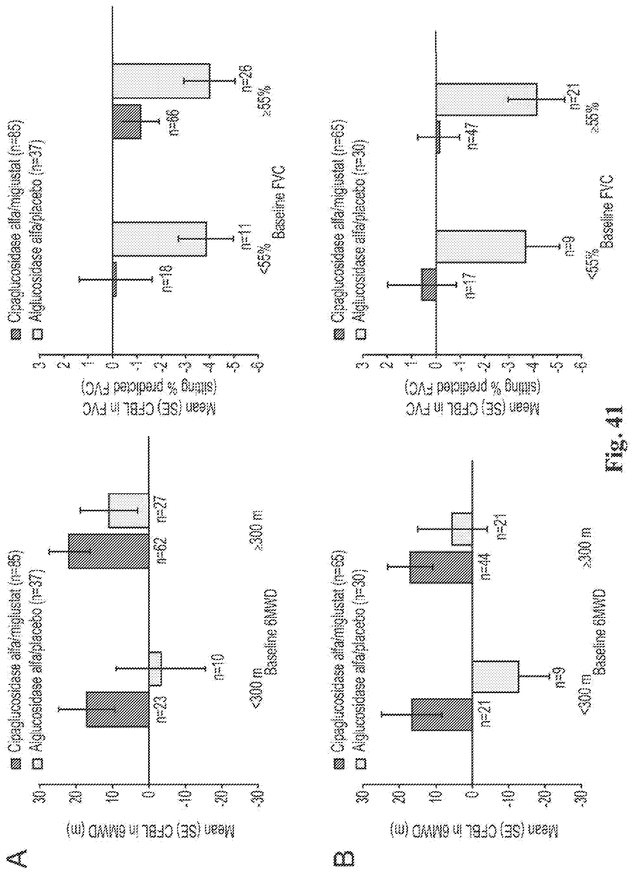

FIG. 41 shows subgroup analyses for the change from baseline in 6MWD and FVC by baseline status in the overall population (n=122) (Set A) and ERT-experienced patients (n=95) (Set B) in the ATB200-03 study.

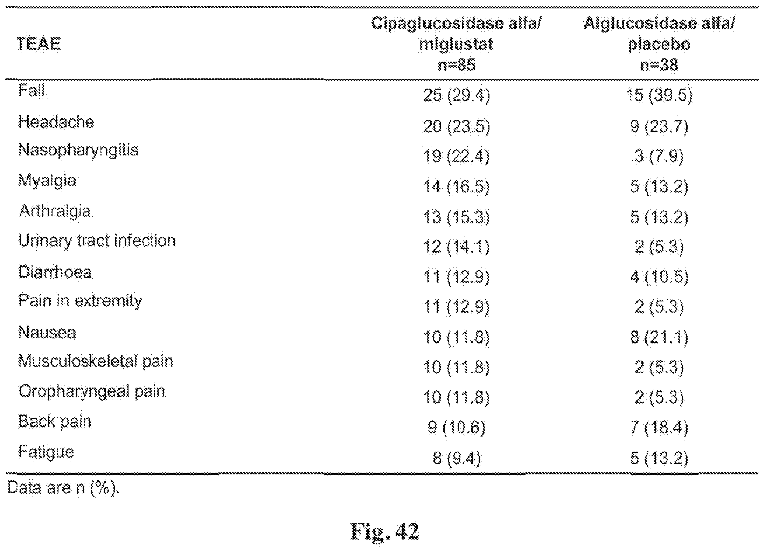

FIG. 42 shows a list of treatment emergent adverse events (TEAEs) in ≥ 10% of patients in any group in the ATB200-03 study.

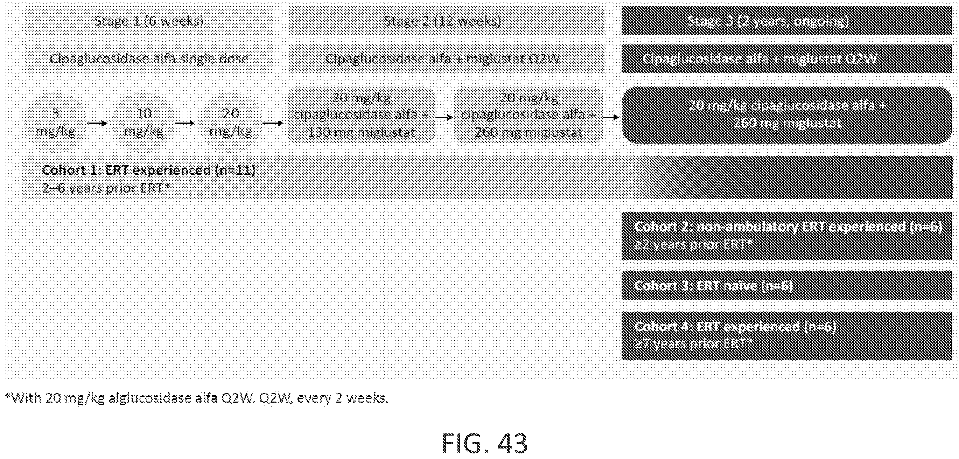

FIG. 43 shows the study design for the Phase I/II ATB200-02 study. The asterisk indicates that prior ERT was with 20 mg/kg alglucosidase alfa Q2W. Q2W, every 2 weeks

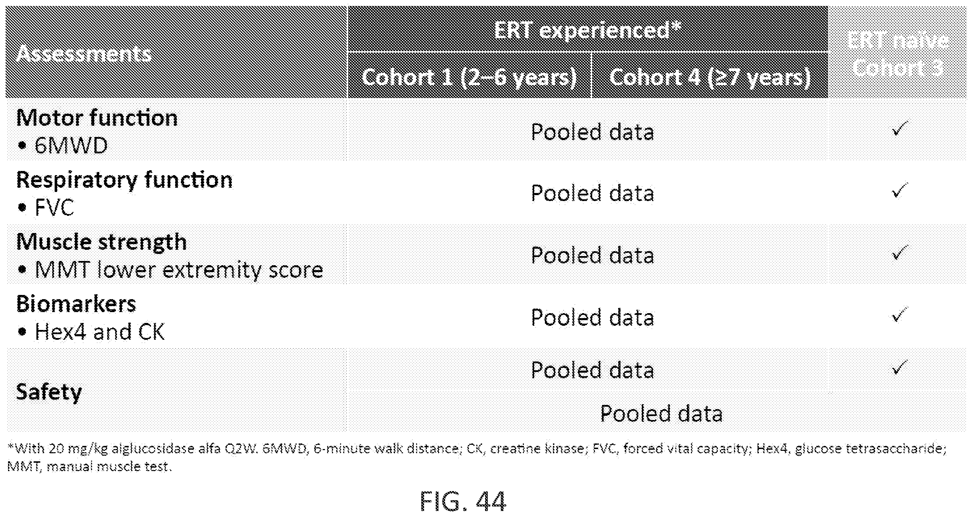

FIG. 44 shows a summary of endpoints and cohorts reported for the ATB200-02 study.

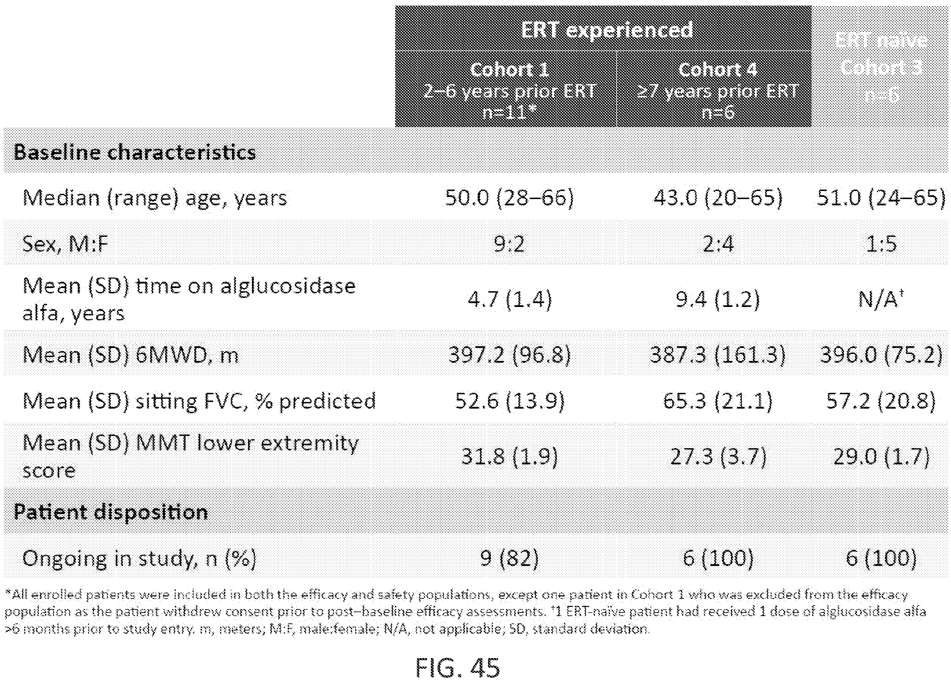

FIG. 45 shows the baseline characteristics and patient disposition for the ATB200-02 study. The asterisk indicates that 1 ERT-naïve patient had received 1 dose of alglucosidase alfa >6 months prior to study entry. M means meters; M:F means male:female ratio; N/A means not applicable; SD means standard deviation.

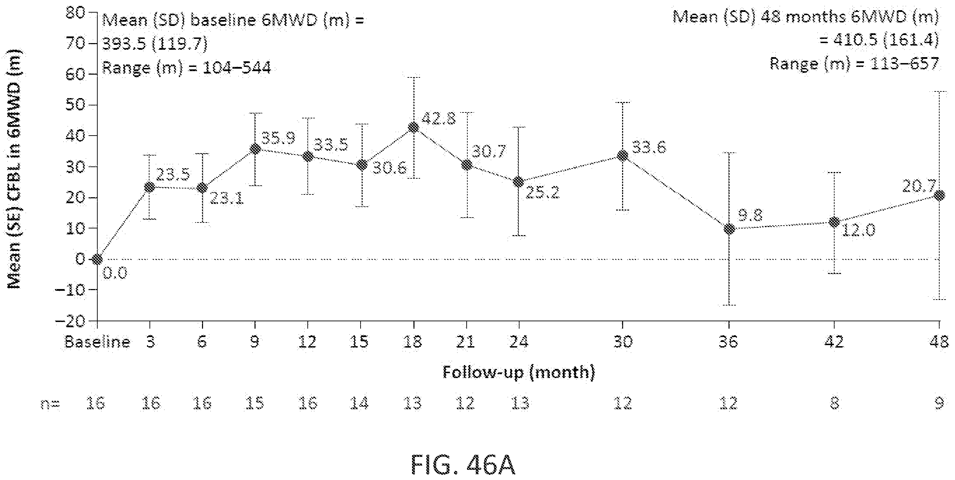

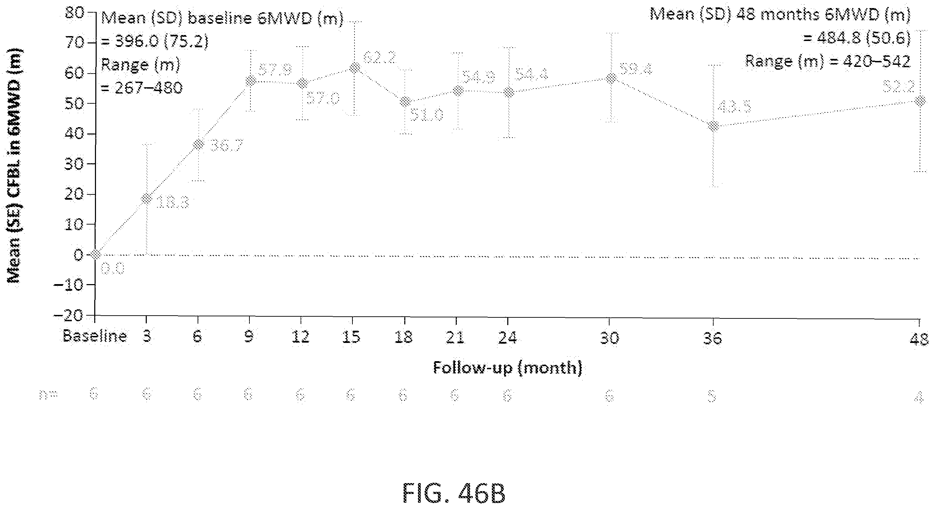

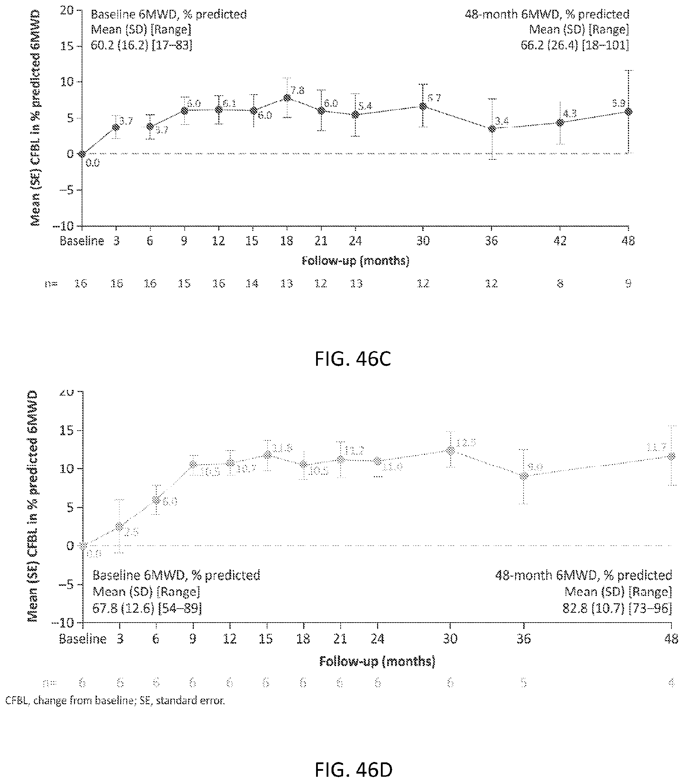

FIG. 46A - FIG. 46D show the mean change from baseline (CFBL) in 6-minute walk distance (6MWD) over time in ERT-experienced (FIG. 46A, 46 C) and ERT-naïve (FIG. 46B, 46D) subjects in the ATB200-02 study.

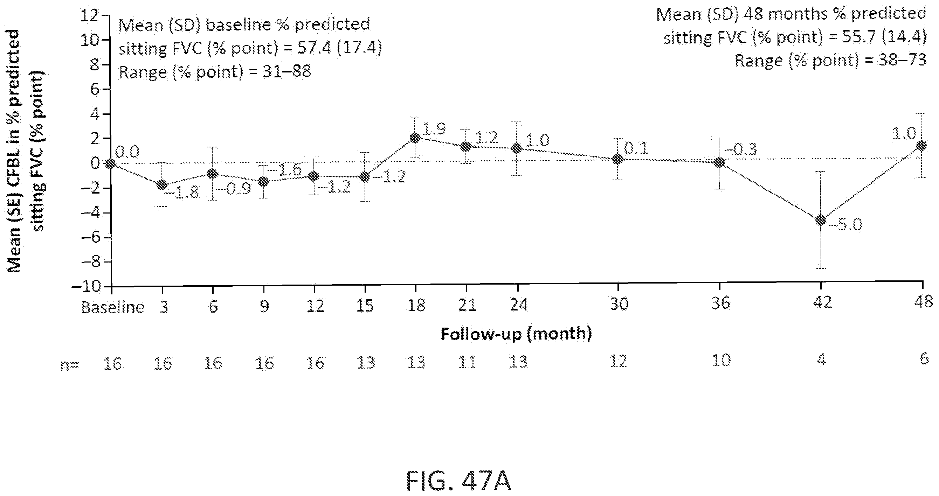

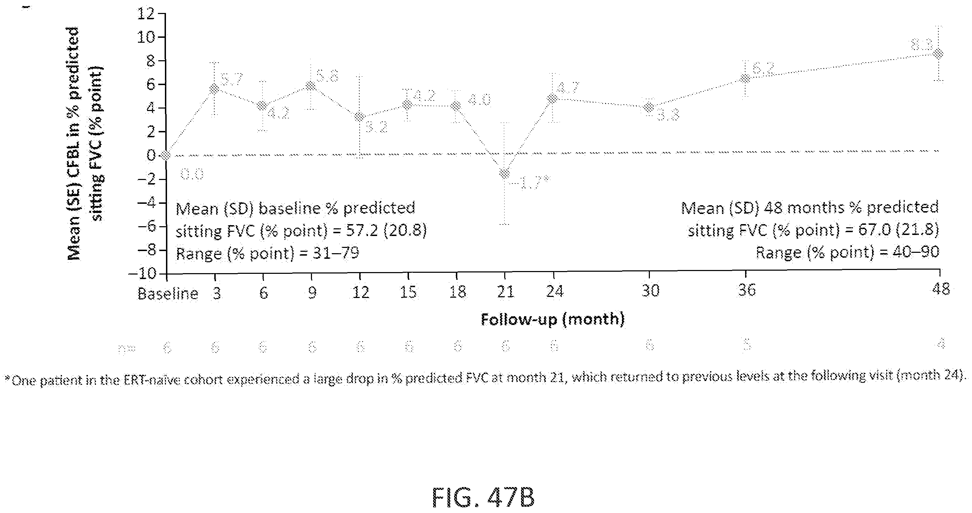

FIG. 47A - FIG. 47B show the mean change from baseline (CFBL) in percentage predicted sitting forced vital capacity (FVC) over time in ERT-experienced (FIG. 47A) and ERT-naïve (FIG. 47B) subjects in the ATB200-02 study.

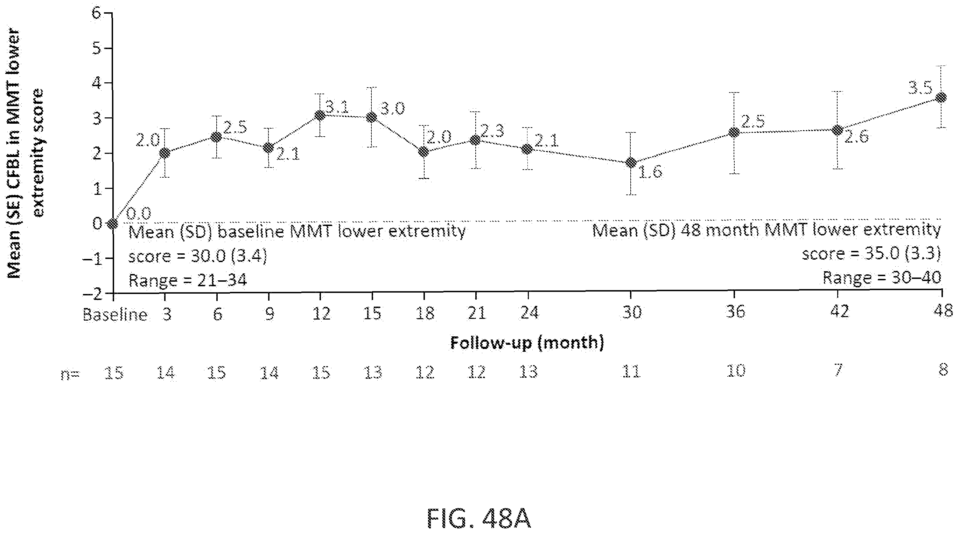

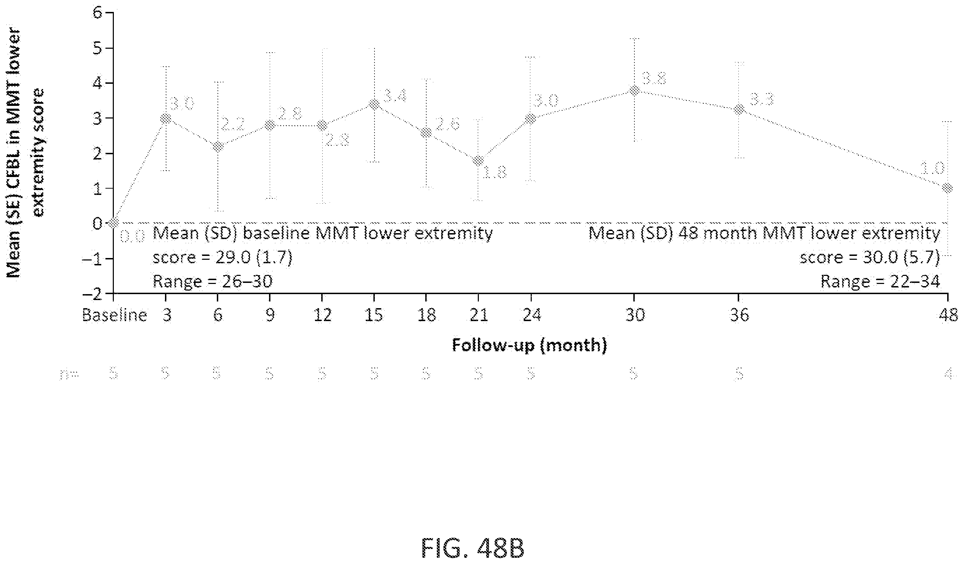

FIG. 48A - FIG. 48B show the mean change from baseline (CFBL) in manual muscle testing (MMT) lower extremity score over time in ERT-experienced (FIG. 48A) and ERT-naïve (FIG. 48B) subjects in the ATB200-02 study.

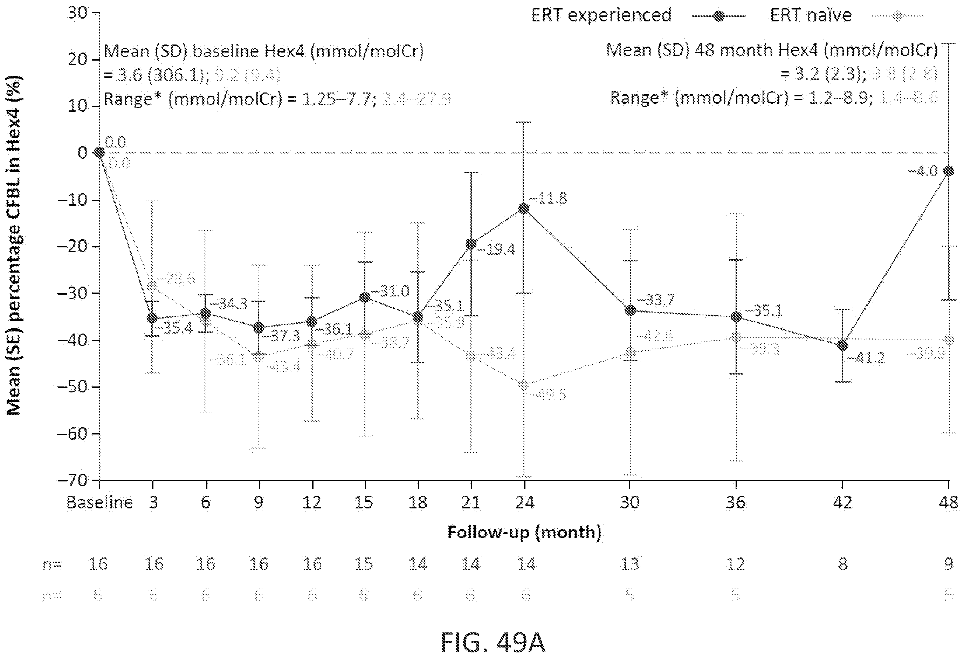

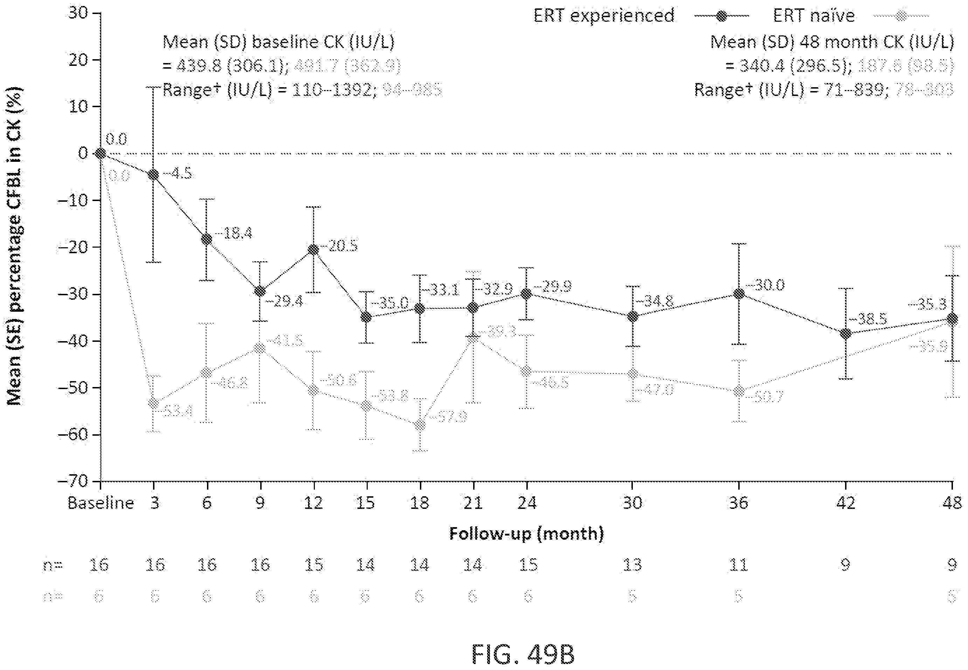

FIG. 49A - FIG. 49B show the mean percentage change from baseline (CFBL) in urine hexose tetrasaccharide (Hex4) levels (FIG. 49A) and plasma creatine kinase (CK) levels (FIG. 49B) in ERT-experienced and ERT-naïve subjects in the ATB200-02 study.

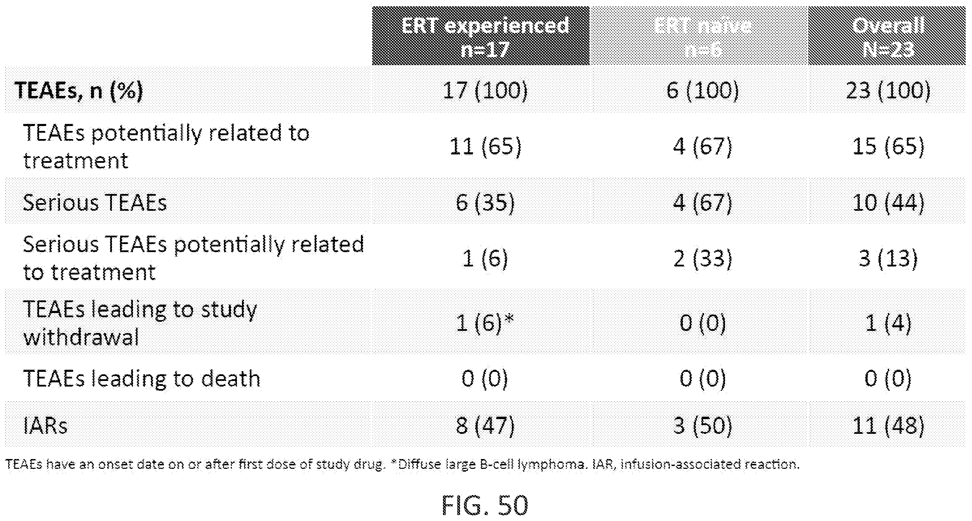

FIG. 50 shows a summary of treatment emergent adverse events (TEAEs) in the ATB200-02 study. Asterisk indicates diffuse large B-cell lymphoma. IAR means infusion-associated reaction; TEAE means treatment-emergent adverse event with onset date on or after first dose of study drug.

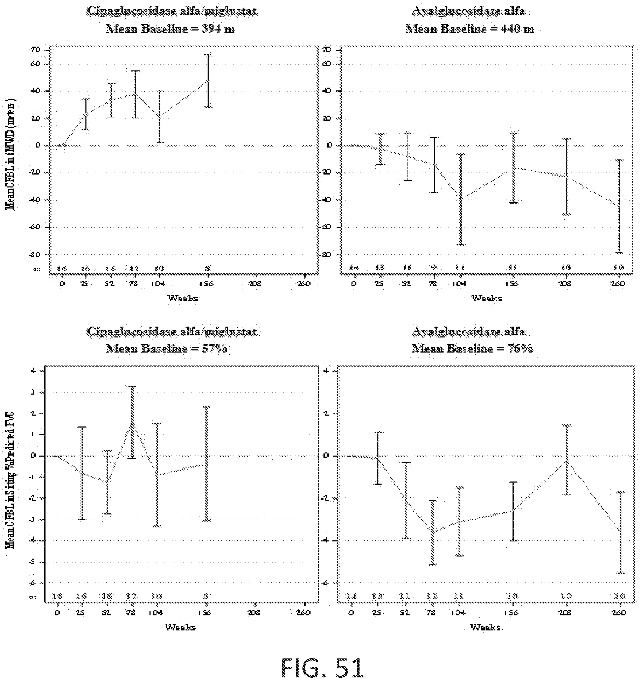

FIG. 51 shows a comparison of the long-term effects of cipaglucosidase alfa/miglustat and avalglucosidase alfa on change from baseline for 6MWD and percentage predicted FVC (sitting) in ERT-experienced subjects.

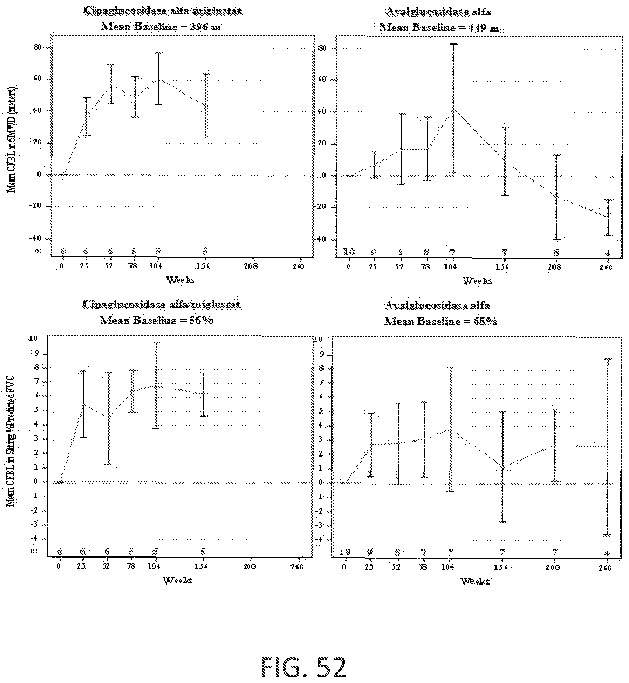

FIG. 52 shows a comparison of the long-term effects of cipaglucosidase alfa/miglustat and avalglucosidase alfa on change from baseline for 6MWD and percentage predicted FVC (sitting) in ERT-naïve subjects.

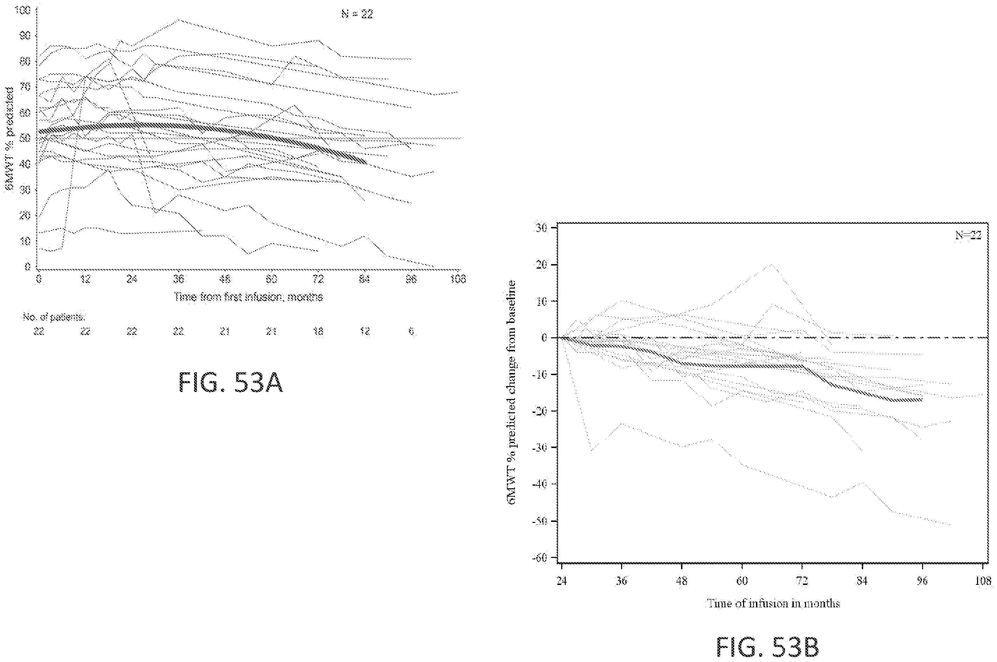

FIG. 53A - FIG. 53B show the 6-minute walk test (6MWT) percentage predicted during treatment of ERT-experienced subjects with alglucosidase alfa. FIG. 53B shows replotted data from FIG. 53A only from year 2 onward.

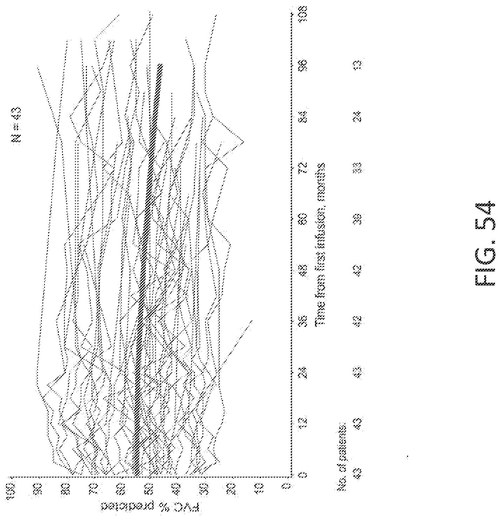

FIG. 54 shows the FVC percentage predicted during treatment of ERT-experienced subjects with alglucosidase alfa.

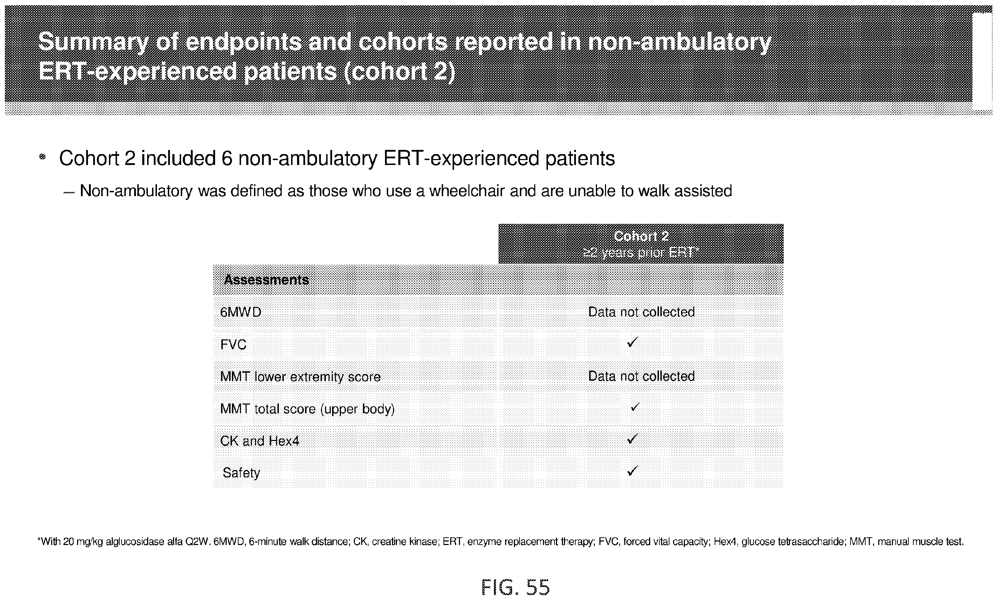

FIG. 55 shows a summary of endpoints and cohorts for Cohort 2 (non-ambulatory ERT-experienced patients) of the ATB200-02 study.

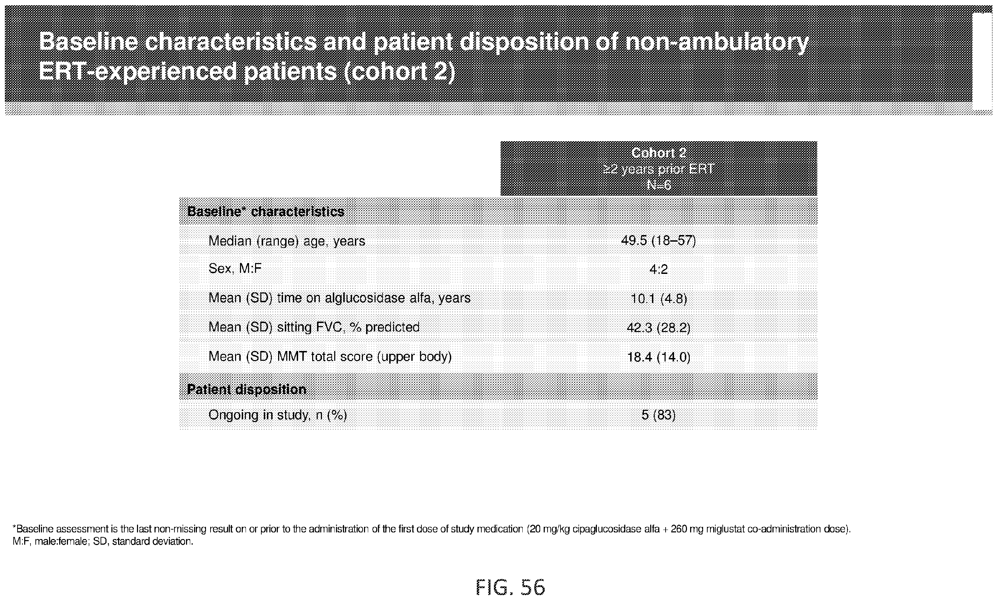

FIG. 56 shows the baseline characteristics and patient disposition for Cohort 2 (non-ambulatory ERT-experienced patients) of the ATB200-02 study. The asterisk indicates that baseline assessment is the last non-missing result on or prior to the administration of the first dose of study medication (20 mg/kg cipaglucosidase alfa + 260 mg miglustat co-administration dose). M:F means male:female ratio; SD means standard deviation.

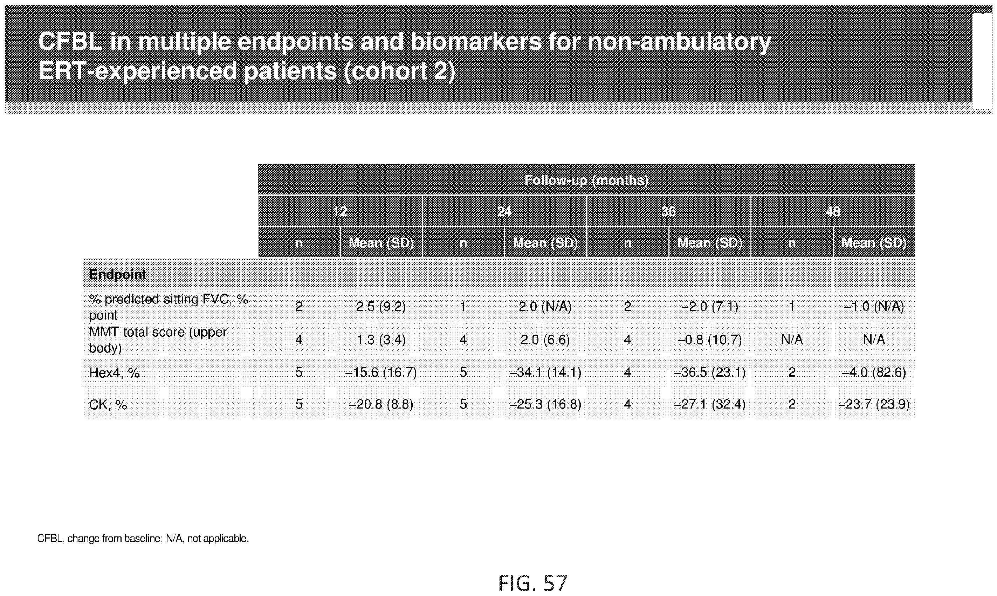

FIG. 57 show the mean change from baseline (CFBL) in percentage predicted sitting forced vital capacity (FVC) over time in Cohort 2 (non-ambulatory ERT-experienced patients).

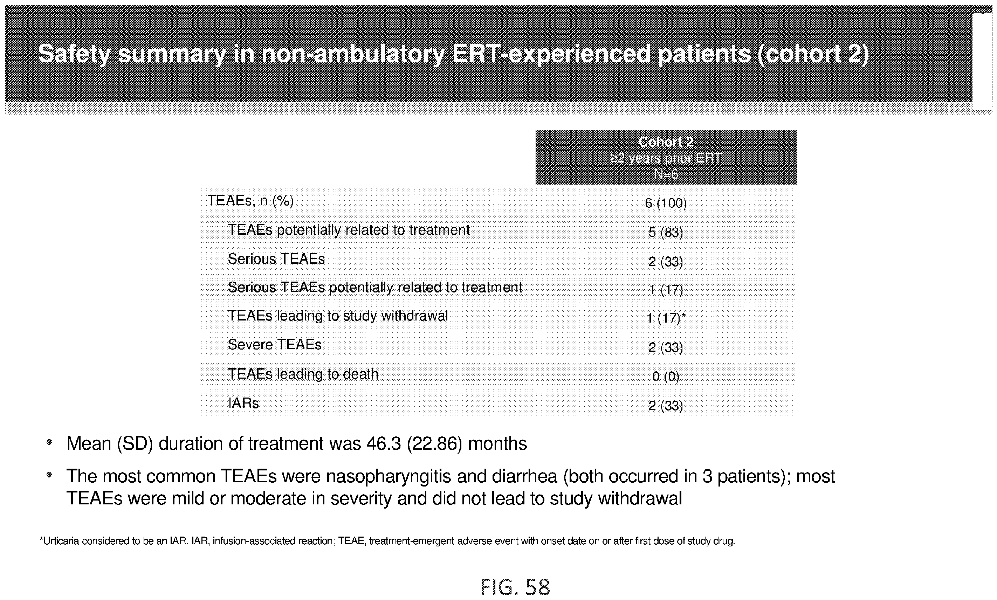

FIG. 58 shows a summary of treatment emergent adverse events (TEAEs) for Cohort 2 (non-ambulatory ERT-experienced patients) in the ATB200-02 study. Asterisk indicates urticaria considered to be an IAR. IAR means infusion-associated reaction; TEAE means treatment-emergent adverse event with onset date on or after first dose of study drug.

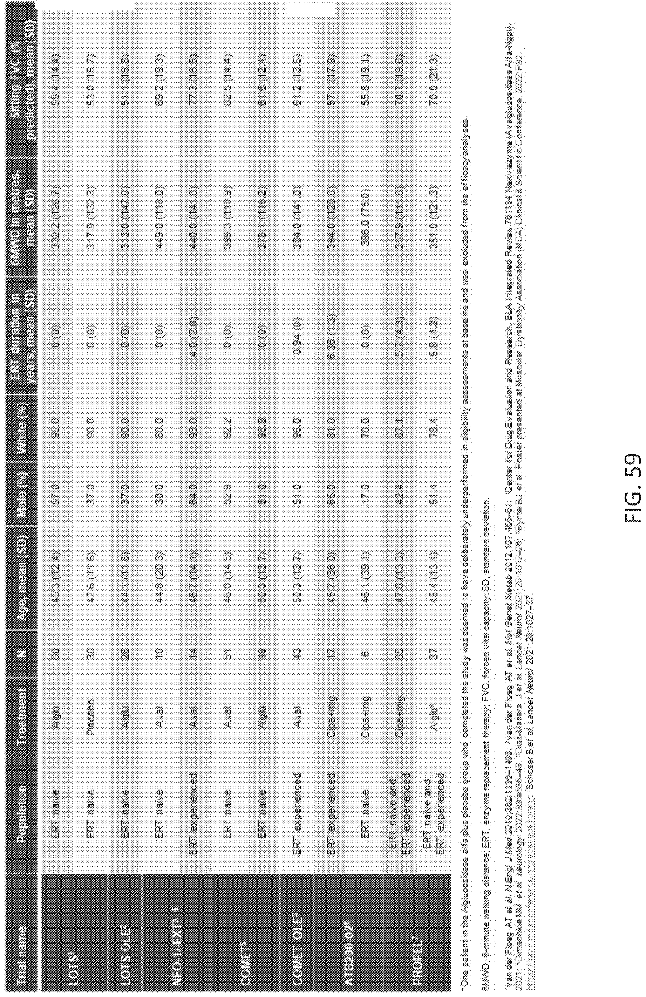

FIG. 59 shows the baseline characteristics of the seven clinical studies identified by the systematic literature review (SLR)

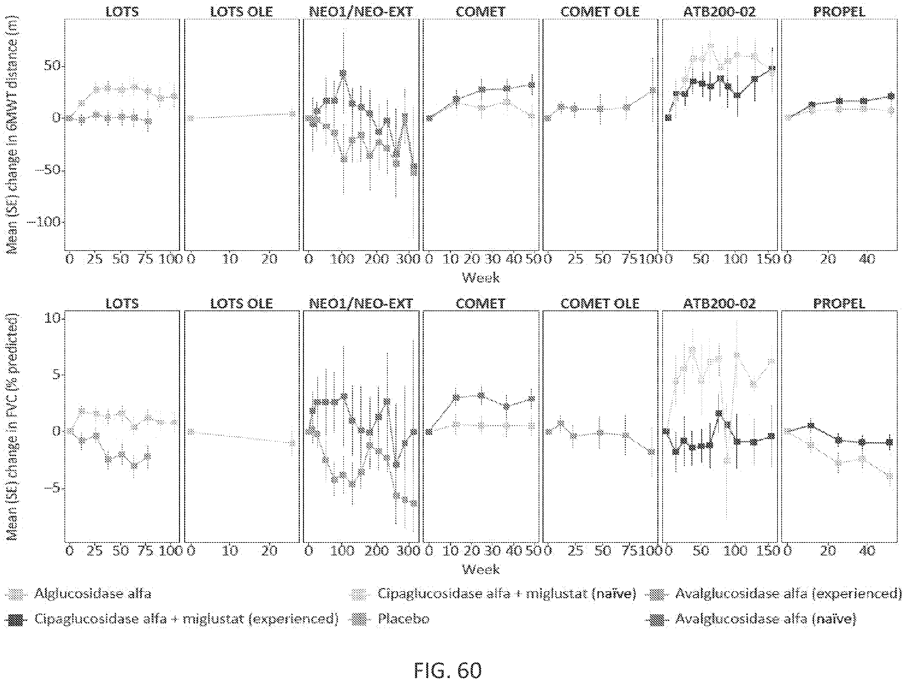

FIG. 60 shows longitudinal efficacy results versus trial for 6MWD (m) and FVC (% predicted) as changed from baseline for each of the identified studies.

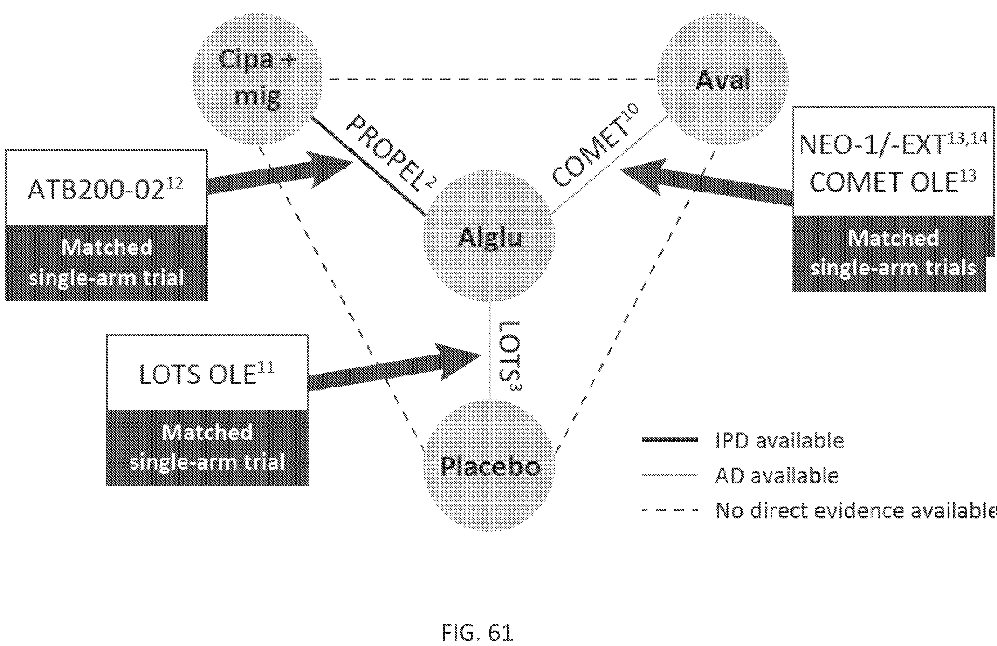

FIG. 61 shows a Network for 6MWD (m) and sitting FVC (% predicted).

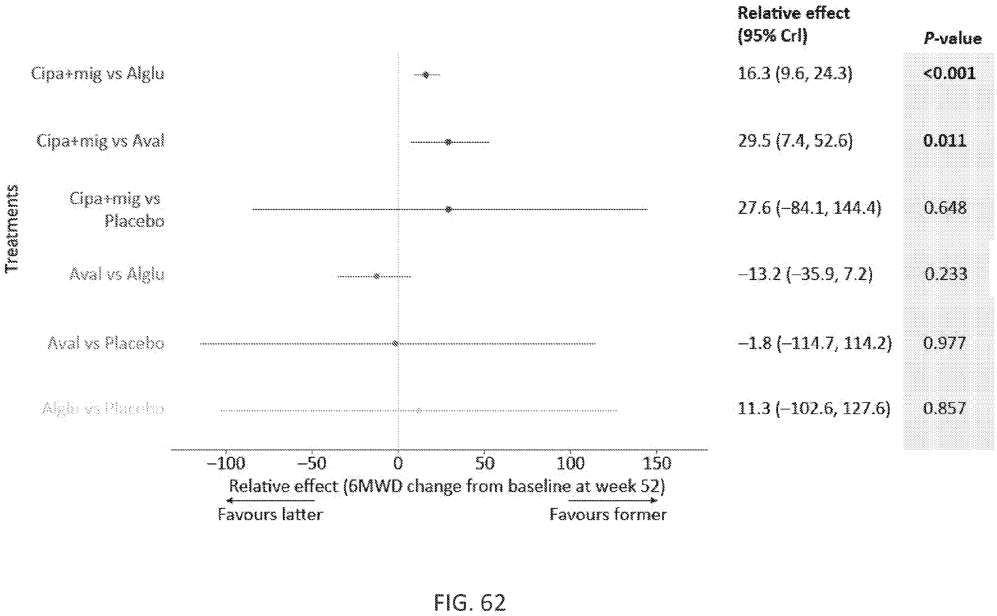

FIG. 62 shows a forest plot of relative effect estimates with 95% credible intervals for 6MWD in the base-case scenario (main analysis).

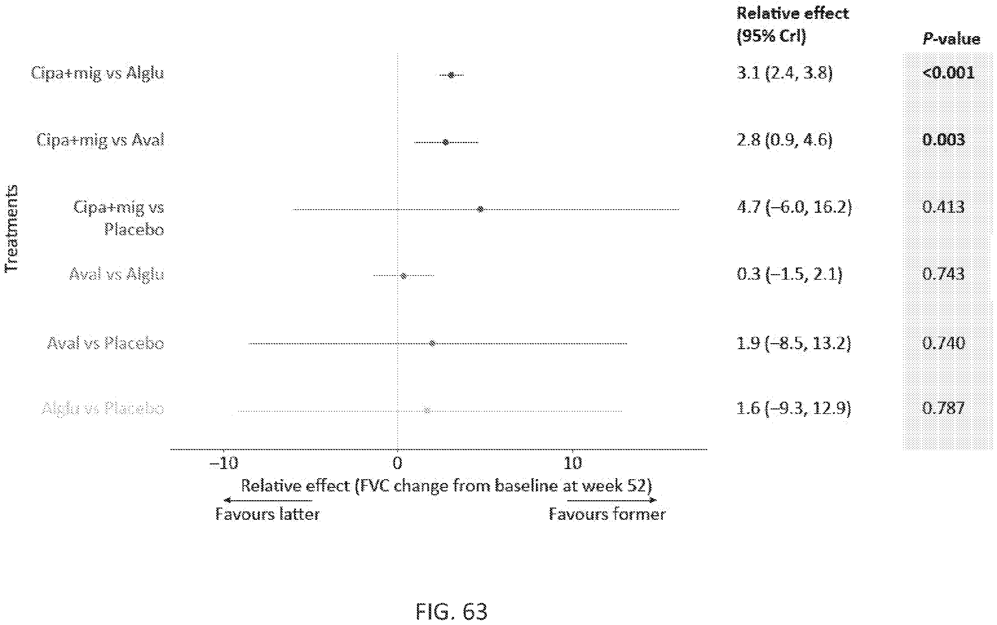

FIG. 63 shows a forest plot of relative effect estimates with 95% credible intervals for FVC in the base-case scenario (main analysis).

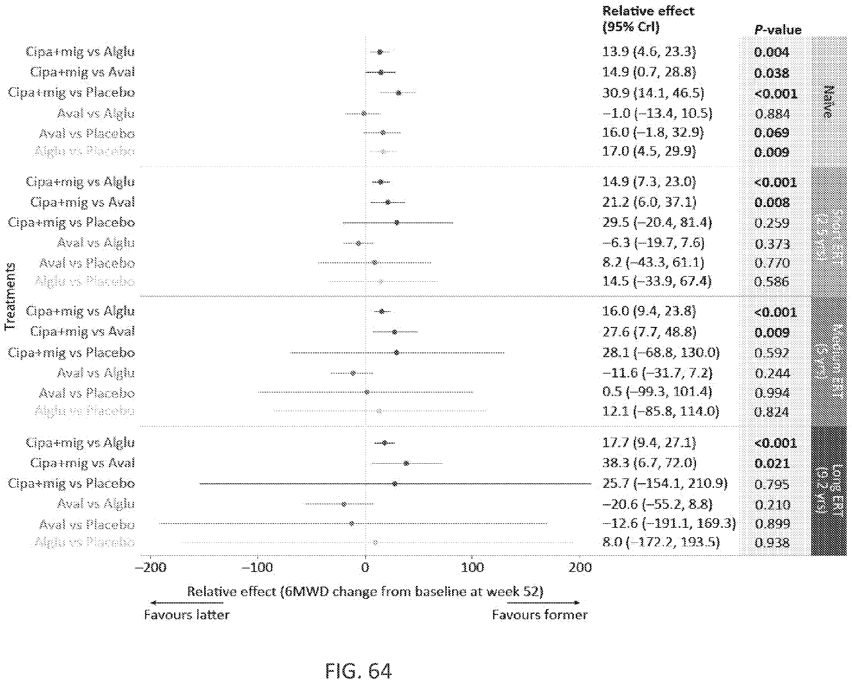

FIG. 64 shows a forest plot of relative effect estimates with 95% credible intervals for 6MWD by ERT duration.

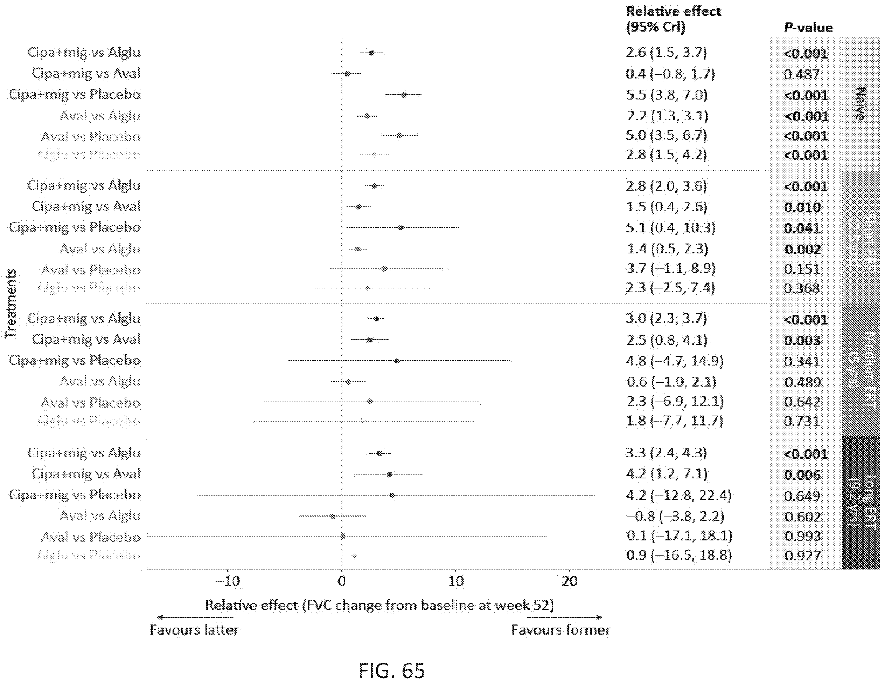

FIG. 65 shows a forest plot of relative effect estimates with 95% credible intervals for FVC by ERT duration.

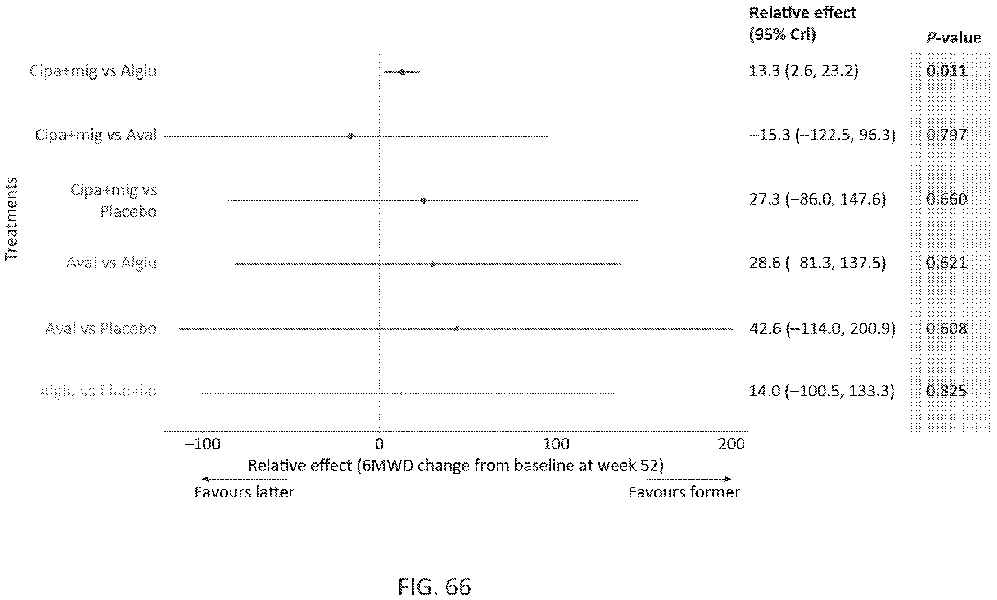

FIG. 66 shows a forest plot of relative effect estimates with 95% credible intervals for 6MWD in the base-case scenario (sensitivity analysis).

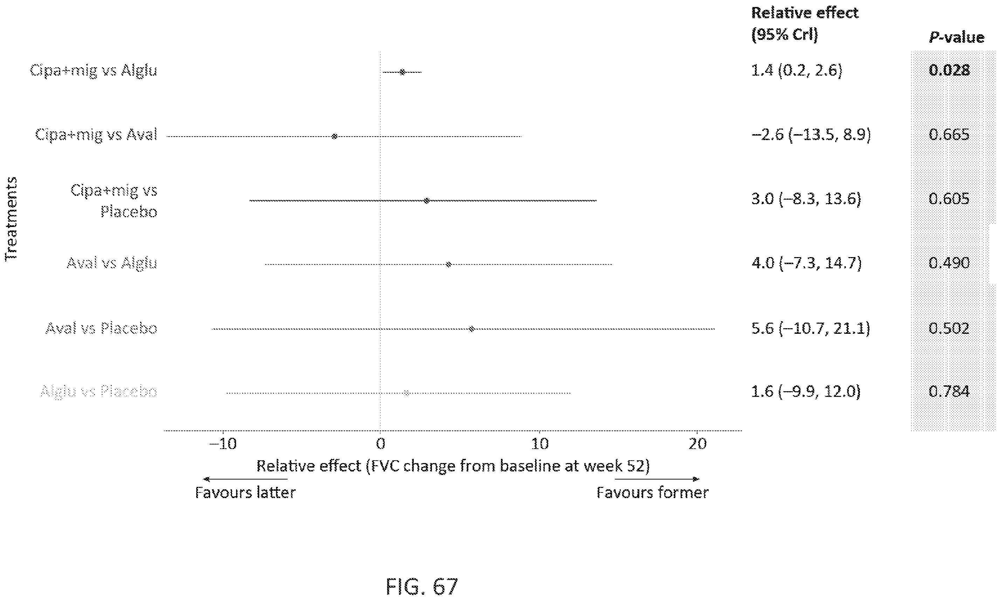

FIG. 67 shows a forest plot of relative effect estimates with 95% credible intervals for FVC in the base-case scenario (sensitivity analysis).

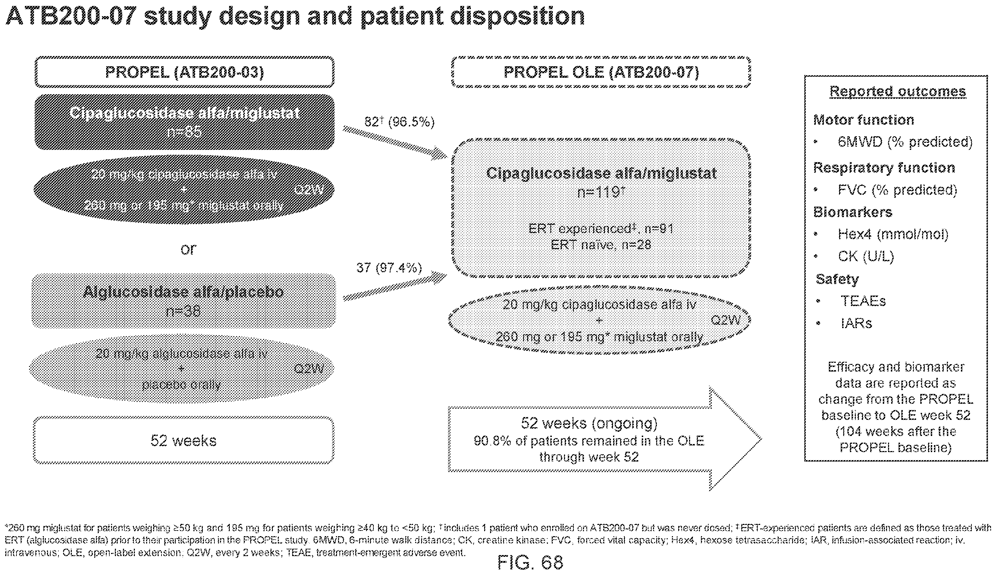

FIG. 68 shows the study design and patient disposition for the ATB200-07 study.

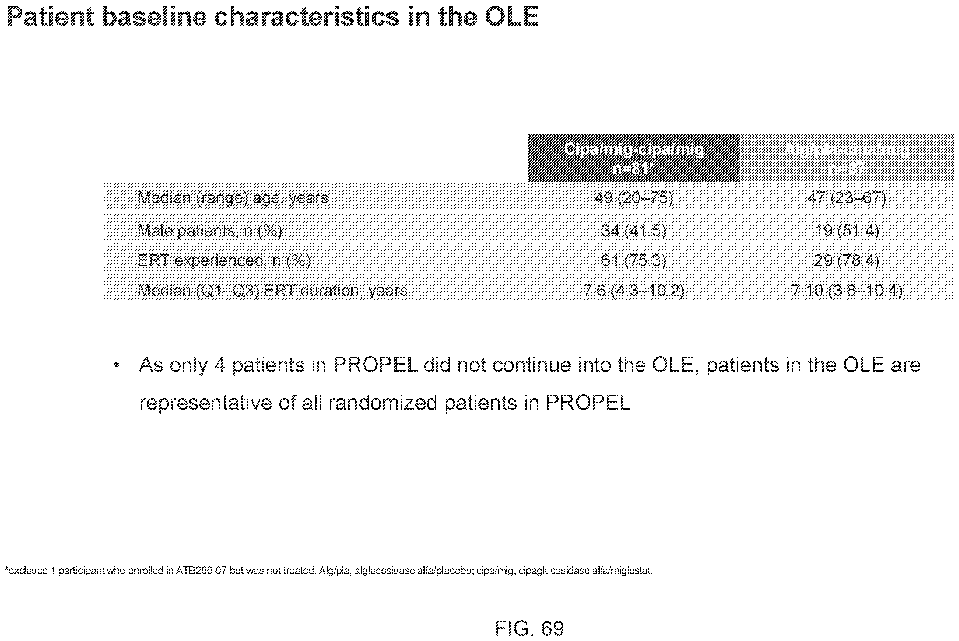

FIG. 69 summarizes the baseline demographics of the ATB200-07 study.

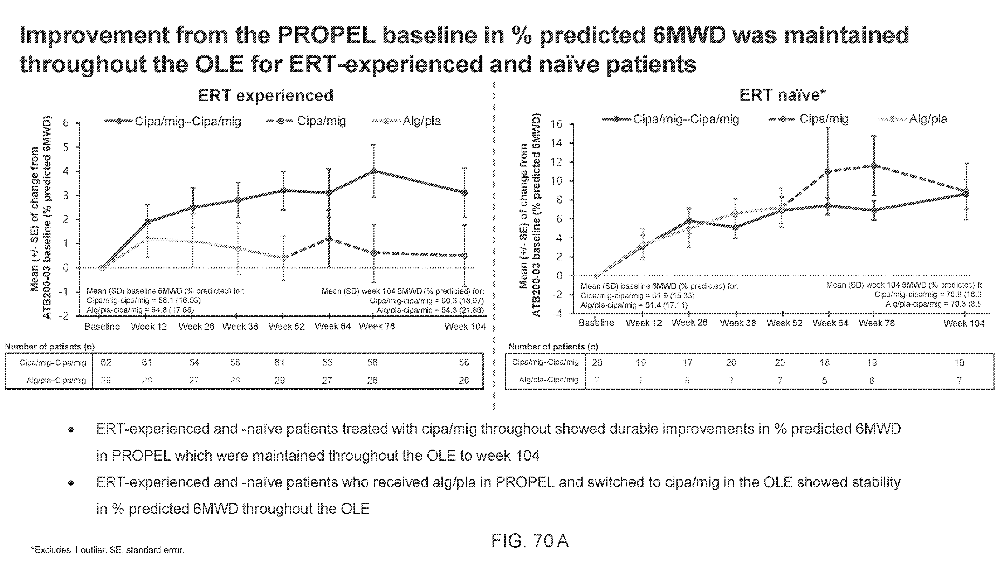

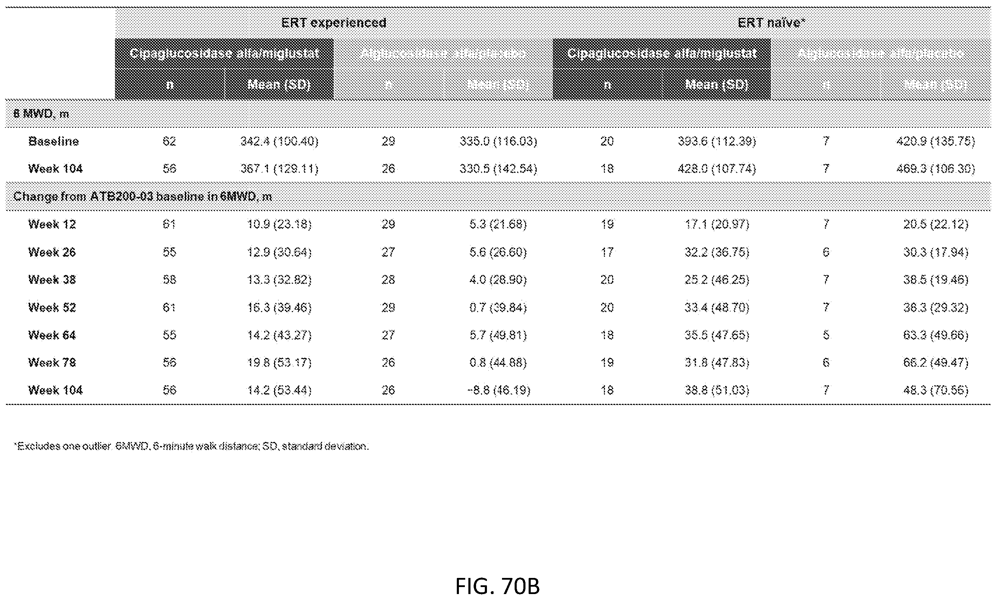

FIG. 70A-70B show the mean change from baseline in % predicted 6MWD (FIG. 70A) and in 6MWD (FIG. 70B) for ERT-experienced and ERT-naïve patients in the ATB200-07 study.

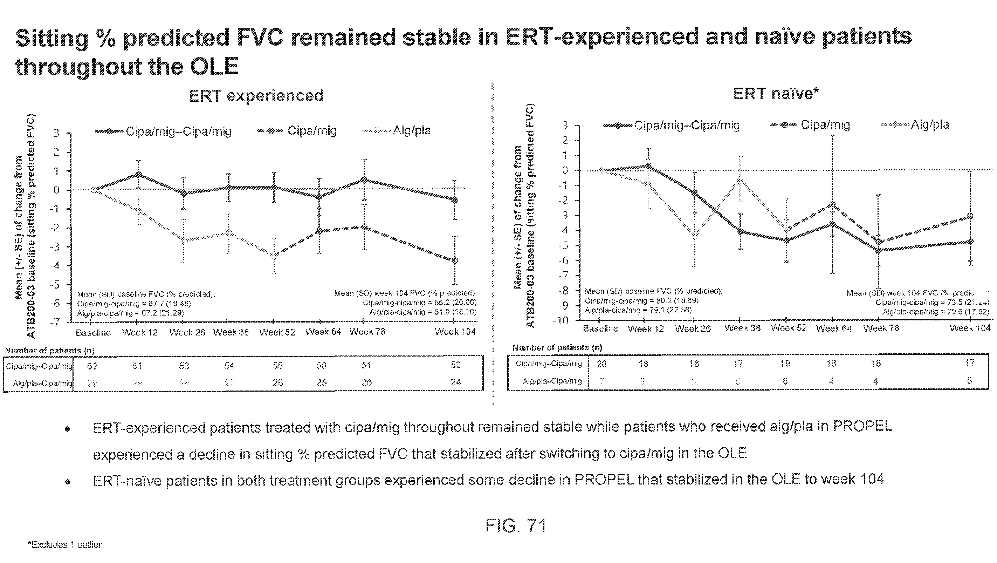

FIG. 71 shows the mean change from baseline % predicted FVC for ERT-experienced and ERT-naïve patients in the ATB200-07 study

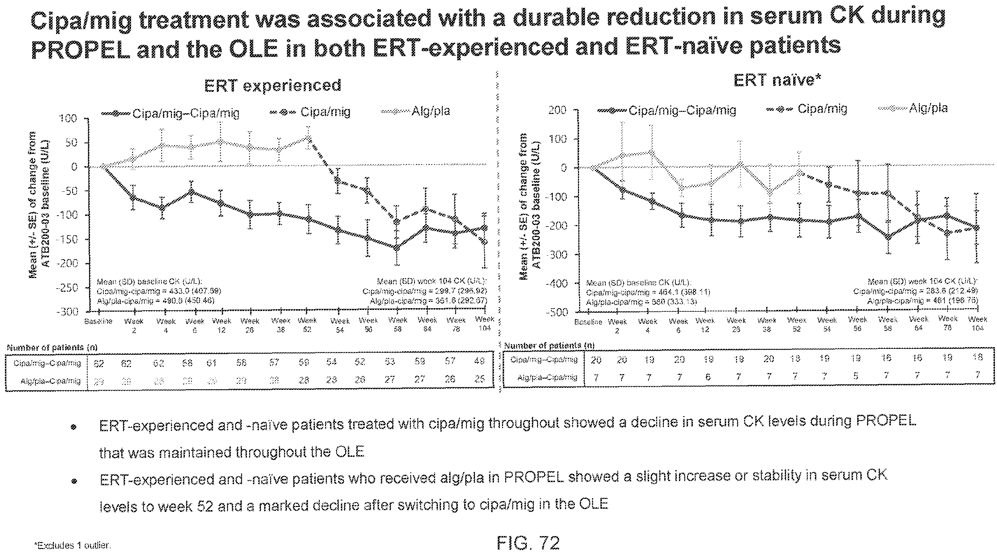

FIG. 72 shows the mean change from baseline in serum CK for ERT-experienced and ERT-naïve patients in the ATB200-07 study

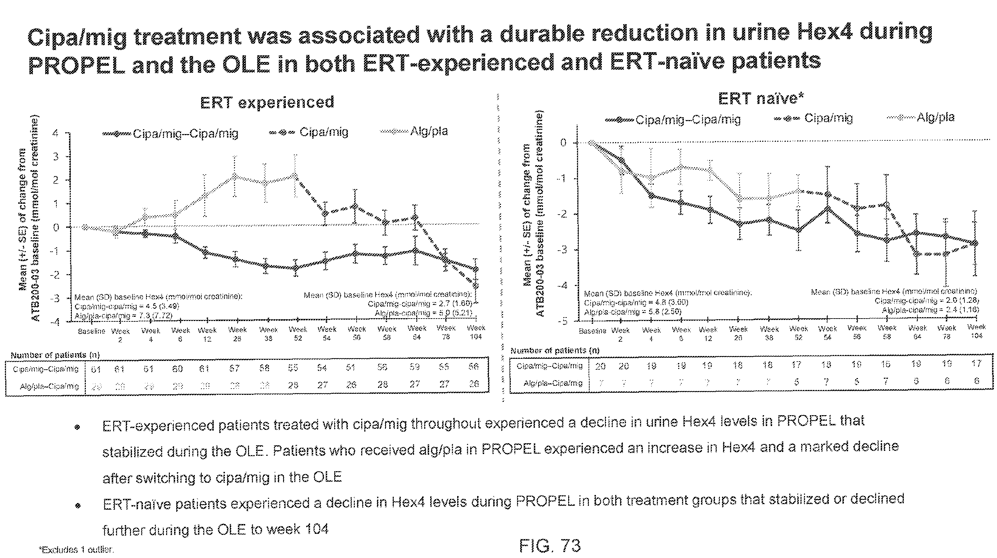

FIG. 73 shows the mean change from baseline in urine Hex4 for ERT-experienced and ERT-naïve patients in the ATB200-07 study.

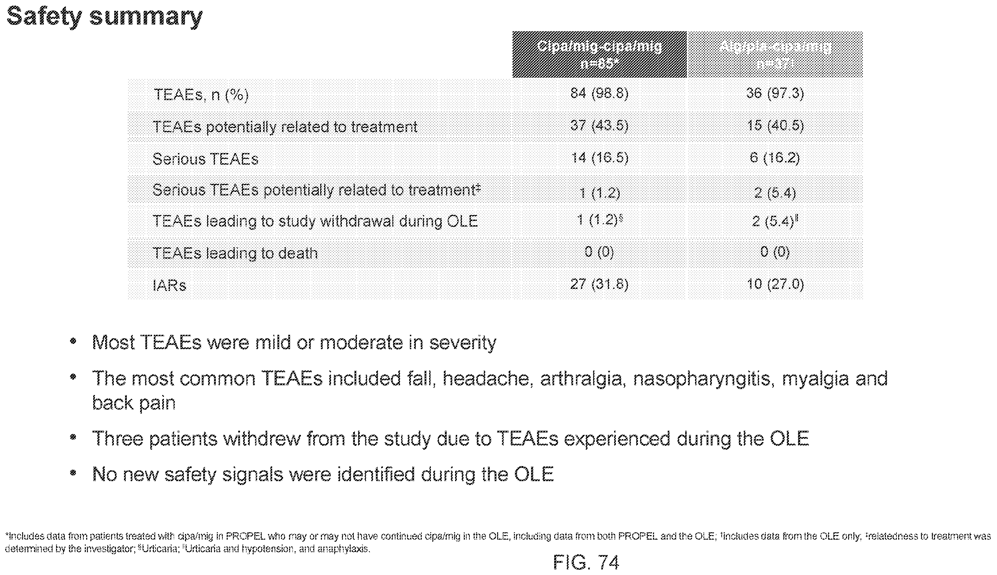

FIG. 74 shows the safety summary from the ATB200-07 study.

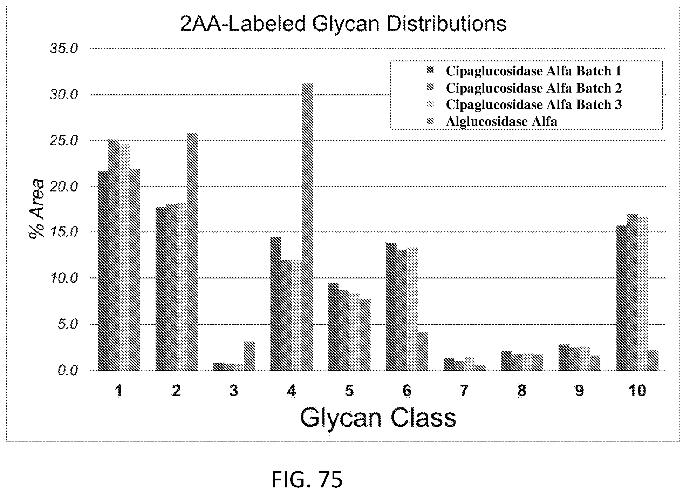

FIG. 75 is a graph showing the 2-AA labeled N-glycan distributions identified by LC-FLD analysis for alglucosidase alfa and three preparations of cipaglucosidase alfa.

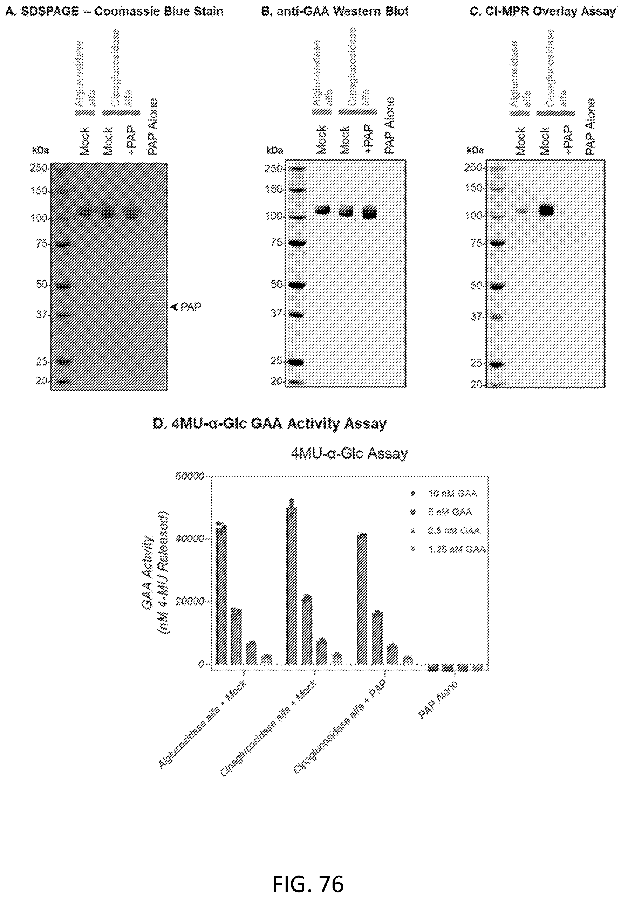

FIG. 76A depicts protein loading control of a western blot for mock-treated alglucosidase alfa, mock-treated cipaglucosidase alfa, and purple acid phosphatase (PAP)-treated cipaglucosidase alfa. Fig. 76B shows a western blot depicting GAA protein levels of samples shown in 76A. 76C shows a variation of a far-western blot to determine CIMPR binding for samples in 76A. 76D is a graph showing 4MU-α-glucosidase enzyme activity for the samples shown in 76A.

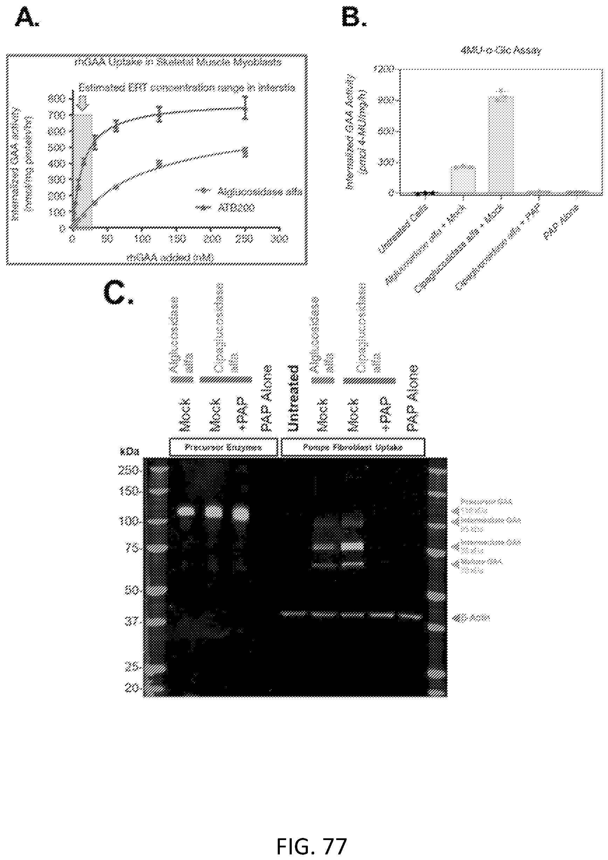

Fig. 77A is a graph showing internalized rhGAA uptake inside skeletal muscle myoblasts at various rhGAA concentrations. Fig. 77B compares GAA activity of inside Pompe disease patient-derived fibroblasts for mock-treated alglucosidase alfa, mock-treated cipaglucosidase alfa, and PAP-treated cipaglucosidase alfa at a 20nM GAA concentration. Fig. 77C is a western blot depicting the GAA content of cell lysates shown in Fig. 77B after uptake.

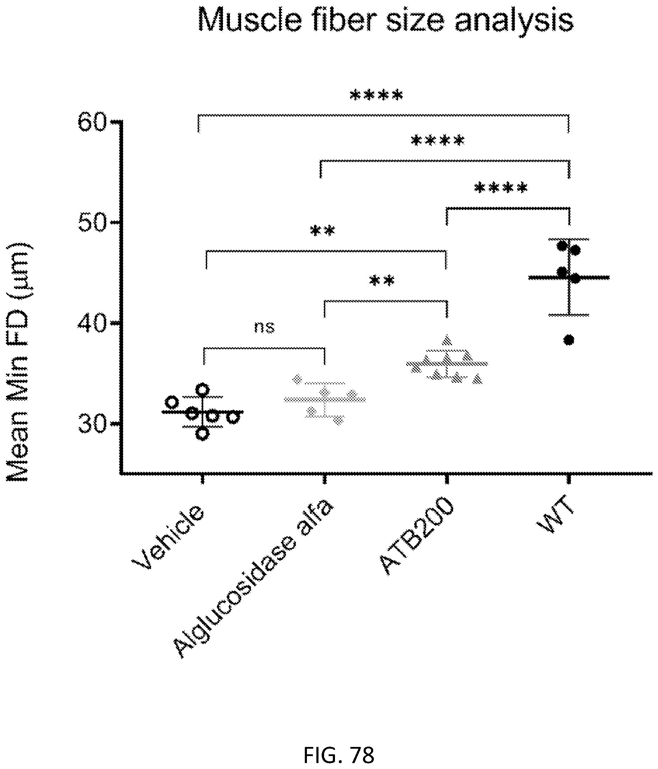

Fig. 78 is a graph showing the effect of long-term cipaglucosidase alfa administration (20 mg/kg, 12 biweekly bolus injections) versus alglucosidase alfa on muscle fiber size by mean minimum fiber diameter (FD) in Gaa KO mouse quadriceps.

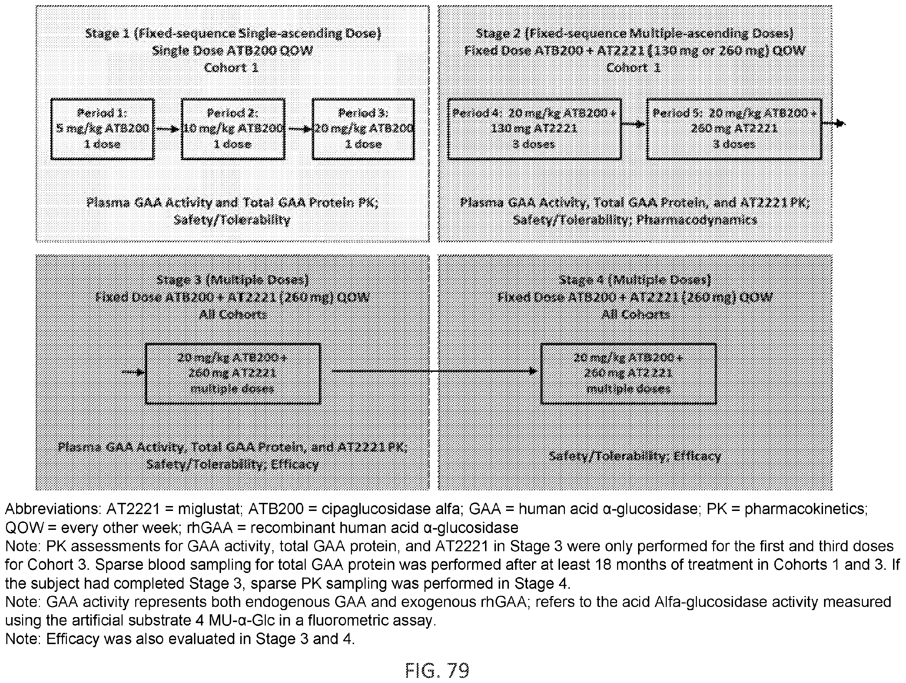

Fig. 79 illustrates a design schematic for Study ATB200-02.

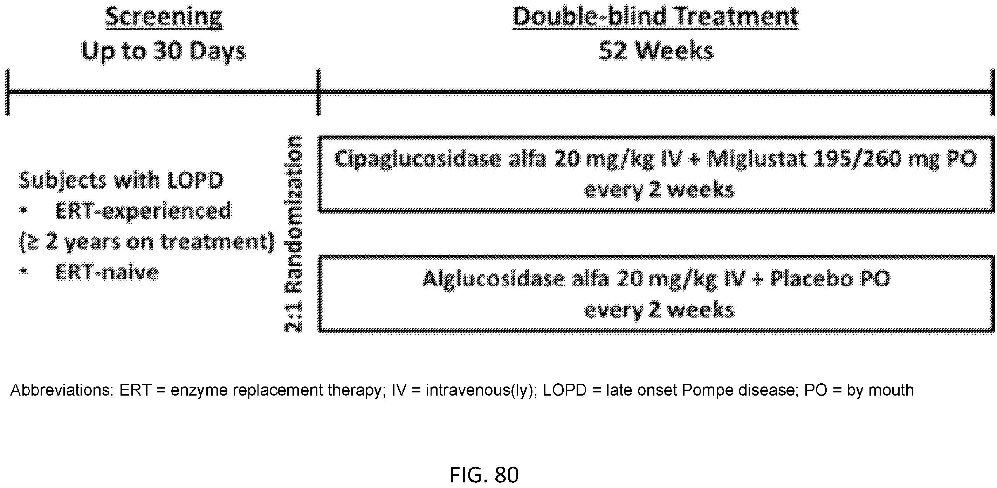

Fig. 80 illustrates a design schematic for Study ATB200-03.

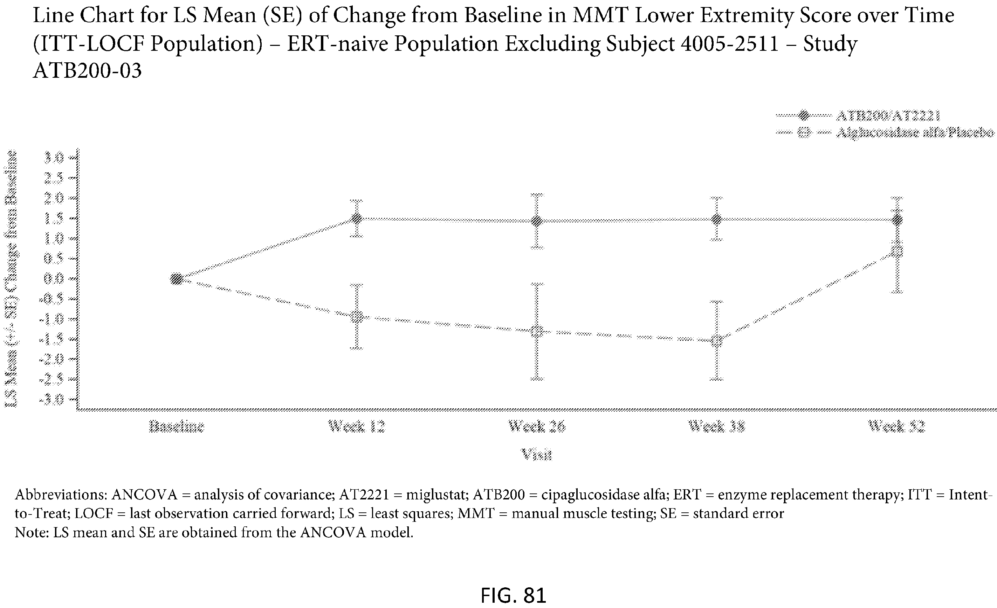

Fig. 81 is a line chart for LS Mean (SE) of change from baseline in MMT Lower Extremity Score over time (ITT-LOCF Population) for the ERT-naïve population excluding subject 4005-2511 in Study ATB200-03.

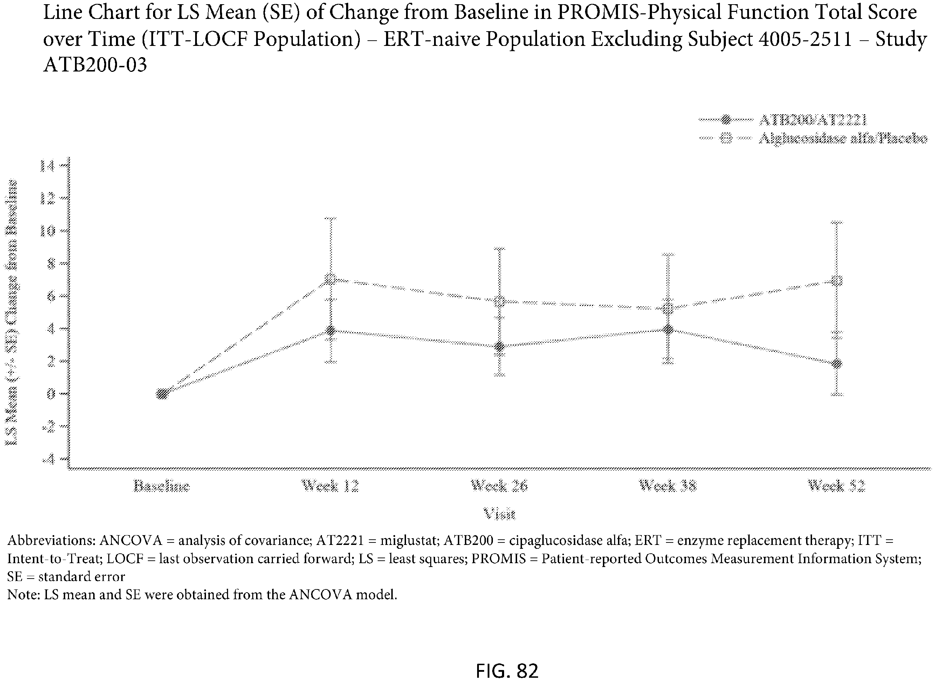

Fig. 82 is a line chart for LS Mean (SE) of change from baseline in PROMIS-Physical Function Total Score over time (ITT-LOCF Population) for the ERT-naïve population excluding subject 4005-2511 in Study ATB200-03.

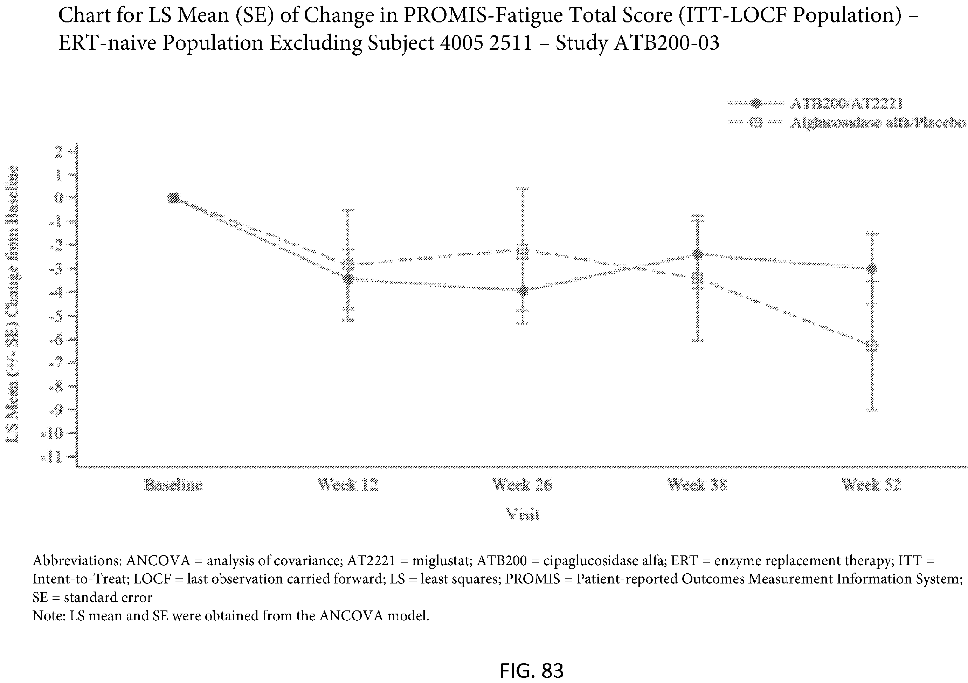

Fig. 83 is a line chart for LS Mean (SE) of change in PROMIS-Fatigue Total Score (ITT-LOCF Population) for the ERT-naïve Population excluding subject 4005-2511 in Study ATB200-03.

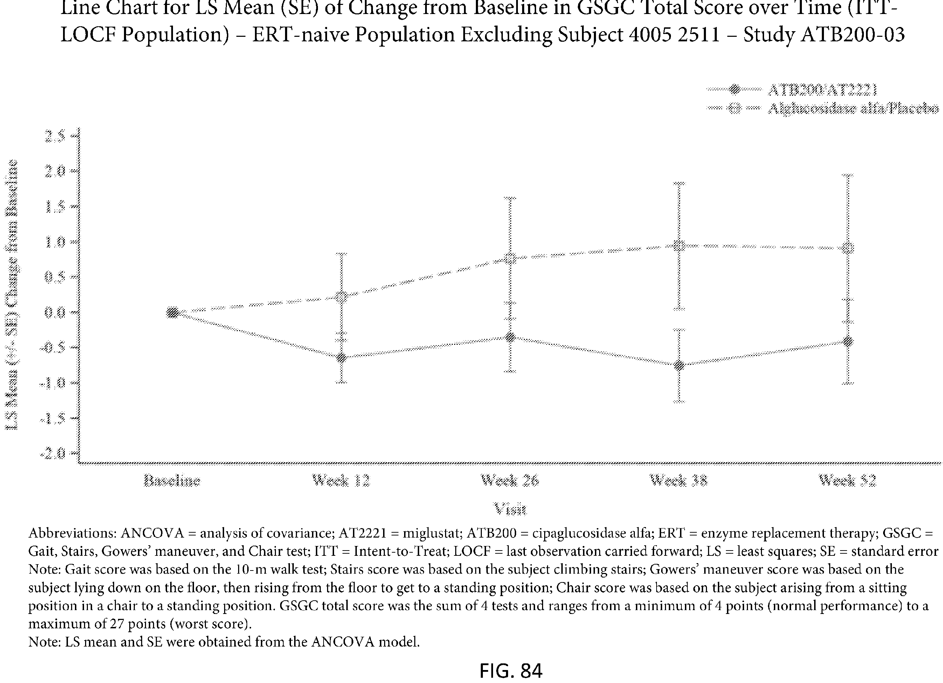

Fig. 84 is a line chart for LS Mean (SE) of change from baseline in GSGC Total Score over time (ITT-LOCF Population) for the ERT-naïve population excluding subject 4005-2511 in Study ATB200-03.

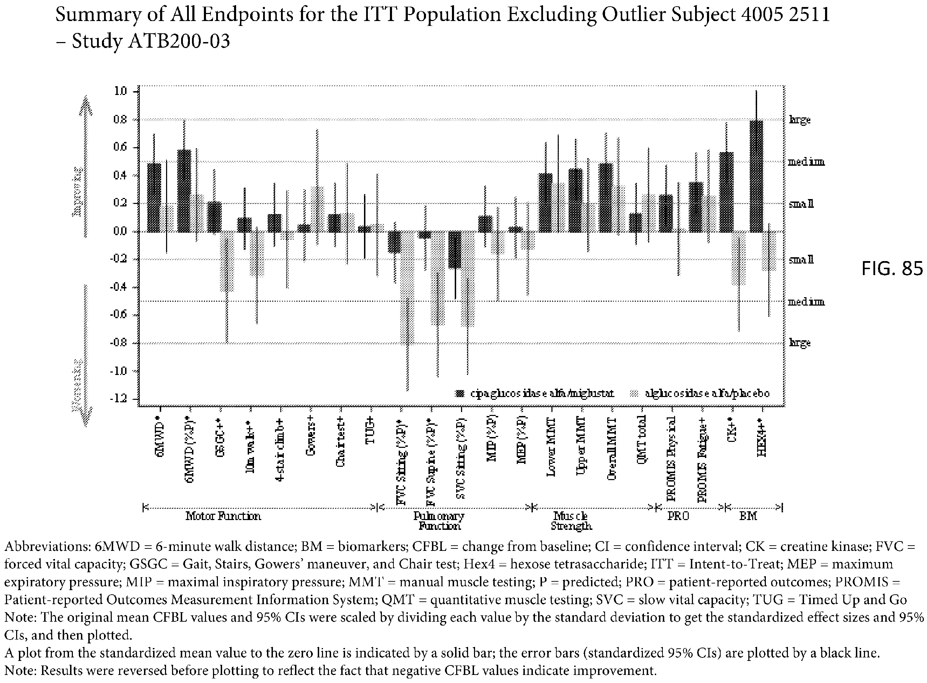

Fig. 85 is a bar chart summarizing all endpoints for the ITT population excluding outlier subject 4005-2511 in Study ATB200-03.

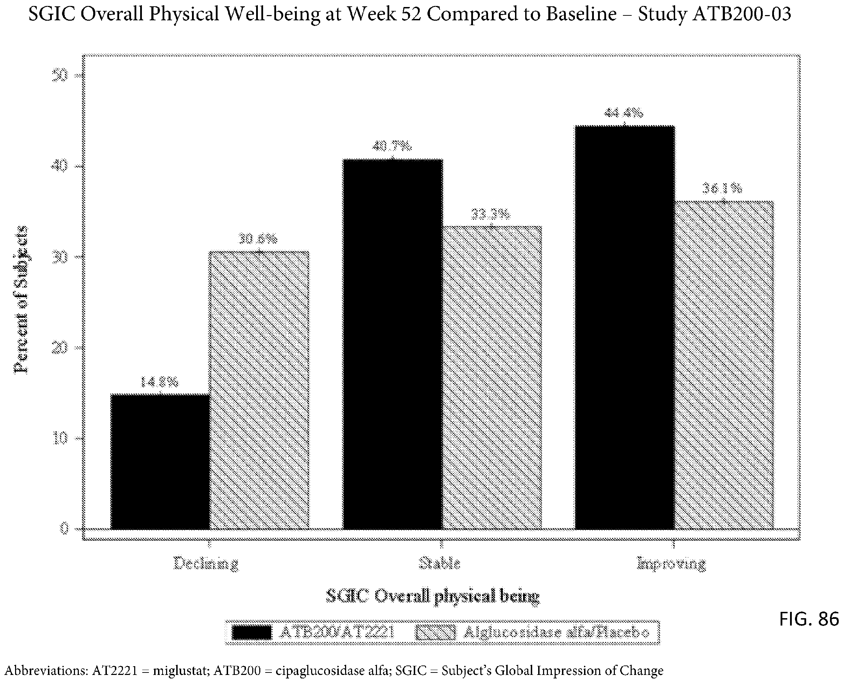

Fig. 86 is a bar chart illustrating SGIC overall physical well-being at week 52 compared to baseline in Study ATB200-03.

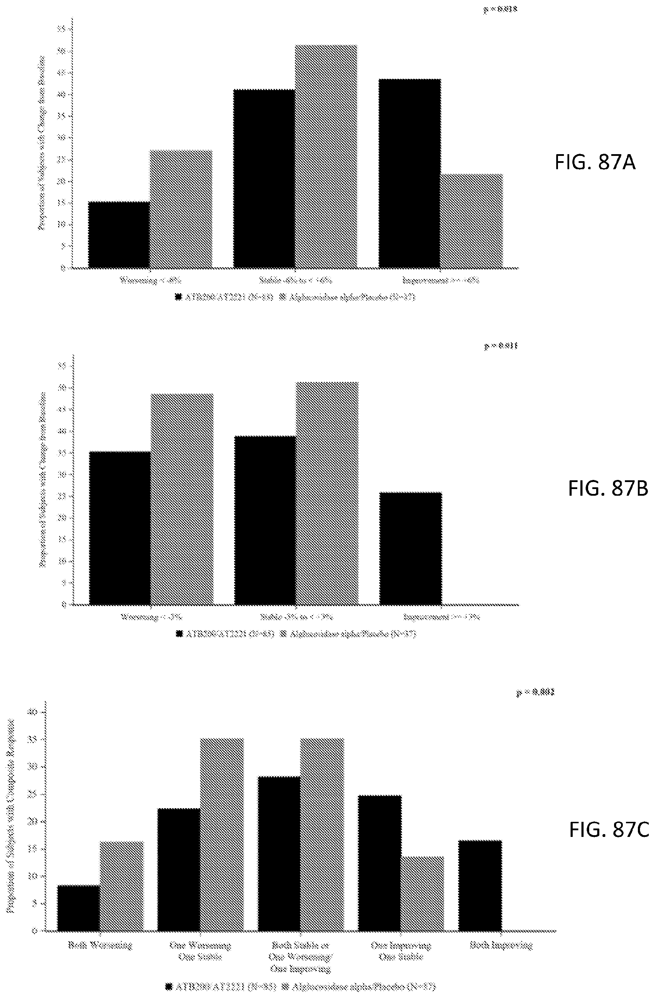

Fig. 87A is a bar chart illustrating the proportion of subjects with change from baseline at week 52 in 6MWD (meters) grouped by consolidated ranges (ITT-LOCF excluding subject 4005-2511) in Study ATB200-03. Fig. 87B is a bar chart illustrating the proportion of subjects with change from baseline at week 52 in Sitting % Predicted FVC grouped by consolidated ranges (ITT-LOCF excluding subject 4005-2511) in Study ATB200-03. Fig. 87C is a bar chart illustrating the proportion of subjects with composite responses on both 6MWD and % Predicted FVC at week 52 (ITT-LOCF excluding subject 4005-2511) in Study ATB200-03.

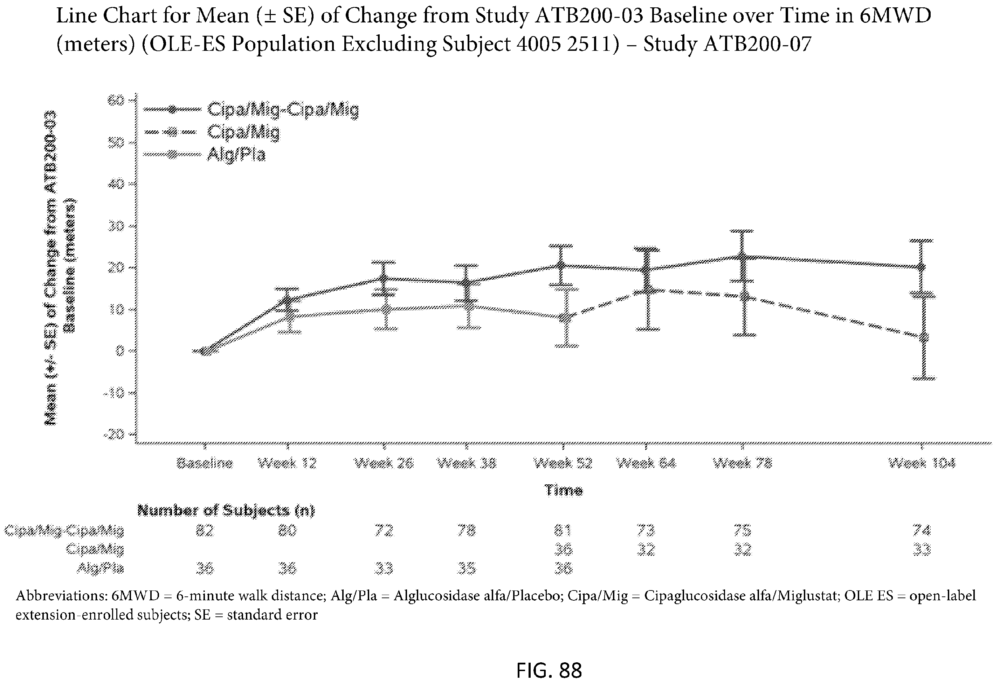

Fig. 88 is a line chart for Mean (± SE) of change from Study ATB200-03 baseline over time in 6MWD (meters) for the OLE-ES population excluding subject 4005-2511 as provided by Study ATB200-07.

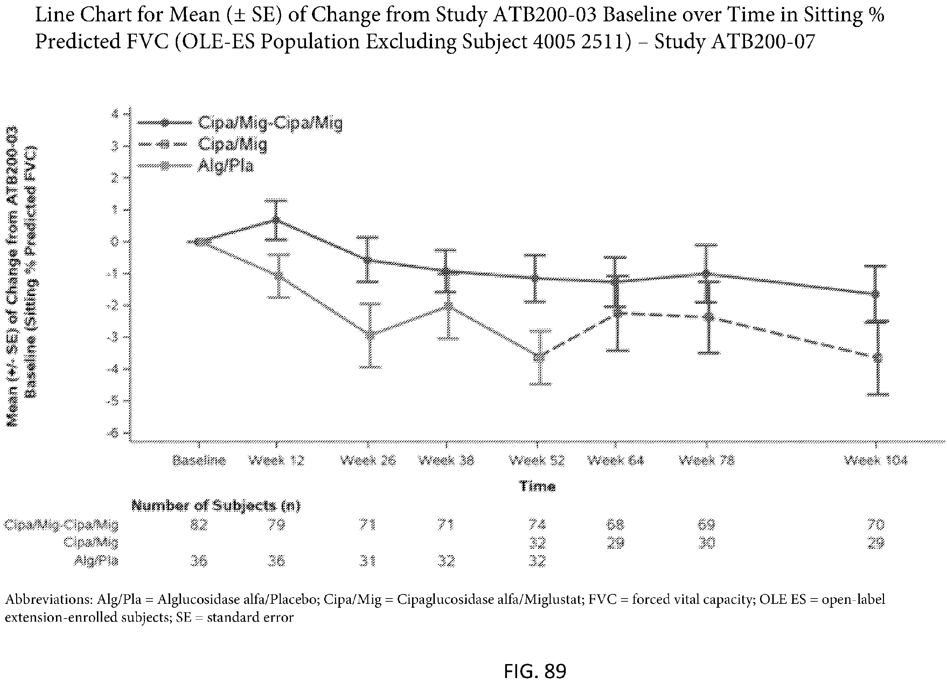

Fig. 89 is a line chart for Mean (± SE) of Change from Study ATB200-03 Baseline over Time in Sitting % Predicted FVC for the OLE-ES population excluding subject 4005-2511 as provided by Study ATB200-07.

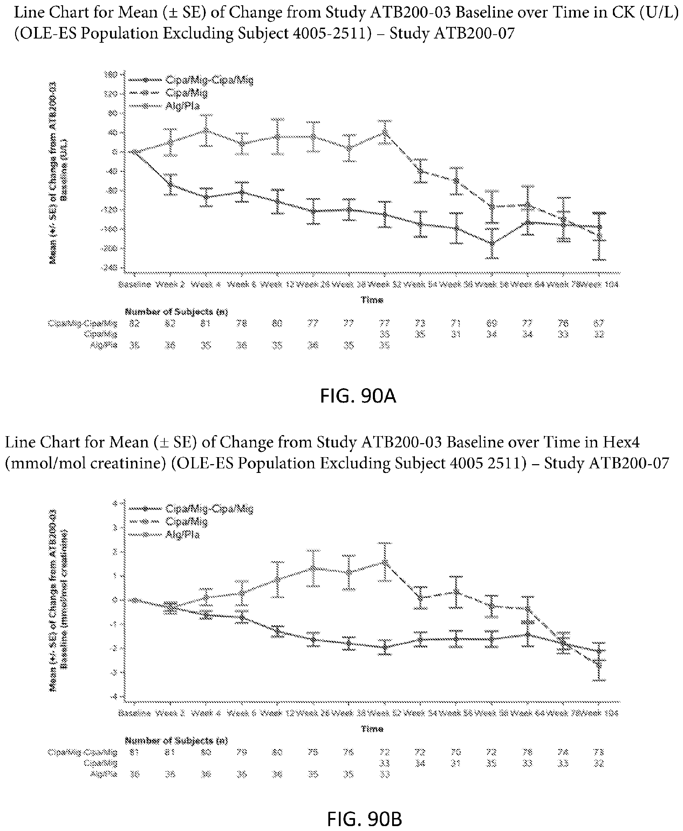

Fig. 90A is a line chart for Mean (± SE) of change from Study ATB200-03 baseline over time in CK (U/L) for the OLE-ES population excluding subject 4005-2511 as provided by Study ATB200-07. Fig. 90B is a line chart for Mean (± SE) of change from Study ATB200-03 baseline over time in Hex4 (mmol/mol creatinine) for the OLE-ES population excluding subject 4005-2511 as provided by Study ATB200-07.

### DETAILED DESCRIPTION

Provided herein are methods for treating Pompe disease comprising administering to an individual a recombinant human α-glucosidase (rhGAA) and an enzyme stabilizer, wherein the methods provide long term benefits (e.g., improvement and/or stabilization of motor function, muscle strength and/or pulmonary function). The methods provided herein also have a favorable safety profile. The prolonged benefits of the methods provided herein are an improvement over the current enzyme replacement therapy (ERT) options for treating Pompe disease.

The current ERT options for treating Pompe disease offer improvements for a limited duration, followed by a slow decline. In 2012, a systematic review of all studies performed in subjects with late-onset Pompe disease (LOPD) was performed (Toscano and Schoser, (2013) J Neurol 260, 951-959). The review included data on 368 subjects with LOPD from published studies, including 27 juvenile subjects (age range: 2 to 17 years old) and 251 adult subjects who received alglucosidase alfa for at least 2 preceding years. Results indicated that > 30% of subjects did not show an initial improvement during treatment with alglucosidase alfa and continued to experience deterioration of muscular and respiratory functions despite treatment. In the group of subjects who initially responded to alglucosidase alfa treatment, several additional longer-term studies showed that improvements usually lasted for only approximately 2 years. Thereafter, subjects generally plateaued before beginning to progressively decline.

In 2012, the United Kingdom Health Technology Assessment program, as part of the National Institute for Health Research (Wyatt et al., (2012) Health Technol Assess 16(39), issued recommendations drawn from review of longitudinal data for 81 patients with Pompe disease (including infantile- and late-onset forms (children and adults) who received the current approved ERT standard of care, alglucosidase alfa. Key markers of Pompe disease progression (forced vital capacity, ventilation dependency, mobility, 6-Minute Walk Test, muscle strength, and body mass index) were assessed and modeled with time of treatment on alglucosidase alfa therapy. Results of this assessment indicated that improvements in FVC, 6-minute walk distance, and muscle strength by patients with LOPD occurred for the first 2 years after commencing ERT with alglucosidase alfa, and decline occurred with continuing treatment beyond this timeframe. Additionally, a 3-year study in 38 subjects with LOPD receiving alglucosidase alfa showed that the subjects demonstrated an improvement in motor function in the first year of treatment, which remained generally stable in the second year and began to decline in the third year (Regnery et al., (2012) Journal of Inherited Metabolic Disease 35: 837-845).

Furthermore, a report providing a 10-year follow-up on a Phase 3 LUMIZYME^{®} (Sanofi Genzyme) study showed that after experiencing some improvement in motor and pulmonary function during the first couple of years of treatment, subjects began to slowly decline with ongoing treatment (van der Ploeg et al., (2017) European journal of neurology 24.6: 768-e31). In the study, from years 3 to 6 on therapy, there was an average decline of approximately 10% in percent predicted baseline 6 minute walk distance, with approximately 80% of subjects experiencing a decline.

The most serious tolerability issue with alglucosidase alfa is the occurrence of infusion-associated reactions (IARs), which, in some instances can include life-threatening anaphylaxis or other severe allergic responses (MYOZYME^{®} Summary of Product Characteristics, December 2018). Management of these events include dose reduction, reduced infusion rates and prolonged infusion times, and dose interruption or discontinuation. Premedication with antihistamines and steroids (prior to infusion) is also regularly used to prevent and reduce the occurrence and severity of IARs and hypersensitivities related to alglucosidase alfa infusion. Despite these measures, patients with Pompe disease may still experience IARs, and some cannot tolerate regular infusions of the currently approved ERT.

In 2017, a systematic review of the literature was undertaken by the European Pompe Consortium, a network of experts from 11 European countries in the field of Pompe disease (van der Ploeg et al., (2017) European journal of neurology 24.6: 768-e31). Based on the data obtained from one clinical study and 43 observational studies, covering a total of 586 individual adult subjects, evidence of an effect of ERT at group level was assessed by the consortium. The current European Pompe Consortium consensus is to discontinue ERT therapy upon the occurrence of severe IARs or the progressive clinical worsening of disease symptoms, as well as occurrence of high-neutralizing antibody (Ab) titers, which effectively inactivate the existing ERT treatment. The European Pompe Consortium consensus recommendation also included consideration for re-initiation of ERT treatment if disease progression and clinical worsening recur after ERT has been stopped.

The cellular uptake of a rhGAA molecule is facilitated by the specialized carbohydrate, mannose-6-phosphate (M6P), which binds to the cation-independent mannose-6-phosphate receptor (CIMPR) present on target cells such as muscle cells. Upon binding, rhGAA molecule is taken up by target cells and subsequently transported into the lysosomes within the cells. Most of the conventional rhGAA products, however, lack a high total content of mono-M6P- and bis-M6P-bearing N-glycans (i.e., N-glycans bearing one M6P residue or N-glycans bearing two M6P residues, respectively), which limits their cellular uptake via CIMPR and lysosomal delivery, thus making conventional enzyme replacement therapy insufficiently effective. For example, while conventional rhGAA products at 20 mg/kg or higher doses do ameliorate some aspects of Pompe disease, they are not able to adequately, among other things, (i) treat the underlying cellular dysfunction, (ii) restore muscle structure, or (iii) reduce accumulated glycogen in many target tissues, such as skeletal muscles, to reverse disease progression. Further, higher doses may impose additional burdens on the subject as well as medical professionals treating the subject, such as lengthening the infusion time needed to administer rhGAA intravenously.

The glycosylation of GAA or rhGAA can be enzymatically modified in vitro by the phosphotransferase and uncovering enzymes described by Canfield, et al., U.S. Patent No. 6,534,300, to generate M6P groups. However, enzymatic glycosylation cannot be adequately controlled and can produce rhGAA having undesirable immunological and pharmacological properties. Enzymatically modified rhGAA may contain only high-mannose oligosaccharide which all could be potentially enzymatically phosphorylated in vitro with a phosphotransferase or uncovering enzyme. The glycosylation patterns produced by in vitro enzymatic treatment of GAA are problematic because the additional terminal mannose residues, particularly non-phosphorylated terminal mannose residues, negatively affect the pharmacokinetics of the modified rhGAA. When such an enzymatically modified product is administered in vivo, these mannose groups increase non-productive clearance of the GAA, increase the uptake of the enzymatically-modified GAA by immune cells, and reduce rhGAA therapeutic efficacy due to less of the GAA reaching targeted tissues, such as skeletal muscle myocytes. For example, terminal non-phosphorylated mannose residues are known ligands for mannose receptors in the liver and spleen which leads to rapid clearance of the enzymatically-modified rhGAA and reduced targeting of rhGAA to target tissue. Moreover, the glycosylation pattern of enzymatically-modified GAA having high mannose N-glycans with terminal non-phosphorylated mannose residues resembles that on glycoproteins produced in yeasts and molds, and increases the risk of triggering immune or allergic responses, such as life-threatening severe allergic (anaphylactic) or hypersensitivity reactions, to the enzymatically modified rhGAA.

Compared with conventional recombinant rhGAA products and in vitro-phosphorylated rhGAA, the rhGAA used in the two-component therapy according to this disclosure has an optimized N-glycan profile for enhanced biodistribution and lysosomal uptake, thereby minimizing non-productive clearance of rhGAA once administered. The present disclosure provides stable or declining Pompe patients an effective therapy that reverses disease progression at the cellular level - including clearing lysosomal glycogen more efficiently than the current standard of care. Patients treated with the two-component therapy of the present disclosure comprising rhGAA and an enzyme stabilizer (e.g., miglustat) exhibit significant health improvements, including improvements and/or stabilization in muscle strength, motor function, and/or pulmonary function, and/or including a reversal in disease progression.

Moreover, a comparison of three randomized clinical trials (LOTS: alglucosidase alfa vs placebo; COMET: avalglucosidase alfa vs alglucosidase alfa; and PROPEL: cipaglucosidase alfa/miglustat vs alglucosidase alfa) share 6MWD and % predicted FVC as key primary or secondary endpoints. Using patient-level data from the PROPEL randomized clinical trial (RCT) plus aggregate published data from other RCTs, Phase I/II and open label extension trials, a multi-level network meta-regression was conducted, adjusting for various baseline covariates including previous ERT duration. The base case scenario had covariates resembling the PROPEL population (naive and ERT-experienced) and analyzed 6MWD and FVC change from baseline at 52 weeks. Cipaglucosidase alfa/miglustat was favored vs. alglucosidase alfa and avalglucosidase alfa for 6MWD and FVC, with 6MWD relative effects of 16.3 meters (95% confidence interval: 9.6-24.3) and 29.5 meters (7.4-52.6), respectively, and FVC relative effects of 3.1% (2.4-3.8) and 2.8% (1.0-4.6), respectively. For naive subjects, relative effects of cipaglucosidase alfa/miglustat remained significant for both endpoints, except cipaglucosidase alfa/miglustat vs. Aval for FVC: 0.4% (-0.8-1.7). These findings suggest cipaglucosidase alfa/miglustat may be clinically differentiated for LOPD patients on key motor and respiratory endpoints, particularly in the ERT-experienced population.

### DEFINITIONS

The terms used in this specification generally have their ordinary meanings in the art, within the context of this disclosure and in the specific context where each term is used. Certain terms are discussed below, or elsewhere in the specification, to provide additional guidance to the practitioner in describing the compositions and methods of the disclosure and how to make and use them. The articles "a" and "an" refer to one or to more than one (i.e., to at least one) of the grammatical object of the article. The term "or" means, and is used interchangeably with, the term "and/or," unless context clearly indicates otherwise. In this application, the use of the singular includes the plural unless specifically stated otherwise. Furthermore, the use of the term "including," as well as other forms, such as "includes" and "included," are not limiting. Any range described herein will be understood to include the endpoints and all values between the endpoints. In the present specification, except where the context requires otherwise due to express language or necessary implication, the word "comprises," or variations such as "comprising," is used in an inclusive sense, i.e., to specify the presence of the stated features but not to preclude the presence or addition of further features in various embodiments of the disclosure.

The term "GAA" refers to human acid α-glucosidase (GAA) enzyme that catalyzes the hydrolysis of α-1,4- and α-1,6-glycosidic linkages of lysosomal glycogen as well as to insertional, relational, or substitution variants of the GAA amino acid sequence and fragments of a longer GAA sequence that exert enzymatic activity. Human acid α-glucosidase is encoded by the GAA gene (National Centre for Biotechnology Information (NCBI) Gene ID 2548), which has been mapped to the long arm of chromosome 17 (location 17q25.2-q25.3). An exemplary amino acid sequence of GAA is NP 000143.2, which is incorporated by reference. This disclosure also encompasses DNA sequences that encode the amino acid sequence of NP 000143.2. More than 500 mutations have currently been identified in the human GAA gene, many of which are associated with Pompe disease. Mutations resulting in misfolding or misprocessing of the acid α-glucosidase enzyme include T1064C (Leu355Pro) and C2104T (Arg702Cys). In addition, GAA mutations which affect maturation and processing of the enzyme include Leu405Pro and Met519Thr. The conserved hexapeptide WIDMNE (SEQ ID NO: 7) at amino acid residues 516-521 is required for activity of the acid α-glucosidase protein. As used herein, the abbreviation "GAA" is intended to refer to human acid α-glucosidase enzyme, while the italicized abbreviation "*GAA*" is intended to refer to the human gene coding for the human acid α-glucosidase enzyme. The italicized abbreviation "*Gaa*" is intended to refer to non-human genes coding for non-human acid α-glucosidase enzymes, including but not limited to rat or mouse genes, and the abbreviation "Gaa" is intended to refer to non-human acid α-glucosidase enzymes.

The term "rhGAA" is intended to refer to the recombinant human acid α-glucosidase enzyme and is used to distinguish synthetic and/or recombinant-produced GAA (e.g., GAA produced from CHO cells or other host cells transformed with DNA encoding GAA) from endogenous GAA. Accordingly, rhGAA does not include endogenous GAA. The term "rhGAA" encompasses a population of individual rhGAA molecules. Characteristics of the population of rhGAA molecules are provided herein. The term "conventional rhGAA product" is intended to refer to products containing alglucosidase alfa, such as LUMIZYME^{®} or MYOZYME^{®}, or avalglucosidase alfa, such as NEXVIAZYME^{®}.

The term "genetically modified" or "recombinant" refers to cells, such as CHO cells, that express a particular gene product, such as rhGAA, following introduction of a nucleic acid comprising a coding sequence which encodes the gene product, along with regulatory elements that control expression of the coding sequence. Introduction of the nucleic acid may be accomplished by any method known in the art including gene targeting and homologous recombination. As used herein, the term also includes cells that have been engineered to express or overexpress an endogenous gene or gene product not normally expressed by such cell, e.g., by gene activation technology.

As used herein, the term "alglucosidase alfa" is intended to refer to a recombinant human acid α-glucosidase identified as [199-arginine,223-histidine]prepro-α-glucosidase (human); Chemical Abstracts Registry Number 420794-05-0. Alglucosidase alfa is approved for marketing in the United States by Sanofi Genzyme, as the products LUMIZYME^{®} and MYOZYME^{®}.

As used herein, the term "avalglucosidase alfa" is intended to refer to a recombinant human acid α-glucosidase identified as avalglucosidase alfa-ngpt; Chemical Abstracts Registry Number 1802558-87-7. Avalglucosidase alfa is approved for marketing in the United States by Sanofi Genzyme , as the product NEXVIAZYME^{®}.

As used herein, the term "ATB200" is intended to refer to a recombinant human acid α-glucosidase described in International Pat. App. No. PCT/2015/053252, U.S. Pat. No. 10,208,299, and U.S. Pat. No. 10,961,522, the disclosures of which are herein incorporated by reference in their entirety. ATB200 is also referred to as "cipaglucosidase alfa." In some embodiments, "ATB200" refers to a rhGAA with a high content of N-glycans bearing mono-M6P and bis-M6P, which is produced from a GA-ATB200 cell line and purified using methods described herein.

As used herein, the term "glycan" is intended to refer to an oligosaccharide covalently bound to an amino acid residue on a protein or polypeptide. As used herein, the term "N- glycan" or "N-linked glycan" is intended to refer to a polysaccharide chain attached to an asparagine residue on a protein or polypeptide through covalent binding to a nitrogen atom of the asparagine residue. In some embodiments, the N-glycan units attached to a rhGAA are determined by liquid chromatography-tandem mass spectrometry (LC-MS/MS) utilizing an instrument such as the Thermo Scientific^{™} Orbitrap Velos Pro^{™} Mass Spectrometer, Thermo Scientific^{™} Orbitrap Fusion^{™} Lumos Tribid^{™} Mass Spectrometer, or Waters Xevo^{®} G2-XS QTof Mass Spectrometer.

As used herein, the term "glycan bearing mono-M6P" or "glycan bearing bis-M6P" is intended to refer to an N-glycan unit of the mono-phosphorylated (mono-M6P) or bis-phosphorylated (bis-M6P) as part of all N-glycan types, unless specifically stated to be the high mannose N-glycan type or the hybrid N-glycan class.

As used herein, forced vital capacity, or "FVC," is the amount of air that can be forcibly exhaled from the lungs of a subject after the subject takes the deepest breath possible.

As used herein, a "six-minute walk test" (6MWT) is a test for measuring the distance an individual is able to walk over a total of six minutes on a hard, flat surface. The test is conducted by having the individual to walk as far as possible in six minutes.

As used herein, a "ten-meter walk test" (10MWT) is a test for measuring the time it takes an individual in walking shoes to walk ten meters on a flat surface.

As used herein, the compound miglustat, also known as N-butyl-1-deoxynojirimycin or NB-DNJ or (2R,3R,4R,5S)-1-butyl-2-(hydroxymethyl)piperidine-3,4,5-triol, is a compound having the following chemical formula: 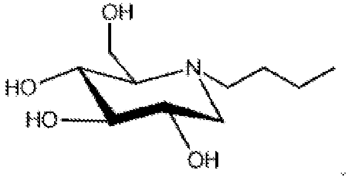 Alternatively shown as:

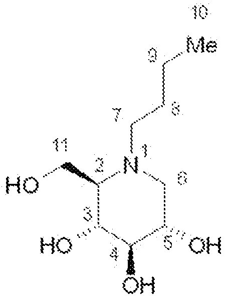

One formulation of miglustat is marketed commercially under the trade name ZAVESCA^{®} as monotherapy for type 1 Gaucher disease. In some embodiments, miglustat is referred to as AT2221.

As discussed below, pharmaceutically acceptable salts of miglustat may also be used in the present disclosure. When a salt of miglustat is used, the dosage of the salt will be adjusted so that the dose of miglustat received by the patient is equivalent to the amount which would have been received had the miglustat free base been used.

As used herein, the compound duvoglustat, also known as 1-deoxynojirimycin or DNJ or (2R,3R,4R,5S)-2-(hydroxymethyl)piperidine-3,4,5-triol, is a compound having the following chemical formula:

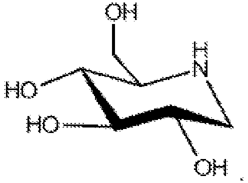

As used herein, the term "enzyme stabilizer" is intended to refer to a molecule that specifically binds to acid α-glucosidase and has one or more of the following effects:
- enhances the formation of a stable molecular conformation of the protein;
- enhances proper trafficking of the protein from the endoplasmic reticulum to another cellular location, preferably a native cellular location, so as to prevent endoplasmic reticulum-associated degradation of the protein;
- prevents aggregation of conformationally unstable or misfolded proteins;
- restores and/or enhances at least partial wild-type function, stability, and/or activity of the protein;
- improves the phenotype or function of the cell harboring acid α-glucosidase; and/or
- stabilizes the acid α-glucosidase *in vitro* and/or *in vivo* (e.g., in a patient's bloodstream).

Enzyme stabilizers are also sometimes known as "pharmacological chaperones."

Thus, an enzyme stabilizer for acid α-glucosidase is a molecule that binds to acid α-glucosidase, resulting in proper folding, trafficking, non-aggregation, and/or activity of acid α-glucosidase. In at least one embodiment, the enzyme stabilizer is miglustat. Another nonlimiting example of an enzyme stabilizer for acid α-glucosidase is duvoglustat.

As used herein, the term "pharmaceutically acceptable" is intended to refer to molecular entities and compositions that are physiologically tolerable and do not typically produce untoward reactions when administered to a human. Preferably, as used herein, the term "pharmaceutically acceptable" means approved by a regulatory agency of the federal or a state government or listed in the U.S. Pharmacopeia or other generally recognized pharmacopeia for use in animals, and more particularly in humans. As used herein, the term "carrier" is intended to refer to a diluent, adjuvant, excipient, or vehicle with which a compound is administered. Suitable pharmaceutical carriers are known in the art and, in at least one embodiment, are described in "Remington's Pharmaceutical Sciences" by E. W. Martin, 18th Edition, or other editions.

The term "pharmaceutically acceptable salt" as used herein is intended to mean a salt which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. Lists of suitable salts are found in, for example, S. M. Berge et al., J. Pharm. Sci., 1977, 66, pp. 1-19, herein incorporated by reference. The term "pharmaceutically-acceptable acid addition salt" as used herein is intended to mean those salts which retain the biological effectiveness and properties of the free bases and which are not biologically or otherwise undesirable, formed with inorganic acids. The term "pharmaceutically-acceptable base addition salt" as used herein is intended to mean those salts which retain the biological effectiveness and properties of the free acids and which are not biologically or otherwise undesirable, formed with inorganic bases.

As used herein, the term "buffer" refers to a solution containing a weak acid and its conjugate base or a weak base and its conjugate acid that helps to prevent changes in pH.

As used herein, the terms "therapeutically effective dose" and "effective amount" are intended to refer to an amount of acid α-glucosidase and/or of miglustat and/or of a two-component therapy thereof, which is sufficient to result in a therapeutic response in a subject.

The therapeutic response may also include molecular responses such as glycogen accumulation, lysosomal proliferation, and formation of autophagic zones. The therapeutic responses may be evaluated by comparing physiological and molecular responses of muscle biopsies before and after treatment with a rhGAA described herein. For instance, the amount of glycogen present in the biopsy samples can be used as a marker for determining the therapeutic response. Another example includes biomarkers such as lysosome-associated protein 1 (LAMP-1), microtubule-associated protein *1* light chain 3 (LC3), and Dysferlin, which can be used as an indicator of lysosomal storage dysfunction. Additional biomarkers include biomarkers of muscle injury or damage such as creatine kinase (CK), lactate dehydrogenase (LDH), alanine aminotransferase (ALT), and aspartate aminotransferase (AST), and/or markers of glycogen accumulation such as urine hexose tetrasaccharide (Hex4). For instance, muscle biopsies collected prior to and after treatment with a rhGAA described herein may be stained with an antibody that recognizes one of the biomarkers. The therapeutic response may also include a decrease in fatigue or improvement in other patient-reported outcomes (e.g., daily living activities, well-being, etc.).

As used herein, the term "enzyme replacement therapy" or "ERT" is intended to refer to the introduction of a non-native, purified enzyme into an individual having a deficiency in such enzyme. The administered protein can be obtained from natural sources or by recombinant expression. The term also refers to the introduction of a purified enzyme in an individual otherwise requiring or benefiting from administration of a purified enzyme. In at least one embodiment, such an individual suffers from enzyme insufficiency. The introduced enzyme may be a purified, recombinant enzyme produced in vitro, or a protein purified from isolated tissue or fluid, such as, for example, placenta or animal milk, or from plants.

As used herein, the term "two-component therapy" is intended to refer to any therapy wherein two or more individual therapies are administered concurrently or sequentially. In some embodiment, the results of the two-component therapy are enhanced as compared to the effect of each therapy when it is performed individually. Enhancement may include any improvement of the effect of the various therapies that may result in an advantageous result as compared to the results achieved by the therapies when performed alone. Enhanced effect or results can include a synergistic enhancement, wherein the enhanced effect is more than the additive effects of each therapy when performed by itself; an additive enhancement, wherein the enhanced effect is substantially equal to the additive effect of each therapy when performed by itself; or less than additive effect, wherein the enhanced effect is lower than the additive effect of each therapy when performed by itself, but still better than the effect of each therapy when performed by itself. Enhanced effect may be measured by any means known in the art by which treatment efficacy or outcome can be measured.

"Pompe disease" refers to an autosomal recessive LSD characterized by deficient acid alpha glucosidase (GAA) activity which impairs lysosomal glycogen metabolism. The enzyme deficiency leads to lysosomal glycogen accumulation and results in progressive skeletal muscle weakness, reduced cardiac function, respiratory insufficiency, and/or CNS impairment at late stages of disease. Genetic mutations in the GAA gene result in either lower expression or produce mutant forms of the enzyme with altered stability, and/or biological activity ultimately leading to disease, (see generally Hirschhorn R, 1995, Glycogen Storage Disease Type II: Acid a-Glucosidase (Acid Maltase) Deficiency, The Metabolic and Molecular Bases of Inherited Disease, Scriver et al., eds., McGraw-Hill, New York, 7th ed., pages 2443-2464). The three recognized clinical forms of Pompe Disease (infantile, juvenile, and adult) are correlated with the level of residual α-glucosidase activity (Reuser A J et al., 1995, Glycogenosis Type II (Acid Maltase Deficiency), Muscle & Nerve Supplement 3, S61-S69). Infantile Pompe disease (type I or A) is most common and most severe, characterized by failure to thrive, generalized hypotonic, cardiac hypertrophy, and cardiorespiratory failure within the second year of life. Juvenile Pompe disease (type II or B) is intermediate in severity and 26iglustatterized by a predominance of muscular symptoms without cardiomegaly. Juvenile Pompe individuals usually die before reaching 20 years of age due to respiratory failure. Adult Pompe disease (type III or C) often presents as a slowly progressive myopathy in the teenage years or as late as the sixth decade (Felicia K J et al., 1995, Clinical Variability in Adult-Onset Acid Maltase Deficiency: Report of Affected Sibs and Review of the Literature, Medicine 74, 131-135). In Pompe disease, it has been shown that α-glucosidase is extensively modified post-translationally by glycosylation, phosphorylation, and proteolytic processing. Conversion of the 110 kilodalton (kDa) precursor to 76 and 70 kDa mature forms by proteolysis in the lysosome is required for optimum glycogen catalysis. As used herein, the term "Pompe disease" refers to all types of Pompe disease. The formulations and dosing regimens disclosed in this application may be used to treat, for example, Type I, Type II or Type III Pompe disease.

Pompe disease is now considered to be a continuous spectrum of phenotypes, with the clinically most severe, rapidly progressive phenotypes being the classic infantile-onset Pompe disease (IOPD) and the less severe, slowly progressive phenotypes being late-onset Pompe disease (LOPD). Late-onset Pompe disease can manifest in childhood or adulthood and does not present with clinically apparent cardiac involvement (Leslie and Bailey, 2017). Late-onset Pompe disease is often referred to as juvenile-onset Pompe disease when occurring in the pediatric subpopulation of the LOPD category. LOPD has a slower rate of progression compared with classic IOPD, with most patients experiencing progressive limb girdle weakness and respiratory failure due to involvement of muscles in the proximal lower and upper limbs, paraspinal muscles, and diaphragm. Clinical manifestations include difficulty walking, climbing stairs, and progressive limitations of motor activities of daily living with progression to a need for ambulatory support followed by wheelchair dependence (Reuser, et al 2001). Clinical manifestations of the disease are compounded by respiratory involvement, initially as sleep disordered breathing and orthopnea (shortness of breath in supine position). The progressive nature of Pompe disease generally results in the use of invasive mechanically assisted ventilation. Biochemical abnormalities include increased level of serum creatine kinase (CK), a biomarker of muscle injury, and urinary hexose tetrasaccharide (Hex4), a biomarker of disease substrate (An, et al 2005; Young, et al 2009). Life expectancy for patients with LOPD can range from early childhood to late adulthood, depending on the age of onset, rate of disease progression, the extent of respiratory muscle involvement, and the presence of co morbidities (Hagemans, et al 2004). If untreated, life expectancy in adults with Pompe disease is greatly reduced (Gungor, et al 2011).

As used herein, "significant" refers to statistical significance. The term refers to statistical evidence that there is a difference between two treatment groups. It can be defined as the probability of making a decision to reject the null hypothesis when the null hypothesis is actually true. The decision is often made using a p-value < 0.05 derived from a suitable statistical analysis for the comparison. See, e.g., Example 9.

A "subject" or "patient" is preferably a human, though other mammals and non-human animals having disorders involving accumulation of glycogen may also be treated. A subject may be a fetus, a neonate, child, juvenile, or an adult with Pompe disease or other glycogen storage or accumulation disorder. One example of an individual being treated is an individual (fetus, neonate, child, juvenile, adolescent, or adult human) having GSD-II (e.g., infantile GSD-II, juvenile GSD-II, or adult-onset GSD-II). The individual can have residual GAA activity, or no measurable activity. For example, the individual having GSD-II can have GAA activity that is less than about 1% of normal GAA activity (infantile GSD-II), GAA activity that is about 1-10% of normal GAA activity (juvenile GSD-II), or GAA activity that is about 10-40% of normal GAA activity (adult GSD-II). In some embodiments, the subject or patient is an "ERT-experienced" or "ERT-switch" patient, referring to a Pompe disease patient who has previously received enzyme replacement therapy. In some embodiments, an "ERT-experienced" or "ERT-switch" patient is a Pompe disease patient who has received or is currently receiving alglucosidase alfa for greater than or equal to 24 months. In some embodiments, an "ERT-experienced" or "ERT-switch" patient is a Pompe disease patient who is declining on currently approved ERT (e.g., MYOZYME^{®} or LUMIZYME^{®}). In some embodiments, the subject is an adult patient (e.g., 18 years of age or older) with a confirmed diagnosis of late onset Pompe disease (acid α-glucosidase (GAA) deficiency), who have previously received enzyme replacement therapy (ERT). In some embodiments, the subject is an adult (e.g., 18 years of age and older) with late-onset Pompe disease (lysosomal acid alpha-glucosidase [GAA] deficiency) weighing ≥ 40 kg whose disease has progressed on enzyme replacement therapy (ERT). In some embodiments, the subject or patient is an "ERT-naïve" patient, referring to a Pompe disease patient who has not previously received enzyme replacement therapy. In certain embodiments, the subject or patient is ambulatory (e.g., an ambulatory ERT-switch patient or an ambulatory ERT-naïve patient). In certain embodiments, the subject or patient is nonambulatory (e.g., a nonambulatory ERT-switch patient). Ambulatory or nonambulatory status may be determined by a six-minute walk test (6MWT). In some embodiments, an ambulatory patient is a Pompe disease patient who is able to walk at least 200 meters in the 6MWT. In some embodiments, a nonambulatory patient is a Pompe disease patient who is unable to walk unassisted or who is wheelchair bound. In some embodiments, the subject is using effective contraception. In some embodiments, the subject and/or the subject's partner are using highly effective contraception, such as one that results in a low failure rate (e.g., < 1% per year) when used consistently and correctly. Examples of highly effective methods of contraception include, but are not limited to: total abstinence; combined (estrogen- and progestogen-containing) hormonal contraception associated with inhibition of ovulation; oral, intravaginal, transdermal progestogen-only hormonal contraception associated with inhibition of ovulation: oral, injectable, implantable intrauterine device; intrauterine hormone-releasing system; bilateral tubal occlusion; and vasectomy. In some embodiments, the subject is post-menopausal. In some embodiments, the subject is not of child-bearing potential. In some embodiments, the subject is permanently sterile. In some embodiments, the subject is not pregnant. In some embodiments, the subject is not breastfeeding.

In some embodiments, a patient has a diagnosis of late onset Pompe disease, based on documentation of at least one of the following: (1) deficiency of GAA enzyme; and/or (2) gene encoding human acid α-glucosidase (GAA) genotyping. In some embodiments, the patient is 18 years of age or older. In some embodiments, the patient has previously received enzyme replacement therapy (ERT). In some embodiments, the patient is declining on currently approved ERT (e.g., MYOZYME^{®} or LUMIZYME^{®}). In some embodiments, if of reproductive potential, both male and female patients have agreed to use a highly effective method of contraception throughout the duration of the treatment and for up to 90 days after their last dose. In some embodiments, patients who are taking β2-receptor agonists or non-selective β-blockers (e.g., propranolol, nadolol, carvedilol) maintain a stable dose as appropriate and determined by the treating physician.

The terms "treat" and "treatment," as used herein, refer to amelioration of one or more symptoms associated with the disease, delay of the onset of one or more symptoms of the disease, and/or lessening of the severity or frequency of one or more symptoms of the disease. For example, treatment can refer to improvement of cardiac status (e.g., increase of end-diastolic and/or end-systolic volumes, or reduction or amelioration of the progressive cardiomyopathy that is typically found in GSD-II) or of pulmonary function (e.g., increase in crying vital capacity over baseline capacity, and/or normalization of oxygen desaturation during crying); improvement in neurodevelopment and/or motor skills (e.g., increase in AIMS score); reduction of glycogen levels in tissue of the individual affected by the disease; or any combination of these effects. In one preferred embodiment, treatment includes improvement of cardiac status, particularly in reduction of GSD-II-associated cardiomyopathy.

The terms "improve," "increase," and "reduce," as used herein, indicate values that are relative to a baseline measurement or the corresponding values from a control treatment, such as a measurement in the same individual prior to initiation of the treatment described herein, a measurement in a control individual (or multiple control individuals) in the absence of the treatment described herein, or a measurement after a control treatment. A control individual is an individual afflicted with the same form of GSD-II (either infantile, juvenile, or adult-onset) as the individual being treated, who is about the same age as the individual being treated (to ensure that the stages of the disease in the treated individual and the control individual(s) are comparable). In some embodiments, a control treatment comprises administering alglucosidase alfa and a placebo for an enzyme stabilizer (see Example 9).

As used herein, the phrases "stabilizing motor function", "stabilizing pulmonary function", and similar terms refer to reducing or arresting the decline in motor and pulmonary function, and/or restoring motor and/or pulmonary function. As untreated Pompe patients are expected to have significant decreases in motor function and pulmonary function over time, enhancements in the rate of motor and/pulmonary function deterioration and/or enhancements in motor and/pulmonary function demonstrate a benefit of therapy as described herein. Moreover, as ERT-experienced Pompe patients often continue to decline in motor and/pulmonary function over time, stabilizing motor and/or pulmonary function using the therapy described herein can include reducing and/or arresting the decline in motor and/or pulmonary function compared to such patients receiving the previous ERT treatment (e.g., MYOZYME^{®} or LUMIZYME^{®}).

As used herein, the terms "about" and "approximately" are intended to refer to an acceptable degree of error for the quantity measured given the nature or precision of the measurements. For example, the degree of error can be indicated by the number of significant figures provided for the measurement, as is understood in the art, and includes but is not limited to a variation of ±1 in the most precise significant figure reported for the measurement. Typical exemplary degrees of error are within 20 percent (%), preferably within 10%, and more preferably within 5% of a given value or range of values. Numerical quantities given herein are approximate unless stated otherwise, meaning that the term "about" or "approximately" can be inferred when not expressly stated.

All references, articles, publications, patents, patent publications, and patent applications cited herein are incorporated by reference in their entireties for all purposes. However, mention of any reference, article, publication, patent, patent publication, and patent application cited herein is not, and should not be taken as an acknowledgment or any form of suggestion that they constitute valid prior art or form part of the common general knowledge in any country in the world.

The section headings used herein are for organizational purposes only and are not to be construed as limiting the subject matter described.

### RECOMBINANT HUMAN ACID A-GLUCOSIDASE (rhGAA)

In some embodiments, the recombinant human acid α-glucosidase (rhGAA) is an enzyme having an amino acid sequence as set forth in SEQ ID NO: 1, SEQ ID NO: 3, SEQ ID NO: 4, SEQ ID NO: 5, or SEQ ID NO: 6. In some embodiments, the rhGAA is encoded by a nucleotide sequence as set forth in SEQ ID NO: 2.

**Table 1. Nucleotide Sequences and Protein Sequences**

| **SEQ ID NO** | **Sequences** |
|---|---|
| 1 | MGVRHPPCSHRLLAVCALVSLATAALLGHILLHDFLLVPRELSGSSPVLEETHPAHQQGA SRPGPRDAQAHPGRPRAVPTQCDVPPNSRFDCAPDKAITQEQCEARGCCYIPAKQGLQGA QMGQPWCFFPPSYPSYKLENLSSSEMGYTATLTRTTPTFFPKDILTLRLDVMMETENRLH FTIKDPANRRYEVPLETPRVHSRAPSPLYSVEFSEEPFGVIVHRQLDGRVLLNTTVAPLF FADQFLQLSTSLPSQYITGLAEHLSPLMLSTSWTRITLWNRDLAPTPGANLYGSHPFYLA LEDGGSAHGVFLLNSNAMDVVLQPSPALSWRSTGGILDVYIFLGPEPKSVVQQYLDVVGY PFMPPYWGLGFHLCRWGYSSTAITRQVVENMTRAHFPLDVQWNDLDYMDSRRDFTFNKDG FRDFPAMVQELHQGGRRYMMIVDPAISSSGPAGSYRPYDEGLRRGVFITNETGQPLIGKV WPGSTAFPDFTNPTALAWWEDMVAEFHDQVPFDGMWIDMNEPSNFIRGSEDGCPNNELEN PPYVPGVVGGTLQAATICASSHQFLSTHYNLHNLYGLTEAIASHRALVKARGTRPFVISR STFAGHGRYAGHWTGDVWSSWEQLASSVPEILQFNLLGVPLVGADVCGFLGNTSEELCVR WTQLGAFYPFMRNHNSLLSLPQEPYSFSEPAQQAMRKALTLRYALLPHLYTLFHQAHVAG ETVARPLFLEFPKDSSTWTVDHQLLWGEALLITPVLQAGKAEVTGYFPLGTWYDLQTVPI EALGSLPPPPAAPREPAIHSEGQWVTLPAPLDTINVHLRAGYIIPLQGPGLTTTESRQQP MALAVALTKGGEARGELFWDDGESLEVLERGAYTQVIFLARNNTIVNELVRVTSEGAGLQ LQKVTVLGVATAPQQVLSNGVPVSNFTYSPDTKVLDICVSLLMGEQFLVSWC |
| 2 | cagttgggaaagctgaggttgtcgccggggccgcgggtggaggtcggggatgaggcagcaggt aggacagtgacctcggtgacgcgaaggaccccggccacctctaggttctcctcgtccgcccgt tgttcagcgagggaggctctgggcctgccgcagctgacggggaaactgaggcacggagcgggc ctgtaggagctgtccaggccatctccaaccatggagtgaggcacccgccctgctcccaccgg ctcctggccgtctgcgccctcgtgtccttggcaaccgctgcactcctggggcacatcctactc catgatttcctgctggttccccgagagctgagtggctcctccccagtcctggaggagactcac ccagctcaccagcagggagccagcagaccagggccccgggatgcccaggcacaccccggccgt cccagagcagtgcccacacagtgcgacgtcccccccaacagccgcttcgattgcgcccctgac aaggccatcacccaggaacagtgcgaggcccgcggctgctgctacatccctgcaaagcagggg ctgcagggagcccagatgggcagccctggtgcttcttcccacccagctaccccagctacaag ctggagaacctgagctcctctgaaatgggctacacggccaccctgacccgtaccacccccacc ttcttccccaaggacatcctgaccctgcggctggacgtgatgatggagactgagaaccgcctc cacttcacgatcaaagatccagctaacaggcgctacgaggtgcccttggagaccccgcgtgtc cacagccgggcaccgtccccactctacagcgtggagttctccgaggagcccttcggggtgatc gtgcaccggcagctggacggccgcgtgctgctgaacacgacggtggcgcccctgttctttgcg gaccagttccttcagctgtccacctcgctgccctcgcagtatatcacaggcctcgccgagcac ctcagtcccctgatgctcagcaccagctggaccaggatcaccctgtggaaccgggaccttgcg cccacgcccggtgcgaacctctacgggtctcaccctttctacctggcgctggaggacggcggg tcggcacacggggtgttcctgctaaacagcaatgccatggatgtggtcctgcagccgagccct gcccttagctggaggtcgacaggtgggatcctggatgtctacatcttcctgggcccagagccc aagagcgtggtgcagcagtacctggacgttgtgggatacccgttcatgccgccatactggggc ctgggcttccacctgtgccgctggggctactcctccaccgctatcacccgccaggtggtggag aacatgaccagggcccacttccccctggacgtccaatggaacgacctggactacatggactcc cggagggacttcacgttcaacaaggatggcttccgggacttcccggccatggtgcaggagctg caccaggcggccggcgctacatgatgatcgtggatcctgccatcagcagctcgggccctgcc gggagctacaggccctacgacgagggtctgcggagggggttttcatcaccaacgagaccggc cagccgctgattgggaaggtatggcccgggtccactgccttccccgacttcaccaaccccaca gccctggcctggtgggaggacatggtggctgagttccatgaccaggtgcccttcgacggcatg tggattgacatgaacgagccttccaacttcatcagaggctctgaggacggctgccccaacaat gagctggagaacccaccctacgtgcctggggtggttgggggaccctccaggcggccaccatc tgtgcctccagccaccagtttctctccacacactacaacctgcacaacctctacggcctgacc gaagccatcgcctcccacagggcgctggtgaaggctcggggacacgcccatttgtgatctcc cgctcgacctttgctggccacggccgatacgccggccactggacgggggacgtgtggagctcc tgggagcagctcgcctcctccgtgccagaaatcctgcagtttaacctgctgggggtgcctctg |
| | gtcggggccgacgtctgcggcttcctggcaacacctcagaggagctgtgtgtgcgctggacc cagctgggggccttctaccccttcatgcggaaccacaacagcctgctcagtctgccccaggag ccgtacagcttcagcgagccggcccagcaggccatgaggaaggccctcaccctgcgctacgca ctcctcccccacctctacacactgttccaccaggcccacgtcgcgggggagaccgtggcccgg cccctcttcctggagttccccaaggactctagcacctggactgtggaccaccagctcctgtgg ggggaggccctgctcatcaccccagtgctccaggccgggaaggccgaagtgactggctacttc cccttgggcacatggtacgacctgcagacggtgccaatagaggcccttggcagcctcccaccc ccacctgcagctccccgtgagccagccatccacagcgagggcagtgggtgacgctgccggcc cccctggacaccatcaacgtccacctccgggctgggtacatcatcccccctgcagggccctggc ctcacaaccacagagtcccgccagcagcccatggccctggctgtggccctgaccaaggtgga gaggcccgagggagctgttctgggacgatggagagagcctggaagtgctggagcgagggcc tacacacaggtcatcttcctggccaggaataacacgatcgtgaatgagctggtacgtgtgacc agtgagggagctggcctgcagctgcagaaggtgactgtcctgggcgtggccacggcgccccag caggtcctctccaacggtgtccctgtctccaacttcacctacagccccgacaccaaggtcctg gacatctgtgtctcgctgttgatgggagagcagtttctcgtcagctggtgttagccgggcgga gtgtgttagtctctccagagggaggctggttccccagggaagcagagcctgtgtgcgggcagc agctgtgtgcgggcctgggggttgcatgtgtcacctggagctgggcactaaccattccaagcc gccgcatcgcttgtttccacctcctgggccggggctctggcccccaacgtgtctaggagagct ttctccctagatcgcactgtgggccggggcctggagggctgctctgtgttaataagattgtaa ggtttgccctcctcacctgttgccggcatgcgggtagtattagccacccccctccatctgttc ccagcaccggagaaggggggtgctcaggtggaggtgtggggtatgcacctgagctcctgcttcg cgcctgctgctctgccccaacgcgaccgcttcccggctgcccagaggctggatgcctgccgg tccccgagcaagcctgggaactcaggaaaattcacaggacttgggagattctaaatcttaagt gcaattattttaataaaagggcatttggaatc |
| 3 | MGVRHPPCSHRLLAVCALVSLATAALLGHILLHDFLLVPRELSGSSPVLEETHPAHQQGA SRPGPRDAQAHPGRPRAVPTQCDVPPNSRFDCAPDKAITQEQCEARGCCYIPAKQGLQGA QMGQPWCFFPPSYPSYKLENLSSSEMGYTATLTRTTPTFFPKDILTLRLDVMMETENRLH FTIKDPANRRYEVPLETPRVHSRAPSPLYSVEFSEEPFGVIVHRQLDGRVLLNTTVAPLF FADQFLQLSTSLPSQYITGLAEHLSPLMLSTSWTRITLWNRDLAPTPGANLYGSHPFYLA LEDGGSAHGVFLLNSNAMDVVLQPSPALSWRSTGGILDVYIFLGPEPKSVVQQYLDVVGY PFMPPYWGLGFHLCRWGYSSTAITRQVVENMTRAHFPLDVQWNDLDYMDSRRDFTFNKDG FRDFPAMVQELHQGGRRYMMIVDPAISSSGPAGSYRPYDEGLRRGVFITNETGQPLIGKV WPGSTAFPDFTNPTALAWWEDMVAEFHDQVPFDGMWIDMNEPSNFIRGSEDGCPNNELEN PPYVPGVVGGTLQAATICASSHQFLSTHYNLHNLYGLTEAIASHRALVKARGTRPFVISR STFAGHGRYAGHWTGDVWSSWEQLASSVPEILQFNLLGVPLVGADVCGFLGNTSEELCVR WTQLGAFYPFMRNHNSLLSLPQEPYSFSEPAQQAMRKALTLRYALLPHLYTLFHQAHVAG ETVARPLFLEFPKDSSTWTVDHQLLWGEALLITPVLQAGKAEVTGYFPLGTWYDLQTVPI EALGSLPPPPAAPREPAIHSEGQWVTLPAPLDTINVHLRAGYIIPLQGPGLTTTESRQQP MALAVALTKGGEARGELFWDDGESLEVLERGAYTQVIFLARNNTIVNELVRVTSEGAGLQ LQKVTVLGVATAPQQVLSNGVPVSNFTYSPDTKVLDICVSLLMGEQFLVSWC |
| 4 | |
| | |
| 5 | QQGASRPGPRDAQAHPGRPRAVPTQCDVPPNSRFDCAPDKAITQEQCEARGCCYIPAKQG LQGAQMGQPWCFFPPSYPSYKLENLSSSEMGYTATLTRTTPTFFPKDILTLRLDVMMETE NRLHFTIKDPANRRYEVPLETPRVHSRAPSPLYSVEFSEEPFGVIVHRQLDGRVLLNTTV APLFFADQFLQLSTSLPSQYITGLAEHLSPLMLSTSWTRITLWNRDLAPTPGANLYGSHP FYLALEDGGSAHGVFLLNSNAMDVVLQPSPALSWRSTGGILDVYIFLGPEPKSVVQQYLD VVGYPFMPPYWGLGFHLCRWGYSSTAITRQVVENMTRAHFPLDVQWNDLDYMDSRRDFTF NKDGFRDFPAMVQELHQGGRRYMMIVDPAISSSGPAGSYRPYDEGLRRGVFITNETGQPL IGKVWPGSTAFPDFTNPTALAWWEDMVAEFHDQVPFDGMWIDMNEPSNFIRGSEDGCPNN ELENPPYVPGVVGGTLQAATICASSHQFLSTHYNLHNLYGLTEAIASHRALVKARGTRPF VISRSTFAGHGRYAGHWTGDVWSSWEQLASSVPEILQFNLLGVPLVGADVCGFLGNTSEE LCVRWTQLGAFYPFMRNHNSLLSLPQEPYSFSEPAQQAMRKALTLRYALLPHLYTLFHQA HVAGETVARPLFLEFPKDSSTWTVDHQLLWGEALLITPVLQAGKAEVTGYFPLGTWYDLQ TVPIEALGSLPPPPAAPREPAIHSEGQWVTLPAPLDTINVHLRAGYIIPLQGPGLTTTES RQQPMALAVALTKGGEARGELFWDDGESLEVLERGAYTQVIFLARNNTIVNELVRVTSEG AGLQLQKVTVLGVATAPQQVLSNGVPVSNFTYSPDTKVLDICVSLLMGEQFLVSWC |
| 6 | QQGASRPGPRDAQAHPGRPRAVPTQCDVPPNSRFDCAPDKAITQEQCEARGCCYIPAKQGLQG AQMGQPWCFFPPSYPSYKLENLSSSEMGYTATLTRTTPTFFPKDILTLRLDVMMETENRLHFT IKDPANRRYEVPLETPHVHSRAPSPLYSVEFSEEPFGVIVRRQLDGRVLLNTTVAPLFFADQF LQLSTSLPSQYITGLAEHLSPLMLSTSWTRITLWNRDLAPTPGANLYGSHPFYLALEDGGSAH GVFLLNSNAMDVVLQPSPALSWRSTGGILDVYIFLGPEPKSVVQQYLDVVGYPFMPPYWGLGF HLCRWGYSSTAITRQVVENMTRAHFPLDVQWNDLDYMDSRRDFTFNKDGFRDFPAMVQELHQG GRRYMMIVDPAISSSGPAGSYRPYDEGLRRGVFITNETGQPLIGKVWPGSTAFPDFTNPTALA WWEDMVAEFHDQVPFDGMWIDMNEPSNFIRGSEDGCPNNELENPPYVPGVVGGTLQAATICAS SHQFLSTHYNLHNLYGLTEAIASHRALVKARGTRPFVISRSTFAGHGRYAGHWTGDVWSSWEQ LASSVPEILQFNLLGVPLVGADVCGFLGNTSEELCVRWTQLGAFYPFMRNHNSLLSLPQEPYS FSEPAQQAMRKALTLRYALLPHLYTLFHQAHVAGETVARPLFLEFPKDSSTWTVDHQLLWGEA LLITPVLQAGKAEVTGYFPLGTWYDLQTVPVEALGSLPPPPAAPREPAIHSEGQWVTLPAPLD TINVHLRAGYIIPLQGPGLTTTESRQQPMALAVALTKGGEARGELFWDDGESLEVLERGAYTQ VIFLARNNTIVNELVRVTSEGAGLQLQKVTVLGVATAPQQVLSNGVPVSNFTYSPDTKVLDIC VSLLMGEQFLVSWC |

In some embodiments, the rhGAA has a GAA amino acid sequence as set forth in SEQ ID NO: 1, as described in U.S. Patent No. 8,592,362 and has GenBank accession number AHE24104.1 (GI:568760974). In some embodiments, the rhGAA has a GAA amino acid sequence as encoded in SEQ ID NO: 2, the mRNA sequence having GenBank accession number Y00839.1. In some embodiments, the rhGAA has a GAA amino acid sequence as set forth in SEQ ID NO: 3. In some embodiments, the rhGAA has a GAA amino acid sequence as set forth in SEQ ID NO: 4, and has National Center for Biotechnology Information (NCBI) accession number NP_000143.2 or UniProtKB Accession Number P10253.

In some embodiments, the rhGAA is initially expressed as having the full-length 952 amino acid sequence of wild-type GAA as set forth in SEQ ID NO: 1 or SEQ ID NO: 4, and the rhGAA undergoes intracellular processing that removes a portion of the amino acids, e.g., the first 56 amino acids. Accordingly, the rhGAA that is secreted by the host cell can have a shorter amino acid sequence than the rhGAA that is initially expressed within the cell. In some embodiments, the shorter protein has the amino acid sequence set forth in SEQ ID NO: 5, which only differs from SEQ ID NO: 1 in that the first 56 amino acids of SEQ ID NO: 1 comprising the signal peptide and precursor peptide have been removed, thus resulting in a protein having 896 amino acids. In some embodiments, the shorter protein has the amino acid sequence set forth in SEQ ID NO: 6, which only differs from SEQ ID NO: 4 in that the first 56 amino acids of SEQ ID NO: 4 comprising the signal peptide and precursor peptide have been removed, thus resulting in a protein having 896 amino acids. Other variations in the number of amino acids are also possible, such as having 1, 2, 3, 4, 5, 6, 7, 8, 9, 10, 11, 12, 13, 14, 15, or more deletions, substitutions and/or insertions relative to the amino acid sequence described by SEQ ID NO: 1, SEQ ID NO: 4, SEQ ID NO: 5, or SEQ ID NO: 6. In some embodiments, the rhGAA product includes a mixture of recombinant human acid α-glucosidase molecules having different amino acid lengths.

In some embodiments, the rhGAA comprises an amino acid sequence that is at least 80%, 85%, 90%, 95%, 98% or 99% identical to SEQ ID NO: 4 or SEQ ID NO: 6. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 80%, 85%, 90%, 95%, 98% or 99% identity to specific polypeptides described herein and preferably exhibiting substantially the same functions, as well as polynucleotide encoding such polypeptides, are contemplated. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure. The polynucleotide sequences of similar polypeptides are deduced using the genetic code and may be obtained by conventional means, in particular by reverse translating its amino acid sequence using the genetic code.

In some embodiments, the rhGAA undergoes post-translational and/or chemical modifications at one or more amino acid residues in the protein. For example, methionine and tryptophan residues can undergo oxidation. As another example, the N-terminal glutamine in SEQ ID NO: 6 can be further modified to form pyro-glutamate. As another example, asparagine residues can undergo deamidation to aspartic acid. As yet another example, aspartic acid residues can undergo isomerization to iso-aspartic acid. As yet another example, unpaired cysteine residues in the protein can form disulfide bonds with free glutathione and/or cysteine. Accordingly, in some embodiments, the enzyme is initially expressed as having an amino acid sequence as set forth in SEQ ID NO: 1, SEQ ID NO: 3, SEQ ID NO: 4, or SEQ ID NO: 5, or an amino acid sequence encoded by SEQ ID NO: 2, and the enzyme undergoes one or more of these post-translational and/or chemical modifications. Such modifications are also within the scope of the present disclosure.

### N-LINKED GLYCOSYLATION OF rhGAA

There are seven potential N-linked glycosylation sites on a single rhGAA molecule. These potential glycosylation sites are at the following positions of SEQ ID NO: 6: N84, N177, N334, N414, N596, N826, and N869. Similarly, for the full-length amino acid sequence of SEQ ID NO: 4, these potential glycosylation sites are at the following positions: N140, N233, N390, N470, N652, N882, and N925. Other variants of rhGAA can have similar glycosylation sites, depending on the location of asparagine residues. Generally, sequences of Asn-X-Ser or Asn-X-Thr in the protein amino acid sequence indicate potential glycosylation sites, with the exception that X cannot be His or Pro.

The rhGAA molecules described herein may have, on average, 1, 2, 3, or 4 mannose-6-phosphate (M6P) groups on their N-glycans. For example, only one N-glycan on a rhGAA molecule may bear M6P (mono-phosphorylated or mono-M6P), a single N-glycan may bear two M6P groups (bis-phosphorylated or bis-M6P), or two different N-glycans on the same rhGAA molecule may each bear single M6P groups. In some embodiments, the rhGAA molecules described herein on average have 3-4 mol M6P groups on their N-glycans per mol rhGAA. Recombinant human acid α-glucosidase molecules may also have N-glycans bearing no M6P groups. In another embodiment, on average the rhGAA comprises greater than 2.5 mol M6P per mol rhGAA and greater than 4 mol sialic acid per mol rhGAA. In some embodiments, on average the rhGAA comprises about 3-3.5 mol M6P per mol rhGAA. In some embodiments, on average the rhGAA comprises about 4-5.4 mol sialic acid per mol rhGAA. On average at least about 3, 4, 5, 6, 7, 8, 9, 10%, or 20% of the total N-glycans on the rhGAA may be in the form of a mono-M6P N-glycan, and on average, at least about 0.5, 1, 1.5, 2.0, 2.5, 3.0, 4, 5, 6, 7, 8, 9, 10, 11, 12, 13, 14, 15, 16, 17, 18, 19 or 20% of the total N-glycans on the rhGAA are in the form of a bis-M6P N-glycan and on average less than 25% of total rhGAA contains no phosphorylated N-glycan binding to CIMPR. In some embodiments, on average about 10% to about 14% of the total N-glycans on the rhGAA are mono-phosphorylated. In some embodiments, on average about 7% to about 25% of the total N-glycans on the rhGAA are bis-phosphorylated. In some embodiments, on average the rhGAA comprises at least about 1.0, 1.1, 1.2 or1.3 mol bis-M6P per mol rhGAA.

The rhGAA described herein may have on average from 0.5 to 7.0 mol M6P per mol rhGAA or any intermediate value or subrange thereof including 0.5, 1.0, 1.5, 2.0, 2.5, 3.0, 3.5, 4.0, 4.5, 5.0, 5.5, 6.0, 6.5, or 7.0 mol M6P per mol rhGAA. The rhGAA can be fractionated to provide rhGAA preparations with different average numbers of mono-M6P-bearing or bis-M6P-bearing N-glycans, thus permitting further customization of rhGAA targeting to the lysosomes in target tissues by selecting a particular fraction or by selectively combining different fractions.

In some embodiments, up to 60% of the N-glycans on the rhGAA may be fully sialylated, for example, up to 10%, 20%, 30%, 40%, 50% or 60% of the N-glycans may be fully sialylated. In some embodiments, no more than 50% of the N-glycans on the rhGAA are fully sialylated. In some embodiments, from 4% to 20% of the total N-glycans are fully sialylated. In other embodiments, no more than 5%, 10%, 20% or 30% of N-glycans on the rhGAA carry sialic acid and a terminal galactose residue (Gal). This range includes all intermediate values and subranges, for example, 7% to 30% of the total N-glycans on the rhGAA can carry sialic acid and terminal galactose. In yet other embodiments, no more than 5%, 10%, 15%, 16%, 17%, 18%, 19%, or 20% of the N-glycans on the rhGAA have a terminal galactose only and do not contain sialic acid. This range includes all intermediate values and subranges, for example, from 8% to 19% of the total N-glycans on the rhGAA in the composition may have terminal galactose only and do not contain sialic acid.

In some embodiments, 30% to 60%, 35% to 60%, 40% to 60%, 45% to 60%, 50% to 60%, or 55% to 60% of the total N-glycans on the rhGAA are complex type N-glycans; or no more than 1%, 2%, 3%, 4%, 5%, 6,%, or 7% of the total N-glycans on the rhGAA are hybrid-type N-glycans; no more than 5%, 10%, 15%, 20%, 25%, or 30% of the total N-glycans on the rhGAA are high mannose-type N-glycans that are non-phosphorylated; at least 5%, 6%, 7%, 8%, 9%, 10%, 11%, 12%, 13%, 14%, or 15% of the total N-glycans on the rhGAA are mono-phosphorylated high mannose-type N-glycans; and/or at least 1%, 2%, 3%, 4%, 5%, 6%, 7%, 8%, 9%, 10%, 11%, 12%, 13%, 14%, 15%, or 20% of the total N-glycans on the rhGAA are bis-phosphorylated high mannose-type N-glycans. These values include all intermediate values and subranges. A rhGAA may meet one or more of the content ranges described above.

In some embodiments, the rhGAA may bear, on average, 2.0 to 8.0 moles of sialic acid residues per mole of rhGAA. This range includes all intermediate values and subranges thereof, including 2.0, 2.5, 3.0, 3.5, 4.0, 4.5, 5.0, 5.5, 6.0, 6.5, 7.0, 7.5, and 8.0 mol sialic acid residues per mol rhGAA. Without being bound by theory, it is believed that the presence of N-glycan units bearing sialic acid residues may prevent non-productive clearance of the rhGAA by asialoglycoprotein receptors.

In one or more embodiments, the rhGAA has a certain N-glycosylation profile at certain potential N-glycosylation sites. In some embodiments, the rhGAA has seven potential N-glycosylation sites. In some embodiments, at least 20% of the rhGAA is phosphorylated at the first potential N-glycosylation site (e.g., N84 for SEQ ID NO: 6 and N140 for SEQ ID NO: 4). For example, at least 20%, 25%, 30%, 35%, 40%, 45%, 50%, 55%, 60%, 65%, 70%, 75%, 80%, 85%, 90%, or 95% of the rhGAA can be phosphorylated at the first potential N-glycosylation site. This phosphorylation can be the result of mono-M6P and/or bis-M6P units. In some embodiments, at least 10%, 15%, 20%, 25%, 30%, 35%, 40%, 45%, 50%, 55%, 60%, 65%, 70%, 75%, 80%, 85%, 90%, or 95% of the rhGAA bears a mono-M6P unit at the first potential N-glycosylation site. In some embodiments, at least 10%, 15%, 20%, 25%, 30%, 35%, 40%, 45%, 50%, 55%, 60%, 65%, 70%, 75%, 80%, 85%, 90%, or 95% of the rhGAA bears a bis-M6P unit at the first potential N-glycosylation site. In some embodiments, the rhGAA comprises on average about 1.4 mol M6P (mono-M6P and bis-M6P) per mol rhGAA at the first potential N-glycosylation site. In some embodiments, the rhGAA comprises on average about at least 0.5 mol bis-M6P per mol rhGAA at the first potential N-glycosylation site. In some embodiments, the rhGAA comprises on average about 0.25 mol mono-M6P per mol rhGAA at the first potential N-glycosylation site. In some embodiments, the rhGAA comprises on average about 0.2 mol to about 0.3 mol sialic acid per mol rhGAA at the first potential N-glycosylation site. In at least one embodiment, the rhGAA comprises a first potential N-glycosylation site occupancy as depicted in Fig. 6A and an N-glycosylation profile as depicted in Fig. 6B. In at least one embodiment, the rhGAA comprises a first potential N-glycosylation site occupancy as depicted in Fig. 19A and an N-glycosylation profile as depicted in Fig. 19B or Fig. 20B.

In some embodiments, at least 20% of the rhGAA is phosphorylated at the second potential N-glycosylation site (e.g., N177 for SEQ ID NO: 6 and N223 for SEQ ID NO: 4). For example, at least 20%, 25%, 30%, 35%, 40%, 45%, 50%, 55%, 60%, 65%, 70%, 75%, 80%, 85%, 90%, or 95% of the rhGAA can be phosphorylated at the second N-glycosylation site. This phosphorylation can be the result of mono-M6P and/or bis-M6P units. In some embodiments, at least 10%, 15%, 20%, 25%, 30%, 35%, 40%, 45%, 50%, 55%, 60%, 65%, 70%, 75%, 80%, 85%, 90%, or 95% of the rhGAA bears a mono-M6P unit at the second N-glycosylation site. In some embodiments, at least 10%, 15%, 20%, 25%, 30%, 35%, 40%, 45%, 50%, 55%, 60%, 65%, 70%, 75%, 80%, 85%, 90%, or 95% of the rhGAA bears a bis-M6P unit at the second N-glycosylation site. In some embodiments, the rhGAA comprises on average about 0.5 mol M6P (mono-M6P and bis-M6P) per mol rhGAA at the second potential N-glycosylation site. In some embodiments, the rhGAA comprises on average about 0.4 to about 0.6 mol mono-M6P per mol rhGAA at the second potential N-glycosylation site. In at least one embodiment, the rhGAA comprises a second potential N-glycosylation site occupancy as depicted in Fig. 6A and an N-glycosylation profile as depicted in Fig. 6C. In at least one embodiment, the rhGAA comprises a second potential N-glycosylation site occupancy as depicted in Fig. 19A and an N-glycosylation profile as depicted in Fig. 19C or Fig. 20B.

In one or more embodiments, at least 5% of the rhGAA is phosphorylated at the third potential N-glycosylation site (e.g., N334 for SEQ ID NO: 6 and N390 for SEQ ID NO: 4). In other embodiments, less than 5%, 10%, 15%, 20%, or 25% of the rhGAA is phosphorylated at the third potential N-glycosylation site. For example, the third potential N-glycosylation site can have a mixture of non-phosphorylated high mannose N-glycans, di-, tri-, and tetra-antennary complex N-glycans, and hybrid N-glycans as the major species. In some embodiments, at least 3%, 5%, 8%, 10%, 15%, 20%, 25%, 30%, 35%, 40%, 45%, or 50% of the rhGAA is sialylated at the third potential N-glycosylation site. In some embodiments, the rhGAA comprises on average about 0.9 to about 1.2 mol sialic acid per mol rhGAA at the third potential N-glycosylation site. In at least one embodiment, the rhGAA comprises a third potential N-glycosylation site occupancy as depicted in Fig. 6A and an N-glycosylation profile as depicted in Fig. 6D. In at least one embodiment, the rhGAA comprises a third potential N-glycosylation site occupancy as depicted in Fig. 19A and an N-glycosylation profile as depicted in Fig. 19D or Fig. 20B.

In some embodiments, at least 20% of the rhGAA is phosphorylated at the fourth potential N-glycosylation site (e.g., N414 for SEQ ID NO: 6 and N470 for SEQ ID NO: 4). For example, at least 20%, 25%, 30%, 35%, 40%, 45%, 50%, 55%, 60%, 65%, 70%, 75%, 80%, 85%, 90%, or 95% of the rhGAA can be phosphorylated at the fourth potential N-glycosylation site. This phosphorylation can be the result of mono-M6P and/or bis-M6P units. In some embodiments, at least 10%, 15%, 20%, 25%, 30%, 35%, 40%, 45%, 50%, 55%, 60%, 65%, 70%, 75%, 80%, 85%, 90%, or 95% of the rhGAA bears a mono-M6P unit at the fourth potential N-glycosylation site. In some embodiments, at least 10%, 15%, 20%, 25%, 30%, 35%, 40%, 45%, 50%, 55%, 60%, 65%, 70%, 75%, 80%, 85%, 90%, or 95% of the rhGAA bears a bis-M6P unit at the fourth potential N-glycosylation site. In some embodiments, at least 3%, 5%, 8%, 10%, 15%, 20%, or 25% of the rhGAA is sialylated at the fourth potential N-glycosylation site. In some embodiments, the rhGAA comprises on average about 1.4 mol M6P (mono-M6P and bis-M6P) per mol rhGAA at the fourth potential N-glycosylation site. In some embodiments, the rhGAA comprises on average about 0.4 to about 0.6 mol bis-M6P per mol rhGAA at the fourth potential N-glycosylation site. In some embodiments, the rhGAA comprises on average about 0.3 to about 0.4 mol mono-M6P per mol rhGAA at the fourth potential N-glycosylation site. In at least one embodiment, the rhGAA comprises a fourth potential N-glycosylation site occupancy as depicted in Fig. 6A and an N-glycosylation profile as depicted in Fig. 6E. In at least one embodiment, the rhGAA comprises a fourth potential N-glycosylation site occupancy as depicted in Fig. 19A and an N-glycosylation profile as depicted in Fig. 19E or Fig. 20B.

In some embodiments, at least 5% of the rhGAA is phosphorylated at the fifth potential N-glycosylation site (e.g., N596 for SEQ ID NO: 6 and N692 for SEQ ID NO: 4). In other embodiments, less than 5%, 10%, 15%, 20%, or 25% of the rhGAA is phosphorylated at the fifth potential N-glycosylation site. For example, the fifth potential N-glycosylation site can have fucosylated di-antennary complex N-glycans as the major species. In some embodiments, at least 3%, 5%, 8%, 10%, 15%, 20%, 25%, 30%, 35%, 40%, 45%, 50%, 55%, 60%, 65%, 70%, 75%, 80%, 85%, 90%, or 95% of the rhGAA is sialylated at the fifth potential N-glycosylation site. In some embodiments, the rhGAA comprises on average about 0.8 to about 0.9 mol sialic acid per mol rhGAA at the fifth potential N-glycosylation site. In at least one embodiment, the rhGAA comprises a fifth potential N-glycosylation site occupancy as depicted in Fig. 6A and an N-glycosylation profile as depicted in Fig. 6F. In at least one embodiment, the rhGAA comprises a fifth potential N-glycosylation site occupancy as depicted in Fig. 19A and an N-glycosylation profile as depicted in Fig. 19F or Fig. 20B.

In some embodiments, at least 5% of the rhGAA is phosphorylated at the sixth N-glycosylation site (e.g., N826 for SEQ ID NO: 6 and N882 for SEQ ID NO: 4). In other embodiments, less than 5%, 10%, 15%, 20% or 25% of the rhGAA is phosphorylated at the sixth N-glycosylation site. For example, the sixth N-glycosylation site can have a mixture of di-, tri-, and tetra-antennary complex N-glycans as the major species. In some embodiments, at least 3%, 5%, 8%, 10%, 15%, 20%, 25%, 30%, 35%, 40%, 45%, 50%, 55%, 60%, 65%, 70%, 75%, 80%, 85%, 90% or 95% of the rhGAA is sialylated at the sixth N-glycosylation site. In some embodiments, the rhGAA comprises on average about 1.5 to about 4.2 mol sialic acid per mol rhGAA at the sixth potential N-glycosylation site. In some embodiments, the rhGAA comprises on average about 0.9 mol acetylated sialic acid per mol rhGAA at the sixth potential N-glycosylation site. In some embodiments, the rhGAA comprises an average of at least 0.05 mol glycan species with poly-N-Acetyl-D-lactosamine (poly-LacNAc) residues per mol rhGAA at the sixth potential N-glycosylation site. In some embodiments, over 10% of the rhGAA comprises a glycan bearing a poly-LacNAc residue at the sixth potential N-glycosylation site. In at least one embodiment, the rhGAA comprises a sixth potential N-glycosylation site occupancy as depicted in Fig. 6A and an N-glycosylation profile as depicted in Fig. 6G. In at least one embodiment, the rhGAA comprises a sixth potential N-glycosylation site occupancy as depicted in Fig. 19A and an N-glycosylation profile as depicted in Fig. 19G or Fig. 20B.

In some embodiments, at least 5% of the rhGAA is phosphorylated at the seventh potential N-glycosylation site (e.g., N869 for SEQ ID NO: 6 and N925 for SEQ ID NO: 4). In other embodiments, less than 5%, 10%, 15%, 20%, or 25% of the rhGAA is phosphorylated at the seventh potential N-glycosylation site. In some embodiments, less than 40%, 45%, 50%, 55%, 60%, or 65% of the rhGAA has any N-glycan at the seventh potential N-glycosylation site. In some embodiments, at least 30%, 35%, or 40% of the rhGAA has an N-glycan at the seventh potential N-glycosylation site. In some embodiments, the rhGAA comprises on average at least 0.5 mol sialic acid per mol rhGAA at the seventh potential N-glycosylation site. In some embodiments, the rhGAA comprises on average at least 0.8 mol sialic acid per mol rhGAA at the seventh potential N-glycosylation site. In some embodiments, the rhGAA comprises on average about 0.86 mol sialic acid per mol rhGAA at the seventh potential N-glycosylation site. In some embodiments, the rhGAA comprises an average of at least 0.3 mol glycan species bearing poly-LacNAc residues per mol rhGAA at the seventh potential N-glycosylation site. In some embodiments, nearly half of the rhGAA comprises a glycan bearing a poly-LacNAc residue at the seventh potential N-glycosylation site. In at least one embodiment, all N-glycans identified at the seventh potential N-glycosylation site are complex N-glycans. In at least one embodiment, the rhGAA comprises a seventh potential N-glycosylation site occupancy as depicted in Fig. 6A or as depicted in Fig. 19A and an N-glycosylation profile as depicted in Fig. 19H or Fig. 20B.

In some embodiments, the rhGAA comprises on average 3-4 mol M6P residues per mol rhGAA and about 4 to about 7.3 mol sialic acid per mol rhGAA. In some embodiments, the rhGAA further comprises on average at least about 0.5 mol bis-M6P per mol rhGAA at the first potential N-glycosylation site, about 0.4 to about 0.6 mol mono-M6P per mol rhGAA at the second potential N-glycosylation site, about 0.9 to about 1.2 mol sialic acid per mol rhGAA at the third potential N-glycosylation site, about 0.4 to about 0.6 mol bis-M6P per mol rhGAA at the fourth potential N-glycosylation site, about 0.3 to about 0.4 mol mono-M6P per mol rhGAA at the fourth potential N-glycosylation site, about 0.8 to about 0.9 mol sialic acid per mol rhGAA at the fifth potential N-glycosylation site, and about 1.5 to about 4.2 mol sialic acid per mol rhGAA at the sixth potential N-glycosylation site. In some embodiments, the rhGAA further comprises on average at least 0.5 mol sialic acid per mol rhGAA at the seventh potential N-glycosylation site. In some embodiments, the rhGAA comprises on average at least 0.8 mol sialic acid per mol rhGAA at the seventh potential N-glycosylation site. In at least one embodiment, the rhGAA further comprises on average about 0.86 mol sialic acid per mol rhGAA at the seventh potential N-glycosylation site. In at least one embodiment, the rhGAA comprises seven potential N-glycosylation sites with occupancy and N-glycosylation profiles as depicted in Figs. 6A-6H. In at least one embodiment, the rhGAA comprises seven potential N-glycosylation sites with occupancy and N-glycosylation profiles as depicted in Figs. 19A-19H and Figs. 20A-20B.

Methods of making rhGAA are disclosed in International Pat. App. No. PCT/2015/053252, U.S. Pat. No. 10,208,299, and U.S. Pat. No. 10,961,522, the entire disclosures of which are incorporated herein by reference.

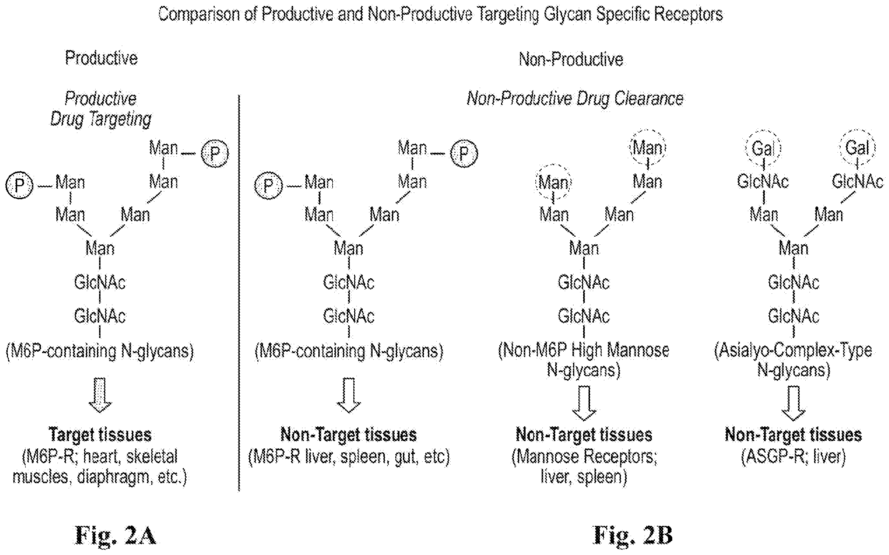

Once inside the lysosome, rhGAA can enzymatically degrade accumulated glycogen. However, conventional rhGAA products have low total levels of mono-M6P- and bis-M6P bearing N-glycans and, thus, target muscle cells poorly, resulting in inferior delivery of rhGAA to the lysosomes. The majority of rhGAA molecules in these conventional products do not have phosphorylated N-glycans, thereby lacking affinity for the CIMPR. Non-phosphorylated high mannose N-glycans can also be cleared by the mannose receptor, which results in non-productive clearance of the ERT (Fig. 2B). In contrast, as shown in Fig. 2A, a rhGAA described herein may contains a higher amount of mono-M6P- and bis-M6P bearing N-glycans, leading to productive uptake of rhGAA into specific tissues such as muscle.

### PRODUCTION AND PURIFICATION OF N-LINKED GLYCOSYLATED rhGAA

As described in International Pat. App. No. PCT/2015/053252, U.S. Pat. No. 10,208,299, and U.S. Pat. No. 10,961,522, the entireties of which are incorporated herein by reference, cells such as Chinese hamster ovary (CHO) cells may be used to produce the rhGAA described therein. Expressing high M6P rhGAA in CHO cells is advantageous over modifying the glycan profile of an rhGAA post-translationally at least in part because only the former may be converted by glycan degradation to a form of rhGAA with optimal glycogen hydrolysis, thus enhancing therapeutic efficacy.

**In** some embodiments, the rhGAA is preferably produced by one or more CHO cell lines that are transformed with a DNA construct encoding the rhGAA described herein. Such CHO cell lines may contain multiple copies of a gene, such as 5, 10, 15, or 20 or more copies, of a polynucleotide encoding GAA. DNA constructs, which express allelic variants of acid α-glucosidase or other variant acid α-glucosidase amino acid sequences such as those that are at least 90%, 95%, 98%, or 99% identical to SEQ ID NO: 4 or SEQ ID NO: 6, may be constructed and expressed in CHO cells. Those of skill in the art may select alternative vectors suitable for transforming CHO cells for production of such DNA constructs.

Methods for making such CHO cell lines are described in International Pat. App. No. PCT/2015/053252, U.S. Pat. No. 10,208,299, and U.S. Pat. No. 10,961,522, the entireties of which are incorporated herein by reference. Briefly, these methods involve transforming a CHO cell with DNA encoding GAA or a GAA variant, selecting a CHO cell that stably integrates the DNA encoding GAA into its chromosome(s) and that stably expresses GAA, and selecting a CHO cell that expresses GAA having a high content of N-glycans bearing mono-M6P or bis-M6P, and, optionally, selecting a CHO cell having N-glycans with high sialic acid content and/or having N-glycans with a low non-phosphorylated high-mannose content. The selected CHO cell lines may be used to produce rhGAA and rhGAA compositions by culturing the CHO cell line and recovering said composition from the culture of CHO cells. In some embodiments, a rhGAA produced from the selected CHO cell lines contains a high content of N-glycans bearing mono-M6P or bis-M6P that target the CIMPR. In some embodiments, a rhGAA produced as described herein has low levels of complex N-glycans with terminal galactose. In some embodiments, the selected CHO cell lines are referred to as GA-ATB200 or ATB200-X5-14. In some embodiments, the selected CHO cell lines encompass a subculture or derivative of such a CHO cell culture. In some embodiments, a rhGAA produced from the selected CHO cell lines is referred to as ATB200.

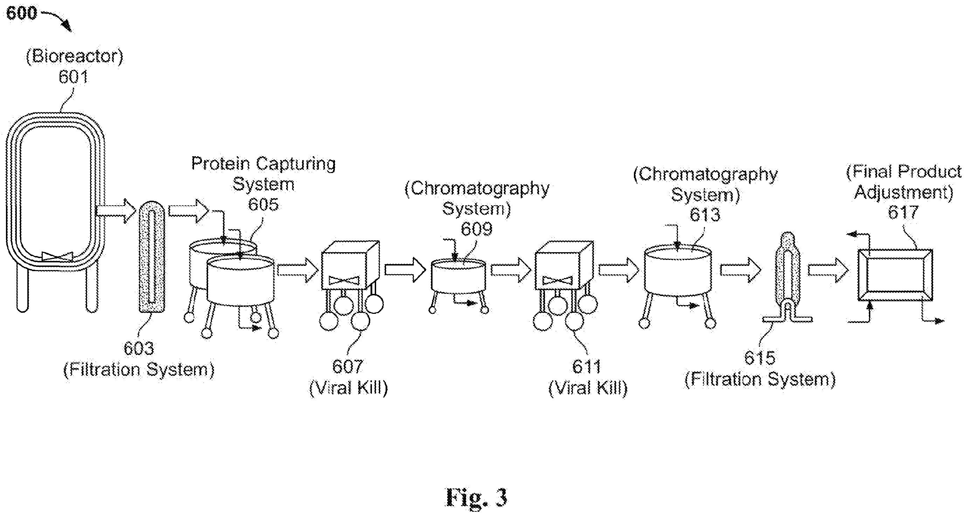

A rhGAA produced as described herein may be purified by following methods described in U.S. Pat. No. 10,227,577 and in U.S. Provisional Application No. 62/506,569, both of which are incorporated herein by reference in their entirety. An exemplary process for producing, capturing, and purifying a rhGAA produced from CHO cell lines is shown in Fig. 3.

Briefly, bioreactor 601 contains a culture of cells, such as CHO cells, that express and secrete rhGAA into the surrounding liquid culture media. The bioreactor 601 may be any appropriate bioreactor for culturing the cells, such as a perfusion, batch or fed-batch bioreactor. The culture media is removed from the bioreactor after a sufficient period of time for cells to produce rhGAA. Such media removal may be continuous for a perfusion bioreactor or may be batch-wise for a batch or fed-batch reactor. The media may be filtered by filtration system 603 to remove cells. Filtration system 603 may be any suitable filtration system, including an alternating tangential flow filtration (ATF) system, a tangential flow filtration (TFF) system, and/or centrifugal filtration system. In various embodiments, the filtration system utilizes a filter having a pore size between about 10 nanometers and about 2 micrometers.

After filtration, the filtrate is loaded onto a protein capturing system 605. The protein capturing system 605 may include one or more chromatography columns. If more than one chromatography column is used, then the columns may be placed in series so that the next column can begin loading once the first column is loaded. Alternatively, the media removal process can be stopped during the time that the columns are switched.

In various embodiments, the protein capturing system 605 includes one or more anion exchange (AEX) columns for the direct product capture of rhGAA, particularly rhGAA having a high M6P content. The rhGAA captured by the protein capturing system 605 is eluted from the column(s) by changing the pH and/or salt content in the column. Exemplary conditions for an AEX column are provided in Table 2.

**Table 2. Exemplary conditions for an AEX column**

| **Procedure** | **Buffer** | **Flow rate (cm/h)** | **Volume (CV)** | **Temperature (°C)** |
|---|---|---|---|---|
| Pre-used Sanitization | 0.1-10 M NaOH | ≤ 25-2500 | ≥ 1-3 (≥ 10-120 min) | 15 - 25 |
| Pre-Equilibration | 20-2000 mM phosphate buffer (PB), pH 6.9-7.3 | ≤ 25-2500 | ≥ 1-5 | 15 - 25 |
| Equilibration | 4-400 mM PB, pH 6.9-7.3 | ≤ 25-2500 | ≥ 1-5 | 2 - 15 |
| Load | NA | ≤ 10-1000 | NA | 2 - 15 |
| Wash1 | 4-400 mM PB, pH 6.9-7.3 | ≤ 25-2500 | ≥ 2-10 | 2 - 15 |
| Wash2 | 4-400 mM PB, pH 6.9-7.3 | ≤ 25-2500 | ≥ 2-10 | 15 - 25 |
| Elution | 4-400 mM PB, 20-2000 mM NaCl, pH 6.1-6.5 | ≤ 25-2500 | NA | 15 - 25 |
| Strip | 4-400 mM PB, 0.1-10 M NaCl, pH 6.1-6.5 | ≤ 25-2500 | ≥ 1-5 | 15 - 25 |
| Post-use Sanitization | 0.1-10 M NaOH | ≤ 25-2500 | ≥ 1-3 (≥ 10-120 min) | 15 - 25 |
| Storage | 0.01-1.0 M NaOH | ≤ 25-2500 | ≥ 1-5 | 15 - 25 |

The eluted rhGAA can be subjected to further purification steps and/or quality assurance steps. For example, the eluted rhGAA may be subjected to a virus kill step 607. Such a virus kill 607 may include one or more of a low pH kill, a detergent kill, or other technique known in the art. The rhGAA from the virus kill step 607 may be introduced into a second chromatography system 609 to further purify the rhGAA product. Alternatively, the eluted rhGAA from the protein capturing system 605 may be fed directly to the second chromatography system 609. In various embodiments, the second chromatography system 609 includes one or more immobilized metal affinity chromatography (IMAC) columns for further removal of impurities. Exemplary conditions for an IMAC column are provided in Table 3 below.

**Table 3. Exemplary conditions for an IMAC column**

| **Procedure** | **Buffer** | **Flow rate (cm/h)** | **Vol (CV)** |
|---|---|---|---|
| Rinse | 4-400 mM PB, pH 6.3-6.7 | ≤ 25-2500 | ≥ 1-5 |
| Pre-use Sanitization | 0.01-1.0 M NaOH | ≤ 25-2500 | ≥ 1-3 (10 - 30 min) |
| Equilibration | 4-400 mM PB, pH 6.5 | ≤ 25-2500 | ≥ 1-5 |
| Wash with WFI | Water For Injection (WFI) | ≤ 25-2500 | ≥ 1-3 |
| Chelating | 0.01-1.0 M Copper Acetate | ≤ 25-2500 | ≥ 1-5 |
| Wash with WFI | WFI | ≤ 25-2500 | ≥ 2-10 |
| Wash with acidic buffer | 2-200 mM Sodium Acetate, 0.05-5 M NaCl, pH 3.5-4.5 | ≤ 25-2500 | ≥ 2-10 |
| Equilibration | 4-400 mM PB, pH 6.3-6.7 | ≤ 25-2500 | ≥ 1-5 |
| Blank run with elution buffer | 4-400 mM PB, 15-1500 mM Glycine, pH 6.1-6.5 | ≤ 25-2500 | ≥ 2-20 |
| Equilibration | 4-400 mM PB, pH 6.3-6.7 | ≤ 25-2500 | ≥ 1-5 |
| Load | NA | ≤ 25-2500 | ≥ 1-5 |
| Washl | 4-400 mM PB, pH 6.3-6.7 | ≤ 25-2500 | ≥ 2-10 |
| Wash2 | 4-400 mM PB, 0.1-10 M NaCl, 5-30% propylene glycol, pH 6.3-6.7 | ≤ 25-2500 | ≥ 2-10 |
| Wash3 | 4-400 mM PB, pH 6.3-6.7 | ≤ 25-2500 | ≥ 2-10 |
| Elution | 4-400 mM PB, 15-1500 mM Glycine, pH 6.1-6.5 | ≤ 25-2500 | NA |
| Strip | 4-400 mM PB, 50-5000 mM imidazole, pH 6.3-6.7 | ≤ 25-2500 | ≥ 1-5 |
| Post-use Sanitization | 0.01-1M NaOH | ≤ 25-2500 | ≥ 1-3 (10 - 30 min) |
| Rinse | 4-400 mM PB, pH 6.3-6.7 | ≤ 25-2500 | ≥ 1-5 |
| Storage | 5-30% ethanol | ≤ 25-2500 | ≥ 1-5 |

After the rhGAA is loaded onto the second chromatography system 609, the recombinant protein is eluted from the column(s). The eluted rhGAA can be subjected to a virus kill step 611. As with virus kill 607, virus kill 611 may include one or more of a low pH kill, a detergent kill, or other technique known in the art. In some embodiments, only one of virus kill 607 or 611 is used, or the virus kills are performed at the same stage in the purification process.

The rhGAA from the virus kill step 611 may be introduced into a third chromatography system 613 to further purify the recombinant protein product. Alternatively, the eluted recombinant protein from the second chromatography system 609 may be fed directly to the third chromatography system 613. In various embodiments, the third chromatography system 613 includes one or more cation exchange chromatography (CEX) columns and/or size exclusion chromatography (SEC) columns for further removal of impurities. The rhGAA product is then eluted from the third chromatography system 613. Exemplary conditions for a CEX column are provided in Table 4 below.

**Table 4. Exemplary conditions for a CEX column**

| **Procedure** | **Buffer** | **Flow rate (cm/h)** | **Vol (CV)** |
|---|---|---|---|
| Pre-used Sanitization | 0.1-10 M NaOH | ≤ 25-2500 | ≥ 1-3 (≥ 10-120 min) |
| Equilibration | 2-200 mM Sodium citrate, pH 4.0-5.0 | ≤ 30-3000 | ≥ 2-10 |
| Load | NA | ≤ 30-3000 | NA |
| Wash | 2-200 mM Sodium citrate, pH 4.0-5.0 | ≤ 30-3000 | ≥ 2-10 |
| Elution | 2-200 mM Sodium citrate, 15-1500 mM NaCl, pH 4.0-5.0 | ≤ 30-3000 | ≥ 2-10 |
| Strip | 2-200 mM Sodium citrate, 0.1-10 M NaCl, pH 4.0-5.0 | ≤ 30-3000 | ≥ 1-5 |
| Post-use Sanitization | 0.1-10 M NaOH | ≤ 25-2500 | ≥ 1-3 (≥ 10-120 min) |
| Storage | 0.01-1.0 M NaOH | ≤ 30-3000 | ≥ 1-5 |

The rhGAA product may also be subjected to further processing. For example, another filtration system 615 may be used to remove viruses. In some embodiments, such filtration can utilize filters with pore sizes between 5 and 50 µm. Other product processing can include a product adjustment step 617, in which the recombinant protein product may be sterilized, filtered, concentrated, stored, and/or have additional components for added for the final product formulation.

### PHARMACEUTICAL COMPOSITIONS

In various embodiments, a pharmaceutical composition comprising the rhGAA described herein, either alone or in combination with other therapeutic agents, and/or a pharmaceutically acceptable carrier, is provided.

In one or more embodiments, a pharmaceutical composition described herein comprises a pharmaceutically acceptable salt.

In some embodiments, the pharmaceutically acceptable salt used herein is a pharmaceutically-acceptable acid addition salt. The pharmaceutically-acceptable acid addition salt may include, but is not limited to, hydrochloric acid, hydrobromic acid, sulfuric acid, sulfamic acid, nitric acid, phosphoric acid, and the like, and organic acids including but not limited to acetic acid, trifluoroacetic acid, adipic acid, ascorbic acid, aspartic acid, benzenesulfonic acid, benzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, hexanoic acid, formic acid, fumaric acid, 2-hydroxyethanesulfonic acid (isethionic acid), lactic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, pivalic acid, propionic acid, pyruvic acid, salicylic acid, stearic acid, succinic acid, sulfanilic acid, tartaric acid, p-toluenesulfonic acid, undecanoic acid, and the like.

In some embodiments, the pharmaceutically acceptable salt used herein is a pharmaceutically-acceptable base addition salt. The pharmaceutically-acceptable base addition salt may include, but is not limited to, ammonia or the hydroxide, carbonate, or bicarbonate of ammonium or a metal cation such as sodium, potassium, lithium, calcium, magnesium, iron, zinc, copper, manganese, aluminum, and the like. Salts derived from pharmaceutically-acceptable organic nontoxic bases include, but are not limited to, salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'- dibenzylethylenediamine, polyamine resins, and the like.

In some embodiments, the rhGAA or a pharmaceutically acceptable salt thereof may be formulated as a pharmaceutical composition adapted for intravenous administration. In some embodiments, the pharmaceutical composition is a solution in sterile isotonic aqueous buffer. Where necessary, the composition may also include a solubilizing agent and a local anesthetic to ease pain at the site of the injection. The ingredients of the pharmaceutical composition may be supplied either separately or mixed together in unit dosage form, for example, as a dry lyophilized powder or water free concentrate in a hermetically sealed container such as an ampule or sachet indicating the quantity of active agent. Where the composition is to be administered by infusion, it may be dispensed with an infusion bottle containing sterile pharmaceutical grade water, saline or dextrose/water. In some embodiments, the infusion may occur at a hospital or clinic. In some embodiments, the infusion may occur outside the hospital or clinic setting, for example, at a subject's residence. Where the composition is administered by injection, an ampule of sterile water for injection or saline may be provided so that the ingredients may be mixed prior to administration.

In some embodiments, the rhGAA or a pharmaceutically acceptable salt thereof is administered every 2 weeks as an IV infusion lasting about 4 hours. In some embodiments, the total volume of infusion is determined by the patient's body weight. Infusion rate can be lowered and infusion duration increased if patient experiences an IAR.

In some embodiments, the initial infusion rate is 1 mg/kg/hour. In some embodiments, the infusion rate is gradually increased by 2 mg/kg/hour every 30 minutes if there are no signs of infusion-associated reactions (IARs) until a maximum rate of 7 mg/kg/hour is reached; then, the infusion rate is maintained at 7 mg/kg/hour until the infusion is complete. In some embodiments, the approximate total infusion duration is 4 hours.

Infusions should be administered in a step-wise manner using an infusion pump. Infusion rates can be increased from initial rate every 30 minutes +/- 5 minutes until the maximum rate is reached as shown in Table 5 below based on patient weight.

**Table 5: Recommended Infusion Volumes and Rates for 20 mg/kg Dose**

| **Subject Weight Range (kg)** | **Total Infusion Volume (mL)** | **Step 1 1 mg/kg/hr (mL/hr)** | **Step 2 3 mg/kg/hr (mL/hr)** | **Step 3 5 mg/kg/hr (mL/hr)** | **Step 4 7 mg/kg/hr (mL/hr)** |
|---|---|---|---|---|---|
| 40 - 50 | 250 | 13 | 38 | 63 | 88 |
| 50.1 - 60 | 300 | 15 | 45 | 75 | 105 |
| 60.1 - 100 | 500 | 25 | 75 | 125 | 175 |
| 100.1 - 120 | 600 | 30 | 90 | 150 | 210 |
| 120.1 - 140 | 700 | 35 | 105 | 175 | 245 |
| 140.1 - 160 | 800 | 40 | 120 | 200 | 280 |
| 160.1 - 180 | 900 | 45 | 135 | 225 | 315 |
| 180.1 -200 | 1,000 | 50 | 150 | 250 | 350 |

The most serious tolerability issue with the rhGAA or a pharmaceutically acceptable salt thereof is the occurrence of infusion-associated reactions (IARs), which, in some instances can include life-threatening anaphylaxis or other severe allergic responses. In some embodiments, prior to administration of the rhGAA or a pharmaceutically acceptable salt thereof, pretreatments with antihistamines, antipyretics, and/or corticosteroids are administered. If pretreatment was used with previous enzyme replacement therapy (ERT), prior to administration of the rhGAA or a pharmaceutically acceptable salt thereof, pretreatments with antihistamines, antipyretics, and/or corticosteroids are administered.

In some embodiments, the rhGAA or a pharmaceutically acceptable salt thereof may be formulated for oral administration. Orally administrable compositions may be formulated in a form of tablets, capsules, ovules, elixirs, solutions or suspensions, gels, syrups, mouth washes, or a dry powder for reconstitution with water or other suitable vehicle before use, optionally with flavoring and coloring agents for immediate-, delayed-, modified-, sustained-, pulsed-, or controlled-release applications. Solid compositions such as tablets, capsules, lozenges, pastilles, pills, boluses, powder, pastes, granules, bullets, dragees, or premix preparations can also be used. Solid and liquid compositions for oral use may be prepared according to methods well known in the art. Such compositions can also contain one or more pharmaceutically acceptable carriers and excipients which can be in solid or liquid form. Tablets or capsules can be prepared by conventional means with pharmaceutically acceptable excipients, including but not limited to binding agents, fillers, lubricants, disintegrants, or wetting agents. Suitable pharmaceutically acceptable excipients are known in the art and include but are not limited to pregelatinized starch, polyvinylpyrrolidone, povidone, hydroxypropyl methylcellulose (HPMC), hydroxypropyl ethylcellulose (HPEC), hydroxypropyl cellulose (HPC), sucrose, gelatin, acacia, lactose, microcrystalline cellulose, calcium hydrogen phosphate, magnesium stearate, stearic acid, glyceryl behenate, talc, silica, corn, potato or tapioca starch, sodium starch glycolate, sodium lauryl sulfate, sodium citrate, calcium carbonate, dibasic calcium phosphate, glycine croscarmellose sodium, and complex silicates. Tablets can be coated by methods well known in the art.

In some embodiments, a pharmaceutical composition described herein may be formulated according to U.S. Pat. No. 10,512,676 and U.S. Provisional Application No. 62/506,574, both incorporated herein by reference in their entirety. For instance, in some embodiments, the pH of a pharmaceutical composition described herein is from about 5.0 to about 7.0 or about 5.0 to about 6.0. In some embodiments, the pH ranges from about 5.5 to about 6.0. In some embodiments, the pH of the pharmaceutical composition is 6.0. In some embodiments, the pH may be adjusted to a target pH by using pH adjusters (e.g., alkalizing agents and acidifying agents) such as sodium hydroxide and/or hydrochloric acid.

The pharmaceutical composition described herein may comprise a buffer system such as a citrate system, a phosphate system, and/or a combination thereof. The citrate and/or phosphate may be a sodium citrate or sodium phosphate. Other salts include potassium and ammonium salts. In one or more embodiments, the buffer comprises a citrate. In further embodiments, the buffer comprises sodium citrate (e.g., a mixture of sodium citrate dehydrate and citric acid monohydrate). In one or more embodiments, buffer solutions comprising a citrate may comprise sodium citrate and citric acid. In some embodiments, both a citrate and phosphate buffer are present.

In some embodiments, a pharmaceutical composition described herein comprises at least one excipient. The excipient may function as a tonicity agent, bulking agent, and/or stabilizer. Tonicity agents are components which help to ensure the formulation has an osmotic pressure similar to or the same as human blood. Bulking agents are ingredients which add mass to the formulations (e.g., lyophilized) and provide an adequate structure to the cake. Stabilizers are compounds that can prevent or minimize the aggregate formation at the hydrophobic air-water interfacial surfaces. One excipient may function as a tonicity agent and bulking agent at the same time. For instance, mannitol may function as a tonicity agent and also provide benefits as a bulking agent.

Examples of tonicity agents include sodium chloride, mannitol, sucrose, and trehalose. In some embodiments, the tonicity agent comprises mannitol. In some embodiments, the total amount of tonicity agent(s) ranges in an amount of from about 10 mg/mL to about 50 mg/mL. In further embodiments, the total amount of tonicity agent(s) ranges in an amount of from about 10, 11, 12, 13, 14, or 15 mg/mL to about 16, 20, 25, 30, 35, 40, 45, or 50 mg/mL.

In some embodiments, the excipient comprises a stabilizer. In some embodiments, the stabilizer is a surfactant. In some embodiments, the stabilizer is polysorbate 80. In one or more embodiments, the total amount of stabilizer ranges from about 0.1 mg/mL to about 1.0 mg/mL. In further embodiments, the total amount of stabilizer ranges from about 0.1, 0.2, 0.3, 0.4, or 0.5 mg/mL to about 0.5, 0.6, 0.7, 0.8, 0.9, or 1.0 mg/mL. In yet further embodiments, the total amount of stabilizer is about 0.1, 0.2, 0.3, 0.4, 0.5, 0.6, 0.7, 0.8, 0.9, or 1.0 mg/mL.

In some embodiments, the pharmaceutical composition comprises one or more of the following excipients: sodium citrate dihydrate, citric acid monohydrate, mannitol or polysorbate-80.

In some embodiments, a pharmaceutical composition comprises (a) a rhGAA (e.g., ATB200 or cipaglucosidase alfa), (b) at least one buffer selected from the group consisting of a citrate, a phosphate, and a combination thereof, and (c) at least one excipient selected from the group consisting of mannitol, polysorbate 80, and a combination thereof, and has a pH of (i) from about 5.0 to about 6.0, or (ii) from about 5.0 to about 7.0. In some embodiments, the composition further comprises water. In some embodiments, the composition may further comprise an acidifying agent and/or alkalizing agent.

In some embodiments, the pharmaceutical composition comprises (a) a rhGAA (e.g., ATB200 or cipaglucosidase alfa) at a concentration of about 5-50 mg/mL, about 5-30 mg/mL, or about 15 mg/mL, (b) sodium citrate buffer at a concentration of about 10-100 mM or about 25 mM, (c) mannitol at a concentration of about 10-50 mg/mL, or about 20 mg/mL, (d) polysorbate 80, present at a concentration of about 0.1-1 mg/mL, about 0.2-0.5 mg/mL, or about 0.5 mg/mL, annaïvee) water, and has a pH of about 6.0. In at least one embodiment, the pharmaceutical composition comprises (a) 15 mg/mL rhGAA (e.g., ATB200 or cipaglucosidase alfa) (b) 25 mM sodium citrate buffer, (c) 20 mg/mL mannitol (d) 0.5 mg/mL polysorbate 80,naïved (e) water, and has a pH of about 6.0. In some embodiments, the composition may further comprise an acidifying agent and/or alkalizing agent.

In some embodiments, the pharmaceutical composition comprising rhGAA (e.g., ATB200 or cipaglucosidase alfa) is diluted prior to administration to a subject in need thereof.

In some embodiments, the pharmaceutical composition described herein may undergo lyophilization (freeze-drying) process to provide a cake or powder. Accordingly, in some embodiments, the pharmaceutical composition described herein pertains to a rhGAA composition after lyophilization. The lyophilized mixture may comprise the rhGAA described herein (e.g., ATB200 or cipaglucosidase alfa), buffer selected from the group consisting of a citrate, a phosphate, and combinations thereof, and at least one excipient selected from the group consisting of trehalose, mannitol, polysorbate 80, and a combination thereof. In some embodiments, other ingredients (e.g., other excipients) may be added to the lyophilized mixture. The pharmaceutical composition comprising the lyophilized formulation may be provided in vial, which then can be stored, transported, reconstituted and/or administered to a patient.

In some embodiments, the pharmaceutical composition comprising a rhGAA (e.g., ATB200 or cipaglucosidase alfa) as described herein is a lyophilized powder in glass vials. In some embodiments, each vial may contain about 105 mg of lyophilized rhGAA (e.g., ATB200 or cipaglucosidase alfa). The powder may be reconstituted in sterile water and then followed by dilution with 0.9% sodium chloride prior to administration by IV infusion. In some embodiments, after reconstitution, the concentrate obtained contains 15 mg of rhGAA (e.g., ATB200 or cipaglucosidase alfa) per mL. In some embodiments, after reconstitution with 7.2 mL of diluent, the vial contains a usable volume of 7.0 mL of concentrate containing 15 mg/mL of rhGAA (e.g., ATB200 or cipaglucosidase alfa). In some embodiments, the diluent is sterile water and/or 0.9% sodium chloride. In some embodiments, each vial may include an overfill to make up for fluid loss during preparation. In some embodiments, the instant disclosure provides a vial (e.g., a glass vial) containing 105 mg lyophilized rhGAA (e.g., ATB200 or cipaglucosidase alfa) composition comprising rhGAA (e.g., ATB200 or cipaglucosidase alfa), sodium citrate dihydrate, citric acid monohydrate, mannitol, polysorbate 80, wherein the amount/concentration of each ingredient may be selected from those described herein.

In some embodiments, the number of vials to be reconstituted are based on patient's body weight and the fact that each vial contains 105 mg of rhGAA (e.g., ATB200 or cipaglucosidase alfa). Accordingly, in some embodiments, patient dose (mg) = Subject weight (kg) x dose (mg/kg); and Number of vials required = patient dose (in mg)/105 (mg per vial). In some embodiments, if the number of vials includes a fraction, round up to the next whole number.

For example, in a 65 kg patient dosed at 20 mg/kg: Patient dose (mg) = 65 kg X 20 mg/kg = 1300 mg total dose; Number of vials required = 1300/105 mg per vial = 12.381 rounded up to 13 vials for the purpose of dispensation. The patient dose is 20 mg/ kg, therefore the full volume of the first 12 vials will be extracted bu^{t} the 13^{th} vial will have 2.7 ml extracted and added to the infusion bag.

In some embodiments, the method of reconstitution comprises or consists essentially of the follow processes: (1) reconstitute each vial by slowly injecting 7.2 mL of Sterile Water for Injection, to the inside wall of each vial and not directly onto the lyophilized cake; (2) roll each vial gently, do not invert, swirl, or shake; (3) dilute an amount of reconstituted rhGAA based on the patient's body weight in 0.9% Sodium Chloride for Injection, immediately after reconstitution to the total infusion volume for 20 mg/kg dose based on patient weight; (4) prior to adding the reconstituted rhGAA, remove air and total amount equal to reconstituted volume 0.9% Sodium Chloride for Injection bag; (5) slowly withdraw the reconstituted solution from each vial avoiding foaming in the syringe; (6) Slowly add the reconstituted cipaglucosidase alfa solution directly into the 0.9% Sodium Chloride for Injection bag (do not add directly into the airspace that may remain within the infusion bag) avoiding foaming in the infusion bag (Alternatively, product dilution can be accomplished using an appropriately sized empty infusion bag to add the required volume of 0.9% Sodium Chloride for Injection and the reconstituted rhGAA based on patient weight.. It is recommended to use pre-filled 0.9% Sodium Chloride for Injection bag); (7) gently invert or massage the infusion bag to mix; and (8) cover the infusion bag and tubing to protect the medication from light. After reconstitution, each vial will yield a concentration of 15mg/mL. The total extractable dose per vial is 105 mg per 7 mL.

In some embodiments, only the exact amount of reconstituted rhGAA based on the patient's body weight is diluted. In some embodiments, for partial withdrawal of the reconstituted vial, amounts are rounded up or down to 1 decimal place. In some embodiments, the infusion bag is not shaken to mix or a pneumatic tube used to transport the infusion bag. In some embodiments, the reconstituted and diluted solutions may contain particles in the form of thin white strands or translucent fibers after initial preparation and increase over time.

The present disclosure also provides a pharmaceutical composition comprising an enzyme stabilizer. In some embodiments, the enzyme stabilizer is miglustat or a pharmaceutically acceptable salt thereof. In another embodiment, the enzyme stabilizer is duvoglustat or a pharmaceutically acceptable salt thereof.

In some embodiments, a rhGAA described herein is formulated in one pharmaceutical composition, while an enzyme stabilizer such as miglustat is formulated in another pharmaceutical composition. In some embodiments, the pharmaceutical composition comprising miglustat is based on a formulation available commercially as ZAVESCA^{®} (Actelion Pharmaceuticals). In some embodiments, the pharmaceutical composition comprising miglustat comprises microcrystalline cellulose, pregelatinized starch, Emprove^{®} sucralose powder, magnesium stearate, and/or colloidal silicon dioxide. In some embodiments, the pharmaceutical composition is a hard gelatin capsule for oral administration, comprising about 65 mg of miglustat, microcrystalline cellulose, pregelatinized starch, Emprove^{®} sucralose powder, magnesium stearate, and/or colloidal silicon dioxide.

In some embodiments, a pharmaceutical composition comprising miglustat comprises 20%-40% by weight miglustat, such as 30-35% by weight miglustat. In some embodiments, a pharmaceutical composition comprising miglustat further comprises 40%-60% by weight microcrystalline cellulose, such as 45-55% by weight microcrystalline cellulose. In some embodiments, a pharmaceutical composition comprising miglustat further comprises 5%-25% by weight pregelatinized starch, such as 10-20% by weight pregelatinized starch. In some embodiments, a pharmaceutical composition comprising miglustat further comprises 0.1%-5% by weight sucralose, such as 0.2-1% by weight sucralose. In some embodiments, a pharmaceutical composition comprising miglustat further comprises 0.1%-5% by weight magnesium stearate, such as 0.2-1% by weight magnesium stearate. In some embodiments, a pharmaceutical composition comprising miglustat further comprises 0.1%-5% by weight colloidal silicon dioxide, such as 0.2-1% by weight colloidal silicon dioxide. In some embodiments, a pharmaceutical composition comprising miglustat is provided in a hard gelatin capsule for oral administration. In some embodiments, the pharmaceutical composition is a hard gelatin capsule for oral administration, comprising about 20%-40% (e.g., 30-35%) by weight of miglustat, 40%-60% (e.g., 45-55%) by weight of microcrystalline cellulose, 5%-25% (e.g., 10-20%) by weight of pregelatinized starch, 0.1%-5% (e.g., 0.2%-1%) by weight of sucralose, 0.1%-5% (e.g., 0.2%-1%) by weight of magnesium stearate, and/or 0.1%-5% (e.g., 0.2%-1%) by weight of colloidal silicon dioxide. In some embodiments, a pharmaceutical composition comprising miglustat is provided in an oral liquid dosage form, such as an oral solution, dispersion, or suspension. In some embodiments, the pharmaceutical composition is an oral liquid dosage form, comprising about 20%-40% (e.g., 30-35%) by weight of miglustat, 40%-60% (e.g., 45-55%) by weight of microcrystalline cellulose, 5%-25% (e.g., 10-20%) by weight of pregelatinized starch, 0.1%-5% (e.g., 0.2%-1%) by weight of sucralose, 0.1%-5% (e.g., 0.2%-1%) by weight of magnesium stearate, and/or 0.1%-5% (e.g., 0.2%-1%) by weight of colloidal silicon dioxide.

In some embodiments, a pharmaceutical composition comprising miglustat comprises about 50 to about 100 mg miglustat, such as about 65 mg miglustat. In some embodiments, a pharmaceutical composition comprising miglustat comprises about 50 to about 150 mg microcrystalline cellulose, such as about 75 to about 125 mg microcrystalline cellulose. In some embodiments, a pharmaceutical composition comprising miglustat comprises about 20 to about 50 mg pregelatinized starch, such as about 30 to about 40 mg pregelatinized starch. In some embodiments, a pharmaceutical composition comprising miglustat comprises about 0.1 to about 5 mg sucralose, such as about 0.5 to about 2 mg sucralose. In some embodiments, a pharmaceutical composition comprising miglustat comprises about 0.1 to about 5 mg magnesium stearate, such as about 0.5 to about 2 mg magnesium stearate. In some embodiments, a pharmaceutical composition comprising miglustat comprises about 0.1 to about 5 mg colloidal silicon dioxide, such as about 0.2 mg to about 1 mg colloidal silicon dioxide. In some embodiments, a pharmaceutical composition comprising miglustat is provided in a hard gelatin capsule for oral administration. In some embodiments, the pharmaceutical composition is a hard gelatin capsule for oral administration, comprising about 50 to about 100 mg (e.g., 65 mg) of miglustat, about 50 to about 150 mg (e.g., 75 mg to about 125 mg) of microcrystalline cellulose, about 20 to about 50 mg (e.g., 30 mg to about 40 mg) of pregelatinized starch, about 0.1 to about 5 mg (e.g., 0.5 mg to about 2 mg) of sucralose, about 0.1 to about 5 mg (e.g., 0.5 mg to about 2 mg) of magnesium stearate, and/or about 0.1 to about 5 mg (e.g., 0.5 mg to about 2 mg) of colloidal silicon dioxide. In some embodiments, a pharmaceutical composition comprising miglustat is provided in an oral liquid dosage form, such as an oral solution, dispersion or suspension. In some embodiments, the pharmaceutical composition is an oral liquid dosage form, comprising about 50 to about 100 mg (e.g., 65 mg) of miglustat, about 50 to about 150 mg (e.g., 75 mg to about 125 mg) of microcrystalline cellulose, about 20 to about 50 mg (e.g., 30 mg to about 40 mg) of pregelatinized starch, about 0.1 to about 5 mg (e.g., 0.5 mg to about 2 mg) of sucralose, about 0.1 to about 5 mg (e.g., 0.5 mg to about 2 mg) of magnesium stearate, and/or about 0.1 to about 5 mg (e.g., 0.5 mg to about 2 mg) of colloidal silicon dioxide.

In some embodiments, a pharmaceutical composition comprising miglustat is provided in a hard gelatin capsule for oral administration comprising about 65 mg miglustat, about 100 mg microcrystalline cellulose, about 32.6 mg pregelatinized starch, about 1 mg sucralose powder, about 1 mg magnesium stearate, and about 0.4 mg colloidal silicon dioxide.

In some embodiments, a pharmaceutical composition comprising miglustat is provided in an oral solution, dispersion or suspension, comprising about 65 mg miglustat, about 100 mg microcrystalline cellulose, about 32.6 mg pregelatinized starch, about 1 mg sucralose powder, about 1 mg magnesium stearate, and about 0.4 mg colloidal silicon dioxide.

In some embodiments, a pharmaceutical composition comprising miglustat is provided in an oral solution dispersion or suspension, comprising about 130 mg miglustat, about 200 mg microcrystalline cellulose, about 65.2 mg pregelatinized starch, about 2 mg sucralose powder, about 2 mg magnesium stearate, and about 0.8 mg colloidal silicon dioxide.

In some embodiments, a pharmaceutical composition comprising miglustat is provided in an oral solution, dispersion or suspension, comprising about 195 mg miglustat, about 300 mg microcrystalline cellulose, about 97.8 mg pregelatinized starch, about 3 mg sucralose powder, about 3 mg magnesium stearate, and about 1.2 mg colloidal silicon dioxide.

In some embodiments, a pharmaceutical composition comprising miglustat is provided in an oral solution, dispersion or suspension, comprising about 260 mg miglustat, about 400 mg microcrystalline cellulose, about 130.4 mg pregelatinized starch, about 4 mg sucralose powder, about 4 mg magnesium stearate, and about 1.6 mg colloidal silicon dioxide.

### METHODS OF TREATMENT

### A. Treatment of Diseases

Another aspect of the disclosure pertains to a method of treatment of a disease or disorder related to glycogen storage dysregulation by administering the rhGAA or pharmaceutical composition described herein. In some embodiments, the disease is Pompe disease (also known as acid maltase deficiency (AMD) and glycogen storage disease type II (GSD II)). In some embodiments, the rhGAA is ATB200 or cipaglucosidase alfa. In some embodiments, the pharmaceutical composition comprises rhGAA (e.g., ATB200 or cipaglucosidase alfa). Also provided herein are uses of rhGAA (e.g., ATB200 or cipaglucosidase alfa) to treat Pompe disease.

In some embodiments, the subject treated by the methods disclosed herein is an ERT-experienced patient. For instance, the subject treated by the methods disclosed herein is an adult patient 18 years of age or older with a confirmed diagnosis of late onset Pompe disease (acid α-glucosidase (GAA) deficiency), who have previously received enzyme replacement therapy (ERT). In some embodiments, the ERT-experienced patient is currently receiving an approved ERT (e.g., MYOZYME^{®} or LUMIZYME^{®}). In some embodiments, the ERT-experienced patient is declining on their current treatment. In some embodiments, the methods disclosed herein are begun approximately 2 weeks after the last ERT dose. In some embodiments, the subject treated by the methods disclosed herein is an ERT-naive patient.

In some embodiments, the patient may be expected to be seen again every 3 months to ensure the clinical benefit provided to the patient and thus continue the treatment. This visit frequency may be required until the medicinal product is commercially available. In some embodiments, a patient's response to treatment is regularly assessed based on an assessment of the main clinical and laboratory parameters of the disease. In some embodiments, an rhGAA as described herein (such as cipaglucosidase alfa) and miglustat are administered every two weeks. In some embodiments, a dosage of rhGAA such as cipaglucosidase alfa is about 20 mg/kg of body weight given as a 4-hour infusion. In some embodiments, if the infusion is delayed, it should not be started more than 3 hours after oral administration of miglustat. In some embodiments, for patients weighing 50 kg or more, four 65 mg capsules (260 mg total).

In some of the above embodiments, for patients weighing between 40 and 50 kg, a dosage of three 65 mg capsules (195 mg total) is administered. In some of the above embodiments, rhGAA (such as cipaglucosidase alfa) is administered via infusion every two weeks.

The rhGAA or pharmaceutical composition described herein is administered by an appropriate route. In one embodiment, the rhGAA or pharmaceutical composition is administered intravenously. In some embodiments, the rhGAA or pharmaceutical composition is administered intravenously using an infusion pump. In some embodiments, when administered intravenously, the infusion bag and tubing are covered to protect from light. In other embodiments, the rhGAA or pharmaceutical composition is administered by direct administration to a target tissue, such as to heart or skeletal muscle (e.g., intramuscular), or nervous system (e.g., direct injection into the brain; intraventricularly; intrathecally). In some embodiments, the rhGAA or pharmaceutical composition is administered orally. More than one route can be used concurrently, if desired.

In some embodiments, the therapeutic effects of the rhGAA or pharmaceutical composition described herein may be assessed based on one or more of the following criteria: (1) cardiac status (e.g., increase of end-diastolic and/or end-systolic volumes, or reduction, amelioration or prevention of the progressive cardiomyopathy that is typically found in GSD-II), (2) pulmonary function (e.g., increase in crying vital capacity over baseline capacity, and/or normalization of oxygen desaturation during crying), (3) neurodevelopment and/or motor skills/function (e.g., increase in AIMS score), (4) reduction of glycogen levels in tissue of the individual affected by the disease, (5) muscle strength; and/or (6) quality of life.

In some embodiments, the therapeutic effects of the rhGAA or pharmaceutical composition described herein may be assessed across measures of motor function, muscle strength, pulmonary function, patient reported outcomes (PROs) and biomarkers. In some embodiments, the therapeutic effects of the rhGAA or pharmaceutical composition described herein may be measured by the 6-minute walk test (6MWT), % predicted 6MWD, 10-meter walk test (10MWT), GSGC, 4 stair climb, Gowers', chair test, and Timed Up and Go (TUG). Treatment with the rhGAA or pharmaceutical composition described herein may also result in improved pulmonary function tests (PFTs) as measured by FVC *(e.g.,* sitting, supine), slow vital capacity (SVC), maximum inspiratory pressure (MIP), maximum expiratory pressure (MEP), and sniff nasal inspiratory pressure (SNIP), as well as improvements in muscle strength in all tested body parts of both ambulatory and non-ambulatory subjects, as measured by MMTs (*e*.*g*., lower MMT, upper MMT, overall MMT) and quantitative muscle testing (QMT).

In some embodiments, the cardiac status of a subject is improved by 10%, 20%, 30%, 40%, or 50% (or any percentage in-between) after administration of one or more dosages of the rhGAA or pharmaceutical composition described herein, as compared to that of a subject treated with a vehicle or that of a subject prior to treatment. The cardiac status of a subject may be assessed by measuring end-diastolic and/or end-systolic volumes and/or by clinically evaluating cardiomyopathy. In some embodiments, the pulmonary function of a subject is improved by 10%, 20%, 30%, 40%, or 50% (or any percentage in-between) after administration of one or more dosages of rhGAA (e.g., ATB200 or cipaglucosidase alfa) or pharmaceutical composition comprising rhGAA (e.g., ATB200 or cipaglucosidase alfa), as compared to that of a subject treated with a vehicle or that of a subject prior to treatment. In certain embodiments, the improvement is achieved after 1 week, 2 weeks, 3 weeks, 1 month, 2 months, or more from administration (or any time period in between). In certain embodiments, rhGAA (e.g., ATB200 or cipaglucosidase alfa) or pharmaceutical composition comprising rhGAA e.g., ATB200 or cipaglucosidase alfa) improves and/or stabilizes the pulmonary function of a subject after 1 week, 2 weeks, 3 weeks, 1 month, 2 months, or more from administration (or any time period in between).

In some embodiments, the pulmonary function of a subject is improved by 10%, 20%, 30%, 40%, or 50% (or any percentage in-between) after administration of one or more dosages of the rhGAA or pharmaceutical composition described herein, as compared to that of a subject treated with a vehicle or that of a subject prior to treatment. The pulmonary function of a subject may be assessed by crying vital capacity over baseline capacity, and/or normalization of oxygen desaturation during crying. In some embodiments, the pulmonary function of a subject is improved by 10%, 20%, 30%, 40%, or 50% (or any percentage in-between) after administration of one or more dosages of rhGAA (e.g., ATB200 or cipaglucosidase alfa) or pharmaceutical composition comprising rhGAA (e.g., ATB200 or cipaglucosidase alfa), as compared to that of a subject treated with a vehicle or that of a subject prior to treatment. In certain embodiments, the improvement is achieved after 1 week, 2 weeks, 3 weeks, 1 month, 2 months, or more from administration (or any time period in between). In certain embodiments, rhGAA (e.g., ATB200 or cipaglucosidase alfa) or pharmaceutical composition comprising rhGAA (e.g., ATB200 or cipaglucosidase alfa) improves the pulmonary function of a subject after 1 week, 2 weeks, 3 weeks, 1 month, 2 months, or more from administration (or any time period in between).

In some embodiments, the neurodevelopment and/or motor skills of a subject is improved by 10%, 20%, 30%, 40%, or 50% (or any percentage in-between) after administration of one or more dosages of the rhGAA (e.g., ATB200 or cipaglucosidase alfa) or pharmaceutical composition rhGAA (e.g., ATB200 or cipaglucosidase alfa) described herein, as compared to that of a subject treated with a vehicle or that of a subject prior to treatment. The neurodevelopment and/or motor skills of a subject may be assessed by determining an AIMS score. The AIMS is a 12-item anchored scale that is clinician-administered and scored (*see* Rush JA Jr., Handbook of Psychiatric Measures, American Psychiatric Association, 2000, 166-168). Items 1-10 are rated on a 5-point anchored scale. Items 1-4 assess orofacial movements. Items 5-7 deal with extremity and truncal dyskinesia. Items 8-10 deal with global severity as judged by the examiner, and the patient's awareness of the movements and the distress associated with them. Items 11-12 are yes/no questions concerning problems with teeth and/or dentures (such problems can lead to a mistaken diagnosis of dyskinesia). In some embodiments, the neurodevelopment and/or motor skills of a subject is improved by 10%, 20%, 30%, 40%, or 50% (or any percentage in-between) after administration of one or more dosages of rhGAA (e.g., ATB200 or cipaglucosidase alfa) or pharmaceutical composition comprising rhGAA (e.g., ATB200 or cipaglucosidase alfa), as compared to that of a subject treated with a vehicle or that of a subject prior to treatment. In certain embodiments, the improvement is achieved after 1 week, 2 weeks, 3 weeks, 1 month, 2 months, or more from administration (or any time period in between). In certain embodiments, rhGAA (e.g., ATB200 or cipaglucosidase alfa) or pharmaceutical composition comprising rhGAA (e.g., ATB200 or cipaglucosidase alfa) improves and/or stabilizes the neurodevelopment and/or motor skills of a subject after 1 week, 2 weeks, 3 weeks, 1 month, 2 months, or more from administration (or any time period in between).

In some embodiments, the glycogen level of a certain tissue of a subject is reduced by 10%, 20%, 30%, 40%, or 50% (or any percentage in-between) after administration of one or more dosages of the rhGAA or pharmaceutical composition described herein, as compared to that of a subject treated with a vehicle or that of a subject prior to treatment. In some embodiment, the tissue is muscle such as quadriceps, triceps, and gastrocnemius. The glycogen level of a tissue can be analyzed using methods known in the art. The determination of glycogen levels is well known based on amyloglucosidase digestion, and is described in publications such as: Amalfitano et al. (1999), Proc Natl Acad Sci USA, 96:8861-8866. In some embodiments, the glycogen level in muscle of a subject is reduced by 10%, 20%, 30%, 40%, or 50% (or any percentage in between) after administration of one or more dosages of rhGAA (e.g., ATB200 or cipaglucosidase alfa) or pharmaceutical composition comprising rhGAA (e.g., ATB200 or cipaglucosidase alfa), as compared to that of a subject treated with a vehicle or that of a subject prior to treatment. In certain embodiments, the reduction is achieved after 1 week, 2 weeks, 3 weeks, 1 month, 2 months, or more from administration (or any time period in between). In certain embodiments, rhGAA (e.g., ATB200 or cipaglucosidase alfa) or pharmaceutical composition comprising rhGAA (e.g., ATB200 or cipaglucosidase alfa) reduces the glycogen level in muscle of a subject after 1 week, 2 weeks, 3 weeks, 1 month, 2 months, or more from administration (or any time period in between).

In some embodiments, the treatment effects of the pharmaceutical compositions of the present application, such as those set forth herein are durable and maintained through 12, 24, 36, 48 or >48 months of treatment. In some embodiments, the treatment effects of the pharmaceutical compositions of the present application, including such as, improvements in motor function (e.g., as measured by 6MWD, GSGC, 10 m walk, 4 stair climb, Gowers', Chair test, TUG), pulmonary or respiratory function or improved or stabilized PFTs (e.g., as measured by % predicted FVC (sitting and supine), SVC, MIP, MEP, and SNIP), biomarker levels (e.g., serum CK and urine Hex4), stable or improved muscle strength in all tested body parts (e.g., as measured by MMT (lower, upper, overall), QMT), and/or patient-reported outcomes (PROs, including the PROMIS-Physical Function Short Form [SF] 20a and PROMIS-Fatigue SF 8a) are sustained up to or through 24, 36, or 48 months of treatment.

In some embodiments, patients treated with a pharmaceutical composition of the present application achieve greater (e.g., greater clinically meaningful) improvements in motor function (e.g., as measured by 6MWD, GSGC, 10 m walk, 4 stair climb, Gowers', Chair test, TUG), pulmonary or respiratory function or improved or stabilized PFTs (e.g., as measured by % predicted FVC (sitting and supine), SVC, MIP, MEP, and SNIP), biomarker levels (e.g., serum CK and urine Hex4), stable or improved muscle strength in all tested body parts (e.g., as measured by MMT (lower, upper, overall), QMT), and/or patient-reported outcomes (PROs, including the PROMIS-Physical Function Short Form [SF] 20a and PROMIS-Fatigue SF 8a), than patients treated with a conventional rhGAA product (e.g., MYOZYME^{®}, LUMIZYME^{®} or NEXVIAZYME^{®}). In some embodiments, patients treated with a pharmaceutical composition of the present application show greater (e.g., greater clinically meaningful) improvements in motor function (e.g., as measured by 6MWD), pulmonary or respiratory function (e.g., as measured by FVC), and/or muscle strength (e.g., as measured by MMT) than patients treated with a conventional rhGAA product (e.g., MYOZYME^{®}, LUMIZYME^{®} or NEXVIAZYME^{®}). In some embodiments, such greater improvements are sustained up to or though 12, 24, 36, 48, or >48 months of treatment.

In some embodiments, ERT-experienced switch patients treated with a pharmaceutical composition of the present application, such as those patients who are switched from a conventional rhGAA product (e.g., MYOZYME^{®}, LUMIZYME^{®} or NEXVIAZYME^{®}) to a pharmaceutical composition of the present application, show greater (e.g., greater clinically meaningful) improvements in motor function (e.g., as measured by 6MWD, GSGC, 10 m walk, 4 stair climb, Gowers', Chair test, TUG), pulmonary or respiratory function or improved or stabilized PFTs (e.g., as measured by % predicted FVC (sitting and supine), SVC, MIP, MEP, and SNIP), biomarker levels (e.g., serum CK and urine Hex4), stable or improved muscle strength in all tested body parts (e.g., as measured by MMT (lower, upper, overall), QMT), and/or patient-reported outcomes (PROs, including the PROMIS-Physical Function Short Form [SF] 20a and PROMIS-Fatigue SF 8a), when compared to patients who remain on the conventional rhGAA product. In some embodiments, patients under a conventional rhGAA product (e.g., MYOZYME^{®}, LUMIZYME^{®} or NEXVIAZYME^{®}), who are no longer improving or worsening with such treatment achieve improvements in motor function (e.g., as measured by 6MWD, GSGC, 10 m walk, 4 stair climb, Gowers', Chair test, TUG), pulmonary or respiratory function or improved or stabilized PFTs (e.g., as measured by % predicted FVC (sitting and supine), SVC, MIP, MEP, and SNIP), biomarker levels (e.g., serum CK and urine Hex4), stable or improved muscle strength in all tested body parts (e.g., as measured by MMT (lower, upper, overall), QMT), and/or patient-reported outcomes (PROs, including the PROMIS-Physical Function Short Form [SF] 20a and PROMIS-Fatigue SF 8a), after switching to a pharmaceutical composition of the present application. In some embodiments, such improvements are sustained up to or through 12, 24, 36, 48, or >48 months.

### B. Biomarkers

Biomarkers of glycogen accumulation in a subject, such as urine hexose tetrasaccharide (Hex4), may be used to assess and compare the therapeutic effects of enzyme replacement therapy in a subject with Pompe disease. In some embodiments, the therapeutic effect of the rhGAA or a pharmaceutical composition comprising rhGAA on glycogen accumulation is assessed by measuring the levels of urinary Hex4 in a subject.

Biomarkers of muscle injury or damage such as creatine kinase (CK), lactate dehydrogenase (LDH), alanine aminotransferase (ALT), and aspartate aminotransferase (AST) may be used to assess and compare the therapeutic effects of enzyme replacement therapy in a subject with Pompe disease. In some embodiments, the therapeutic effect of the rhGAA or a pharmaceutical composition comprising rhGAA on muscle damage is assessed by measuring the levels of CK, LDH, ALT, and/or AST in a subject. In at least one embodiment, the therapeutic effect of the rhGAA or a pharmaceutical composition comprising rhGAA on muscle damage is assessed by measuring the levels of CK in a subject.

Biomarkers such as LAMP-1, LC3, and Dysferlin may also be used to assess and compare the therapeutic effects of the rhGAA or pharmaceutical composition described herein. In Pompe disease, the failure of GAA to hydrolyze lysosomal glycogen leads to the abnormal accumulation of large lysosomes filled with glycogen in some tissues. (Raben et al., JBC 273: 19086-19092, 1998.) Studies in a mouse model of Pompe disease have shown that the enlarged lysosomes in skeletal muscle cannot adequately account for the reduction in mechanical performance, and that the presence of large inclusions containing degraded myofibrils (i.e., autophagic buildup) contributes to the impairment of muscle function. (Raben et al., Human Mol Genet 17: 3897-3908, 2008.) Reports also suggest that impaired autophagy flux is associated with poor therapeutic outcome in Pompe patients. (Nascimbeni et al., Neuropathology and Applied Neurobiology doi: 10.1111/nan.12214, 2015; Fukuda et al., Mol Ther 14: 831-839, 2006.) In addition, late-onset Pompe disease is prevalent in unclassified limb-girdle muscular dystrophies (LGMDs) (Preisler et al., Mol Genet Metab 110: 287-289, 2013), which is a group of genetically heterogeneous neuromuscular diseases with more than 30 genetically defined subtypes of varying severity. IHC examination revealed substantially elevated sarcoplasmic presence of dysferlin in the skeletal muscle fibers of *Gaa* KO mice.

Various known methods can be used to measure the gene expression level and/or protein level of such biomarkers. For instance, a sample from a subject treated with the rhGAA or pharmaceutical composition described herein can be obtained, such as biopsy of tissues, in particular muscle. In some embodiments, the sample is a biopsy of muscle in a subject. In some embodiments, the muscle is selected from quadriceps, triceps, and gastrocnemius. The sample obtained from a subject may be stained with one or more antibodies or other detection agents that detect such biomarkers or be identified and quantified by mass spectrometry. The samples may also or alternatively be processed for detecting the presence of nucleic acids, such as mRNAs, encoding the biomarkers via, e.g., RT-qPCR methods.

In some embodiments, the gene expression level and/or protein level of one or more biomarkers is measured in a muscle biopsy obtained from an individual prior to and post treatment with the rhGAA or pharmaceutical composition described herein. In some embodiments, the gene expression level and/or protein level of one or more biomarkers is measured in a muscle biopsy obtained from an individual treated with a vehicle. In some embodiments, the gene expression level and/or protein level of one or more biomarkers is reduced by 10%, 20%, 30%, 40%, or 50% (or any percentage in-between) after administration of one or more dosages of the rhGAA or pharmaceutical composition described herein, as compared to that of a subject treated with a vehicle or that of a subject prior to treatment. In some embodiments, the gene expression level and/or protein level of one or more biomarkers is reduced by 10%, 20%, 30%, 40%, or 50% (or any percentage in-between) after administration of one or more dosages of rhGAA (e.g., ATB200 or cipaglucosidase alfa) or pharmaceutical composition comprising rhGAA (e.g., ATB200 or cipaglucosidase alfa), as compared to that of a subject treated with a vehicle or that of a subject prior to treatment. In certain embodiments, the reduction is achieved after 1 week, 2 weeks, 3 weeks, 1 month, 2 months, or more from administration (or any time period in between). In certain embodiments, rhGAA (e.g., ATB200 or cipaglucosidase alfa) or pharmaceutical composition comprising rhGAA (e.g., ATB200 or cipaglucosidase alfa) reduces the gene expression level and/or protein level of one or more biomarkers after 1 week, 2 weeks, 3 weeks, 1 month, 2 months, or more from administration (or any time period in between).

### C. Dosages of rhGAA

The pharmaceutical formulation or reconstituted composition is administered in a therapeutically effective amount (e.g., a dosage amount that, when administered at regular intervals, is sufficient to treat the disease, such as by ameliorating symptoms associated with the disease, delaying the onset of the disease, and/or lessening the severity or frequency of symptoms of the disease). The amount which is therapeutically effective in the treatment of the disease may depend on the nature and extent o' the disease's effects, and can be determined by standard clinical techniques. In addition, *in vitro* or *in vivo* assays may optionally be employed to help identify optimal dosage ranges. In at least one embodiment, a rhGAA described herein or pharmaceutical composition comprising the rhGAA is administered at a dose of about 1 mg/kg to about 100 mg/kg, such as about 5 mg/kg to about 30 mg/kg, typically about 5 mg/kg to about 20 mg/kg. In at least one embodiment, the rhGAA or pharmaceutical composition described herein is administered at a dose of about 5 mg/kg, about 10 mg/kg, about 15 mg/kg, about 20 mg/kg, about 25 mg/kg, about 30 mg/kg, about 35 mg/kg, about 40 mg/kg, about 50 mg/kg, about 50 mg/kg, about 60 mg/kg, about 70 mg/kg, about 80 mg/kg, about 90 mg/kg, or about 100 mg/kg. In some embodiments, the rhGAA is administered at a dose of 5 mg/kg, 10 mg/kg, 20 mg/kg, 50 mg/kg, 75 mg/kg, or 100 mg/kg. In at least one embodiment, the rhGAA or pharmaceutical composition is administered at a dose of about 20 mg/kg. In some embodiments, the rhGAA or pharmaceutical composition is administered concurrently or sequentially with an enzyme stabilizer. In some embodiments, the enzyme stabilizer is miglustat. In at least one embodiment, the miglustat is administered as an oral dose of about 260 mg. In at least one embodiment, the miglustat is administered as an oral dose of about 195 mg. The effective dose for a particular individual can be varied (e.g., increased or decreased) over time, depending on the needs of the individual. For example, in times of physical illness or stress, or if anti-acid α-glucosidase antibodies become present or increase, or if disease symptoms worsen, the amount of rhGAA and/or miglustat can be adjusted.

In some embodiments, the therapeutically effective dose of the rhGAA or pharmaceutical composition described herein is lower than that of conventional rhGAA products. For instance, if the therapeutically effective dose of a conventional rhGAA product is 20 mg/kg, the dose of the rhGAA or pharmaceutical composition described herein required to produce the same as or better therapeutic effects than the conventional rhGAA product may be lower than 20 mg/kg. Therapeutic effects may be assessed based on one or more criteria discussed above (e.g., cardiac status, glycogen level, or biomarker expression). In some embodiments, the therapeutically effective dose of the rhGAA or pharmaceutical composition described herein is at least about 5%, 10%, 15%, 20%, 30%, 40%, 50%, 60%, 70%, 80%, 90%, or more lower than that of conventional rhGAA products.

In some embodiments, the therapeutic effect of the rhGAA or pharmaceutical composition described herein comprises an improvement and/or stabilization in motor function, an improvement and/or stabilization in muscle strength (upper-body, lower-body, or total-body), an improvement and/or stabilization in pulmonary function, decreased fatigue, reduced levels of at least one biomarker of muscle injury, reduced levels of at least one biomarker of glycogen accumulation, or a combination thereof. In some embodiments, the therapeutic effect of the rhGAA or pharmaceutical composition described herein comprises a reversal of lysosomal pathology in a muscle fiber, a faster and/or more effective reduction in glycogen content in a muscle fiber, an increase in six-minute walk test distance, a decrease in timed up and go test time, a decrease in four-stair climb test time, a decrease in ten-meter walk test time, a decrease in gait-stair-gower-chair score, an increase in upper extremity strength, an improvement and/or stabilization in shoulder adduction, an improvement and/or stabilization in shoulder abduction, an improvement and/or stabilization in elbow flexion, an improvement and/or stabilization in elbow extension, an improvement and/or stabilization in upper body strength, an improvement and/or stabilization in lower body strength, an improvement and/or stabilization in total body strength, an improvement in upright (sitting) forced vital capacity, an improvement and/or stabilization in maximum expiratory pressure, an improvement and/or stabilization in maximum inspiratory pressure, a decrease in fatigue severity scale score, a reduction in urine hexose tetrasaccharide levels, a reduction in creatine kinase levels, a reduction in alanine aminotransferase levels, a reduction in asparate aminotransferase levels, or any combination thereof.

In some embodiments, the rhGAA or pharmaceutical composition described herein achieves desired therapeutic effects faster than conventional rhGAA products when administered at the same dose. Therapeutic effects may be assessed based on one or more criteria discussed above (e.g., cardiac status, glycogen level, or biomarker expression). For instance, if a single dose of a conventional rhGAA product decreases glycogen levels in tissue of a treated individual by 10% in a week, the same degree of reduction may be achieved in less than a week when the same dose of the rhGAA or pharmaceutical composition described herein is administered. In some embodiments, when administered at the same dose, the rhGAA or pharmaceutical composition described herein may achieve desired therapeutic effects at least about 1.25, 1.5, 1.75, 2.0, 3.0, or more faster than conventional rhGAA products.

In some embodiments, the therapeutically effective amount of rhGAA (or composition or medicament comprising rhGAA) is administered more than once. In some embodiments, the rhGAA or pharmaceutical composition described herein is administered at regular intervals, depending on the nature and extent o' the disease's effects, and on an ongoing basis. Administration at a "regular interval," as used herein, indicates that the therapeutically effective amount is administered periodically (as distinguished from a one-time dose). The interval can be determined by standard clinical techniques. In certain embodiments, rhGAA is administered bimonthly, monthly, bi-weekly, weekly, twice weekly, or daily. In some embodiments, the rhGAA is administered intravenously twice weekly, weekly, or every other week. The administration interval for a single individual need not be a fixed interval, but can be varied over time, depending on the needs of the individual. For example, in times of physical illness or stress, if anti-rhGAA antibodies become present or increase, or if disease symptoms worsen, the interval between doses can be decreased.

In some embodiments, when used at the same dose, the rhGAA or pharmaceutical composition as described herein may be administered less frequently than conventional rhGAA products and yet capable of producing the same as or better therapeutic effects than conventional rhGAA products. For instance, if a conventional rhGAA product is administered at 20 mg/kg weekly, the rhGAA or pharmaceutical composition as described herein may produce the same as or better therapeutic effects than the conventional rhGAA product when administered at 20 mg/kg, even though the rhGAA or pharmaceutical composition is administered less frequently, e.g., biweekly or monthly. Therapeutic effects may be assessed based on one or more criterion discussed above (e.g., cardiac status, glycogen level, or biomarker expression). In some embodiments, an interval between two doses of the rhGAA or pharmaceutical composition described herein is longer than that of conventional rhGAA products. In some embodiments, the interval between two doses of the rhGAA or pharmaceutical composition is at least about 1.25, 1.5, 1.75, 2.0, 3.0, or more longer than that of conventional rhGAA products.

In some embodiments, under the same treatment condition (e.g., the same dose administered at the same interval), the rhGAA or pharmaceutical composition described herein provides therapeutic effects at a degree superior than that provided by conventional rhGAA products. Therapeutic effects may be assessed based on one or more criteria discussed above (e.g., cardiac status, glycogen level, or biomarker expression). For instance, when compared to a conventional rhGAA product administered at 20 mg/kg weekly, the rhGAA or pharmaceutical composition administered at 20 mg/kg weekly may reduce glycogen levels in tissue of a treated individual at a higher degree. In some embodiments, when administered under the same treatment condition, the rhGAA or pharmaceutical composition described herein provides therapeutic effects that are at least about 1.25, 1.5, 1.75, 2.0, 3.0, or more greater than those of conventional rhGAA products.

### D. Two-Component Therapy

In one or more embodiments, the rhGAA or pharmaceutical composition comprising the rhGAA described herein is administered concurrently or sequentially with an enzyme stabilizer. In some embodiments, the rhGAA or pharmaceutical composition is administered via a different route as compared to the enzyme stabilizer. For instance, an enzyme stabilizer may be administered orally while the rhGAA or pharmaceutical composition is administered intravenously.

In various embodiments, the enzyme stabilizer is miglustat. Without wishing to be bound by any theory, it is believed that when co-administered, miglustat stabilizes rhGAA (e.g., ATB200 or cipaglucosidase alfa) from denaturation in systemic circulation, which enhances the delivery of the active component rhGAA (e.g., ATB200 or cipaglucosidase alfa) to lysosomes.

**In** some embodiments, the miglustat is administered at an oral dose of about 50 mg to about 600 mg. In at least one embodiment, the miglustat is administered at an oral dose of about 200 mg to about 600 mg, or at an oral dose of about 200 mg, about 250 mg, about 300 mg, about 350 mg, about 400 mg, about 450 mg, about 500 mg, about 550 mg, or about 600 mg. In at least one embodiment, the miglustat is administered at an oral dose of about 233 mg to about 500 mg. In at least one embodiment, the miglustat is administered at an oral dose of about 250 to about 270 mg, or at an oral dose of about 250 mg, about 255 mg, about 260 mg, about 265 mg or about 270 mg. In at least one embodiment, the miglustat is administered as an oral dose of about 260 mg.

It will be understood by those skilled in the art that an oral dose of miglustat in the range of about 200 mg to 600 mg or any smaller range therewith can be suitable for an adult patient depending on his/her body weight. For instance, for patients having a significantly lower body weight than about 70 kg, including but not limited to infants, children, or underweight adults, a smaller dose may be considered suitable by a physician. Therefore, in at least one embodiment, the miglustat is administered as an oral dose of from about 50 mg to about 200 mg, or as an oral dose of about 50 mg, about 75 mg, about 100 mg, about 125 mg, about 130 mg, about 150 mg, about 175 mg, about 195 mg, about 200 mg, or about 260 mg. In at least one embodiment, the miglustat is administered as an oral dose of from about 65 mg to about 195 mg, as an oral dose of from about 65 mg to about 260 mg , as an oral dose of from about 195 mg to about 260 mg, or as an oral dose of about 65 mg, about 130 mg, about 195 mg, or about 260 mg.

In some embodiments, the starting dose of rhGAA (e.g., ATB200 or cipaglucosidase alfa) and miglustat may be based on weight (see, for example, **Table** ). In some embodiments, the miglustat dose may be adjusted and the weight increase or decrease may be confirmed at 2 consecutive infusion visits before the miglustat dose is changed.

**Table 6: Exemplary rhGAA and Miglustat Dose Regimen**

| **Age Group** | **Weight Group (kg)** | **Starting Dose for rhGAA (e.g., ATB200 or cipaglucosidase alfa) (mg/kg)** | **Miglustat Dose (mg)** |
|---|---|---|---|
| **Adults** | 40 to <50 | 20 | 195^{a} |
| | ≥ 50 | 20 | 260^{b} |

| | | | |
|---|---|---|---|
| ^{a} Dose administered as three 65 mg capsules or as a 195 mg oral liquid dosage form ^{b} Dose administered as four 65 mg capsules or as a 260 mg oral liquid dosage form | | | |

Miglustat exhibited linear pharmacokinetics with plasma area under the concentration-time curve (AUC) and maximum concentration (Cₘₐₓ) increased approximately proportional with increasing doses from 130 mg (0.5-fold of the recommended dose of 260 mg in patients weighing ≥ 50 kg) to 260 mg. At the recommended 260 mg dose, the mean Cₘₐₓ was approximately 3 mcg/mL and the mean AUC was approximately 25 mcg*hr/mL. The mean time to reach the maximum concentration ranged from 2 hours to 3 hours.

Plasma concentrations of miglustat increased in patients with renal impairment. Accordingly, the apparent clearance of miglustat decreased with decreasing renal function. The available data estimated that the AUC₀₋₂₄ₕᵣ of miglustat increased by 21%, 26%, and 31% in patients with mild (creatinine clearance based on the Cockcroft-Gault equation, CLcr 60-89 mL/minute), moderate (CLcr 30-59 mL/minute), and severe (CLcr 15-29 mL/minute) renal impairment, respectively, compared to patients with normal renal function. In some embodiments, the dosage of miglustat in patients with moderate or severe renal impairment is reduced as compared to patients with normal renal function. Exemplary doses for patients with moderate or severe renal impairment are provided in Table 7 below:

**Table 7: Recommend Miglustat Dosage in Patients with Moderate or Severe Renal Impairment**

| | **Moderate Renal Impairment¹ (CLcr 30-59 mL/minute)** | **Severe Renal Impairment¹ (CLer 15-30 mL/minute)** |
|---|---|---|
| Patients weighing ≥ 50 kg | 195 mg | 195 mg |
| Patients weighing ≥ 40 kg to <50 kg | 130 mg | 130 mg |

| | | |
|---|---|---|
| ¹ Renal function classified by creatinine clearance based on the Cockcroft-Gault equation. | | |

In some embodiments, for patients with mild renal impairment (creatinine clearance based on the Cockcroft-Gault equation, CLcr 60-89 mL/minute), the recommended miglustat dosage is the same as for patients with normal renal function.

In one or more embodiments, the rhGAA dose is not adjusted for patients with renal impairment.

Available pharmacodynamic/toxicological data in animals have shown excretion of cipaglucosidase alfa in milk. It is not known whether miglustat is secreted in breast milk. A risk to newborns/breastfed infants cannot be ruled out. The developmental and health benefits of breastfeeding should be considered, as well as the clinical need for the mother to receive co-administration therapy with cipaglucosidase alfa and miglustat, and any adverse reactions experienced by the breastfed child potentially related to the co-administration of cipaglucosidase alfa and miglustat or the underlying maternal condition. In some embodiments, breastfeeding is not allowed while taking cipaglucosidase alfa and miglustat.

There are no clinical data on the effects of co-administration therapy of cipaglucosidase alfa and miglustat on fertility. Preclinical data did not reveal any significant adverse reactions with cipaglucosidase alfa.

No effect on sperm concentration, motility, or morphology was observed in 7 healthy adult men who received 100 mg of miglustat orally twice daily for 6 weeks. Preclinical data in rats have shown that miglustat negatively affects sperm characteristics (motility and morphology), thereby reducing fertility.

In some embodiments, the rhGAA is administered intravenously at a dose of about 5 mg/kg to about 20 mg/kg and the miglustat is administered orally at a dose of about 50 mg to about 600 mg. In some embodiments, the rhGAA is administered intravenously at a dose of about 5 mg/kg to about 20 mg/kg and the miglustat is administered orally at a dose of about 50 mg to about 200 mg. In some embodiments, the rhGAA is administered intravenously at a dose of about 5 mg/kg to about 20 70iglustat the miglustat is administered orally at a dose of about 200 mg to about 600 mg. In some embodiments, the rhGAA is administered intravenously at a dose of about 5 mg/kg to about 20 mg/kg and the miglustat is administered orally at a dose of about 200 mg to about 500 mg. In one embodiment, the rhGAA is administered intravenously at a dose of about 20 mg/kg and the miglustat is administered orally at a dose of about 260 mg. In some embodiments, the rhGAA is administered intravenously at a dose of about 5 mg/kg to about 20 mg/kg and the miglustat is administered orally at a dose of about 130 mg to about 200 mg. In one embodiment, the rhGAA is administered intravenously at a dose of about 20 mg/kg and the miglustat is administered orally at a dose of about 195 mg.

The area under the plasma concentration-time curve (AUC) and maximum plasma concentration (Cmax) of cipaglucosidase alfa following administration with 20 mg/kg of cipaglucosidase alfa in combination with a single oral dose of 260 mg of miglustat in adult patients with LOPD are summarized in Table 8 below:

**Table 8. Mean (CV%) Pharmacokinetic Parameters of Cipaglucosidase Alfa in ERT-Experienced Adult Patients with LOPD**

| | **Cipaglucosidase Alfa 20 mg/kg in combination with Miglustat 260 mg** | **Cipaglucosidase Alfa 20 mg/kg without Miglustat** |
|---|---|---|
| **Cₘₐₓ (mcg/mL)** | 345 (18.5) | 325 (13.5) |
| **AUC_{0-inf}(mcg*h/mL)** | 1812 (20.8) | 1410 (15.9) |

In some embodiments, the miglustat and the rhGAA are administered concurrently. For instance, the miglustat may administered within 10, 9, 8, 7, 6, 5, 4, 3, 2, or 1 minute(s) before or after administration of the rhGAA. In some embodiments, the miglustat is administered within 5, 4, 3, 2, or 1 minute(s) before or after administration of the rhGAA.

In some embodiments, the miglustat and the rhGAA are administered sequentially. In at least one embodiment, the miglustat is administered prior to administration of the rhGAA. In at least one embodiment, the miglustat is administered less than three hours prior to administration of the rhGAA. In at least one embodiment, the miglustat is administered about two hours prior to administration of the rhGAA. In at least one embodiment, the miglustat is administered in a range of 50 minutes to 90 minutes prior to administration of the rhGAA. For instance, the miglustat may be administered about 1.5 hours, about 1 hour, about 50 minutes, about 30 minutes, or about 20 minutes prior to administration of the rhGAA. In at least one embodiment, the miglustat is administered about one hour prior to administration of the rhGAA. In some emb71iglustat the miglustat is orally administered about one hour prior to administration of the rhGAA.

In some embodiments, the miglustat is administered after administration of the rhGAA. In at least one embodiment, the miglustat is administered within three hours after administration of the rhGAA. In at least one embodiment, the miglustat is administered within two hours after administration of the rhGAA. For instance, the miglustat may be administered within about 1.5 hours, about 1 hour, about 50 minutes, about 30 minutes, or about 20 minutes after administration of the rhGAA.

In some embodiments, the subject fasts for at least two hours before administration of miglustat. In some embodiments, the subject fasts for at least two hours after administration of miglustat. In some embodiments, the subject fasts for at least two hours before and at least two hours after administration of miglustat.

In some embodiments, the subject fasts for at least two hours before and at least two hours after administration of miglustat, and the miglustat is administered about one hour prior to administration of the rhGAA. In some embodiments, the fasting, miglustat administration and rhGAA administration follows the following dosing timeline:

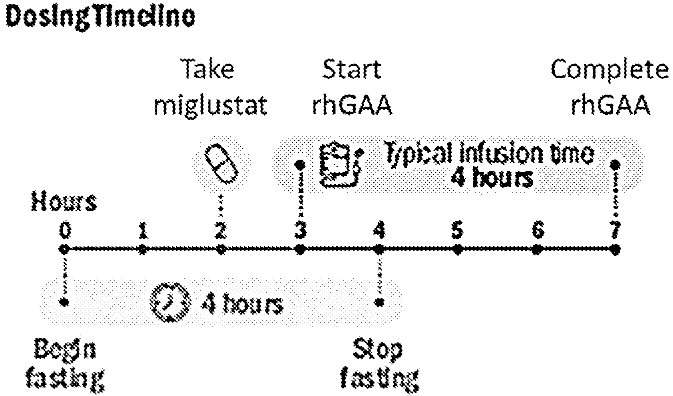
Dosing Timeline
Take
miglustat
Start
rhGAA
Complete
rhGAA
 Typical infusion time
4 hours
Hours
0 1 2 3 4 5 6 7
 4 hours
Begin
fasting
Stop
fasting

In some embodiments, the two-component therapy according to this disclosure improves one or more disease symptoms in a subject with Pompe disease compared to (1) baseline, or (2) a control treatment comprising administering alglucosidase alfa and a placebo for the enzyme stabilizer. In such control treatment, a placebo was administered in place of the enzyme stabilizer.

Provided herein is a method for improving and/or stabilizing motor function and/or pulmonary function for at least 24 months, at least 36 months, or at least 48 months in a subject having Pompe disease, the method comprising administering to the subject a population of recombinant human acid α-glucosidase (rhGAA) molecules, concurrently or sequentially with an enzyme stabilizer; wherein each rhGAA molecule comprises seven potential N-glycosylation sites; wherein 40%-60% of the N-glycans on the rhGAA molecules are complex type N-glycans; wherein the rhGAA molecules comprise at least 0.5 mol bis-mannose-6-phosphate (bis-M6P) per mol of rhGAA at the first potential N-glycosylation site as determined using liquid chromatography tandem mass spectrometry (LC-MS/MS); and wherein the method improves and/or stabilizes motor function and/or pulmonary function in the subject compared to baseline.

In some embodiments, the subject treated by two-component therapy is an ERT-experienced patient. In some embodiments, the ERT-experienced subject had been previously treated with alglucosidase alfa. In some embodiments, the ERT-experienced subject had been previously treated with alglucosidase alfa for from about 2 years to about 6 years. In some embodiments, the ERT-experienced subject had been previously treated with alglucosidase alfa for at least about 7 years. In some embodiments, the ERT-experienced subject is non-ambulatory. In some embodiments, the ERT-experienced subject is ambulatory. In some embodiments, the subject is an ERT-naïve subject.

In some embodiments, the subject treated by two-component therapy is an ERT-naive patient.

In some embodiments, the two-component therapy according to this disclosure improves and/or stabilizes the subject's motor function, as measured by a 6-minute walk test (6MWT). In some embodiments, compared to baseline, the subject's 6-minute walk distance (6MWD) is increased by at least 5, 6, 7, 8, 9, 10, 11, 12, 13, 14, 15, 16, 17, 18, 19, 20, 21, 22, 23, 24, 25, 30, 35, 40, 45 or 50 meters or at least 1%, 2%, 3%, 4%, 5%, 6%, 7%, 8%, 9%, or 10% after 12, 26, 38, or 52 weeks, or after 12, 18, 24, 30, 36 or 48 months of treatment. In some embodiments, the subject's 6MWD is increased by at least 20 meters or at least 5% after 52 weeks of treatment. In some embodiments, the subject's 6MWD is increased by at least 20 meters or at least 5% after 12, 18, 24, 30, 36 or 48 months of treatment. In some embodiments, compared to the control treatment, the subject's 6MWD is improved by at least 5, 6, 7, 8, 9, 10, 12, 14, 16, 18, 20, 30, 40, or 50 meters after 12, 26, 38, or 52 weeks, or after 18, 24, 30, 36 or 48 months of treatment. In some embodiments, compared to the control treatment, the subject's 6MWD is improved by at least 13 meters after 52 weeks of treatment. In some embodiments of methods for treating an ERT-experienced subject, the motor function is measured by a 6-minute walk test; and the improvement from baseline in 6-minute walk distance (6MWD) is at least 15, 16, 17, 18, 19, 20, 21, 22, 23, 24, 25, 26, 27, 28, 29, 30, 31, 32, 33, 34, or 35 meters at 12, 18, 24, 30, 36 or 48 months after initiation of treatment. In some embodiments of methods for treating an ERT-experienced subject, the motor function is measured by a 6-minute walk test; and the improvement from baseline in 6-minute walk distance (6MWD) is at least 5, 6, 7, 8, 9, 10, 15, 20, 25, 30, 35, 40, 41, 42, 43, 44, 45, 46, or 47 meters at 36 or 48 months after initiation of treatment.

In some embodiments of methods for treating an ERT-naïve subject, the motor function is measured by a 6-minute walk test; and the improvement from baseline in 6-minute walk distance (6MWD) is at least 40, 41, 42, 43, 44, 45, 46, 47, 48, 49, 50, 51, 52, 53, 54, 55, 56, 57, 58, 59, or 60 meters at 12, 24, 36 or 48 months after initiation of treatment. In some embodiments of methods for treating an ERT-naïve subject, the motor function is measured by a 6-minute walk test; and the improvement from baseline in 6-minute walk distance (6MWD) is at least 34, 35, 40, 41, 42, 43, 44 or 45 meters at 36 or 48 months after initiation of treatment. In some embodiments, the subject has a baseline 6MWD less than 300 meters. In some embodiments, the subject has a baseline 6MWD greater than or equal to 300 meters.

In some embodiments, the two-component therapy according to this disclosure stabilizes the subject's pulmonary function, as measured by a forced vital capacity (FVC) test. In some embodiments, after 12, 26, 38, or 52 weeks, or after 18, 24, 30, 36, or 48 months of treatment, the subject's percent-predicted FVC is either increased compared to baseline, or decreased by less than 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, or 10% compared to baseline. In some embodiments, after 52 weeks of treatment, the subject's percent-predicted FVC is decreased by less than 1% compared to baseline. In some embodiments, the subject's percent-predicted FVC is increased by at least 1%, at least 2%, at least 3% or at least 5%, compared to baseline after 12, 18, 24, 30, 36 or 48 months of treatment. In some embodiments, compared to the control treatment, the subject's percent-predicted FVC is significantly improved and/or stabilized after treatment. In some embodiments, compared to the control treatment, the subject's percent-predicted FVC is significantly improved by at least 0.5%, 1%, 2%, 3%, 4%, 5%, or 6% after 12, 26, 38, or 52 weeks, or after 18, 24, 30, 36, or 48 months of treatment. In some embodiments, compared to the control treatment, the subject's percent-predicted FVC is significantly improved by at least 3% after 52 weeks of treatment. In some embodiments, compared to the control treatment, the subject's percent-predicted FVC is significantly improved by at least 3% or at least 5% after 12, 18, 24, 30, 36 or 48 months of treatment. In some embodiments of methods for treating an ERT-experienced subject, the pulmonary function is measured by a sitting forced vital capacity (FVC) test, and the subject's percent-predicted FVC is stable compared to baseline at 24, 36, or 48 months after initiation of treatment. In some embodiments of methods for treating an ERT-naïve subject, the pulmonary function is measured by a sitting forced vital capacity (FVC) test; and the improvement from baseline in the subject's percent-predicted FVC is at least 4.0, 4.1, 4.2, 4.3, 4.4, 4.5, 4.6, 4.7, 4.8, 4.9, 5.0, 5.5, 6.0, 6.4, 6.5, 6.6, 6.7, or 6.8% at 24 months after initiation of treatment. In some embodiments of methods for treating an ERT-naïve subject, the pulmonary function is measured by a sitting forced vital capacity (FVC) test; and the improvement from baseline in the subject's percent-predicted FVC is at least 5.7, 5.8, 5.9, 6.0, 6.1, or 6.2% at 36 or 48 months after initiation of treatment. In some embodiments, the subject has a baseline FVC less than 55%. In some embodiments, the subject has a baseline FVC greater than or equal to 55%. In some embodiments, the subject has a baseline FVC less than 50%. In some embodiments, the subject has a baseline FVC greater than or equal to 50%.

In some embodiments, the two-component therapy according to this disclosure improves and/or stabilizes the subject's muscle strength, as measured by a manual muscle test (MMT). In some embodiments, compared to baseline, the subject's MMT lower extremity score is improved as indicated by an increase of at least 0.1, 0.3, 0.5, 0.7, 1.0, 1.0, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5 points after 12, 26, 38 or 52 weeks, or after 12, 18, 24, 30, 36 or 48 months of treatment. In some embodiments, compared to the control treatment, the subject's MMT lower extremity score is significantly improved and/or stabilized after treatment. In some embodiments of methods for treating an ERT-experienced subject, the muscle strength is measured by MMT; and the improvement from baseline in an MMT lower extremity score is at least 1.9, 2, 2.1, 2.2, or 2.3 points at 24 months after initiation of treatment. In some embodiments of methods for treating an ERT-experienced subject, the muscle strength is measured by an MMT; and the improvement from baseline in an MMT lower extremity score is at least 1.5, 1.6, 1.7, 1.8, or 1.9 points at 36 or 48 months after initiation of treatment. In some embodiments of methods for treating an ERT-naive subject, the muscle strength is measured by an MMT; and the improvement from baseline in an MMT lower extremity score is at least 1.0, 1.1, 1.2, 1.3, 1.4, or 1.5 points at 24 months after initiation of treatment. In some embodiments of methods for treating an ERT-naïve subject, the muscle strength is measured by an MMT; and the improvement from baseline in an MMT lower extremity score is at least 2.8, 2.9, 3.0, 3.1, 3.2, or 3.3 points at 36 or 48 months after initiation of treatment. In some embodiments, the subject has a baseline MMT lower extremity score less than 25. In some embodiments, the subject has a baseline MMT lower extremity score greater than or equal to 25.

In some embodiments, the two-component therapy according to this disclosure improves and/or stabilizes the subject's motor function, as measured by a gait, stair, gower, chair (GSGC) test. In some embodiments, compared to baseline, the subject's GSGC score is improved as indicated by a decrease of at least 0.1, 0.3, 0.5, 0.7, 1.0, 1.5, or 2.5 points after 12, 26, 38 or 52 weeks, or after 18, 24, 30, 36, or 48 months of treatment. In some embodiments, compared to baseline, the subject's GSGC score is improved as indicated by a decrease of at least 0.5 points after 52 weeks of treatment. In some embodiments, compared to the control treatment, the subject's GSGC score is significantly improved after treatment. In some embodiments, compared to the control treatment, the subject's GSGC score is significantly improved as indicated by a decrease of at least 0.3, 0.5, 0.7, 1.0, 1.5, 2.5, or 5 points after 12, 26, 38, or 52 weeks, or after 18, 24, 30, 36, or 48 months of treatment. In some embodiments, compared to the control treatment, the subject's GSGC score is significantly improved as indicated by a decrease of at least 1.0 point after 52 weeks of treatment.

In some embodiments, the two-component therapy according to this disclosure improves the subject's cardiac function. In some embodiments, the therapy improves the subject's cardiac function as measured by left ventricular mass index (LVMi).

In some embodiments, the two-component therapy according to this disclosure reduces the level of at least one marker of muscle damage after treatment. In some embodiments, marker of muscle damage comprises one or more of creatine kinase (CK), alanine aminotransferase (ALT), and aspartate aminotransferase (AST). In some embodiments, the at least one marker of muscle damage comprises CK. In some embodiments, compared to baseline, the subject's CK level is reduced by at least 10%, 15%, 20%, 25%, 30%, 40%, or 50% after 12, 26, 38, or 52 weeks, or after 18, 24, 30, or 36 months of treatment. In some embodiments, compared to baseline, the subject's CK level is reduced by at least 20% after 52 weeks of treatment. In some embodiments, compared to the control treatment, the subject's CK level is significantly reduced after treatment. In some embodiments, compared to the control treatment, the subject's CK level is significantly reduced by at least 10%, 15%, 20%, 25%, 30%, 40%, or 50% after 12, 26, 38, or 52 weeks, or after 18, 24, 30, or 36 months of treatment. In some embodiments, compared to the control treatment, the subject's CK level is significantly reduced by at least 30% after 52 weeks of treatment.

In some embodiments, the two-component therapy according to this disclosure reduces the level of at least one marker of glycogen accumulation after treatment. In some embodiments, the at least one marker of glycogen accumulation comprises urine hexose tetrasaccharide (Hex4). In some embodiments, compared to baseline, the subject's urinary Hex4 level is reduced by at least 10%, 15%, 20%, 25%, 30%, 40%, 50%, or 60% after 12, 26, 38, or 52 weeks, or after 18, 24, 30, or 36 months of treatment. In some embodiments, compared to baseline, the subject's urinary Hex4 level is reduced by at least 30% after 52 weeks of treatment. In some embodiments, compared to the control treatment, the subject's urinary Hex4 level is significantly reduced after treatment. In some embodiments, compared to the control treatment, the subject's urinary Hex4 level is significantly reduced by at least 10%, 15%, 20%, 25%, 30%, 40%, 50%, or 60% after 12, 26, 38, or 52 weeks, or after 18, 24, 30, or 36 months of treatment. In some embodiments, compared to the control treatment, the subject's urinary Hex4 level is significantly reduced by at least 40% after 52 weeks of treatment.

In some embodiments, the two-component therapy according to this disclosure improves and/or stabilizes one or more disease symptoms in an ERT-experienced patient subject with Pompe disease compared to (1) baseline, or (2) a control treatment comprising administering alglucosidase alfa and a placebo for the enzyme stabilizer.

In some embodiments, the two-component therapy according to this disclosure improves a quality of life and/or patient reported outcome measurement, such as by the European Quality of Life - Five Dimensions Five Level (EQ 5D 5L/EQ-5D-Y) Questionnaire and/or the Patient-Reported Outcomes Measurement Information System (PROMIS-Physical Function, PROMIS Fatigue, PROMIS Dyspnea).

In some embodiments, the two-component therapy according to this disclosure reduces, delays and/or maintains the need for user of devices for mobility or respiratory support, such as by monitoring onset of/changes in use of assistive device for mobility and type of device and/or monitoring onset of/changes in use of respiratory support and type of support.

In some embodiments, the two-component therapy for an ERT-experienced subject with Pompe disease improves and/or stabilizes the subject's motor function, as measured by a 6MWT. In some embodiments, compared to baseline, the subject's 6MWD is increased by at least 10, 11, 12, 13, 14, 15, 16, 17, 18, 19, 20, 21, 22, 23, 24, 25, 30, or 50 meters or at least 1%, 2%, 3%, 4%, 5%, 6%, 7%, 8%, 9%, or 10% after 12, 26, 38, or 52 weeks, or after 18, 24, 30, 36, or 48 months of treatment. In some embodiments, the subject's 6MWD is increased by at least 15 meters or at least 5% after 52 weeks of treatment. In some embodiments, the subject's 6MWD is increased by at least 25 meters or at least 6% after 24 months of treatment. In some embodiments, the subject's 6MWD is increased by at least 10 meters or at least 2% after 36 months of treatment. In some embodiments, the subject's 6MWD is increased by at least 20 meters or at least 5% after 52 weeks of treatment. In some embodiments, compared to the control treatment, the subject's 6MWD is significantly improved after treatment. In some embodiments, compared to the control treatment, the subject's 6MWD is significantly improved by at least 10, 12, 14, 15, 16, 18, 20, 30, 40, or 50 meters after 12, 26, 38, or 52 weeks, or after 18, 24, 30, 36, or 48 months of treatment. In some embodiments, compared to the control treatment, the subject's 6MWD is significantly improved by at least 15 meters after 52 weeks of treatment. In some embodiments, the subject has a baseline 6MWD less than 300 meters. In some embodiments, the subject has a baseline 6MWD greater than or equal to 300 meters.

In some embodiments, the two-component therapy for an ERT-experienced subject with Pompe disease improves and/or stabilizes the subject's pulmonary function, as measured by an FVC test. In some embodiments, after 12, 26, 38, or 52 weeks, or after 18, 24, 30, 36, or 48 months of treatment, the subject's percent-predicted FVC is increased by at least 0.1%, 0.2%, 0.3%, 0.4%, 0.5%, 1%, 2%, 3%, 4%, or 5% compared to baseline. In some embodiments, after 52 weeks of treatment, the subject's percent-predicted FVC is increased by at least 0.1% compared to baseline. In some embodiments, compared to the control treatment, the subject's percent-predicted FVC is significantly improved and/or stabilized after treatment. In some embodiments, compared to the control treatment, the subject's percent-predicted FVC is significantly improved by at least 1%, 2%, 3%, 4%, 5%, 6%, 8%, or 10% after 12, 26, 38, or 52 weeks, or after 18, 24, 30, 36, or 48 months of treatment. In some embodiments, compared to the control treatment, the subject's percent-predicted FVC is significantly improved by at least 4% after 52 weeks of treatment. In some embodiments, the subject has a baseline FVC less than 55%. In some embodiments, the subject has a baseline FVC greater than or equal to 55%.

In some embodiments, the two-component therapy for an ERT-experienced subject with Pompe disease improves and/or stabilizes the subject's motor function, as measured by a GSGC test. In some embodiments, compared to baseline, the subject's GSGC score is improved as indicated by a decrease of at least 0.1, 0.3, 0.5, 0.7, 1.0, 1.5, or 2.5 points after 12, 26, 38, or 52 weeks, or after 18, 24, 30, 36, or 48 months of treatment. In some embodiments, compared to baseline, the subject's GSGC score is improved as indicated by a decrease of at least 0.5 points after 52 weeks of treatment. In some embodiments, compared to the control treatment, the subject's GSGC score is significantly improved and/or stabilized after treatment. In some embodiments, compared to the control treatment, the subject's GSGC score is significantly improved as indicated by a decrease of at least 0.3, 0.5, 0.7, 1.0, 1.5, 2.5, or 5 points after 12, 26, 38, or 52 weeks, or after 18, 24, 30, 36, or 48 months of treatment. In some embodiments, compared to the control treatment, the subject's GSGC score is significantly improved as indicated by a decrease of at least 1.0 point after 52 weeks of treatment.

In some embodiments, the two-component therapy for an ERT-experienced subject with Pompe disease reduces the level of at least one marker of muscle damage after treatment. In some embodiments, the at least one marker of muscle damage comprises CK. In some embodiments, compared to baseline, the subject's CK level is reduced by at least 10%, 15%, 20%, 25%, 30%, 40%, or 50% after 12, 26, 38, or 52 weeks, or after 18, 24, 30, 36, or 48 months of treatment. In some embodiments, compared to baseline, the subject's CK level is reduced by at least 15% after 52 weeks of treatment. In some embodiments, compared to baseline, the subject's CK level is reduced by at least 25% after 24 months of treatment. In some embodiments, compared to baseline, the subject's CK level is reduced by at least 30% after 36 months of treatment. In some embodiments, compared to baseline, the subject's CK level is reduced by at least 35% after 48 months of treatment. In some embodiments, compared to the control treatment, the subject's CK level is significantly reduced after treatment. In some embodiments, compared to the control treatment, the subject's CK level is significantly reduced by at least 10%, 15%, 20%, 25%, 30%, 40%, or 50% after 12, 26, 38, or 52 weeks, or after 18, 24, 30, 36, or 48 months of treatment. In some embodiments, compared to the control treatment, the subject's CK level is significantly reduced by at least 30% after 52 weeks of treatment.

In some embodiments, the two-component therapy for an ERT-experienced subject with Pompe disease reduces the level of at least one marker of glycogen accumulation after treatment. In some embodiments, the at least one marker of glycogen accumulation comprises urinary Hex4. In some embodiments, compared to baseline, the subject's urinary Hex4 level is reduced by at least 10%, 15%, 20%, 25%, 30%, 40%, 50%, or 60% after 12, 26, 38, or 52 weeks, or after 18, 24, 30, or 36 months of treatment. In some embodiments, compared to baseline, the subject's urinary Hex4 level is reduced by at least 25% after 52 weeks of treatment. In some embodiments, compared to baseline, the subject's urinary Hex4 level is reduced by at least 35% after 36 months of treatment. In some embodiments, compared to baseline, the subject's urinary Hex4 level is reduced by at least 30% after 48 months of treatment. In some embodiments, compared to the control treatment, the subject's urinary Hex4 level is significantly reduced after treatment. In some embodiments, compared to the control treatment, the subject's urinary Hex4 level is significantly reduced by at least 10%, 15%, 20%, 25%, 30%, 40%, 50%, or 60% after 12, 26, 38, or 52 weeks, or after 18, 24, 30, or 36 months of treatment. In some embodiments, compared to the control treatment, the subject's urinary Hex4 level is significantly reduced by at least 40% after 52 weeks of treatment.

### E. Kits

Another aspect of the disclosure pertains to kits suitable for performing the rhGAA therapy described herein. In one or more embodiments, the kit comprises a container (e.g., vial, tube, bag, etc.) comprising the rhGAA or pharmaceutical composition (either before or after lyophilization) and instructions for reconstitution, dilution and administration. In one or more embodiments, the kit comprises a container (e.g., vial, tube, bag, etc.) comprising an enzyme stabilizer (e.g., miglustat) and a pharmaceutical composition comprising rhGAA (either before or after lyophilization), and instructions for reconstitution, dilution, and administration of rhGAA with the enzyme stabilizer.

### EXAMPLES

The present invention is further illustrated by the following non-limiting examples.

### Example 1: Preparation of CHO Cells producing rhGAA having a high content of mono- or bis-M6P-bearing N-glycans.

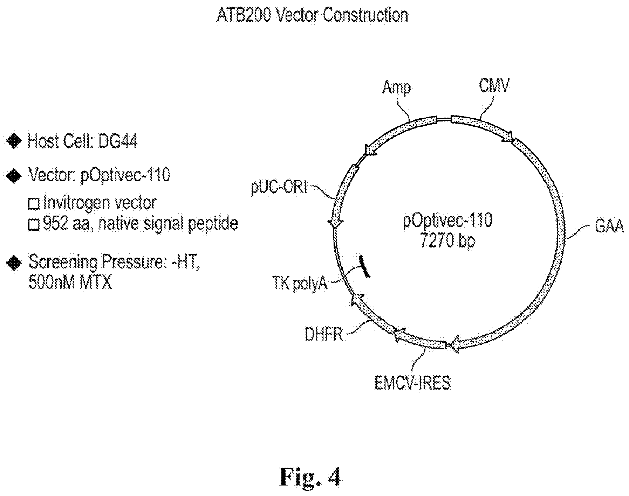

DG44 CHO (DHFR-) cells were transfected with a DNA construct that expresses rhGAA. The DNA construct is shown in Fig. 4. After transfection, CHO cells containing a stably integrated GAA gene were selected with hypoxanthine/thymidine deficient (-HT) medium). GAA expression in these cells was induced by methotrexate treatment (MTX, 500 nM).

Cell pools that expressed high amounts of GAA were identified by GAA enzyme activity assays and were used to establish individual clones producing rhGAA. Individual clones were generated on semisolid media plates, picked by ClonePix system, and were transferred to 24-deep well plates. The individual clones were assayed for GAA enzyme activity to identify clones expressing a high level of GAA. Conditioned media for determining GAA activity used a 4-MU-α-Glucosidase substrate. Clones producing higher levels of GAA as measured by GAA enzyme assays were further evaluated for viability, ability to grow, GAA productivity, N-glycan structure and stable protein expression. CHO cell lines, including CHO cell line GA-ATB200, expressing rhGAA with enhanced mono-M6P or bis-M6P N-glycans were isolated using this procedure.

### Example 2: Purification of rhGAA

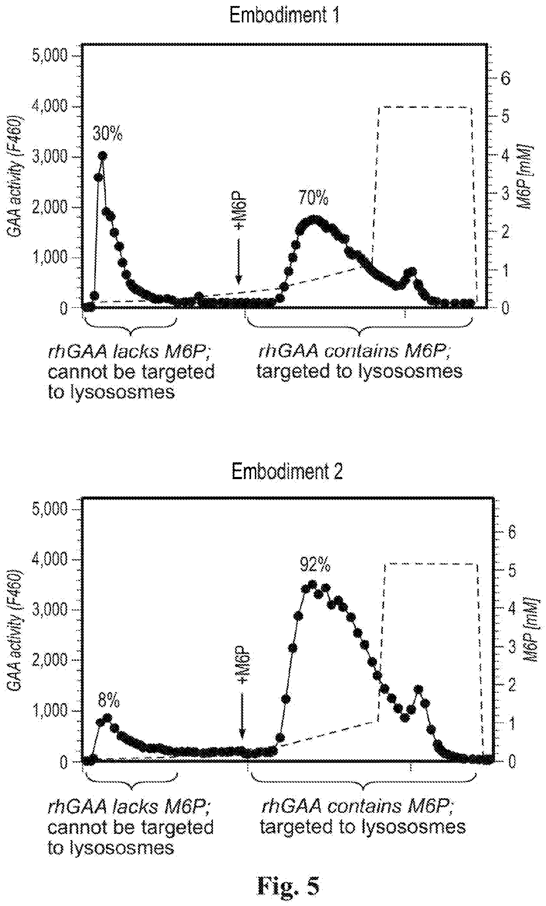

Multiple batches of the rhGAA according to the disclosure were produced in shake flasks and in perfusion bioreactors using CHO cell line GA-ATB200, the product of which is referred to as "ATB200." Weak anion exchange ("WAX") liquid chromatography was used to fractionate ATB200 rhGAA according to terminal phosphate and sialic acid. Elution profiles were generated by eluting the ERT with increasing amount of salt. The profiles were monitored by UV (A280nm). Similar CIMPR receptor binding (at least ~70%) profiles were observed for purified ATB200 rhGAA from different production batches (Fig. 5), indicating that ATB200 rhGAA can be consistently produced.

### Example 3: Oligosaccharide Characterization of ATB200 rhGAA

ATB200 rhGAA was analyzed for site-specific N-glycan profiles using different LC-MS/MS analytical techniques. The results of the first two LC-MS/MS methods are shown in Figs. 6A-6H. The results of a third LC-MS/MS method with 2-AA glycan mapping are shown in Figs. 19A-19H, Fig. 20A-20B, and Table 9.

In the first LC-MS/MS analysis, the protein was denatured, reduced, alkylated, and digested prior to LC-MS/MS analysis. During protein denaturation and reduction, 200 µg of protein sample, 5 µL of 1 mol/L tris-HCl (final concentration 50 mM), 75 µL of 8 mol/L guanidine HCl (final concentration 6 M), 1 µL of 0.5 mol/L EDTA (final concentration 5 mM), 2 µL of 1 mol/L DTT (final concentration 20 mM), and Milli-Q^{®} water were added to a 1.5 mL tube to provide a total volume of 100 µL. The sample was mixed and incubated at 56°C for 30 minutes in a dry bath. During alkylation, the denatured and reduced protein sample was mixed with 5 µL of 1 mol/L iodoacetamide (IAM, final concentration 50 mM), then incubated at 10-30°C in the dark for 30 minutes. After alkylation, 400 µL of precooled acetone was added to the sample and the mixture was frozen at -80°C refrigeration for 4 hours. The sample was then centrifuged for 5 min at 13000 rpm at 4°C and the supernatant was removed. 400 µL of precooled acetone was added to the pellets, which was then centrifuged for 5 min at 13000 rpm at 4°C and the supernatant was removed. The sample was then air dried on ice in the dark to remove acetone residue. Forty microliters of 8M urea and 160 µL of 100 mM NH₄HCO₃ were added to the sample to dissolve the protein. During trypsin digestion, 50 µg of the protein was then added with trypsin digestion buffer to a final volume of 100 µL, and 5 µL of 0.5 mg/mL trypsin (protein to enzyme ratio of 20/1 w/w) was added. The solution was mixed well and incubated overnight (16 ± 2 hours) at 37°C. Two and a half microliters of 20% TFA (final concentration 0.5%) were added to quench the reaction. The sample was then analyzed using the Thermo Scientific^{™} Orbitrap Velos Pro^{™} Mass Spectrometer.

In the second LC-MS/MS analysis, the ATB200 sample was prepared according to a similar denaturation, reduction, alkylation, and digestion procedure, except that iodoacetic acid (IAA) was used as the alkylation reagent instead of IAM, and then analyzed using the Thermo Scientific^{™} Orbitrap Fusion^{™} Lumos Tribid^{™} Mass Spectrometer.

The results of the first and second analyses are shown in Figs. 6A-6H. In Figs. 6A-6H, the results of the first analysis are represented by left bar (dark grey) and the results from the second analysis are represented by the right bar (light grey). The symbol nomenclature for glycan representation is in accordance with Varki, A., Cummings, R.D., Esko J.D., et al., Essentials of Glycobiology, 2nd edition (2009).

As can be seen from Figs. 6A-6H, the two analyses provided similar results, although there was some variation between the results. This variation can be due to a number of factors, including the instrument used and the completeness of N-glycan analysis. For example, if some species of phosphorylated N-glycans were not identified and/or not quantified, then the total number of phosphorylated N-glycans may be underrepresented, and the percentage of rhGAA bearing the phosphorylated N-glycans at that site may be underrepresented. As another example, if some species of non-phosphorylated N-glycans were not identified and/or not quantified, then the total number of non-phosphorylated N-glycans may be underrepresented, and the percentage of rhGAA bearing the phosphorylated N-glycans at that site may be overrepresented.

Fig. 6A shows the N-glycosylation site occupancy of ATB200. As can be seen from Fig. 6A, the first, second, third, fourth, fifth, and sixth N-glycosylation sites are mostly occupied, with both analyses detecting around or over 90% and up to about 100% of the ATB200 enzyme having an N-glycan detected at each potential N-glycosylation site. However, the seventh potential N-glycosylation site is N-glycosylated about half of the time.

Fig. 6B shows the N-glycosylation profile of the first potential N-glycosylation site, N84. As can be seen from Fig. 6B, the major N-glycan species is bis-M6P N-glycans. Both the first and second analyses detected over 75% of the ATB200 having bis-M6P at the first site, corresponding to an average of about 0.8 mol bis-M6P per mol ATB200 at the first site.

Fig. 6C shows the N-glycosylation profile of the second potential N-glycosylation site, N177. As can be seen from Fig. 6C, the major N-glycan species are mono-M6P N-glycans and non-phosphorylated high mannose N-glycans. Both the first and second analyses detected over 40% of the ATB200 having mono-M6P at the second site, corresponding to an average of about 0.4 to about 0.6 mol mono-M6P per mol ATB200 at the second site.

Fig. 6D shows the N-glycosylation profile of the third potential N-glycosylation site, N334. As can be seen from Fig. 6D, the major N-glycan species are non-phosphorylated high mannose N-glycans, di-, tri-, and tetra-antennary complex N-glycans, and hybrid N-glycans. Both the first and second analyses detected over 20% of the ATB200 having a sialic acid residue at the third site, corresponding to an average of about 0.9 to about 1.2 mol sialic acid per mol ATB200 at the third site.

Fig. 6E shows the N-glycosylation profile of the fourth potential N-glycosylation site, N414. As can be seen from Fig. 6E, the major N-glycan species are bis-M6P and mono-M6P N-glycans. Both the first and second analyses detected over 40% of the ATB200 having bis-M6P at the fourth site, corresponding to an average of about 0.4 to about 0.6 mol bis-M6P per mol ATB200 at the fourth site. Both the first and second analyses also detected over 25% of the ATB200 having mono-M6P at the fourth site, corresponding to an average of about 0.3 to about 0.4 mol mono-M6P per mol ATB200 at the fourth site.

Fig. 6F shows the N-glycosylation profile of the fifth potential N-glycosylation site, N596. As can be seen from Fig. 6F, the major N-glycan species are fucosylated di-antennary complex N-glycans. Both the first and second analyses detected over 70% of the ATB200 having a sialic acid residue at the fifth site, corresponding to an average of about 0.8 to about 0.9 mol sialic acid per mol ATB200 at the fifth site.

Fig. 6G shows the N-glycosylation profile of the sixth potential N-glycosylation site, N826. As can be seen from Fig. 6G, the major N-glycan species are di-, tri-, and tetra-antennary complex N-glycans. Both the first and second analyses detected over 80% of the ATB200 having a sialic acid residue at the sixth site, corresponding to an average of about 1.5 to about 1.8 mol sialic acid per mol ATB200 at the sixth site.

An analysis of the N-glycosylation at the seventh site, N869, showed approximately 40% N-glycosylation, with the most common N-glycans being A4S3S3GF (12%), A5S3G2F (10%), A4S2G2F (8%) and A6S3G3F (8%).

Fig. 6H shows a summary of the phosphorylation at each of the seven potential N-glycosylation sites. A s can be seen from Fig. 6H, both the first and second analyses detected high phosphorylation levels at the first, second, and fourth potential N-glycosylation sites. Both analyses detected over 80% of the ATB200 was mono- or bis-phosphorylated at the first site, over 40% of the ATB200 was mono-phosphorylated at the second site, and over 80% of the ATB200 was mono- or bis-phosphorylated at the fourth site.

Another N-glycosylation analysis of ATB200 was performed according to an LC-MS/MS method as described below. This analysis yielded an average N-glycosylation profile over ten lots of ATB200 (Figs. 19A-19H, Figs. 20A-20B).

N-linked glycans from ATB200 were released enzymatically with PNGase-F and labeled with 2-Anthranilic acid (2-AA). The 2-AA labeled N-glycans were further processed by solid phase extraction (SPE) to remove excess salts and other contaminants. The purified 2-AA N-glycans were dissolved in acetonitrile/water (20/80; v/v), and 10 micrograms were loaded on an amino-polymer analytical column (apHera^{™}, Supelco) for High Performance Liquid Chromatography with Fluorescence detection (HPLC-FLD) and High Resolution Mass Spectrometry (HRMS) analysis.

The liquid chromatographic (LC) separation was performed under normal phase conditions in a gradient elution mode with mobile phase A (2% acetic acid in acetonitrile) and mobile phase B (5% acetic acid; 20 millimolar ammonium acetate in water adjusted to pH 4.3 with ammonium hydroxide). The initial mobile phase composition was 70% A/30% B. For the fluorescence detection, the parameters for the detector (RF-20Axs, Shimadzu) were Excitation (Ex):320 nm; Emission (Em):420 nm. The HRMS analysis was carried out using a Quadrupole Time of Flight mass spectrometer (Sciex X500B QTOF) operating in Independent Data Acquisition (IDA) mode. The acquired datafiles were converted into mzML files using MSConvert from ProteoWizard, and then GRITS Toolbox 1.2 Morning Blend software (UGA) was utilized for glycan database searching and subsequent annotation of identified N-glycans. The N-glycans were identified using both precursor monoisotopic masses (m/z) and product ion m/z. Experimental product ions and fragmentation patterns were confirmed in-silico using the GlycoWorkbench 2 Application.

To determine the relative quantitation of N-linked glycans from ATB200, data acquired from the HPLC-FLD-QTOF MS/MS experiment was processed as follows. All of the N-glycan peaks in the FLD chromatogram were integrated, and each peak was assigned a percentage of the total area of all peaks in the FLD chromatogram. The fluorescent signal, expressed as a peak area, is a quantitative measure of the amount of each N-glycan in the sample. However, in most cases, multiple N-glycan species were contained in the same FLD peak. Therefore, the mass spectrometer data was also required to obtain relative quantitation of each N-glycan species (Table 9). The ion intensity signal for each N-glycan was "extracted" from the data to create a chromatographic peak called an extracted ion chromatogram (XIC). The XIC aligned with the FLD chromatographic peak and was specific to only one N-glycan species. The XIC peak created from the ion intensity signal was then integrated and this peak area is a relative quantitative measure of the amount of glycan present. Both the FLD peak areas and mass spectrometer XIC peak areas were used to enable relative quantitation of all the N-linked glycan species of ATB200 reported herein.

The results of this LC-MS/MS analysis are provided in Table 9 below. The symbol nomenclature for glycan representation is in accordance with Wopereis W, et al. 2006. Abnormal glycosylation with hypersialylated O-glycans in patients with Sialuria. Biochimica et Biophysica Acta. 1762:598-607; Gornik O, et al. 2007. Changes of serum glycans during sepsis and acute pancreatitis. Glycobiology. 17:1321-1332; Kattla JJ, et al. 2011. Biologic protein glycosylation. In: Murray Moo-Young (ed.), Comprehensive Biotechnology, Second Edition, 3:467-486; Tharmalingam-Jaikaran T, et al. N-glycan profiling of bovine follicular fluid at key dominant follicle developmental stages. 2014. Reproduction. 148:569-580; Clerc F, et al. Human plasma protein N-glycosylation. 2015. Glycoconj J. DOI 10.1007/s10719-015-9626-2; and Blackler RJ, et al. 2016. Single-chain antibody-fragment M6P-1 possesses a mannose 6-phosphate monosaccharide-specific binding pocket that distinguishes N-glycan phosphorylation in a branch-specific manner. Glycobiology. 26-2:181-192.

**Table 9: Type and Prevalence of Oligosaccharides identified on ATB200 based on 2-AA glycan mapping and LC-MS/MS identification**

| **High Mannose N-Glycans** | **% Total** | **Complex N-Glycans** | **% Total** | **Complex N-Glycans** | **% Total** | **Complex N-Glycans** | **% Total** |
|---|---|---|---|---|---|---|---|
| 2P-M7 | 11.39 | FA2G2S1 | 3.89 | A3G3S1+1Ac | 0.65 | FA2G2S1+1Ac | 0.29 |
| P-M7 | 7.97 | FA2G2S2 | 3.42 | A3G2S2+1Ac | 0.64 | A4G3 | 0.29 |
| M6 | 6.89 | A2G2S2 | 3.32 | A1G1S1 | 0.63 | A4G4+3KDN | 0.29 |
| P-M6 | 3.42 | FA2G2 | 2.77 | A4G3S1 | 0.61 | A4G4S3 | 0.28 |
| M5 | 2.06 | FA4G4S3 | 2.26 | FA3G3 | 0.61 | FA5G4 | 0.24 |
| P-M5 | 1.67 | A2G2S1 | 2.25 | A1G1 | 0.6 | A4G3S2 | 0.21 |
| 2P-M8 | 1.27 | FA3G3S1 | 2.12 | FA2G2S2+1Ac | 0.57 | FA1 | 0.21 |
| P-M8 | 1.17 | A3G3S2 | 1.8 | A3G2S1 | 0.57 | FA4G4 | 0.21 |
| BP-M6 | 0.9 | FA2G1 | 1.66 | A3G2S1 | 0.56 | A3G1 | 0.21 |
| M7 | 0.81 | A2G2 | 1.46 | A2G2S2+1Ac | 0.5 | FA4G3S2 | 0.21 |
| BP-M7 | 0.69 | FA3G3S1 | 1.42 | FA3G2 | 0.45 | FA3G2S2 | 0.21 |
| M4 | 0.14 | A4G4S1 | 1.28 | A3G3+3KDN | 0.45 | A1 | 0.2 |
| BP2-M5 | 0.04 | FA3G3S2 | 1.25 | A4G3S1 | 0.45 | A4G2 | 0.19 |
| BP2-M6 | 0.01 | FA4G4(1LN)S3 | 1.1 | A2G1S1 | 0.41 | FA4G3 | 0.19 |
| | | | | | | | |
| **Hybrid N-Glycans** | **% Total** | FA4G4S1 | 1.08 | A3G2 | 0.4 | FA3 | 0.18 |
| FA1P-M6 | 2.16 | A3G3 | 1.08 | FA4G4S1+LN | 0.4 | A1G1S1 | 0.18 |
| M5A1G1S1 | 1.56 | FA4G4S4 | 1.07 | FA3G2S1 | 0.39 | A4G1S1 | 0.16 |
| FP-M6A1G1S1 | 0.42 | FA3G3S3 | 1.04 | FA2 | 0.38 | FA1G1 | 0.15 |
| A1M5 | 0.36 | FA4G4S2 | 0.94 | FA4G4S2+LN | 0.38 | FA3G1 | 0.14 |
| A1G1M5 | 0.32 | A2G1 | 0.94 | A3G2S2 | 0.37 | FA5G4S2 | 0.12 |
| P-M6A1G1S1 | 0.17 | FA2G1S1 | 0.94 | A2 | 0.34 | A3G1S1 | 0.11 |
| | | | | | | | |
| **Summary** | **Total** | A4G4 | 0.91 | FA4G4(2LN)S3 | 0.33 | A3 | 0.11 |
| **High Mannose N-Glycans** | 38% | FA1G1S1 | 0.91 | FA2G2Sg1 | 0.32 | FA3G3S3+1Ac | 0.1 |
| **Hybrid N-Glycans** | 5% | FA2G2S2+2Ac | 0.76 | FA4G4(1LN)S4 | 0.31 | A2G2S1+1Ac | 0.09 |
| **Complex N-Glycans** | 57% | A4G4S2 | 0.69 | A3G3S3 | 0.29 | FA3G1S1 | 0.06 |

Based on this 2-AA and LC-MS/MS analysis, and as further summarized, the ATB200 tested has an average M6P content of 3-5 mol per mol of ATB200 (accounting for both mono-M6P and bis-M6P) and sialic acid content of 4-7 mol per mol of ATB200.

As shown in Figs. 19A-19H and summarized in Fig. 20B, the first potential N-glycosylation site of ATB200 has an average M6P content of about 1.4 mol M6P/mol ATB200, accounting for an average mono-M6P content of about 0.25 mol mono-M6P/mol ATB200 and an average bis-M6P content of about 0.56 mol bis-M6P/mol ATB200; the second potential N-glycosylation site of ATB200 has an average M6P content of about 0.5 mol M6P/mol ATB200, with the primary phosphorylated N-glycan species being mono-M6P N-glycans; the third potential N-glycosylation site of ATB200 has an average sialic acid content of about 1 mol sialic acid/mol ATB200; the fourth potential N-glycosylation site of ATB200 has an average M6P content of about 1.4 mol M6P/mol ATB200, accounting for an average mono-M6P content of about 0.35 mol mono-M6P/mol ATB200 and an average bis-M6P content of about 0.52 mol bis-M6P/mol ATB200; the fifth potential N-glycosylation site of ATB200 has an average sialic acid content of about 0.86 mol sialic acid/mol ATB200; the sixth potential N-glycosylation site of ATB200 has an average sialic acid content of about 4.2 mol sialic acid/mol ATB200; and the seventh potential N-glycosylation site of ATB200 has an average sialic acid content of about 0.86 mol sialic acid/mol ATB200.

Also according to this 2-AA and LC-MS/MS analytical technique, an average of about 65% of the N-glycans at the first potential N-glycosylation site of ATB200 are high mannose N-glycans, about 89% of the N-glycans at the second potential N-glycosylation site of ATB200 are high mannose N-glycans, over half of the N-glycans at the third potential N-glycosylation site of ATB200 are sialylated (with nearly 20% fully sialylated) and about 85% of the N-glycans at the third potential N-glycosylation site of ATB200 are complex N-glycans, about 84% of the N-glycans at the fourth potential N-glycosylation site of ATB200 are high mannose N-glycans, about 70% of the N-glycans at the fifth potential N-glycosylation site of ATB200 are sialylated (with about 26% fully sialylated) and about 100% of the N-glycans at the fifth potential N-glycosylation site of ATB200 are complex N-glycans, about 85% of the N-glycans at the sixth potential N-glycosylation site of ATB200 are sialylated (with nearly 27% fully sialylated) and about 98% of the N-glycans at the sixth potential N-glycosylation site of ATB200 are complex N-glycans, and about 87% of the N-glycans at the seventh potential N-glycosylation site of ATB200 are sialylated (with nearly 8% fully sialylated) and about 100% of the N-glycans at the seventh potential N-glycosylation site of ATB200 are complex N-glycans.

### Example 4: Analytical Comparison of ATB200 and MYOZYME^{®}/ LUMIZYME^{®}

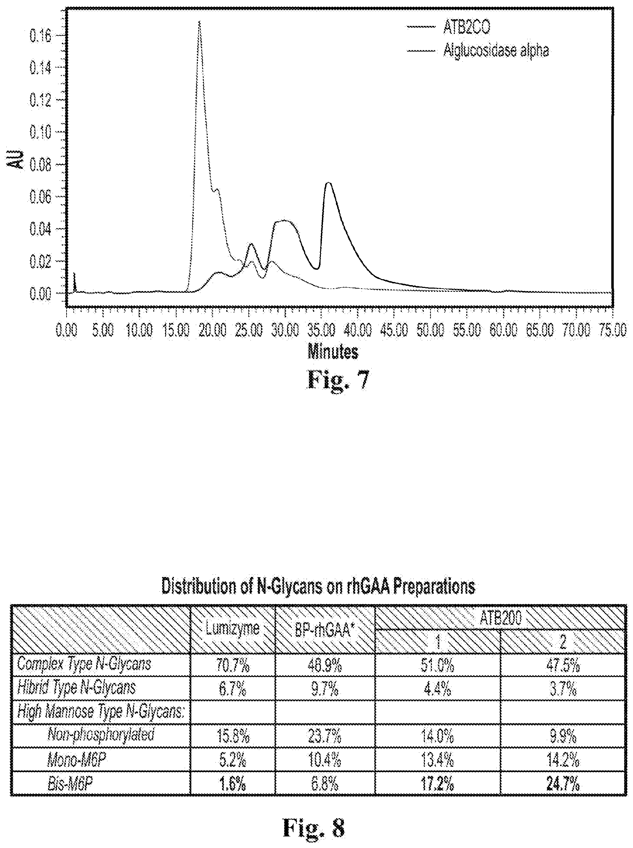

Purified ATB200 and LUMIZYME^{®} N-glycans were evaluated by MALDI-TOF to determine the individual N-glycan structures found on each ERT. LUMIZYME^{®} was obtained from a commercial source. As shown in Fig. 7, ATB200 exhibited four prominent peaks eluting to the right of LUMIZYME^{®}. This confirms that ATB200 was phosphorylated to a greater extent than LUMIZYME^{®} since this evaluation is by terminal charge rather than CIMPR affinity. As summarized in Fig. 8, ATB200 samples were found to contain lower amounts of non-phosphorylated high-mannose type N-glycans than LUMIZYME^{®}.

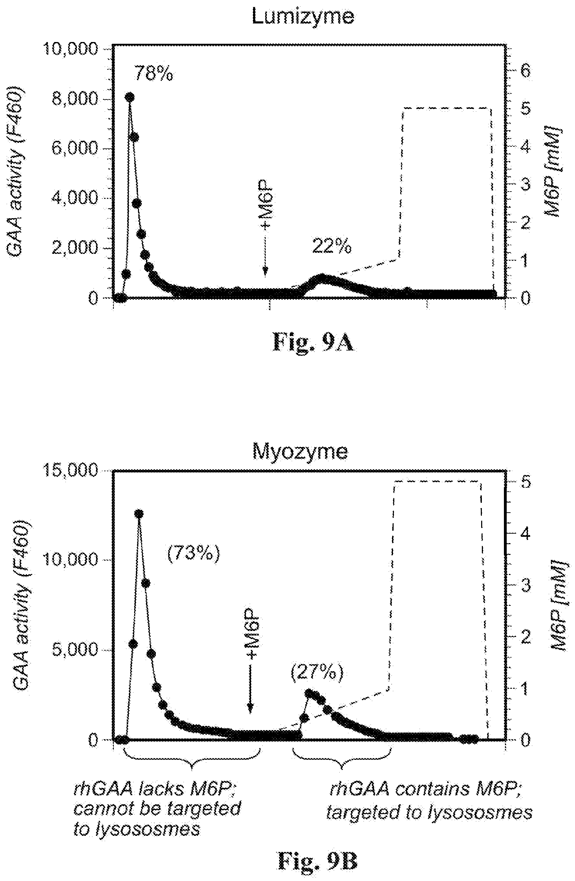

To evaluate the ability of the conventional rhGAAs in MYOZYME^{®} and LUMIZYME^{®} to interact with the CIMPR, the two conventional rhGAA preparations were injected onto a CIMPR affinity column (which binds rhGAA having M6P groups) and the flow through collected. The bound material was eluted with a free M6 gradient. Fractions were collected in 96-well plate and GAA activity assayed by 4MU-α-glucosidase substrate. The relative amounts of unbound (flow through) and bound (M6P eluted) rhGAA were determined based on GAA activity and reported as the fraction of total enzyme. Figs. 9A and 9B show the binding profile of rhGAAs in MYOZYME^{®} and LUMIZYME^{®}: 73% of the rhGAA in MYOZYME^{®} (Fig. 9B) and 78% of the rhGAA in LUMIZYME^{®} (Fig. 9A) did not bind to the CIMPR. Indeed, only 27% of the rhGAA in MYOZYME^{®} and 22% of the rhGAA in LUMIZYME^{®} contained M6P that can be productive to target it to the CIMPR on muscle cells. In contrast, as shown in Fig. 5, under the same condition, more than 70% of the rhGAA in ATB200 was found to bind to the CIMPR.

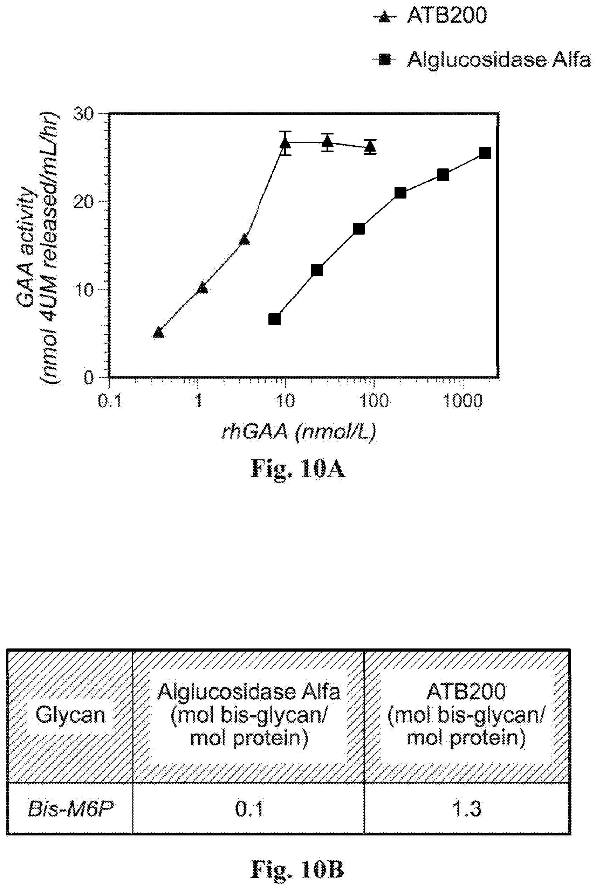

In addition to having a greater percentage of rhGAA that can bind to the CIMPR, it is important to understand the quality of that interaction. LUMIZYME^{®} and ATB200 receptor binding was determined using a CIMPR plate binding assay. Briefly, CIMPR-coated plates were used to capture GAA. Varying concentrations of rhGAA were applied to the immobilized receptor and unbound rhGAA was washed off. The amount of remaining rhGAA was determined by GAA activity. As shown in Fig. 10, ATB200 bound to CIMPR significantly better than LUMIZYME^{®}.

Overall, the higher content of M6P N-glycans in ATB200 than in LUMIZYME^{®} indicates that the higher portion of rhGAA molecules in ATB200 can target muscle cells. As shown above, the high percentage of mono-phosphorylated and bis-phosphorylated structures determined by MALDI agree with the CIMPR profiles which illustrated significantly greater binding of ATB200 to the CIMPR receptor. N-glycan analysis via MALDI-TOF mass spectrometry confirmed that on average each ATB200 molecule contains at least one natural bis-M6P N-glycan structure. This higher bis-M6P N-glycan content on ATB200 directly correlated with high-affinity binding to CIMPR in M6P receptor plate binding assays (K_{D} about 2-4 nM).

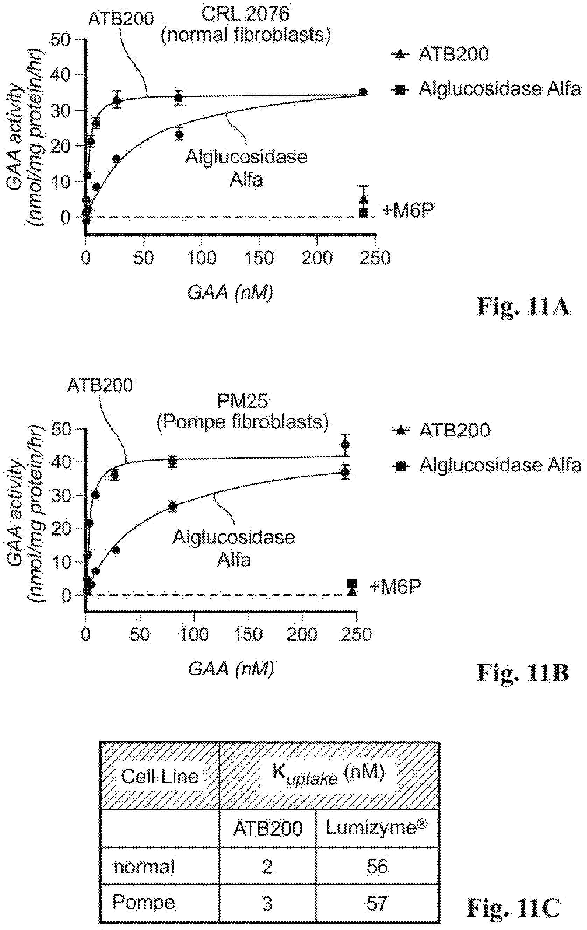

The relative cellular uptake of ATB200 and LUMIZYME^{®} rhGAA were compared using normal and Pompe fibroblast cell lines. Comparisons involved 5-100 nM of ATB200 according to the disclosure with 10-500 nM conventional rhGAA product LUMIZYME^{®}. After 16-hr incubation, external rhGAA was inactivated with TRIS base and cells were washed 3-times with PBS prior to harvest. Internalized GAA measured by 4MU-α-Glucoside hydrolysis and was graphed relative to total cellular protein and the results appear in Figs. 11A-11C.

ATB200 was also shown to be efficiently internalized into cells. As depicted in Figs. 11A-11B, ATB200 is internalized into both normal and Pompe fibroblast cells and is internalized to a greater degree than the conventional rhGAA product LUMIZYME^{®}. ATB200 saturates cellular receptors at about 20 nM, while about 250 nM of LUMIZYME^{®} is needed to saturate cellular receptors. The uptake efficiency constant (Kᵤₚₜₐₖₑ) extrapolated from these results is 2-3 nm for ATB200 and 56 nM for LUMIZYME^{®}, as shown by Fig. 11C. These results suggest that ATB200 is a well-targeted treatment for Pompe disease.

### Example 5: ATB200 and Enzyme Stabilizer

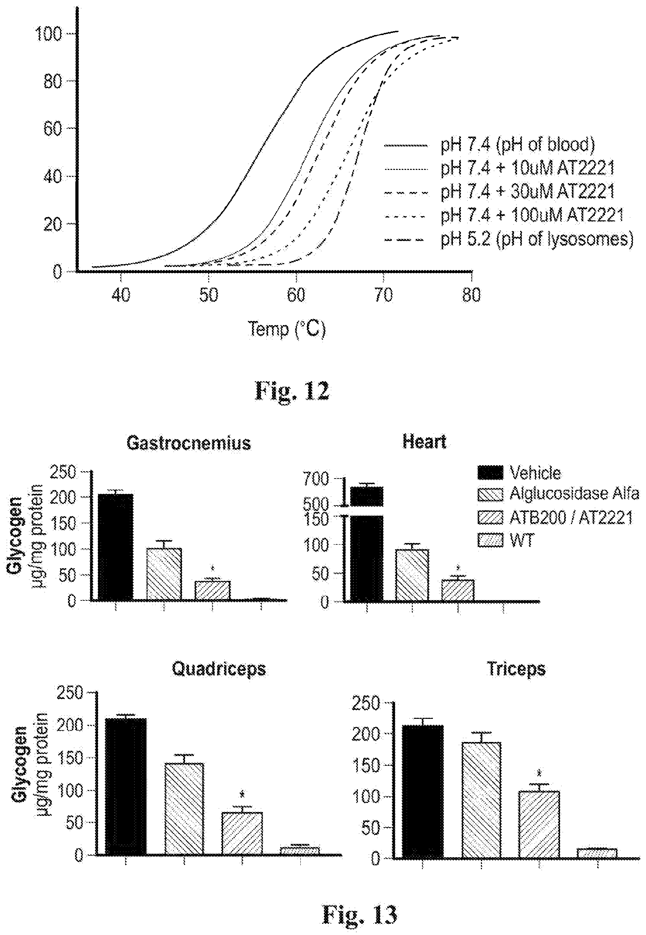

The stability of ATB200 in acidic or neutral pH buffers was evaluated in a thermostability assay using SYPRO Orange, as the fluorescence of the dye increases when proteins denature. As shown in Fig. 12, the addition of miglustat stabilized ATB200 at pH 7.4 in a concentration-dependent manner, comparable to the stability of ATB200 at pH 5.2, a condition that mimics the acidic environment of the lysosome. As summarized in Table 10, the addition of miglustat increased the melting temperature (*Tₘ*) of ATB200 by nearly 10°C.

**Table 10. Stability of ATB200 with miglustat**

| **Test Condition** | **Tm (°C)** |
|---|---|
| pH 7.4 | 56.2 |
| pH 7.4 + 10 µM miglustat | 61.6 |
| pH 7.4 + 30 µM miglustat | 62.9 |
| pH 7.4 + 100 µM miglustat | 66.0 |
| pH 5.2 | 67.3 |

### Example 6: Co-administration of ATB200 and miglustat in Gaa KO Mice

The therapeutic effects of ATB200 and miglustat were evaluated and compared against those of Alglucosidase alfa in *Gaa* KO mice. For the study, male *Gaa* KO (3- to 4-month old) and age-matched wild-type (WT) mice were used. Alglucosidase alfa was administered via bolus tail vein intravenous (IV) injection. In the co-administration regimen, miglustat was administered via oral gavage (PO) 30 minutes prior to the IV injection of ATB200. Treatment was given biweekly. Treated mice were sacrificed after 14 days from the last administration and various tissues were collected for further analysis. Table 11 summarizes the study design:

**Table 11. Co-administration Study Design**

| **Genotype** | **Treatment** | **Drug Dosage per Administration (bi-weekly)** | **Number of Administration** |
|---|---|---|---|
| *Gaa KO* | Vehicle | N/A | 6 |
| *Gaa KO* | Alglucosidase alfa | 20 mg/kg | 6 |
| *Gaa KO* | ATB200/miglustat | 20 mg/kg (ATB200) | 6 |
| | | 10 mg/kg (miglustat) | |
| WT (Sve 129) | Not Treated | N/A | N/A |

Tissue glycogen content in tissues samples was determined using amyloglucosidase digestion, as discussed above. As shown in Fig. 13, a combination of 20 mg/kg ATB200 and 10 mg/kg miglustat significantly decreased the glycogen content in four different tissues (quadriceps, triceps, gastrocnemius, and heart) as compared to the same dosage of alglucosidase alfa.

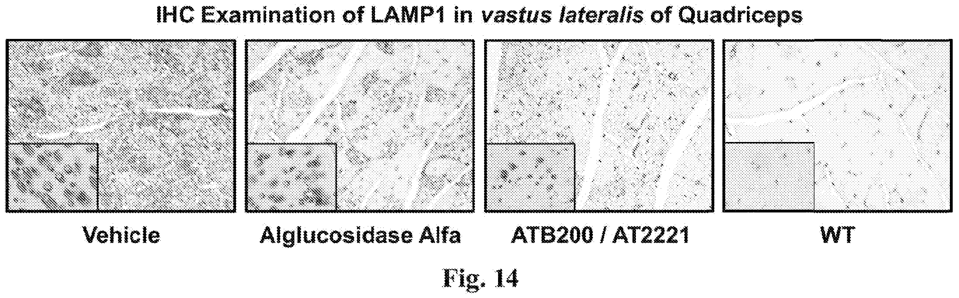

Tissue samples were also analyzed for biomarker changes following the methods discussed in: Khanna R, et al. (2012), "The pharmacological chaperone AT2220 increases recombinant human acid α-glucosidase uptake and glycogen reduction in a mouse model of Pompe disease," Plos One 7(7): e40776; and Khanna, R et al. (2014), "The Pharmacological Chaperone AT2220 Increases the Specific Activity and Lysosomal Delivery of Mutant Acid α-Glucosidase, and Promotes Glycogen Reduction in a Transgenic Mouse Model of Pompe Disease," PLoS ONE 9(7): e102092. As shown in Fig. 14, a profound increase in and enlargement of LAMP1-positive vesicles was seen in muscle fibers of *Gaa* KO animals compared to WT, indicative of lysosomal proliferation. Co-administration of ATB200 / miglustat led to more fibers with normalized LAMP1 level, while the remaining LAMP1-positive vesicles also reduced in size (insets).

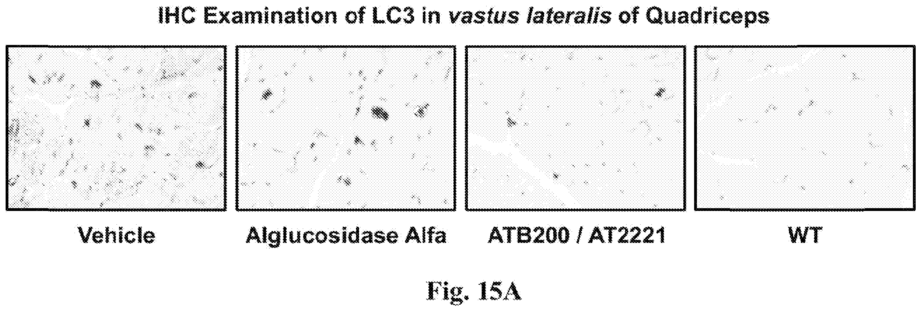

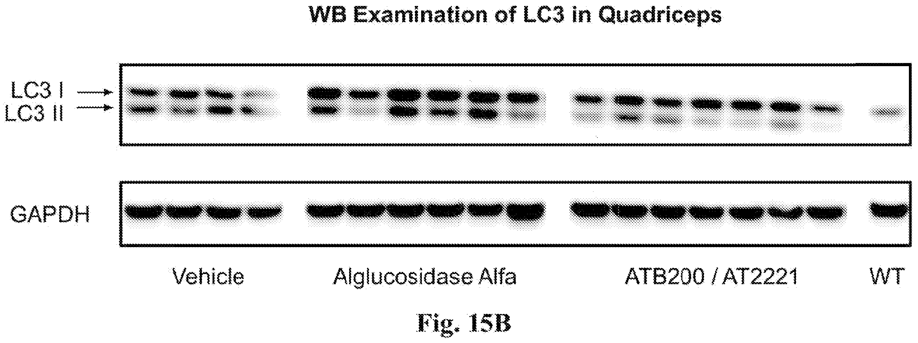

Similarly, intense LC3-positive aggregates in the muscle fibers of untreated *Gaa* KO mice signify the presence of autophagic zones and autophagy build-up. LC3-positive aggregates (red) were preferentially reduced in mice treated with ATB200 / miglustat co-administration as compared to mice treated with alglucosidase alfa (Fig. 15A). A similar observation was made when the expression of LC3 was assessed using western blot. As shown in Fig. 15B, the majority of animals treated with ATB200 / miglustat showed a significant decrease in levels of LC3 II, the lipidated form that is associated with autophagosomes, suggesting an improved autophagy flux. In comparison, the effect of alglucosidase alfa on autophagy was modest.

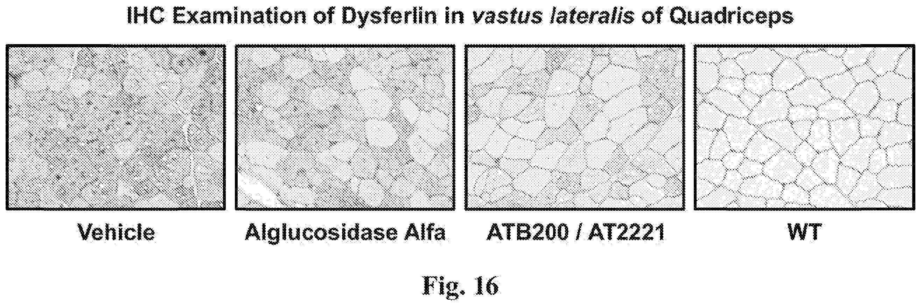

Dysferlin, a protein involved in membrane repair and whose deficiency/mistrafficking is associated with a number of muscular dystrophies, was also assessed. As shown in Fig. 16, dysferlin (brown) was heavily accumulated in the sarcoplasm of *Gaa* KO mice. Compared to alglucosidase alfa, ATB200 / miglustat was able to restore dysferlin to the sarcolemma in a greater number of muscle fibers.

These data are consistent with improvements at the cellular level demonstrated in human Pompe disease patients treated with ATB200 and miglustat, (e.g., the patients exhibit reduced levels of biomarkers of glycogen accumulation and muscle injury), leading not only to effective treatment of Pompe disease but also a reversal in disease progression. Clinical data in human Pompe disease patients are summarized in Examples 8 and 9, below.

### Example 7: Single Fiber Analysis

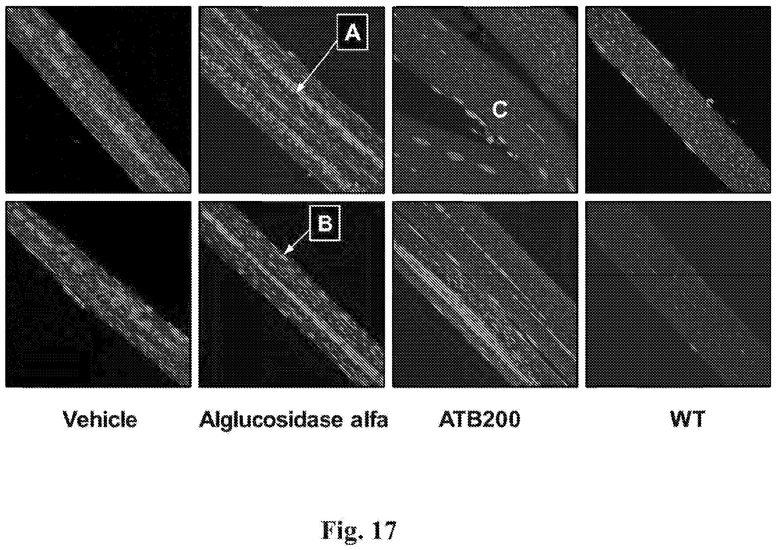

As shown in Fig. 17, majority of the vehicle-treated mice showed grossly enlarged lysosomes (see, for example "B") and the presence of massive autophagic buildup (see, for example "A"). MYOZYME^{®}-treated mice did not show any significant difference as compared to vehicle-treated mice. In contrast, most fibers isolated from mice treated with ATB200 showed dramatically decreased lysosome size (see, for example, "C"). Furthermore, the area with autophagic buildup was also reduced to various degrees (see, for example, "C"). As a result, a significant portion of muscle fibers analyzed (36-60%) from ATB200-treated mice appeared normal or near-normal. Table 12 below summarizes the single fiber analysis shown in Fig. 17.

**Table 12. Single Fiber Analysis**

| **Treatment** | **Animal Analyzed** | **Total Number of Fibers Analyzed (n)** | **Lysosome Enlargement** | **Fibers with Autophagy Buildup** | **Fibers with Normal or Near-normal Appearance** |
|---|---|---|---|---|---|
| WT | 2 | 65 | - | - | 100% |
| Vehicle | 2 | 65 | + | >90% | <10% |
| Alglucosidase alfa | 4 | 150 | + | >90% | <10% |
| ATB200 | 5 | 188 | Dramatic size decrease in most fibers | 40-64%* | 36-60% |

| | | | | | |
|---|---|---|---|---|---|
| * This included fibers with varying degree of reduction in autophagic buildup. Overall, the extent of the buildup was smaller in ATB200-treated group compared to Vehicle- or alglucosidase alfa-treated group. | | | | | |

In a separate study shown in Fig. 78, the administration of 12 biweekly bolus injections of ATB200 (20 mg/kg) to Gaa KO mice showed significantly improved muscle fiber size as was quantified from histological sections of the quadricceps compared to mice treated with alglucosidase alfa (20 mg/kg). Gaa mice, like patients with Pompe disease, show a decrease in muscle fiber size as a consequence of muscle atrophy. The significantly greater mean minimum Feret's diameter of ATB200-treated mice versus alglucosidase alfa-treated mice demonstrates a clear functional difference in efficacy.

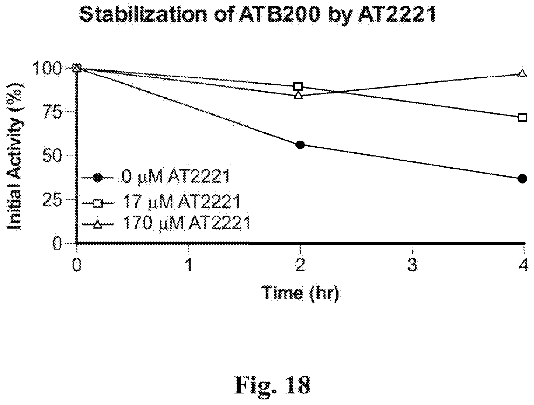

Overall, the data indicate that ATB200, with its higher M6P content, both alone and further stabilized by the enzyme stabilizer miglustat at the neutral pH of blood, is more efficient in tissue targeting and lysosomal trafficking compared to alglucosidase alfa when administered to *Gaa* KO mice, consistent with the stabilization of ATB200 by miglustat as depicted in Fig. 18. As a result, administration of ATB200 and co-administration of ATB200/miglustat was more effective than alglucosidase alfa in correcting some of the disease-relevant pathologies, such as glycogen accumulation, lysosomal proliferation, and formation of autophagic zones. Due to these positive therapeutic effects, administration of ATB200 and ATB200/miglustat co-administration is shown to improve the chance of muscle fiber recovery from damage and even to reverse damage by clearing glycogen that had accumulated in the cell due to lack of optimal GAA activity. As with Example 6, these data are also consistent with improvements at the cellular level demonstrated in human Pompe disease patients that lead to both effective treatment of Pompe disease and reversal in disease progression following administration of ATB200 and miglustat. Clinical data in human Pompe disease patients are summarized in Examples 8 and 9, below.

### Example 8: The ATB200-02 Trial

A phase 1/2 (ATB200-02, NCT-02675465) open-label, fixed-sequence, ascending-dose clinical study was conducted to assess safety, tolerability, pharmacokinetics, pharmacodynamics, and interim efficacy of IV infusion of ATB200 with miglustat in adult subjects with Pompe disease. The data was reported in International Publication No. WO 2020/163480, the disclosure of which is herein incorporated by reference.

### Example 9: The ATB200-03 Trial: a phase 3 in-human study of ATB200/miglustat in patients with Pompe disease

The ATB200-03 trial was a phase 3 double-blind, randomized, multicenter, international study of ATB200/miglustat in adult subjects with late-onset Pompe disease (LOPD) who had received enzyme replacement therapy with alglucosidase alfa (i.e., ERT-experienced) or who had never received ERT (i.e., ERT naive), compared with alglucosidase alfa/placebo.

### Study Design

As shown in Fig. 21, the trial consisted of a screening period up to 30 days, a 12-month treatment period, and a 30-day safety follow-up period. Eligible subjects were randomly assigned in a 2:1 ratio to receive ATB200/miglustat or alglucosidase alfa/placebo and stratified by ERT status (ERT-experienced, ERT-naïve) and baseline 6-minute walk distance (6MWD) (75 to < 150 meters, 150 to < 400 meters, ≥ 400 meters).

Efficacy assessments (i.e., functional assessments) included evaluation of ambulatory function (6MWT), motor function tests (Gait, Stair, Gower, and Chair maneuver (GSGC) test and Timed Up and Go (TUG) test), muscle strength (manual muscle testing and quantitative muscle testing), and pulmonary function tests (FVC, SVC, MIP, MEP, and SNIP). Patient reported outcomes (Rasch-built Pompe-specific Activity (R Pact) Scale, EuroQol 5 Dimensions 5 Levels Instrument (EQ-5D-5L), Patient-Reported Outcomes Measurement Information System (PROMIS^{®}) instruments for physical function, fatigue, dyspnea, and upper extremity, and Subject's Global Impression of Change) were recorded. The Physician's Global Impression of Change were also performed.

Pharmacodynamic assessments included measurement of biomarkers of muscle injury (creatine kinase (CK) and disease substrate (urinary hexose tetrasaccharide (Hex4)). Sparse blood samples were collected for determination of total GAA protein levels and miglustat concentrations in plasma for a population PK analysis in ERT-experienced subjects. Serial blood sampling for characterization of the PK profile of total GAA protein and miglustat were done in ERT-naïve subjects.

Safety assessments included monitoring of adverse events (AEs), including infusion associated reactions (IARs), clinical laboratory tests (chemistry, hematology, and urinalysis), vital signs, physical examinations including weight, electrocardiograms (ECGs), and immunogenicity. Concomitant medications and nondrug therapies were also be recorded.

### Subject Selection

Subjects who participated in the study met all of the following inclusion criteria and none of the exclusion criteria. In total, 122 subjects participated in the ATB200-03 trial. Among them, 85 subjects (ERT-experienced: 65; ERT-naïve: 20) received the ATB200/miglustat treatment, and 37 subjects (ERT-experienced: 30; ERT-naïve: 7) received the alglucosidase alfa/placebo treatment. As shown in Fig. 22, the baseline 6MWD and FVC data was representative of the population and generally similar in the two treatment arms.

### Inclusion Criteria:

1. Subject provided signed informed consent prior to any study-related procedures being performed.
2. Male and female subjects were ≥ 18 years old and weighed ≥ 40 kg at screening.
3. Female subjects of childbearing potential and male subjects agreed to use medically accepted methods of contraception during the study and for 90 days after the last dose of study drug.
4. Subject had a diagnosis of LOPD based on documentation of one of the following:
   a. deficiency of GAA enzyme
   b. GAA genotyping
5. Subject was classified as one of the following with respect to ERT status:
   a. ERT-experienced, defined as had received standard of care ERT (alglucosidase alfa) at the recommended dose and regimen (ie, 20 mg/kg dose every 2 weeks) for ≥ 24 months
      - Specific to Australia, ERT-experienced, defined as had received standard of care ERT (alglucosidase alfa) at the recommended dose and regimen, at a dose of 20 mg/kg based on lean or ideal body weight every 2 weeks
   b. ERT-naïve, defined as never had received investigational or commercially available ERT
6. Subject had a sitting FVC ≥ 30% of the predicted value for healthy adults (National Health and Nutrition Examination Survey III) at screening.
7. Subject performed two 6MWTs at screening that were valid, as determined by the clinical evaluator, and that met all of the following criteria:
   a. both screening values of 6MWD were ≥ 75 meters
   b. both screening values of 6MWD were ≤ 90% of the predicted value for healthy adults
   c. the lower value of 6MWD was within 20% of the higher value of 6MWD

### Exclusion Criteria:

1. Subject had received any investigational therapy or pharmacological treatment for Pompe disease, other than alglucosidase alfa, within 30 days or 5 half-lives of the therapy or treatment, whichever was longer, before Day 1 or was anticipated to do so during the study.
2. Subject had received gene therapy for Pompe disease.
3. Subject was taking any of the following prohibited medications within 30 days before Day 1:
   - miglitol
   - miglustat
   - acarbose
   - voglibose
4. Subject required the use of invasive or noninvasive ventilation support for > 6 hours per day while awake.
5. Subject had a hypersensitivity to any of the excipients in ATB200, alglucosidase alfa, or miglustat.
6. Subject had a medical condition or any other extenuating circumstance that, in the opinion of the investigator or medical monitor, posed an undue safety risk to the subject or compromised his/her ability to comply with or adversely impact protocol requirements. This included clinical depression (as diagnosed by a psychiatrist or other mental health professional) with uncontrolled or poorly controlled symptoms.
7. Subject, if female, was pregnant or breastfeeding at screening.
8. Subject, whether male or female, was planning to conceive a child during the study.
9. Subject refused to undergo genetic testing.

### Investigational Product, Dosage, and Mode of Administration

Subjects were randomized with a randomization ratio of at least 2:1 to receive either ATB200/miglustat or alglucosidase alfa/placebo. Table 13 below summarizes the treatment of the enrolled subjects.

**Table 13. Treatment Assignment and Regimen**

| **Treatment Assignment** | **Treatment** | **Regimen** |
|---|---|---|
| ATB200/miglustat | miglustat^{a} | Subjects ≥ 50 kg: 260 mg (4 × 65-mg oral capsules) 1 hour prior to ATB200 infusion every 2 weeks |
| | | Subjects ≥ 40 kg to < 50 kg: 195 mg (3 × 65-mg oral capsules) 1 hour prior to ATB200 infusion every 2 weeks |
| | ATB200 | 20 mg/kg IV infusion over a 4-hour duration every 2 weeks |
| Alglucosidase alfa/placebo | Placebo | Subjects ≥ 50 kg: Placebo (4 oral capsules) 1 hour prior to alglucosidase alfa infusion every 2 weeks |
| | | Subjects ≥ 40 kg to < 50 kg: Placebo (3 oral capsules) 1 hour prior to alglucosidase alfa infusion every 2 weeks |
| | Alglucosidase alfa | 20 mg/kg IV infusion over a 4-hour duration every 2 weeks |

| | | |
|---|---|---|
| Abbreviations: IV = intravenous ^{a}Note: Subjects were required to fast at least 2 hours before and 2 hours after administration of miglustat or placebo. | | |

### Data Evaluation and Statistical Considerations

The primary efficacy endpoint was the change from baseline to Week 52 in 6MWD. The primary endpoint was tested for superiority of ATB200/miglustat vs Alglucosidase alfa/placebo, using mixed-effect model for repeated measures (MMRM) and pre-specified nonparametric test in case of violation of normality.

Key secondary efficacy endpoints in a pre-specified hierarchical order of importance were as follows. These secondary endpoints were analyzed using analysis of covariance (ANCOVA) model with last observation carried forward (ITT LOCF).
- change from baseline to Week 52 in sitting FVC (% predicted)
- change from baseline to Week 52 in the manual muscle test score for the lower extremities
- change from baseline to Week 52 in the total score for the PROMIS - physical function
- change from baseline to Week 52 in the total score for the PROMIS - fatigue
- change from baseline to Week 52 in GSGC total score

Other secondary efficacy endpoints were as follows:
- change from baseline to Week 52 in the following variables related to motor function:
   - time to complete the 10-meter walk (ie, assessment of gait) of the GSGC test
   - time to complete the 4-stair climb of the GSGC test
   - time to complete the Gower's maneuver of the GSGC test
   - time to arise from a chair as part of the GSGC test
   - time to complete the TUG test
- change from baseline to Week 52 in the following variables related to muscle strength:
   - manual muscle test score for the upper extremities
   - manual muscle test total score
   - quantitative muscle test value (kg) for the upper extremities
   - quantitative muscle test value (kg) for the lower extremities
   - quantitative muscle test total value (kg)
- change from baseline to Week 52 in the following variables from patient-reported outcome measures:
   - total score for the PROMIS - dyspnea
   - total score for the PROMIS - upper extremity
   - R-PAct Scale total score
   - EQ-5D-5L health status
- actual value of the subject's functional status (improving, stable, or declining) pertaining to the effects of study drug in the following areas of life at Week 52, as measured by the Subject's Global Impression of Change
   - overall physical wellbeing
   - effort of breathing
   - muscle strength
   - muscle function
   - ability to move around
   - activities of daily living
   - energy level
   - level of muscular pain
- actual value of the subject's functional status (improving, stable, or declining) at Week 52, as measured by the Physician's Global Impression of Change
- change from baseline to Week 52 in the following measures of pulmonary function, as follows:
   - sitting FVC (% predicted)
   - MIP (cmH2O)
   - MIP (% predicted)
   - MEP (cmH2O)
   - MEP (% predicted)
   - SNIP (cmH2O)

Pharmacodynamic endpoints were as follows:
- change from baseline to Week 52 in serum CK level
- change from baseline to Week 52 in urinary Hex4 level

For ERT-experienced subjects, pharmacokinetic endpoints from a population PK analysis of total GAA protein level and miglustat concentration were collected. For ERT-naïve subjects, PK parameters for plasma total GAA protein concentration and miglustat were calculated.

The safety profile of ATB200/miglustat was characterized using incidence of treatment emergent adverse events (TEAEs), serious adverse events (SAEs), and AEs leading to discontinuation of study drug, frequency and severity of immediate and late IARs, and any abnormalities noted in other safety assessments. The impact of immunogenicity to ATB200 and alglucosidase alfa on safety and efficacy was also assessed.

Statistical methods included the following considerations on sample randomization, sample size calculation, efficacy analyses, and safety analyses.

Randomization. The following two factors were identified as design stratification variables: 1. baseline 6MWD (75 to < 150 meters, 150 to < 400 meters, ≥ 400 meters); and 2. ERT status (ERT-experienced, ERT-naïve). These two factors formed six factorial combinations (i.e., levels, strata). A centralized block randomization procedure was used to balance the above risk factors, 1) to reduce bias and increase the precision of statistical inference, and 2) to allow various planned and unplanned subset analyses. The block randomization scheme was performed for each of the 6 strata. The randomization ratio is 2:1 ATB200/miglustat to alglucosidase alfa/placebo, fixed.

Sample Size Calculation. A 2-group t-test with a 2-sided significance level of 0.05 and a 2:1 randomization scheme (66 subjects in the ATB200/miglustat group and 33 subjects in the alglucosidase alfa/placebo group, for a total sample size of 99 subjects) was determined to have approximately 90% power to detect a standardized effect size of 0.7 between the 2 groups in a superiority test. This calculation was performed using Nquery 8^{©®}. Assuming a 10% dropout rate, the sample size would be approximately 110 subjects.

Efficacy Analyses. The primary efficacy endpoint (i.e., change from baseline to Week 52 in 6MWD) was analyzed using a parametric analysis of covariance (ANCOVA) model to compare between the new treatment and the control. This model would typically adjust for baseline 6MWD (as a continuous covariate), and the 2 factors used to stratify randomization: ERT status (ERT naive vs. ERT-experienced) and baseline 6MWD (75 to < 150 meters, 150 to < 400 meters, ≥ 400 meters). However, the baseline 6MWD could not be used in the model twice (both as a continuous and a categorical variable) due to the expected high point biserial correlation between them. Thus, the 6MWD continuous variable remained in the model but the categorical 6MWD was removed. The ANCOVA model then had terms for treatment, baseline 6MWD (continuous), and ERT status (categorical).

Additionally, potential treatment-by-covariate interactions (i.e., treatment by ERT status and treatment-by-baseline 6MWD continuous) were examined. If an interaction term was statistically significant (e.g., p < 0.10, 2 sided), and there was logical biological interpretation, then the interaction term could potentially be added in the final ANCOVA model that would be used for the primary endpoint analysis. The data was then be analyzed based on the ANCOVA model, and all the relevant estimates (e.g., LS means for each treatment group, LS means difference, 95% confidence intervals (CIs) for the LS mean difference, and p-value for comparing between the 2 treatment groups) were provided.

To support the interpretation of clinical benefit, a composite subject-level response was defined based on the totality of the treatment outcome data. Subjects were classified by an ordinal response variable consisting of significant improvement, moderate improvement, or minor/no improvement based on treatment outcomes.

Key secondary endpoints were analyzed according to the hierarchical order, using stepwise closed testing procedure to control the type I error rate. Key secondary and other secondary endpoints were analyzed separately with a similar method used for the primary endpoint analysis.

Safety Analyses. Safety data was summarized using counts and percentages for categorical data and descriptive statistics (mean, standard deviation, median, minimum, maximum) for continuous data.

### Efficacy Results from the ATB200-03 Trial

**In** the overall population, ATB200/miglustat treatment showed improvement in 6MWD and stabilization in percent-predicted FVC, relative to baseline at week 52 (Fig. 23A) and over time (Fig. 23B). Compared to alglucosidase alfa/placebo, ATB200/miglustat treatment showed greater improvement in 6MWD in the overall population at week 52 (Fig. 23A). Furthermore, as shown in Fig. 23A, ATB200/miglustat treatment showed clinically significant improvement in percent-predicted FVC in the overall population at week 52, compared to alglucosidase alfa/placebo.

In the ERT-experienced population, ATB200/miglustat treatment showed improvement in 6MWD and stabilization in percent-predicted FVC, relative to baseline at week 52 (Fig. 24). Compared to alglucosidase alfa/placebo, ATB200/miglustat treatment showed improvements over time in 6MWD and stabilization over time in percent-predicted FVC in the ERT-experienced population (Fig. 25). Furthermore, as shown in Fig. 24, ATB200/miglustat treatment showed clinically significant improvement in both 6MWD and percent-predicted FVC in the ERT-experienced population at week 52, compared to alglucosidase alfa/placebo.

As shown in Figs. 26A and 26B, in the smaller ERT-naive population (n=27), ATB200/miglustat treatment showed improvement in 6MWD and stabilization in percent-predicted FVC, relative to baseline at week 52 (Fig. 26A) and over time (Fig. 26B). Variability between the two treatment groups was greater and no clinically significant improvement was observed in 6MWD or percent-predicted FVC (Fig. 26A).

As shown in Fig. 28, in the overall population ERT-experienced populations, lower MMT numerically favored ATB200/miglustat treatment, compared to alglucosidase alfa/placebo.

The mean change (SD) in MMT score for lower extremities from baseline to week 52 showed a numerical improvement of 1.6 (3.78) in subjects treated with co-administration of cipaglucosidase alfa and miglustat compared to 0.9 (2.58) in those treated with alglucosidase alfa and placebo (p = 0.191).

As shown in Fig. 29, in the overall and ERT-experienced populations, ATB200/miglustat treatment showed clinically significant improvement in GSGC at week 52, compared to alglucosidase alfa/placebo.

The mean change in total GSGC score from baseline to week 52 showed an improvement of -0.53 (2.5) in subjects treated with co-administration of cipaglucosidase alfa and miglustat compared to 0.77 (1.8) in those treated with alglucosidase alfa and placebo (p = 0.009).

As shown in Fig. 30, in the overall and ERT-experienced populations, PROMIS physical function numerically favored ATB200/miglustat treatment, compared to alglucosidase alfa/placebo.

As shown in Fig. 31, in the overall and ERT experienced populations, PROMIS fatigue improved similarly between the two treatment groups.

### Biomarker Results from the ATB200-03 Trial

In the overall and ERT-experienced populations, ATB200/miglustat treatment showed improvement in biomarkers of muscle damage (CK) and disease substrate (Hex4) over time (Figs. 32 and 33). Furthermore, as shown in Fig. 32 and 33, in the overall and ERT-experienced populations, reductions in CK and urinary Hex4 were significantly greater with ATB200/miglustat treatment at week 52, compared to alglucosidase alfa/placebo.

In ERT-experienced patients, the baseline mean urinary Glc4 concentration was 4.6 mmol/mol and 7.2 mmol/mol in the ATB200/miglustat treatment and alglucosidase alfa with placebo treatment, respectively. At Week 52, the mean urinary Glc4 concentration was 2.9 mmol/mol and 9.1 mmol/mol in the ATB200/miglustat treatment and alglucosidase alfa with placebo treatment group, respectively.

As summarized in Fig. 34, in the overall and ERT-experienced populations, endpoints across motor function, pulmonary function, muscle strength, patient-reported outcomes (PROs) and biomarkers consistently favored ATB200/miglustat treatment over alglucosidase alfa/placebo. Furthermore, of the 17 efficacy and biomarker endpoints assessed, 16 favored ATB200/miglustat treatment over alglucosidase alfa/placebo.

### Safety Results from the ATB200-03 Trial

As shown in Fig. 35, the overall safety profile of ATB200/miglustat treatment group was similar to that of the alglucosidase alfa/placebo group.

Fig. 36 - Fig. 40 describe additional aspects of the ATB200-03 Trial.

### Example 10: Results of PROPEL Phase 3 Clinical Trials

AT-GAA showed clinically meaningful & significant improvements in both musculoskeletal and respiratory measures in late-onset Pompe disease compared to standard of care in pivotal phase 3 PROPEL study. PROPEL is also referred to as "ATB200-03", see Example 9.

Patients switching to AT-GAA from the approved standard of care ERT (alglucosidase alfa) walked on average 17 meters farther (p=0.046).

Patients switching to AT-GAA also showed an improvement in percent-predicted forced vital capacity (FVC), the most important measure of respiratory function in Pompe disease, compared to a decline in patients treated with alglucosidase alfa (FVC Diff. 4.1%; p=0.006).

AT-GAA showed a nominally statistically significant and clinically meaningful difference for superiority on the first key secondary endpoint of FVC compared to patients treated with alglucosidase alfa (FVC Diff. 3.0%; p=0.023).

In the combined study population of ERT switch and ERT naive patients, AT-GAA outperformed alglucosidase alfa by 14 meters (21m compared to 7m) on the primary endpoint and was not statistically significant for superiority (p=0.072).

Improvements in the two important biomarkers of Pompe disease (Hex-4 and CK) for the combined study population significantly favored AT-GAA compared to alglucosidase alfa (p<0.001).

PROPEL was a 52-week, double-blind randomized global study designed to assess the efficacy, safety and tolerability of AT-GAA compared to the current standard of care, alglucosidase alfa, an enzyme replacement therapy (ERT). The study enrolled 123 adult Pompe patients who still had the ability to walk and to breathe without mechanical ventilation and was conducted at 62 clinical sites in 24 countries on 5 continents. It was the largest controlled clinical study ever conducted in a lysosomal disorder.

Patients enrolled in PROPEL were randomized 2:1 so that for every two patients randomized to be treated with AT-GAA, one was randomized to be treated with alglucosidase alfa. Of the Pompe patients enrolled in PROPEL, 77% were being treated with alglucosidase alfa (n=95) immediately prior to enrollment and 23% had never been treated with any ERT (n=28). 117 patients completed the PROPEL study and all 117 have voluntarily enrolled in the long-term extension study and are now being treated solely with AT-GAA for their Pompe disease.

### Pre-Specified Analyses of 6-Minute Walk Distance (6MWD) and Percent-Predicted Forced Vital Capacity (FVC) in the Combined ERT Switch and ERT Naive Study Population:

The primary endpoint of the study was the mean change in 6-minute walk distance as compared with baseline measurements at 52 weeks across the combined ERT switch and ERT naive patient populations. In this combined population patients taking AT-GAA (n=85) walked on average 21 meters farther at 52 weeks compared to 7 meters with those treated with alglucosidase alfa (n=37) (Table 14). This primary endpoint in the combined population was assessed for superiority and while numerically greater, statistical significance for superiority on this combined population was not achieved for the AT-GAA arm as compared to the alglucosidase alfa arm (p=0.072).

Per the hierarchy of the statistical analysis plan, the first key secondary endpoint of the study was the mean change in percent-predicted FVC at 52 weeks across the combined population. In this combined population patients taking AT-GAA demonstrated a nominally statistically significant and clinically meaningful difference for superiority over those treated with alglucosidase alfa. AT-GAA significantly slowed the rate of respiratory decline in patients after 52 weeks. Patients treated with AT-GAA showed a 0.9% absolute decline in percent-predicted FVC compared to a 4.0% absolute decline in the alglucosidase alfa arm (p=0.023) (Table 15). Percent-predicted FVC is the most important measure of respiratory muscle function in Pompe disease and was the basis of approval for alglucosidase alfa.

**Table 14. 6MWD (m) in the Overall ERT Switch and ERT Naïve Study Population**

| **Treatment** | **Baseline** | **CFBL at Week 52** | **Difference** | **P-Value** |
|---|---|---|---|---|
| AT-GAA (n=85) | 357.9 (111.8) | +20.8 (4.6) | +13.6 (8.3) | p=0.072 |
| Alglucosidase alfa (n=37) | 351.0 (121.3) | +7.2 (6.6) | | |

**Table 15. FVC (% predicted) in the Overall ERT Switch and ERT Naïve Study Population**

| **Treatment** | **Baseline** | **CFBL at Week 52** | **Difference** | **P-Value** |
|---|---|---|---|---|
| AT-GAA (n=85) | 70.7 (19.6) | -0.9 (0.7) | +3.0 (1.2) | p=0.023 |
| Alglucosidase alfa (n=37) | 69.7 (21.5) | -4.0 (0.8) | | |

### Pre-Specified Analyses of 6-Minute Walk Distance (6MWD) and Percent-Predicted Forced Vital Capacity (FVC) in the ERT Switch Study Population (n=95):

The PROPEL switch patients entered the study having been treated with alglucosidase alfa for a minimum of two years. More than two thirds (67%+) of those patients had been on ERT treatment for more than five years prior to entering the PROPEL study (mean of 7.4 years).

A pre-specified analysis of the patients switching from alglucosidase alfa on 6-minute walk distance showed that after 52 weeks from switching, AT-GAA treated patients (n=65) walked 16.9 meters farther than their baseline, compared to 0.0 meters for those patients who were randomized to remain on alglucosidase alfa (n=30) (p=0.046) (Table 16).

A pre-specified analysis of the patients switching from alglucosidase alfa on percent-predicted FVC showed that AT-GAA treated patients stabilized and slightly improved their respiratory function on this important measure while those patients remaining on alglucosidase alfa continued to significantly decline in respiratory muscle function. AT-GAA patients showed a 0.1% absolute increase in percent-predicted FVC while the alglucosidase alfa patients showed a 4.0% absolute decline over the course of the year (p=0.006) (Table 17).

**Table 16. 6MWD (m) in the ERT Switch Study Population**

| **Treatment** | **Baseline** | **CFBL at Week 52** | **Difference** | **P-Value** |
|---|---|---|---|---|
| AT-GAA (n=65) | 346.9 (110.2) | +16.9 (5.0) | +16.9 (8.8) | p=0.046 |
| Alglucosidase alfa (n=30) | 334.6 (114.0) | 0.0 (7.2) | | |

**Table 17. FVC (% predicted) in the ERT Switch Study Population**

| **Treatment** | **Baseline** | **CFBL at Week 52** | **Difference** | **P-Value** |
|---|---|---|---|---|
| AT-GAA (n=65) | 67.9 (19.1) | +0.1 (0.7) | +4.1 (1.2) | p=0.006 |
| Alglucosidase alfa (n=30) | 67.5 (21.0) | -4.0 (0.9) | | |

### Pre-Specified Analyses of 6-Minute Walk Distance (6MWD) and Percent-Predicted Forced Vital Capacity (FVC) in the ERT Treatment Naive Population (n=28):

A pre-specified analysis of the patients previously never treated with any ERT on 6-minute walk distance showed that after 52 weeks AT-GAA treated patients (n=20) walked 33 meters farther than their baseline. The alglucosidase alfa treated patients (n=7) walked 38 meters further than their baseline. The difference between the two groups was not statistically significant (p=0.60) (Table 18).

A pre-specified analysis of the patients never previously treated with any ERT showed similar declines in percent-predicted forced vital capacity (FVC) at 52 weeks of -4.1% for AT-GAA treated patients and -3.6% for alglucosidase alfa treated patients (Table 19). The difference between the two groups was not statistically significant (p=0.57).

**Table 18. 6MWD (m) in the ERT Treatment Naive Population**

| **Treatment** | **Baseline** | **CFBL at Week 52** | **Difference** | **P-Value** |
|---|---|---|---|---|
| AT-GAA (n=20) | 393.6 (112.4) | +33.4 (10.9) | -4.9 (19.7) | p=0.60 |
| Alglucosidase alfa (n=7) | 420.9 (135.7) | +38.3 (11.1) | | |

**Table 19. FVC (% predicted) in the ERT Treatment Naive Population**

| **Treatment** | **Baseline** | **CFBL at Week 52** | **Difference** | **P-Value** |
|---|---|---|---|---|
| AT-GAA (n=20) | 80.2 (18.7) | -4.1 (1.5) | -0.5 (2.7) | p=0.57 |
| Alglucosidase alfa (n=7) | 79.1 (22.6) | -3.6 (1.8) | | |

| | | | | |
|---|---|---|---|---|
| Note: One patient in the alglucosidase alfa arm was excluded from the study analysis due to use of an investigational anabolic like steroid that impacted his baseline performance. | | | | |

### Pre-Specified Analyses of Other Key Secondary and Biomarker Endpoints Across the Overall ERT Switch and ERT Naive Study Population:

### Musculoskeletal & Other Key Secondary Endpoints:

**GSGC (Gait, Stairs, Gower's Chair):** GSGC is an important and commonly used endpoint in Pompe Disease capturing strength, coordination and mobility. AT-GAA treated patients demonstrated statistically significant improvements on the scores in this important assessment, compared to a worsening for alglucosidase alfa treated patients in the overall population (p<0.05).

**Lower MMT (Manual Muscle Testing), PROMIS Physical Function:** On both of these validated measures of muscle strength and patient reported outcomes, AT-GAA treated patients improved numerically more than alglucosidase alfa treated patients, though the results were not statistically significant.

**PROMIS Fatigue:** Fatigue as measured by this scale slightly favored AT-GAA treated patients over alglucosidase alfa treated patients.

### Biomarkers of Treatment Effects on Disease:

**Urine Hex-4:** For the combined study population of both ERT switch and ERT naive patients, those patients receiving AT-GAA showed substantial improvements on this biomarker, with a mean reduction of Hex-4 of - 31.5% after 52 weeks compared to an increase of +11.0% (i.e., worsening) in Hex-4 in the alglucosidase alfa treated patients (p=<0.001). Urine Hex-4 is a common biomarker in Pompe disease and is used as an indirect measure of the degree of skeletal glycogen clearance in Pompe patients receiving ERT. Glycogen is the substrate that accumulates in the lysosomes of muscles of Pompe patients.

**CK (Creatine Kinase):** After 52 weeks, AT-GAA treated patients showed substantial improvements on this biomarker as well with a mean - 22.4% reduction in CK compared to an increase (i.e., worsening) of +15.6% in the alglucosidase alfa treated patients. (p<0.001). CK is an enzyme that leaks out of damaged muscle cells and is elevated in Pompe patients.

AT-GAA demonstrated a similar safety profile to alglucosidase alfa. Two patients receiving AT-GAA (2.4%) discontinued treatment due to an adverse event compared to one (2.6%) for alglucosidase alfa unrelated to treatment. Injection associated reactions (IARs) were reported in 25% of AT-GAA participants and 26% of alglucosidase alfa patients.

### Post hoc subgroup analyses:

**Baseline 6MWD and FVC categories:** ERT-naïve population (n=27): three patients had a baseline 6MWD of <300 m and three had a baseline FVC of <55%; analyses of CFBL were not performed in these subgroups owing to the small patient numbers. Baseline 6MWD ≥300 m: both the cipaglucosidase alfa/miglustat (AT-GAA) (n=18) and alglucosidase alfa/placebo (n=6) groups had similar improvements over time (mean [SE] CFBL to week 52: +34.4 [12.1] m and +30.8 [9.6] m, respectively). Baseline FVC ≥55%: both the cipaglucosidase alfa/miglustat (n=19) and alglucosidase alfa/placebo (n=5) groups declined over time (mean [SE] CFBL to week 52: -3.7 [1.5] % and -3.3 [2.6] %, respectively). Outcomes consistently favored cipaglucosidase alfa/miglustat in the overall and ERT-experienced populations in patients with baseline 6MWD of <300 m and ≥ 300 m, and FVC of <55% and ≥55%, as shown in Fig. 41.

In the overall study population including ERT-naïve and ERT-experienced patients, cipaglucosidase alfa/miglustat showed positive trends or clinically meaningful improvements on motor and respiratory functions compared with approved ERT, regardless of baseline 6MWD and % FVC assessments, and across both prespecified and post hoc subgroup analyses.

Cipaglucosidase alfa/miglustat demonstrated a similar safety profile to that of alglucosidase alfa/placebo (Fig. 42).

### About AT-GAA

AT-GAA is an investigational two-component therapy that consists of cipaglucosidase alfa (ATB200), a unique recombinant human acid alpha-glucosidase (rhGAA) enzyme with optimized carbohydrate structures, particularly bis-phosphorylated mannose-6 phosphate (bis-M6P) glycans, to enhance uptake into cells, administered in conjunction with miglustat (AT2221), a stabilizer of cipaglucosidase alfa. In preclinical studies, AT-GAA was associated with increased levels of the mature lysosomal form of GAA and reduced glycogen levels in muscle, alleviation of the autophagic defect and improvements in muscle strength.

### About Pompe Disease

Pompe disease is an inherited lysosomal disorder caused by deficiency of the enzyme acid alpha-glucosidase (GAA). Reduced or absent levels of GAA levels lead to accumulation of glycogen in cells, which is believed to result in the clinical manifestations of Pompe disease. The disease can be debilitating and is characterized by severe muscle weakness that worsens over time. Pompe disease ranges from a rapidly fatal infantile form with significant impacts to heart function to a more slowly progressive, late-onset form primarily affecting skeletal muscle. It is estimated that Pompe disease affects approximately 5,000 to 10,000 people worldwide.

### Example 11: Results of an open-label Phase I/II study (ATB200-02)

ATB200-02 (NCT02675465) is an open-label, Phase I/II clinical trial that aimed to evaluate the safety, tolerability, pharmacokinetics, pharmacodynamics, and efficacy of cipaglucosidase alfa/miglustat in adults with Pompe disease. Cipaglucosidase alfa/miglustat is an investigational, two-component therapy for late-onset Pompe disease (LOPD) comprised of intravenous cipaglucosidase alfa, a rhGAA, administered in conjunction with oral miglustat, an enzyme stabilizer.

FIG. 43 shows the study design for the Phase I/II ATB200-02 study. The study is conducted in 16 centers across 5 countries. Four cohorts of patients with Pompe disease were enrolled in the ATB200-02 study:
- Cohort 1: ERT-experienced, aged 18-65 years, with 2-6 years prior ERT with 20 mg/kg alglucosidase alfa every 2 weeks (n=11)
- Cohort 2: Non-ambulatory ERT-experienced, aged 18-65 years, with ≥2 years prior ERT with 20 mg/kg alglucosidase alfa every 2 weeks (n=6)
- Cohort 3: ERT-naïve, aged 18-65 years (n=6)
- Cohort 4: ERT-experienced, aged 18-75 years, with ≥7 years prior ERT with 20 mg/kg alglucosidase alfa every 2 weeks (n=6)

Eligible ambulatory patients had a 6-minute walk distance (6MWD) of at least 200 m (cohorts 1 and 3) or 75 m (cohort 4) and upright forced vital capacity (FVC) of 30-80% of predicted normal value.

The change from baseline (CFBL) in 6MWD, % predicted sitting FVC, manual muscle test (MMT) and biomarkers - urine glucose tetrasaccharide (Hex4) and serum creatine kinase (CK) - were assessed at regular intervals. Data with up to 36 months of follow-up in ERT-experienced and ERT-naïve patients is provided herein. FIG. 44 shows a summary of endpoints and cohorts reported. FIG. 45 shows the baseline characteristics and patient disposition. Due to the staggered timing of patient enrollment, the number of patients with data currently available decreases at later time-points in this ongoing study.

ERT-experienced patients showed durable mean improvements from baseline in 6MWD up to 48 months. After 12-, 24-, 36- and 48-months of follow-up, 6MWD improved numerically from baseline in 13/16, 9/13, 6/12 and 6/9 ERT-experienced patients, respectively (FIG. 46A). The mean increases were 33 meters (m) by month 12, 25 m by month 24, 9 m by month 36 and 20 m by month 48.

For ERT-experienced patients, change from baseline (CFBL) in FVC was generally stable for up to 48 months of follow-up. After 12-, 24-, 36- and 48-months of follow-up, FVC improved (>3%) or remained stable (±3%) from baseline in 9/16, 11/13, 8/10 and 4/6 patients, respectively (FIG. 47A). The mean changes were -1.2% by month 12, +1.0 % by month 24, - 0.3% by month 36 and +1.0% by month 48.

In ERT-experienced patients, mean change in MMT lower extremity score improved numerically from baseline and improvements were maintained for up to 48-months of follow-up (FIG. 48A). After 12, 24, 36 and 48 months of follow-up, MMT lower extremity score improved numerically from baseline in 14/15, 11/13, 10/10 and 8/8 ambulatory patients, respectively. The mean increases were 3.1 pts by month 12, 2.1 pts by month 24, 2.5 pts by month 36 and 3.5 pts by month 48.

ERT-naïve patients showed durable mean improvements from baseline in 6MWD up to 48 months. After 12-, 24-, 36- and 48-months of follow-up, 6MWD improved numerically from baseline in 6/6, 6/6, 4/5 and 4/4 ERT-naïve patients, respectively (FIG. 46B). The mean increases were 57 m by month 12, 54 m by month 24, 43m by month 36 and 52 by month 48.

In ERT-naïve patients, mean CFBL in FVC improved numerically from baseline for up to 48 months of follow-up. After 12-, 24- 36-, and 48-months of follow-up, FVC improved (>3%) or remained stable (±3%) from baseline in 5/6, 6/6, 5/5 and 4/4 patients, respectively (FIG. 47B). As indicated by the asterisk in FIG. 47B, one patient in the ERT-naïve cohort experienced a large drop in % predicted FVC at month 21, which returned to previous levels at the following visit (month 24). The mean changes were 3.2% by month 12, 4.7% by month 24, 6.2% by month 36 and 8.3% by month 48.

In ERT-naïve patients, mean change in MMT lower extremity score improved numerically from baseline and improvements were maintained for up to 48-months of follow-up (FIG. 48B). After 12, 24, 36 and 48 months of follow-up, MMT lower extremity score improved numerically from baseline in 4/5, 4/5, 4/4 and 3/4 ambulatory patients, respectively. The mean increases were 2.8 pts by month 12, 3.0 pts by month 24, 3.3 pts by month 36 and 1.0 pts by month 48.

During 48 months of follow-up, cipaglucosidase alfa/miglustat was generally associated with mean reductions from baseline in urine Hex4, with greater reductions in ERT-naïve patients. After 12-, 24-, 36- and 48-months of follow-up, Hex4 levels decreased numerically from baseline in 16/16, 11/14, 11/12 and 6/9 ERT-experienced patients, and in 5/6, 5/6, 4/5 and 4/5 ERT-naïve patients, respectively (FIG. 49A).

During 48 months of follow-up, cipaglucosidase alfa/miglustat was associated with either stable levels of, or mean reductions from baseline, in plasma CK, with greater reductions in ERT-naïve patients. After 12-, 24-, 36- and 48-months of follow-up, CK levels decreased numerically from baseline in 13/15, 14/15, 9/11 and 8/9 ERT-experienced patients, and in 6/6, 6/6, 5/5 and 4/5 ERT-naïve patients, respectively (FIG. 49B).

FIG. 50 shows a summary of treatment emergent adverse events (TEAEs) with onset date on or after first dose of study drug in the ATB200-02 study. Mean (SD) duration of treatment was 37.2 (14.48),19.9 (4.13) and 36.9 (12.14) months in cohorts 1 (prior ERT 2-6 years), 4 (prior ERT ≥7 years) and 3 (ERT naive), respectively. The most common TEAEs included fall, nasopharyngitis, arthralgia, headache and diarrhea; most TEAEs were mild or moderate in severity and did not lead to study withdrawal.

Results from up to 48-months of follow-up in ambulatory patients from the ATB200-02 study of cipaglucosidase alfa plus miglustat indicate the following. ERT-experienced patients had durable mean improvements from baseline in motor function that were sustained for up to 48 months of follow-up, while respiratory function was stable and maintained over the same period: an improvement relative to the expected decline in many patients receiving long-term ERT. ERT-naïve patients showed durable mean improvements from baseline in motor and respiratory function that were sustained for up to 48 months of follow-up. Mean levels of two biomarkers, Hex4 and CK, were either stable or decreased from baseline up to 48 months of follow-up, with decreases most notable in the ERT-naïve cohort. The safety profile of cipaglucosidase alfa plus miglustat was similar to that reported for alglucosidase alfa.

FIG. 55 shows a summary of endpoints and cohorts reported for Cohort 2 (non-ambulatory ERT-experienced patients). FIG. 56 shows the baseline characteristics and patient disposition for Cohort 2.

For these non-ambulatory ERT-experienced patients, change from baseline (CFBL) in FVC improved numerically from baseline and/or remained stable for up to 48 months of follow-up (FIG. 57). The mean changes were +2.5% by month 12, +2.0 % by month 24, -2.0% by month 36 and -1.0% by month 48.

In these non-ambulatory ERT-experienced patients, mean change in MMT total score (upper body) improved numerically from baseline and/or remained stable for up to 36-months of follow-up (FIG. 57). The mean changes were +1.3 pts by month 12, +2.0 pts by month 24 and -0.8 pts by month 36.

During 48 months of follow-up in these these non-ambulatory ERT-experienced patients, cipaglucosidase alfa/miglustat was generally associated with mean reductions from baseline in urine Hex4 and plasma CK (FIG. 57). After 12-, 24-, 36- and 48-months of follow-up, Hex4 levels decreased numerically from baseline with mean reductions of -15.6%, -34.1%, -36.5% and -4.0%, respectively. After 12-, 24-, 36- and 48-months of follow-up, plasma CK levels decreased numerically from baseline with mean reductions of -20.8%, -25.3%, -27.% and -23.7%, respectively.

FIG. 58 shows a summary of treatment emergent adverse events (TEAEs) with onset date on or after first dose of study drug or Cohort 2 (in non-ambulatory ERT-experienced patients) in the ATB200-02 study. Mean (SD) duration of treatment was 46.3 (22.86) months. The most common TEAEs included nasopharyngitis and diarrhea (both occurred in 3 patients); most TEAEs were mild or moderate in severity and did not lead to study withdrawal.

Results from up to 48-months of follow-up in non-ambulatory ERT-experienced patients from the ATB200-02 study of cipaglucosidase alfa plus miglustat indicate the following: These patients had durable mean improvements from baseline and/or stabilization in motor function and pulmonary function that were sustained for up to 48 months of follow-up: an improvement relative to the expected decline in many patients receiving long-term ERT. Mean levels of two biomarkers, Hex4 and CK, were either stable or decreased from baseline up to 48 months of follow-up. Cipaglucosidase alfa plus miglustat was generally well-tolerated in this patient group.

### Example 12: Long-term effects of ERT in Pompe disease patients

FIG. 51 shows a comparison of the long-term effects of cipaglucosidase alfa/miglustat and avalglucosidase alfa on change from baseline for 6MWD and percentage predicted FVC (sitting) in ERT-experienced subjects. FIG. 52 shows a comparison of the long-term effects of cipaglucosidase alfa/miglustat and avalglucosidase alfa on change from baseline for 6MWD and percentage predicted FVC (sitting) in ERT-naïve subjects. The comparison shows that ERT-experienced patients switching from alglucosidase alfa to avalglucosidase alfa continue to experience progressive loss of motor function as assessed by 6MWD, similar to the progression shown above for patients remaining on alglucosidase alfa long-term, whereas patients switching from alglucosidase alfa to cipaglucosiadase alfa/miglustat experience a categorical change showing progressive gains in motor function as assessed by 6MWD. Similar trends are observed for FVC, where patients switching to avalglucosidase alfa experience progressive loss of pulmonary function as assessed by FVC whereas patients switching from alglucosidase alfa to cipaglucosiadase alfa/miglustat experience a stability in pulmonary function as assessed by FVC.

FIG. 53A - FIG. 53B show the 6-minute walk test (6MWT) percentage predicted during treatment with alglucosidase alfa. FIG. 53B shows replotted data from FIG. 53A only from year 2 onward. FIG. 54 shows the FVC percentage predicted during treatment with alglucosidase alfa. The data from year 2 onward show the decline expected in ERT-experienced patients remaining on alglucosidase alfa.

### Example 13: Comparison of Alglucosidase Alfa (Alglu), Avalglucosidase Alfa (Aval) and Cipaglucosidase Alfa + Miglustat (Cipa+mig)

In the absence of head-to-head trials comparing Aval to Cipa+mig, an indirect treatment comparison (ITC) is a suitable approach to better understand clinical differentiation of the three treatments for LOPD. ITCs are widely requested by health technology assessment agencies (HTAs) to support comparative health economic evaluation.

An ITC providing relative effect estimates in the target population of interest (ie an LOPD population including a mix of ERT-naïve and ERT-experienced subjects as in the pivotal Phase III trial comparing Cipa+mig with Alglu [PROPEL]) was performed.

A systematic literature review (SLR) was conducted to identify relevant published clinical studies of ERTs in LOPD. Outcomes assessed were change from baseline in 6-minute walking distance (6MWD) (m) and in forced vital capacity (FVC; % predicted) at week 52, acknowledged by clinicians, HTA agencies and payers as key LOPD trial endpoints. Aggregate results on 6MWD and FVC change from baseline over time and baseline characteristics (age, gender, ethnicity, previous ERT duration, baseline 6MWD and baseline FVC) were extracted from included studies.

A multi-level network meta regression (ML-NMR) was performed, which is an extension of standard network meta-analyses (NMAs) that take into account the effect of study-level covariates, and that can be applied to any connected network with any mixture of individual patient-level data (IPD) and aggregate data. ML-NMR is an accepted method by the National Institute for Health and Care Excellence (NICE) in support of cost effectiveness analysis. Single-arm study results were matched to appropriate comparator arms of the comparative studies to allow for inclusion into the network. Mean treatment differences with associated 95% credible intervals (CrIs) were calculated for 6MWD and FVC change from baseline at week 52.

A base-case scenario was evaluated in which all covariates were set to the target population of the PROPEL trial. To study the impact of previous ERT duration on relative effects, ERT duration value was varied, keeping remaining covariate values as in the base-case scenario. A sensitivity analysis was performed by excluding all matched single-arm evidence from the network to assess its impact on the results.

Both fixed effects (FE) and random effects (RE) ML-NMR models were applied and the deviance information criteria (DIC) was used to assess goodness-of-fit of the models and to identify the appropriate model (FE or RE model) for the data. Models were implemented in a Bayesian framework using Stan with help of the R package multinma.

The SLR identified seven clinical studies for which baseline characteristics are shown in Figure 59. These studies included but were not limited to three randomised clinical trials (LOTS: Alglu versus Placebo; COMET: Aval versus Alglu; PROPEL: Cipa+mig versus Alglu). Each share 6MWD and FVC as key primary or secondary endpoints (see Figures 60 and 61) but differ in their trial populations (PROPEL is the only randomised controlled trial [RCT] that comprised both ERT-naïve and -experienced subjects). Efficacy results of the included studies are shown in Figure 60.

For both endpoints, the network is the same and shown in Figure 61. Evidence from the single-arm studies LOTS OLE, NEO-1/-EXT, COMET OLE and ATB200-02 was included into the network, as shown in the blue boxes, by matching the single-arm results to appropriate comparator results from the head-to-head trials.

In the base-case scenario, the covariates were set to the baseline characteristics of the target population (ie the PROPEL trial; see Table 20), and time was set to 52 weeks.

**Table 20: Base-case Scenario Covariate Setting**

| Age (years) | % Male | % White | ERT duration (years) | 6MWD (m) | FVC (% predicted) |
|---|---|---|---|---|---|
| 46.95 | 45.08 | 84.43 | 5.744 | 355.8 | 70.42 |

Based on the DIC, an RE model was chosen for 6MWD and an FE model for FVC. For both endpoints (Figures 62 and 63): Cipa+mig showed a statistically significant favourable effect versus Alglu and Aval; and Cipa+mig showed a numerically favourable effect versus placebo.

Note that the 95% CrIs of the relative effect estimates versus placebo are generally much wider than those versus Alglu or versus Aval. This reflects the larger uncertainty of those estimates, since data on placebo were only available for ERT-naïve subjects and previous ERT duration of the base-case scenario is relatively long (5.7 years).

Relative effect estimates for different previous ERT durations (previous ERT duration = 0 years [ie naive patients], 2.5 years, 5 years, and 9.2 years) are shown in Figure 64 (6MWD) and Figure 65 (FVC).

RE models were chosen for both 6MWD and FVC based on the DIC. Figures 66 and 67 provide an overview of the relative effect estimates with 95% CrIs for the base-case scenario using the sensitivity analysis and show:
- Inclusion of matched single-arm evidence into the network for the main analysis reduces uncertainty of the relative effect estimates
- Cipa+mig: statistically favourable versus Alglu; numerically unfavourable versus Aval; numerically favourable versus placebo (6MWT and FVC)
- Aval: numerically favourable versus Alglu and placebo (6MWT and FVC)
- Alglu: numerically favourable versus placebo

In conclusion, the ML-NMR comparison presented here showed that Cipa+mig was statistically significantly favourable versus Alglu and Aval for 6MWD and FVC in the base-case scenario of the main analysis. Cipa+mig was also statistically significantly favourable over Alglu and Aval for 6MWD and FVC for different ERT durations, with one exception: for FVC, Cipa+mig was only numerically favorable vs. Aval in the ERT-naïve setting.

The sensitivity analysis (only including RCT data) demonstrates that the inclusion of matched single-arm evidence into the network for the main analysis reduces uncertainty of the relative effect estimates. Overall, these results point to Cipa+mig potentially having a differentiated clinical profile versus the other ERTs, particularly for individuals with some level of previous ERT treatment. Further analyses are anticipated to test and refine the findings, when additional longer-term data are published.

### Example 14: Long-Term Comparison of Cipaglucosidase Alfa + Miglustat (Cipa+mig) and Alglucosidase Alfa (Alglu)

The Phase III double-blind PROPEL study (NCT03729362; ATB200-03) described in Examples 9 and 10 above compared the investigational two-component therapy cipaglucosidase alfa/miglustat (cipa/mig) with alglucosidase alfa/placebo (alglu) in adults with late-onset Pompe disease (LOPD) over 52 weeks. The ongoing open-label extension (OLE) of PROPEL (NCT04138277; ATB200-07) evaluates long-term safety and efficacy of cipa/mig. Outcomes include 6 minute walk distance (6MWD), forced vital capacity (FVC), creatine kinase (CK) and hexose tetrasaccharide (Hex4) levels and safety. Data are reported as change from the PROPEL baseline to OLE week 52 (104 weeks after the PROPEL baseline). The study design and patient disposition are as shown in FIG. 68, and baseline characteristics are shown in FIG. 69. Results are shown in FIGS. 70-74 and described in further detail below. In the OLE (N=119; 91 enzyme replacement therapy [ERT]-experienced and 28 ERT-naïve), 82/85 (96.5%) patients previously treated with cipa/mig continued cipa/mig, and 37/38 (97.4%) switched from alglu to cipa/mig; 90.8% of patients remained in the OLE study through week 52. Mean change in % predicted 6MWD was +3.1(8.07 standard deviation) for cipa/mig-cipa/mig and -0.5(7.76) for alglucipa/mig in ERT-experienced patients and +8.6(8.57) for cipa/mig-cipa/mig and +8.9(11.65) for alglu-cipa/mig in ERT-naïve patients. Mean change in % predicted FVC was -0.6(7.50) for cipa/mig-cipa/mig and -3.8(6.23) for alglu-cipa/mig in ERT-experienced patients and -4.8(6.48) and -3.1(6.66) in ERT-naïve patients. Mean reduction in CK (U/L) for ERT-experienced and ERT-naïve patients was -132.1(215.74) and -216.9(243.66) for cipa/mig-cipa/mig and -161.0(269.52) and -218.6(316.47) for alglu-cipa/mig, respectively. Mean reduction in Hex4 (mmol/mol) for ERT-experienced and ERT-naïve patients was -1.9(3.22) and -2.9(2.45) for cipa/mig-cipa/mig and -2.6(3.75) and -2.9(2.22) for alglu-cipa/mig, respectively. During PROPEL through week 52 of the OLE, treatment-emergent adverse events occurred in 84 (98.8%) cipa/mig-cipa/mig and 36 (97.3%) alglu-cipa/mig patients. Three patients discontinued the OLE due to infusion-associated reactions (urticaria, urticaria and hypotension, and anaphylaxis, respectively).

ERT-experienced patients who were treated with cipa/mig through PROPEL and the OLE showed improvements from baseline in 6MWD and biomarker levels and remained stable in % predicted FVC through PROPEL, and remained stable in these outcomes through the OLE to week 104. ERT-experienced patients who were treated with alg/pla during PROPEL remained stable in 6MWD and worsened in % predicted FVC and biomarker levels and stabilized or improved after switching to cipa/mig in the OLE. For ERT-naive patients who were treated with cipa/mig through PROPEL and the OLE, 6MWD and biomarker levels improved through PROPEL and remained stable through the OLE. % predicted FVC declined through PROPEL and stabilized over the OLE. ERT-naive patients who were treated with alg/pla during PROPEL and switched to cipa/mig in the OLE showed a similar pattern to patients who were treated with cipa/mig throughout. No new safety signals were identified. Data demonstrate treatment with cipa/mig up to 104 weeks was associated with a durable effect and was well tolerated, supporting long-term benefits of treatment for patients with LOPD.

### Example 15: Comparison of N-glycan profile distribution in Cipaglucosidase Alfa and Alglucosidase Alfa

Alglucosidase alfa and three preparations of cipaglucosidase alfa were analyzed using LC-FLD to evaluate their N-glycan profile broken down into 10 distinct classes primarily based on charge and hydrophilicity. LC-FLD analysis demonstrated consistently and categorically higher levels of bis-M6P (class 10) N-glycan species in cipalucosidase alfa compared to alglucosidase alfa. Other differences in relative abundance distinguishing cipaglucosidase alfa and alglucosidase alfa were observed of mono-sialylated (Class 2), di-sialylated (Class 4), and mono-phosphorylated (mono-M6P, Class 6) glycan species. The results are shown in Fig. 75 and Table 21.

The three preparations of cipaglucosidase alfa included in the analysis and reported in Fig. 75 and Table 21 are 110C161009a (non-Clinical drug substance generated in the first 1000L bioreactor engineering run with a 1000L working volume), 110C171011a (clinical drug substance), and 2S1802 (clinical drug product).

Analysis was completed using hydrophilic interaction liquid chromatography (HILIC) column under the control of an ultra-high performance liquid chromatography (UHPLC) system coupled with fluorescence detection (LC-FLD) to separate fluorescently labeled 2-AA (2-anthranillic acid) N-glycans released from cipaglucosidase alfa by the amidase PNGase F. The classes (1-10) are labeled in Table 21.

**Table 21: Summary of 2-AA Glycan Percent Abundances identified in cipaglucosidase alfa preparations and alglucosidase alfa based on LC-FLD analysis.**

| **Glycan Class** | **Description** | **Cipaglucosidase Alfa** | | | **Alglucosidase Alfa** |
|---|---|---|---|---|---|
| | | Batch 1 | Batch 2 | Batch 3 | |
| **1** | Neutral High Mannose or desialylated complex | 21.7 | 25.1 | 24.6 | 21.9 |
| **2** | Mono-Sialylated | 17.8 | 18.1 | 18.2 | 25.8 |
| **3** | Mono- or Di-Sialylated | 0.8 | 0.7 | 0.7 | 3.1 |
| **4** | Di-Sialylated | 14.4 | 11.9 | 12.0 | 31.2 |
| **5** | Tri-Sialylated | 9.5 | 8.7 | 8.4 | 7.8 |
| **6** | Mono-Phosphorylated | 13.8 | 13.1 | 13.4 | 4.2 |
| **7** | Tetra-Sialylated | 1.4 | 1.0 | 1.4 | 0.6 |
| **8** | Mono-Phosphorylated (Hybrid) | 2.1 | 1.7 | 1.9 | 1.7 |
| **9** | Bis-Phosphorylated (Hybrid) | 2.8 | 2.5 | 2.6 | 1.6 |
| **10** | Bis-Phosphorylated | 15.7 | 17.0 | 16.8 | 2.1 |
| | Total | 100.0 | 100.0 | 100.0 | 100.0 |

In the LC-FLD analysis, the protein was denatured, reduced, and treated with enzyme PNGase F to release N-linked glycans. The deglycosylated protein was analyzed by SDS-PAGE. The released glycans were labeled with 2-AA and analyzed by normal-phase chromatography. Briefly, 50 µg of ATB200 was mixed with 15 µL of 10× solution of Denaturing Buffer containing 5% SDS and 400 mM dithiothreitol (DTT) in a total volume of 150 µL, heated at 100°C for 10 minutes, and cooled to 20°C. To each denatured ATB200 sample, 20 µL of 10× NP-40 and NEB Glyco Reaction Buffer 2 was added to a final reaction volume of 190 µL. Ten µL of PNGase F was added to release N-linked glycans, and the reaction was incubated at 37°C for 16-18 hours. Released glycans were purified using a 10 kDa MWCO ultrafiltration centrifugal device. 50 µL of the deglycosylation reaction was added to the centrifugal device and centrifuged for 10 minutes at 14,000 RCF. A total of 150 µL of ddH₂O in three 50 µL steps was added to wash out the glycans from deglycosylated ATB200 by centrifuging the ultrafiltration device at 14,000 RCF for 10 minutes at each wash step. The purified glycans were dried by speed-vac and labeled with 2AA. The labeled glycans were precipitated in 95% CAN/5% H₂O and pelleted by centrifugation for 10 minutes at 14,000 RCF at room temperature. The pellet was washed with 95% CAN in water, centrifuged again, and dried using a speed vac. The dried, labeled glycans were dissolved in 100 µL 20% CAN in water and stored at 4°C until chromatographic separation and analysis. During the labeling reaction, 2 µL each of the glycosylated and deglycosylated ATB200 samples were resolved by 4-12% SDS-PAGE. The gel was stained using Imperial Blue stain and visualized using a BioRad ChemiDoc MP imaging system. Glycan class assignment was based on previous work and according to established peak shape and chromatographic retention times. Peak integration and quantitation of glycans was performed using Chromeleon.

Overall, the N-glycan profile analysis by LC-FLD of 2-AA labeled N-glycansdistinguishes cipaglucosidase alfa from alglucosidase alfa. As shown above, the cipaglucosidase alfa has higher M6P glycan content, emphasizing the structural differences. In particular, cipaglucosidase alfa has higher bis-M6P glycans which are attributed to the significantly greater CIMPR binding and uptake into muscle cells.

### Example 16: Role of M6P in CIMPR binding and cellular uptake of cipaglucosidase alfa

The removal of phosphate groups on cipaglucosidase alfa resulted in the blocking of CIMPR binding and prevented rhGAA uptake into fibroblasts derived from Pompe patients, demonstrating the significance of phosphorylated glycans for a therapeutically relevant biological effect. Dephosphorylated cipaglucosidase alfa was obtained through removal of phosphate groups on mono- and bis-M6P by the enzyme purple acid phosphatase (PAP).

Removal of phosphoesters mono- and bis-M6P was completed by the enzyme purple acid phosphatase (PAP). PAP hydrolyzes phosphate esters and anhydrides. Under acidic conditions, the phosphates on the phosphoesters mono- and bis-M6P are removed by PAP, shown by the increase in electrophoretic mobility in Fig. 76A and 76B, while retaining the enzyme activity of cipaglucosidase alfa as demonstrated by 4MU-α-Glucoside hydrolysis in Fig. 76D. Cipaglucosidase alfa was treated with PAP for 18 hours, and alglucosidase alfa in the same reaction conditions but without PAP addition was used as a mock-treated control. PAP treatment results in complete removal of the bis-M6P peak and nearly all of the mono-M6P peak from the cipaglucosidase alfa LC-FLD chromatogram. Additional peaks are clearly observed in the neutral high mannose glycan region resulting from the removal of phosphates from mono- and bis-phosphorylated high mannose glycans.

The result of the CIMPR Overlay Assay shown in Fig. 76C, shows cipaglucosidase alfa binds with high affinity to CIMPR as shown by an intense band, while PAP-treated glucosidase alfa has no detectable band, indicating no binding to CIMPR. Additionally, alglucosidase alfa has a band with a much lower intensity than that shown for cipaglucosidase alfa indicating a lower binding affinity for CIMPR. The significantly lower CIMPR binding of alglucosidase alfa and dephosphorylated cipaglucosidase alfa compared to mock-treated cipaglucosidase alfa clearly demonstrates N-glycan structures mono- and bis-M6P are critical for the characteristics of cipaglucosidase alfa important for its therapeutic biological effect.

The CIMPR Overlay Assay is a variation on a far-western blot where proteins are separated on an SDS-PAGE, transferred to a nitrocellulose membrane, and incubated with purified CIMPR in the place of a primary antibody as in a conventional western blot. Binding of CIMPR to M6P-containing glycans on rhGAA proteins is then visualized by an anti-CIMPR antibody.

The relative cellular uptake into Pompe patient fibroblasts of dephosphorylated cipaglucosidase alfa compared to mock-treated cipaglucosidase alfa and mock-treated alglucosidase alfa was assessed. Comparisons at a concentration of 20 nM of rhGAA was used to mimic the interstitial concentration of cipaglucosidase alfa that crosses into tissues, as shown in Fig. 77A. After an 18-hr incubation for enzyme uptake, cells were washed for 2 hours, harvested, lysed, and then processed for GAA activity and western blot analysis. Internalized GAA measured by 4MU-α-Glucoside hydrolysis and was graphed relative to total cellular protein and the results appear in Fig 77B. CIMPR binding of the cell lysates (3 ug) was analyzed by western blot analysis as shown in Fig. 77C.

Dephosphorylated cipaglucosidase alfa was shown to not be efficiently internalized into cells. As depicted in Fig. 77B and 77C by 4MU-α-Glucoside hydrolysis and CIMPR binding, mock-treated glucosidase alfa is internalized into Pompe fibroblast cells and is internalized to a greater degree than the conventional alfaglucosidase alfa. However, cell uptake of PAP-treated cipaglucosidase alfa is abolished. These results demonstrate the essential role of M6P and suggests that cipaglucosidase alfa has better cellular uptake compared to alfaglucosidase alfa due to the mono- and bis-M6P N-glycan structures and quantities.

### Example 17: Summary of Clinical Development Program

There are 3 clinical trials (Studies ATB200-02, ATB200-03, and ATB200-07) including both ERT-experienced and ERT-naïve (Studies ATB200-02 and ATB200-03) or only ERT-experienced (Study ATB200-07) adult subjects with Pompe disease (≥ 18 years) that will be included in this Summary of Clinical Efficacy (SCE) and details are presented in Table 22. One hundred fifty-two adult subjects were enrolled into the clinical development program for cipaglucosidase alfa/miglustat with 29 adult subjects exposed to cipaglucosidase alfa/miglustat in the ongoing Phase 1/2 Study ATB200-02 and 123 adult subjects exposed to cipaglucosidase alfa/miglustat (85 subjects) or alglucosidase alfa/placebo (38 subjects) in the completed Phase 3 study, ATB200-03. Of the 123 subjects (85 treated with cipaglucosidase alfa/miglustat; 38 treated with alglucosidase alfa/placebo) who enrolled in Study ATB200-03, a total of 117 subjects completed the study and then enrolled in Study ATB200-07. Two additional subjects (1107-1681 and 2010-1352) did not complete Study ATB200-03 but enrolled in Study ATB200-07, bringing the total of subjects enrolled in Study ATB200-07 (OLE-ES Population) to 119 (91 ERT-experienced and 28 ERT-naïve).

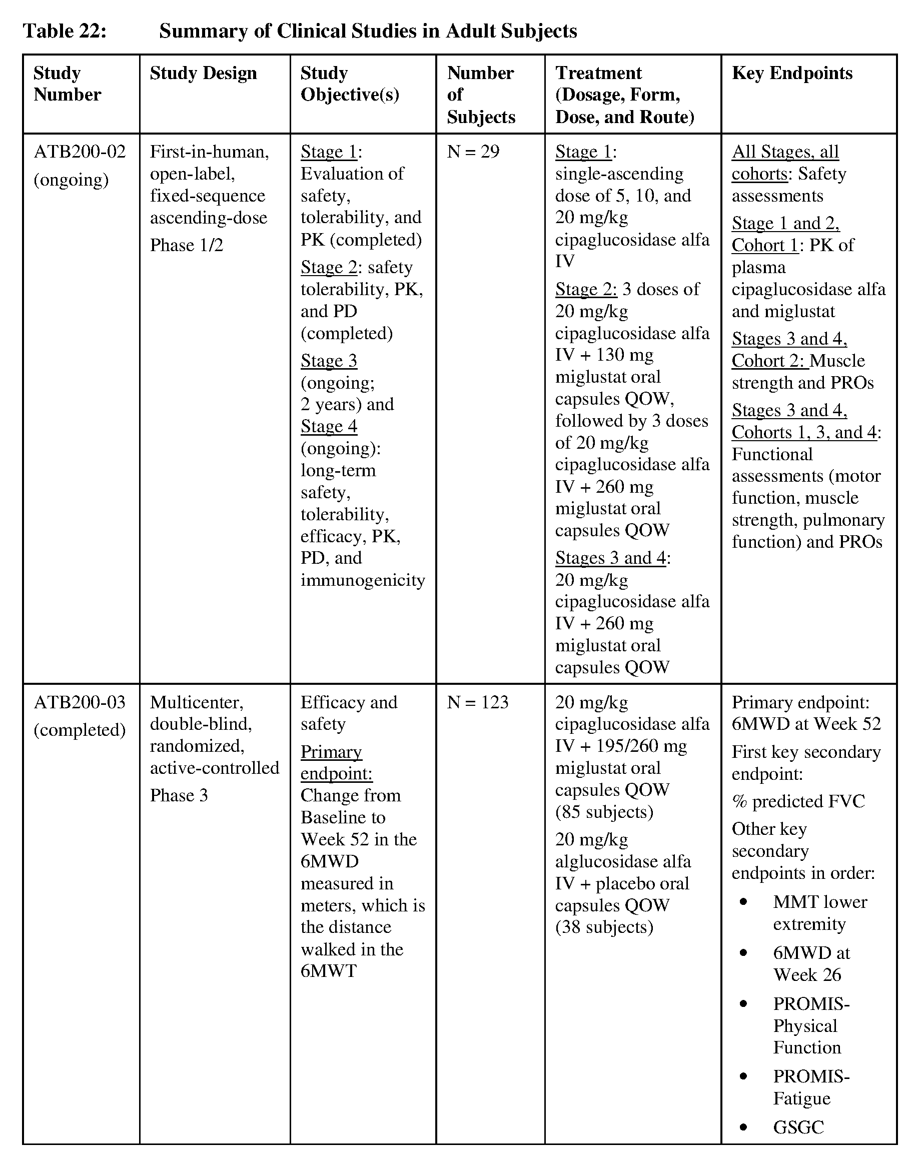

**Table 22: Summary of Clinical Studies in Adult Subjects (Continued)**

| **Study Number** | **Study Design** | **Study Objective(s)** | **Number of Subjects** | **Treatment (Dosage, Form, Dose, and Route)** | **Key Endpoints** |
|---|---|---|---|---|---|
| ATB200-07 (ongoing) | Multicenter, open-label extension study to Study ATB200-03 Phase 3 | Evaluation of Safety and Efficacy | N = 119^{a} | 20 mg/kg cipaglucosidase alfa IV + 195/260 mg miglustat oral capsules QOW | Long-term safety, efficacy, PD, PK, PROs |

| | | | | | |
|---|---|---|---|---|---|
| Abbreviations: 6MWD = 6-minute walk distance; 6MWT = 6-minute walk test; GSGC = Gait, Stairs, Gowers' maneuver, and Chair test; IV = intravenous(ly); LOPD = late-onset Pompe disease; MMT = manual muscle testing; N = number of subjects; PD = pharmacodynamic(s); PK = pharmacokinetic(s); PRO = patient-reported outcome; PROMIS = Patient-reported Outcomes Measurement Information System; QOW = once every other week ^{a} Two subjects discontinued from Study ATB200-03 and enrolled in Study ATB200-07. A total of 119 subjects enrolled and 118 were dosed in Study ATB200-07. Note: In Studies ATB200-03 and ATB200-07, miglustat dosing is adjusted to 195 mg in subjects < 50 kg. | | | | | |

### Phase 1/2 Study ATB200-02

As described above with respect to Examples 8 and 11, Study ATB200-02 is an ongoing open-label, fixed-sequence, single- and multiple-ascending dose, first-in-human study to evaluate the safety, tolerability, PK, PD, efficacy, and immunogenicity of IV cipaglucosidase alfa alone and when co-administered with oral miglustat in adult subjects with Pompe disease. The study design includes 4 stages and 4 cohorts, with Stages 1 and 2 only for Cohort 1 and Stages 3 and 4 for all 4 cohorts (FIG. 79). Data on the efficacy of cipaglucosidase alfa/miglustat summarized in the SCE were obtained from Stages 3 and 4.

The 4 cohorts in the study are as follows:
- Cohort 1: ERT-experienced (2 to 6 years) ambulatory subjects (11 subjects)
- Cohort 2: ERT-experienced (≥ 2 years) non-ambulatory subjects (6 subjects)
- Cohort 3: ERT-naïve ambulatory subjects (6 subjects)
- Cohort 4: ERT-experienced (≥ 7 years) ambulatory subjects (6 subjects)

Subjects were administered cipaglucosidase alfa as a single agent or cipaglucosidase alfa co-administered with miglustat according to the treatment assignment in Table 22.

**Table 22: Treatment Assignment for Stages 1, 2, 3, and 4 - Study ATB200-02**

| **Cohorts** | **Stage 1 (6 weeks)** | | | **Stage 2 (12 weeks)** | |
|---|---|---|---|---|---|
| | **Period 1 Single Dose** | **Period 2 Single Dose** | **Period 3 Single dose** | **Period 4 3 Multiple Doses Co-administration** | **Period 5 3 Multiple Doses Co-administration** |
| Cohort 1 (n = 11) | 5 mg/kg ATB200 | 10 mg/kg ATB200 | 20 mg/kg ATB200 | 20 mg/kg ATB200 + 130 mg AT2221 | 20 mg/kg ATB200 + 260 mg AT2221 |
| Cohort 2 (n = 6) | NA | | | | |
| Cohort 3 (n = 6) | NA | | | | |
| Cohort 4 (n = 6) | NA | | | | |

| **Cohorts** | **Stage 3 (2 years)** | **Stage 4 (Until Approval)** |
|---|---|---|
| | **Multiple Dose, 24 months, Extension Co-administration** | **Multiple Dose, Long-term, Extension Co-administration** |
| Cohort 1 (n = 11) | 20 mg/kg ATB200 + 260 mg AT2221 | 20 mg/kg ATB200 + 260 mg AT2221 |
| Cohort 2 (n = 6) | 20 mg/kg ATB200 + 260 mg AT2221 | 20 mg/kg ATB200 + 260 mg AT2221 |
| Cohort 3 (n = 6) | 20 mg/kg ATB200 + 260 mg AT2221 | 20 mg/kg ATB200 + 260 mg AT2221 |
| Cohort 4 (n = 6) | 20 mg/kg ATB200 + 260 mg AT2221 | 20 mg/kg ATB200 + 260 mg AT2221 |

| | | |
|---|---|---|
| Abbreviations: AT2221 = miglustat; ATB200 = cipaglucosidase alfa; NA = not applicable; n = number of subjects; SSC = Safety Steering Committee Note: At least 1 of the 2 sentinel subjects will complete Period 5, Stage 2 dosing and the safety data will be reviewed by the SSC before any newly enrolled subjects in Cohorts 2 and 3 can be dosed. The first 2 subjects in Cohorts 2 and 3 will also serve as sentinel subjects for their respective cohorts. | | |

The primary objectives of the Phase 1/2 Study ATB200-02 were to evaluate the safety, tolerability, PK, PD, and efficacy of cipaglucosidase alfa alone and when co-administered with oral miglustat.

Dose selection for the pivotal Phase 3 Study ATB200-03 was based on PK, PD/biomarker, efficacy, and safety data from Study ATB200-02. The PK of cipaglucosidase alfa was well characterized in Stages 1 and 2 of Study ATB200-02, which showed increases in overall cipaglucosidase alfa exposure and additional increases in exposure with the addition of miglustat. This increase in cipaglucosidase alfa plasma exposure with miglustat is consistent with data from nonclinical studies where the increased exposure was associated with incremental reductions in glycogen and increases in muscle strength.

Study ATB200-02 provides supportive efficacy data from Stages 3 and 4 showing that the increases in exposure observed in Stages 1 and 2 led to clinically meaningful improvement in a wide range of endpoints following treatment with cipaglucosidase alfa/miglustat in Stages 3 and 4. These improvements in efficacy are observed out to 48 months where data are available, supporting long-term efficacy of cipaglucosidase alfa/miglustat.

### Phase 3 Study ATB200-03

The pivotal Phase 3 Study ATB200-03 is complete. Study ATB200-03 was a double-blind, randomized, multicenter, global controlled trial to evaluate the efficacy and safety of cipaglucosidase alfa/miglustat compared with alglucosidase alfa/placebo (approved therapy) in adult subjects with LOPD who had previously received alglucosidase alfa (ie, ERT-experienced) or who had never received ERT (ie, ERT-naïve). Myozyme (alglucosidase alfa) was sourced directly from Sanofi Genzyme via a third-party vendor. A scientific assessment was performed by Amicus on documentation from Sanofi Genzyme. Results demonstrated that US product (Lumizyme) and non-US product (Myozyme) are comparable. Subjects were randomly assigned in a 2:1 ratio to receive cipaglucosidase alfa/miglustat or alglucosidase alfa/placebo every other week for 52 weeks (FIG. 80).

The primary endpoint of Study ATB200-03 was to assess the efficacy of cipaglucosidase alfa/miglustat co-administration on motor function as measured by the 6MWT, compared with alglucosidase alfa/placebo. The first key secondary endpoint was to assess the efficacy of cipaglucosidase alfa/miglustat co-administration on pulmonary function as measured by sitting % predicted FVC compared with alglucosidase alfa/placebo. Additional key secondary endpoints included MMT lower extremity score, 6MWD at Week 26, total score of the PROMIS^{®}-Physical Function Short Form 20a, total score of the PROMIS-Fatigue Short Form 8a, and GSGC total score. Other secondary endpoints included additional assessments of motor function, pulmonary function, muscle strength, and PROs. Biomarkers of muscle damage (CK) and disease substrate (Hex4) were also assessed.

### Phase 3 Study ATB200-07

Study ATB200-07 is an ongoing open-label extension (OLE) study to assess the long-term safety and efficacy of cipaglucosidase alfa/miglustat in adult subjects with LOPD who completed Study ATB200-03. Subjects who were treated with alglucosidase alfa/placebo in Study ATB200-03 were switched to cipaglucosidase alfa/miglustat in Study ATB200-07. The study doses and schedule are maintained from Study ATB200-03.

### Summary of Study Results

The results of the ongoing Phase 1/2 open-label Study ATB200-02, the completed pivotal Phase 3 Study ATB200-03, and the Phase 3 long-term extension Study ATB200-07 are summarized in this section. Data from these studies are presented individually and as pooled data.

### Example 18: Study ATB200-02

Study ATB200-02 is a Phase 1/2 study and the efficacy of cipaglucosidase alfa/miglustat is mainly based on results from Stages 3 and 4 of the study.

As of December 2021, 29 subjects were dosed with cipaglucosidase alfa/miglustat, 3 subjects discontinued prematurely, and 26 subjects are currently ongoing in Stage 4. Three (10.3%) subjects discontinued the study during Stage 3. The mean (standard deviation [SD]) duration of exposure was 48.4 (18.04) months.

In Study ATB200-02, all ERT-experienced and ERT-naïve subjects demonstrated a significant impact of Pompe disease based on baseline characteristics. For Cohorts 1 and 4 (16 ERT-experienced ambulatory subjects), the mean (SD) % predicted 6MWD at baseline was 60.2% (16.20%). For Cohort 3 (6 ERT-naïve ambulatory subjects), the mean (SD) % predicted 6MWD at baseline was 67.8% (12.61%).

The duration of ERT treatment for ERT-experienced subjects prior to study enrollment was > 2 years, with an overall median dose of 20 mg/kg. The mean (SD) duration of ERT treatment ranged from 5.1 (1.27) years in Cohort 1 to 10.6 (2.06) years in Cohort 4.

The results of Study ATB200-02 referenced below are based on the data cutoff of 13 December 2021 at which point all subjects, except those in Cohort 4, had completed at least 48 months of follow up (or had discontinued). Data out to 48 months supporting long-term efficacy are presented here and included in the pooled analysis below. The data from Study ATB200-02 evaluating the main clinical domains of Pompe disease such as motor function, pulmonary function, and muscle strength support the results obtained with Study ATB200-03.

### Motor Function

In Study ATB200-02, motor function was evaluated in all ambulatory subjects (ie, Cohorts 1, 3, and 4) (Table 23, FIGS. 46A-46D). Following 48 months of treatment with co-administration of 20 mg/kg IV-infused cipaglucosidase alfa and 260 mg miglustat in Stage 3 and Stage 4, clinically meaningful improvements in motor function as measured by the 6MWT were observed in ERT-experienced subjects (Cohorts 1 and 4), ERT-naïve subjects (Cohort 3), and overall (Cohorts 1, 3, and 4) (Table 23). Initial improvement was observed in 1-2 years and was maintained above baseline up to Month 48.

Both the ERT-experienced and ERT-naïve cohorts showed durable mean improvements from baseline in % predicted 6MWD up to 48 months (FIGS. 46C and 46D; Table 26B), with 88.9% of ERT-experienced patients and 100% of ERT-naïve patients experiencing an improvement from baseline in mean % predicted 6MWD at Month 48. After 12, 24, 36, and 48 months of follow-up, mean 6MWD in meters improved numerically from baseline in 13/16, 9/13, 6/12 and 6/9 ERT-experienced patients, respectively. After 12, 24, 36 and 48 months of follow-up, mean 6MWD in meters improved numerically from baseline in 6/6, 6/6, 4/5 and 4/4 ERT-naïve patients, respectively.

**Table 23: Summary of 6-MWD (meters) from Baseline to Month 48 - Stage 3 and Stage 4 (Efficacy Population) - Study ATB200-02**

| **Visit/Statistic** | | **Cohort 1 + Cohort 4 (N = 16)** | **Cohort 3 (N = 6)** | **Cohort 1 + Cohort 3 + Cohort 4 (N = 22)** |
|---|---|---|---|---|
| Baseline | | | | |
| | n | 16 | 6 | 22 |
| | Mean (SD) | 393.5 (119.66) | 396.0 (75.20) | 394.2 (107.59) |
| | 95% CI for mean | 329.7, 457.2 | 317.1, 474.9 | 346.5, 441.9 |
| | Median (Q1, Q3) | 394.5 (330.0, 491.5) | 395.2 (378.3, 460.0) | 395.2 (332.0, 480.0) |
| | Min, max | 104, 544 | 267, 480 | 104, 544 |
| Change from baseline to Month 12 | | | | |
| | n | 16 | 6 | 22 |
| | Mean (SD) | 33.5 (49.62) | 57.0 (29.96) | 39.9 (45.69) |
| | 95% CI for mean | 7.0, 59.9 | 25.6, 88.5 | 19.6, 60.1 |
| | Median (Q1, Q3) | 20.6 (8.3, 51.9) | 55.6 (30.8, 78.9) | 27.4 (12.0, 77.8) |
| | Min, max | -44, 135 | 27, 95 | -44, 135 |
| Change from baseline to Month 24 | | | | |
| | n | 13 | 6 | 19 |
| | Mean (SD) | 25.2 (63.30) | 54.4 (36.18) | 34.4 (56.82) |
| | 95% CI for mean | -13.0, 63.5 | 16.4, 92.4 | 7.1, 61.8 |
| | Median (Q1, Q3) | 18.5 (-7.0, 58.0) | 49.0 (22.6, 78.2) | 22.6 (5.2, 78.2) |
| | Min, max | -98, 135 | 21, 107 | -98, 135 |
| Change from baseline to Month 36 | | | | |
| | n | 12 | 5 | 17 |
| | Mean (SD) | 9.8 (85.98) | 43.5 (45.19) | 19.7 (76.44) |
| | 95% CI for mean | -44.8, 64.5 | -12.6, 99.6 | -19.6, 59.0 |
| | Median (Q1, Q3) | 11.6 (-13.7, 51.5) | 59.0 (20.5, 80.0) | 24.2 (-12.0, 59.0) |
| | Min, max | -204, 131 | -24, 82 | -204, 131 |

**Table 23: Summary of 6-MWD (meters) from Baseline to Month 48 - Stage 3 and Stage 4 (Efficacy Population) - Study ATB200-02 (Continued)**

| **Visit/Statistic** | | **Cohort 1 + Cohort 4 (N = 16)** | **Cohort 3 (N = 6)** | **Cohort 1 + Cohort 3 + Cohort 4 (N = 22)** |
|---|---|---|---|---|
| Change from baseline to Month 48 | | | | |
| | n | 9 | 4 | 13 |
| | Mean (SD) | 20.7 (101.84) | 52.2 (46.59) | 30.4 (87.67) |
| | 95% CI for mean | -57.6, 99.0 | -21.9, 126.3 | -22.6, 83.4 |
| | Median (Q1, Q3) | 27.0 (-3.3, 83.7) | 40.5 (16.3, 88.0) | 27.0 (13.7, 83.7) |
| | Min, max | -215, 138 | 14, 114 | -215, 138 |

| | | | | |
|---|---|---|---|---|
| Abbreviations: 6MWD = 6-minute walk distance; CI = confidence interval; max = maximum; min = minimum; N = total number of subjects enrolled; n = subset of subjects; Q1 = first quartile; Q3 = third quartile; SD = standard deviation Note: 6-minute walk distance (6MWD) is the distance in meters walked in the 6-minute walk test. It was performed by ambulatory subjects only. | | | | |

Co-administration of 20 mg/kg IV-infused cipaglucosidase alfa and 260 mg miglustat (ie, in Stage 2 Period 5, Stage 3, and Stage 4) also resulted in improvements in other measures of motor function in ERT-experienced and ERT-naïve ambulatory subjects, including 10MWT, GSGC, and TUG. Initial improvement was observed in 1 to 2 years and was maintained above baseline up to Month 48.

### Pulmonary Function

Following 48 months of treatment in Stage 3 and Stage 4, co-administration of 20 mg/kg IV-infused cipaglucosidase alfa and 260 mg miglustat (ie, in Stage 2 Period 5, Stage 3, and Stage 4) resulted in stable or improved % predicted FVC for ERT-experienced subjects (Cohorts 1 and 4), ERT-naïve subjects (Cohort 3), and overall (Cohorts 1, 3, and 4) (Table 24, FIGS. 47A-47B). Values were maintained at or above baseline up to Month 48. For Cohort 2 (non-ambulatory subjects), sitting % predicted FVC was stable in 2 subjects with available data out to 36 months.

Mean CFBL in % predicted sitting FVC was generally stable (CFBL ± 3% points) for up to 48 months of follow-up in the ERT-experienced cohorts (FIG. 47A, Table 26B), with improvements (CFBL >3% points) at Months 48 in 66.7% of ERT-experienced and 75% of ERT-naive patients. After 12, 24, 36 and 48 months of follow-up, % predicted sitting FVC improved (>3% points) or remained stable (±3% points) from baseline in 9/16, 11/13, 8/10 and 4/6 ERT-experienced patients respectively. This is further supported by maximum expiratory pressure (MEP) results that also improved numerically from baseline at 12, 24, 36, and 48 months follow-up in ERT-experienced patients (Table 26B).

In the ERT-naïve cohort, mean CFBL in % predicted sitting FVC improved at 3 months and then remained generally stable up to 48 months of follow up (FIG. 47B, Table 26B), except for one patient who experienced a large drop in % predicted sitting FVC at Month 21, which returned to previous levels at the following visit at Month 24. Mean % predicted sitting FVC improvements were more marked in the ERT-naïve cohort compared with the ERT-experienced cohort over 48 months of follow up (FIG. 47B, Table 26B). After 12, 24, 36 and 48 months of follow-up, % predicted sitting FVC improved (>3% points) or remained stable (±3% points) from baseline in 5/6, 6/6, 5/5 and 4/4 in ERT-naïve patients, respectively. Percent predicted sitting FVC data were supported by similar outcomes in other pulmonary measures including % predicted maximum inspiratory pressure (MIP) and MEP. MIP improved numerically in ERT-naive patients at 12, 24, 36 and 48 months follow up (Table 26B).

Limited long-term efficacy data are available for the non-ambulatory ERT-experienced cohort 2. Percent predicted sitting FVC data were available for two non-ambulatory ERT-experienced patients after 36 months and one patient at 48 months of follow up. After 36 months of follow up, one patient was improved and the other worsened compared with baseline. The patient with available data after 48 months of follow up, was generally stable compared with baseline (Table 26B).

**Table 24: Summary of Sitting % Predicted FVC from Baseline to Month 48 - Stage 3 and Stage 4 (Ambulatory Subjects; Efficacy Population) - Study ATB200-02**

| **Visit/Statistic** | | **Cohort 1 + Cohort 4 (N = 16)** | **Cohort 3 (N = 6)** | **Cohort 1 + Cohort 3 + Cohort 4 (N = 22)** |
|---|---|---|---|---|
| Baseline | | | | |
| | n | 16 | 6 | 22 |
| | Mean (SD) | 57.4 (17.42) | 57.2 (20.84) | 57.3 (17.89) |
| | 95% CI for mean | 48.1, 66.7 | 35.3, 79.0 | 49.4, 65.3 |
| | Median (Q1, Q3) | 56.0 (46.0, 70.0) | 59.0 (38.0, 77.0) | 56.0 (45.0, 70.0) |
| | Min, max | 31, 88 | 31, 79 | 31, 88 |
| Change from baseline to Month 12 | | | | |
| | n | 16 | 6 | 22 |
| | Mean (SD) | -1.2 (5.95) | 3.2 (8.42) | 0.0 (6.79) |
| | 95% CI for mean | -4.4, 2.0 | -5.7, 12.0 | -3.0, 3.0 |
| | Median (Q1, Q3) | -1.0 (-5.5, 4.0) | 4.5 (-4.0, 11.0) | -0.5 (-5.0, 5.0) |
| | Min, max | -12, 8 | -9, 12 | -12, 12 |
| Change from baseline to Month 24 | | | | |
| | n | 13 | 6 | 19 |
| | Mean (SD) | 1.0 (7.96) | 4.7 (5.09) | 2.2 (7.24) |
| | 95% CI for mean | -3.8, 5.8 | -0.7, 10.0 | -1.3, 5.6 |
| | Median (Q1, Q3) | 1.0 (-2.0, 6.0) | 4.0 (0.0, 9.0) | 2.0 (-2.0, 6.0) |
| | Min, max | -14, 15 | -1, 12 | -14, 15 |
| Change from baseline to Month 36 | | | | |
| | n | 10 | 5 | 15 |
| | Mean (SD) | -0.3 (6.68) | 6.2 (3.35) | 1.9 (6.48) |
| | 95% CI for mean | -5.1, 4.5 | 2.0, 10.4 | -1.7, 5.5 |
| | Median (Q1, Q3) | -1.5 (-3.0, 2.0) | 5.0 (4.0, 6.0) | 2.0 (-3.0, 6.0) |
| | Min, max | -11, 13 | 4, 12 | -11, 13 |
| Change from baseline to Month 48 | | | | |
| | n | 6 | 4 | 10 |
| | Mean (SD) | 1.0 (6.42) | 8.3 (4.50) | 3.9 (6.61) |
| | 95% CI for mean | -5.7, 7.7 | 1.1, 15.4 | -0.8, 8.6 |
| | Median (Q1, Q3) | 3.5 (-7.0, 5.0) | 9.5 (5.0, 11.5) | 4.5 (2.0, 8.0) |
| | Min, max | -7, 8 | 2, 12 | -7, 12 |

| | | | | |
|---|---|---|---|---|
| Abbreviations: CI = confidence interval; FVC = forced vital capacity; max = maximum; min = minimum; N = total number of subjects enrolled; n = subset of subjects; PFT = pulmonary function test; Q1 = first quartile; Q3 = third quartile; SD = standard deviation Note: PFTs were to be summarized at baseline, every 3 months in Stage 3, and every 6 months in Stage 4 for all ambulatory subjects and for non-ambulatory subjects without invasive ventilatory support. Higher values indicate improving vital capacity. | | | | |

### Muscle Strength

Following 48 months of treatment in Stage 3 and Stage 4, co-administration of 20 mg/kg IV-infused cipaglucosidase alfa and 260 mg miglustat (ie, in Stage 2 Period 5, Stage 3, and Stage 4) resulted in stable or improved muscle strength in the lower and upper extremities and proximal muscle groups of ambulatory ERT-experienced subjects (Cohorts 1 and 4) and ERT-naïve subjects (Cohort 3) as measured by MMTs and QMTs. Stable results were observed in non-ambulatory subjects (Cohort 2; upper extremities only). Results for lower extremity MMT are shown in Table 25 and FIGS. 48A and 48B.

Irrespective of ERT-treatment status at baseline, ambulatory patients experienced initial early improvements in muscle strength based on MMT lower extremity scores, which were maintained for up to 48 months of follow-up (FIGS. 48A and 48B, Table 26B). After 12, 24, 36 and 48 months of follow-up, the MMT lower extremity score improved numerically from baseline in 14/15, 11/13, 10/10 and 8/8 ERT-experienced patients and 4/5, 4/5, 4/4 and 3/4 ERT-naive patients, respectively.

**Table 25: Summary of MMT Lower Extremity Score from Baseline to Month 48 - Stage 3 and Stage 4 (Ambulatory Subjects; Efficacy Population) - Study ATB200-02**

| **Visit/Statistic** | | **Cohort 1 + Cohort 4 (N = 16)** | **Cohort 3 (N = 6)** | **Cohort 1 + Cohort 3 + Cohort 4 (N = 22)** |
|---|---|---|---|---|
| Baseline | | | | |
| | n | 15 | 5 | 20 |
| | Mean (SD) | 30.0 (3.44) | 29.0 (1.73) | 29.8 (3.09) |
| | 95% CI for mean | 28.1, 31.9 | 26.8, 31.2 | 28.3, 31.2 |
| | Median (Q1, Q3) | 30.0 (28.0, 32.0) | 30.0 (29.0, 30.0) | 30.0 (28.5, 32.0) |
| | Min, max | 21, 34 | 26, 30 | 21, 34 |
| Change from baseline to Month 12 | | | | |
| | n | 15 | 5 | 20 |
| | Mean (SD) | 3.1 (2.34) | 2.8 (4.97) | 3.0 (3.04) |
| | 95% CI for mean | 1.8, 4.4 | -3.4, 9.0 | 1.6, 4.4 |
| | Median (Q1, Q3) | 2.0 (2.0, 5.0) | 4.0 (0.0, 5.0) | 3.0 (2.0, 5.0) |
| | Min, max | -2, 6 | -4, 9 | -4, 9 |
| Change from baseline to Month 24 | | | | |
| | n | 13 | 5 | 18 |
| | Mean (SD) | 2.1 (2.18) | 3.0 (3.94) | 2.3 (2.68) |
| | 95% CI for mean | 0.8, 3.4 | -1.9, 7.9 | 1.0, 3.7 |
| | Median (Q1, Q3) | 2.0 (1.0, 4.0) | 3.0 (2.0, 3.0) | 2.0 (1.0, 4.0) |
| | Min, max | -2, 6 | -2, 9 | -2, 9 |
| Change from baseline to Month 36 | | | | |
| | n | 10 | 4 | 14 |
| | Mean (SD) | 2.5 (3.69) | 3.3 (2.75) | 2.7 (3.36) |
| | 95% CI for mean | -0.1, 5.1 | -1.1, 7.6 | 0.8, 4.7 |
| | Median (Q1, Q3) | 1.5 (0.0, 4.0) | 3.5 (1.0, 5.5) | 2.0 (0.0, 4.0) |
| | Min, max | 0, 12 | 0, 6 | 0, 12 |
| Change from baseline to Month 48 | | | | |
| | n | 8 | 4 | 12 |
| | Mean (SD) | 3.5 (2.51) | 1.0 (3.83) | 2.7 (3.08) |
| | 95% CI for mean | 1.4, 5.6 | -5.1, 7.1 | 0.7, 4.6 |
| | Median (Q1, Q3) | 3.0 (2.0, 5.0) | 2.0 (-2.0, 4.0) | 3.0 (1.0, 4.0) |
| | Min, max | 0, 8 | -4, 4 | -4, 8 |

| | | | | |
|---|---|---|---|---|
| Abbreviations: CI = confidence interval; FVC = forced vital capacity; max = maximum; min = minimum; N = total number of subjects enrolled; n = subset of subjects; PFT = pulmonary function test; Q1 = first quartile; Q3 = third quartile; SD = standard deviation Note: MMTs were to be summarized at baseline, every 3 months in Stage 3, and every 6 months in Stage 4 for all subjects. Higher values indicate less disease impact on muscle functions. | | | | |

Favorable findings observed through 48 months of treatment in 6MWD and FVC are bolstered by favorable directional trends in several other secondary endpoints for muscle strength, motor function, and respiratory function. These findings were further supported by PRO results (FSS, R-PAct, RHS, and SGIC) as well as PGIC results, which demonstrated meaningful improvements in subject-perceived fatigue, ability to perform daily life activities, and overall physical well-being, supportive of the overall benefit of this treatment.

PROs supported the improvements observed for motor function, muscle strength and pulmonary function tests. At baseline, all patients were significantly impacted by fatigue which improved after 48 months of follow up as shown by favorable mean CFBL FSS. After 48 months of follow up, all ambulatory patients reported stable R-PAct scores and RHS compared with baseline scores (Table 26B). SGIC outcomes for overall physical wellbeing improved in most patients across all cohorts after 48 months of follow up (Table 26C). PGIC results indicated an improvement or stabilization for all cohorts after 48 months of follow up and supported the results of other efficacy outcomes (Table 26C).

PRO results are available for two non-ambulatory ERT-experienced patients up to 48 months of follow up and demonstrate improved FSS and stable RHS scores compared with baseline. R-PAct scores also showed a mean improvement from baseline up to 48 months of follow up in the two non-ambulatory ERT-experienced patients, indicating an improved ability to perform daily activities and participate in social situations (Table 26B). One patient reported no change, and one reported an improvement from baseline in SGIC overall physical wellbeing. The PGIC results for the two ERT-experienced non-ambulatory patients showed no change for one patient and a decline for the other (Table 26C).

### Pharmacodynamic Outcomes

During the 48 months of follow-up, cipaglucosidase alfa plus miglustat was associated with mean reductions from baseline in plasma CK in both ERT-experienced and ERT-naive ambulatory cohorts (FIG. 49B, Table 26B). After 12, 24, 36, and 48 months of follow-up, plasma CK levels decreased numerically from baseline in 13/15, 14/15, 9/11 and 8/9 ERT-experienced patients and 6/6, 6/6, 5/5 and 4/5 ERT-naïve patients, respectively. Among patients who had CK data available at baseline and Month 48, six of nine (66.7%) ERT-experienced patients had abnormal CK levels at baseline and two of these (33.3%) had normal CK levels at Month 48; two of five (40.0%) ERT-naïve patients had abnormal CK levels at baseline that remained abnormal at Month 48. Similar trends were observed with urine Hex4, which decreased numerically from baseline after 12, 24, 36, and 48 months of follow-up in 16/16, 11/14, 11/12 and 6/9 ERT-experienced patients and 5/6, 5/6, 4/5 and 4/5 ERT-naïve patients, respectively (FIG. 49A, Table 26B). Among patients who had Hex4 data available at baseline and at Month, five out of nine (55.6%) ERT-experienced patients had abnormal Hex4 levels at baseline and four of these (80.0%) had normal Hex4 levels at Month 48; four out of five (80.0%) ERT-naïve ambulatory patients, had abnormal Hex4 measurements at baseline and one of these (25.0%) returned to normal Hex4 levels at Month 48.

Non-ambulatory ERT-experienced patients showed durable reductions from baseline in plasma CK levels: 20.8% (n=5), 25.3% (n=5), 27.1% (n=3) and 23.7% (n=2) at 12, 24, 36, and 48 months, respectively. In addition, a consistent decrease was also seen in urine Hex4 levels from baseline over the follow-up period. After 12, 24, 36 and 48 months, Hex4 levels decreased by 15.6% (n=5), 34.1% (n=5), 36.5% (n=4) and 9.4% (n=2) from baseline, respectively (Table 26B).

### Pharmacokinetic outcomes

Blood sampling for plasma GAA protein and miglustat concentrations were taken for cohort 1 (ERT-experienced) and cohort 3 (ERT-naïve) only. To prevent any undue burden on patients, blood sampling for PK was not performed for non-ambulatory patients (cohort 2) and, due to similarity to cohort 1, those in cohort 4. Plasma total GAA protein, measured by signature peptide T09, increased in a dose-dependent manner after single ascending doses of 5 mg/kg, 10 mg/kg and 20 mg/kg cipaglucosidase alfa. Generally, the median time of peak signature peptide T09 concentrations (tₘₐₓ) was similar across all treatments and consistent with the duration of cipaglucosidase infusion of approximately 4 hours. Exposures (Cₘₐₓ and the area under the plasma drug concentration-time curve [AUC]) increased in a dose-dependent manner. Plasma total GAA protein exposures were similar between cohort 1 and cohort 3 ambulatory patients. Plasma miglustat exposures increased in a dose dependent manner in cohort 1 and cohort 3 ambulatory patients. 48-month sparse PK sampling in Cohort 1 subjects indicated exposures were similar to exposures early in the study (Stage 2), and confirmed lack of immunogenicity impact on cipaglucosidase alfa exposures.

Administration of miglustat one hour before the start of cipaglucosidase alfa infusion resulted in greater AUCs than 20 mg/kg cipaglucosidase alfa alone. The distribution phase half-life was notably higher following miglustat co-administration. Miglustat was rapidly cleared from the circulation.

The efficacy data across the important domains of Pompe disease showed that co-administration of 20 mg/kg IV-infused cipaglucosidase alfa and 260 mg miglustat resulted in clinically meaningful improvements in motor function, PFTs, and muscle strength. Improvements in ambulatory subjects were demonstrated across various efficacy domains, and evidence presented in Table 26A generally supports maintenance of improvements through 48 months (where data were available).

**Table 26A: Summary of Efficacy Assessments in Ambulatory Subjects - Change from Baseline in Stage 3 and Stage 4 at Month 24 and 48 - Study ATB200-02**

| **Clinical Response Assessments (Selected Pharmacodynamic and Efficacy)** | **Mean (SD) Baseline and Change from Baseline at 24 and 48 Months** | | | | | | | | |
|---|---|---|---|---|---|---|---|---|---|
| | **ERT-experienced Cohort 1 + Cohort 4 (N = 16)^{a}** | | | **ERT-naïve Cohort 3 (N = 6)^{a}** | | | **Overall Cohort 1 + Cohort 3 + Cohort 4 (n = 22)^{a}** | | |
| | **BSL** | **24-mo CFB** | **48-mo CFB** | **BSL** | **24-mo CFB** | **48-mo CFB** | **BSL** | **24-mo CFB** | **48-mo CFB** |
| 6MWD (m) | 393.5 (119.66) (n=16) | 25.2 (63.30) (n=13) | 20.7 (101.84) (n = 9) | 396.0 (75.20) (n = 6) | 54.4 (36.18) (n = 6) | 52.2 (46.59) (n = 4) | 394.2 (107.59) (n = 22) | 34.4 (56.82) (n=19) | 30.4 (87.67) (n=13) |
| Sitting FVC (% predicted) | 57.4 (17.42) (n = 16) | 1.0 (7.96) (n=13) | 1.0 (6.42) (n = 6) | 57.2 (20.84) (n = 6) | 4.7 (5.09) (n = 6) | 8.3 (4.50) (n = 4) | 57.3 (17.89) (n = 22) | 2.2 (7.24) (n=19) | 3.9 (6.61) (n=10) |
| MMT (total score) | 65.1 (6.46) (n = 15) | 2.5 (3.45) (n=13) | 4.0 (3.70) (n = 8) | 67.2 (3.70) (n = 5) | 2.0 (5.70) (n = 5) | -1.3 (4.99) (n = 4) | 65.6 (5.88) (n = 20) | 2.4 (4.02) (n=18) | 2.3 (4.71) (n=12) |
| GSGC | 14.3 (5.22) (n = 16) | -1.6 (5.20) (n=14) | -1.0 (6.02) (n = 9) | 12.8 (3.54) (n = 6) | -1.7 (2.07) (n = 6) | -2.4 (2.70) (n = 5) | 13.9 (4.79) (n = 22) | -1.7 (4.43) (n=20) | -1.5 (5.00) (n=14) |

| | | | | | | | | | |
|---|---|---|---|---|---|---|---|---|---|
| Abbreviations: 6MWD = 6-minute walk distance; BSL = baseline; CFB = change from baseline; ERT = enzyme replacement therapy; FVC = forced vital capacity; GSGC = Gait, Stair, Gowers' maneuver, and Chair test; MMT = manual muscle testing; mo = month(s); N = number of subjects Note: For GSGC, a negative number indicates an improvement. ^{a} n at baseline. Any differences in the n at baseline are indicated in parentheses. | | | | | | | | | |

**Table 26B: Summary of Clinical Outcomes for Ambulatory and Non-Ambulatory**

| **Outcome measure** | **Ambulatory patients** | | | | | | | |
|---|---|---|---|---|---|---|---|---|
| | ERT-experienced | | | | ERT-naïve | | | |
| **Timepoint (months)** | 12 | 24 | 36 | 48 | 12 | 24 | 36 | 48 |
| **Motor function** | | | | | | | | |
| Percent predicted | | | | | | | | |
| 6MWD | | | | | | | | |
| n | 16 | 13 | 12 | 9 | 6 | 6 | 5 | 4 |
| CFBL mean (SD) | 6.1 (7.84) | 5.4 (10.56) | 3.4 (14.66) | 5.9 (17.36) | 10.7 (3.93) | 11.0 (5.06) | 9.0 (7.98) | 11.7 (7.69) |
| 6MWD, meters n | | | | | | | | |
| CFBL mean (SD) | 16 | 13 | 12 | 9 | 6 | 6 | 5 | 4 |
| | 33.5 (49.62) | 25.2 (63.30) | 9.8 (85.98) | 20.7 (101.94) | 57.0 (29.96) | 54.4 (36.18) | 43.5 (45.19) | 52.2 (46.59) |
| 10-Meter Walk Test, seconds n | | | | | | | | |
| CFBL mean (SD) | 16 | 14 | 12 | 9 | 6 | 6 | 5 | 5 |
| | -0.1 (1.47) | 1.0 (3.19) | 3.9 (12.56) | 0.6 (1.74) | -0.9 (0.75) | -0.5 (1.00) | 0.1 (0.61) | 0.7 (1.66) |
| Gait, Stairs, Gowers, and Chair | | | | | | | | |
| n | 16 | 14 | 12 | 9 | 6 | 6 | 5 | 5 |
| CFBL mean (SD) | -1.3 (4.39) | -1.6 (5.20) | -1.1 (5.18) | -1.0 (6.02) | -0.8 (2.23) | -1.7 (2.07) | -2.0 (2.12) | -2.4 (2.70) |
| Timed Up and Go, seconds | | | | | | | | |
| n | 15 | 13 | 12 | 9 | 6 | 6 | 5 | 5 |
| CFBL mean (SD) | -1.5 (2.68) | 0.1 (2.24) | -1.5 (3.68) | -0.6 (2.41) | -0.7 (1.95) | -1.0 (2.00) | 0.5 (3.59) | 0.3 (2.33) |

| **Pulmonary function** | | | | | | | | |
|---|---|---|---|---|---|---|---|---|
| Percent predicted sitting | | | | | | | | |
| FVC | 16 | 13 | 10 | 6 | 6 | 6 | 5 | 4 |
| n | -1.2 (5.95) | 1.0 (7.96) | -0.3 (6.68) | 1.0 (6.42) | 3.2 (8.42) | 4.7 (5.09) | 6.2 (3.35) | 8.3 (4.50) |
| CFBL mean (SD) | | | | | | | | |
| Percent predicted maximum inspiratory pressure % | | | | | | | | |
| n | 16 | 13 | 11 | 6 | 6 | 6 | 5 | 4 |
| CFBL mean (SD) | 3.7 (11.61) | 4.5 (20.67) | -0.5 (22.23) | -3.8 (5.41) | 8.0 (15.46) | 18.7 (23.90) | 12.5 (20.71) | 16.1 (17.23) |
| Percent predicted maximum expiratory pressure % | | | | | | | | |
| n | 16 | 13 | 11 | 6 | 6 | 6 | 5 | 4 |
| CFBL mean (SD) | 9.4 (21.37) | 16.8 (21.88) | 13.7 (26.79) | 17.9 (30.81) | 11.6 (18.16) | 17.9 (16.25) | 14.2 (20.27) | 24.8 (24.15) |
| Percent predicted Sniff Nasal Inspiratory Pressure % | | | | | | | | |
| n | 16 | 13 | 11 | 6 | 6 | 6 | 5 | 4 |
| CFBL mean (SD) | 2.1 (18.56) | 5.3 (23.86) | -2.3 (23.65) | 7.3 (21.79) | 9.4 (13.04) | 8.1 (11.48) | 4.3 (9.30) | 4.6 (7.76) |

| Muscle strength | | | | | | | | |
|---|---|---|---|---|---|---|---|---|
| Lower body MMT score | | | | | | | | |
| n | 15 | 13 | 10 | 8 | 5 | 5 | 4 | 4 |

| **Patient-reported outcomes** | | | | | | | | |
|---|---|---|---|---|---|---|---|---|
| CFBL mean (SD) | 3.1 (2.34) | 2.1 (2.18) | 2.5 (3.69) | 3.5 (2.51) | 2.8 (4.97) | 3.0 (3.94) | (3.3 (2.75) | (1.0) 3.83) |
| Upper body MMT score | | | | | | | | |
| n | 15 | 13 | 11 | 8 | 6 | 6 | 5 | 5 |
| CFBL mean (SD) | 1.3 (2.52) | 0.5 (2.30) | 0.8 (2.75) | 0.5 (2.00) | 0.0 (1.67) | -1.5 (2.17) | 1.0 (1.87) | -1.4 (2.61) |
| Total MMT score | | | | | | | | |
| n | 15 | 13 | 10 | 8 | 5 | 5 | 4 | 4 |
| CFBL mean (SD) | 4.3 (3.20) | 2.5 (3.45) | 3.6 (5.23) | 4.0 (3.70) | 2.4 (4.39) | 2.0 (5.70) | 3.5 (3.00) | -1.3 (4.99) |
| Rasch-built Pompe-specific Activity | | | | | | | | |
| n | | | | | | | | |
| CFBL mean (SD) | 15 | 13 | 11 | 9 | 6 | 6 | 5 | 5 |
| | 1.3 (2.97) | 2.2 (1.52) | -0.1 (3.24) | -0.3 (2.96) | 2.0 (3.46) | 2.2 (3.43) | 1.4 (1.52) | -0.6 (5.22) |
| Rotterdam Handicap Scale | | | | | | | | |
| n | | | | | | | | |
| CFBL mean (SD) | 15 | 14 | 11 | 9 | 6 | 6 | 5 | 5 |
| | -0.3 (3.01) | -1.1 (3.91) | -2.3 (4.44) | -2.3 (3.16) | -0.4 (2.06) | -0.4 (2.14) | -1.2 (0.84) | -1.3 (2.56) |
| Fatigue Severity Scale | | | | | | | | |
| n | | | | | | | | |
| CFBL mean (SD) | 15 | 14 | 11 | 9 | 6 | 6 | 5 | 5 |
| | -2.9 (10.55) | -3.1 (8.82) | 1.4 (16.74) | -3.4 (10.10) | -6.3 (7.06) | -5.5 (11.40) | -6.2 (10.57) | 1.2 (7.95) |

| **Biomarkers** | | | | | | | | |
|---|---|---|---|---|---|---|---|---|
| Serum creatine kinase, U/L | | | | | | | | |
| n | | | | | | | | |
| % CFBL mean (SD) | 15 | 15 | 11 | 9 | 6 | 6 | 5 | 5 |
| | -20.5 (35.38) | -29.9 (21.67) | -30.0 (35.59) | -35.3 (27.11) | -50.6 (20.18) | -46.5 (19.32) | -50.7 (14.70) | -35.9 (36.12) |
| Urinary Hex4, mmol/mol creatine | | | | | | | | |
| n | 16 | 14 | 12 | 9 | 6 | 6 | 5 | 5 |
| % CFBL mean (SD) | -36.11 (20.40) | -11.8 (68.57) | -35.1 (42.20) | -4.0 (82.57) | -40.7 (40.60) | -49.5 (48.06) | -39.3 (59.19) | -39.9 (44.68) |

| **Outcome measure** | **Non-ambulatory patients** | | | |
|---|---|---|---|---|
| **Timepoint (months)** | 12 | 24 | 36 | 48 |
| **Motor function** | n/a | n/a | n/a | n/a |
| Percent predicted 6MWD | n/a | n/a | n/a | n/a |
| n | | | | |
| CFBL mean (SD) | | | | |
| 6MWD, meters | n/a | n/a | n/a | n/a |
| n | | | | |
| CFBL mean (SD) | | | | |
| 10-Meter Walk Test, seconds | n/a | n/a | n/a | n/a |
| n | | | | |
| CFBL mean (SD) | | | | |
| Gait, Stairs, Gowers, and Chair | n/a | n/a | n/a | n/a |
| n | | | | |
| CFBL mean (SD) | | | | |
| Timed Up and Go, seconds | n/a | n/a | n/a | n/a |
| n | | | | |
| CFBL mean (SD) | | | | |
| **Pulmonary function** | | | | |
| Percent predicted sitting FVC | | | | |
| n | 2 | 1 | 2 | 1 |
| CFBL mean (SD) | 2.5 (9.19) | 2.0 (N/A) | -2.0 (7.07) | -1.0 (N/A) |
| Percent predicted maximum inspiratory pressure % | | | | |
| n | 3 | 2 | 3 | 2 |
| CFBL mean (SD) | 1.1 (4.71) | 3.3 (1.39) | -0.4 (5.20) | 3.9 (2.35) |
| Percent predicted maximum expiratory pressure % | | | | |
| n | 3 | 2 | 3 | 2 |
| CFBL mean (SD) | 10.6 (10.28) | 21.1 (25.72) | 11.5 (22.30) | 21.0 (27.00) |
| Percent predicted Sniff Nasal Inspiratory Pressure % | | | | |
| n | 3 | 2 | 3 | 2 |
| CFBL mean (SD) | 4.5 (13.96) | 7.8 (13.65) | -0.4 (4.03) | -4.1 (3.26) |
| Muscle strength | | | | |
| Lower body MMT score | | | | |
| n | N/A | N/A | N/A | N/A |
| CFBL mean (SD) | N/A | N/A | N/A | N/A |
| Upper body MMT score | | | | |
| n | 4 | 4 | 4 | N/A |
| CFBL mean (SD) | 1.3 (3.40) | 2.0 (6.63) | -0.8 (10.72) | N/A |
| Total MMT score | | | | |
| n | 4 | 4 | 4 | N/A |
| CFBL mean (SD) | 1.3 (3.40) | 2.0 (6.63) | -0.8 (10.72) | N/A |

| **Patient-reported outcomes** | | | | |
|---|---|---|---|---|
| Rasch-built Pompe-specific Activity | | | | |
| n | 5 | 4 | 4 | 2 |
| CFBL mean (SD) | 1.4 (1.95) | 0.8 (0.96) | 0.8 (0.96) | 2.0 (4.24) |
| Rotterdam Handicap Scale | | | | |
| n | 4 | 4 | 4 | 2 |
| CFBL mean (SD) | 0.6 (3.15) | 0.6 (4.66) | 0.6 (1.67) | -1.5 (4.95) |
| Fatigue Severity Scale | | | | |
| n | 5 | 5 | 5 | 2 |
| CFBL mean (SD) | -9.2 (11.37) | -23.0 (17.78) | -10.0 (9.35) | -12.0 (15.56) |
| **Biomarkers** | | | | |
| Serum creatine kinase, U/L | | | | |
| n | 5 | 5 | 4 | 2 |
| % CFBL mean (SD) | -20.8 (8.84) | -25.3 (16.83) | -27.1 (32.43) | -23.7 (23.90) |
| Urinary Hex4, mmol/mol creatine | | | | |
| n | 5 | 5 | 4 | 2 |
| % CFBL mean (SD) | -15.6 (16.67) | -34.1 (14.08) | -36.5 (23.13) | -9.4 (1.71) |

**Table 26C: Summary of Subject and Physician Global Impression of Change at 48 months follow-up**

| | **Ambulatory ERT-experienced** | | **Non-ambulatory ERT-experienced** | **Ambulatory ERT-naïve** |
|---|---|---|---|---|
| | **Cohort 1** | **Cohort 4** | **Cohort 2** | **Cohort 3** |
| | **N=7** | **N=6** | **N=5** | **N= 6** |
| **SGIC; Overall physical wellbeing** | | | | |
| Improved, n (%) | 6 (85.7) | 4 (66.7) | 3 (60.0) | 5 (83.3) |
| No change, n (%) | 1 (14.3) | 1 (16.7) | 2 (40.0) | 1 (16.7) |
| Declined, n (%) | 0(0) | 1 (16.7) | 0 (0) | 0 (0) |

| **PGIC** | | | | |
|---|---|---|---|---|
| Improved, n (%) | 4 (57.1) | 2 (33.3) | 3 (60.0) | 6 (100.0) |
| No change, n (%) | 3 (42.9) | 4 (66.7) | 2 (40.0) | 0 (0) |
| Declined, n (%) | 0(0) | 0 (0) | 0 (0) | 0(0) |

### Example 19: Study ATB200-03

Study ATB200-03 is the pivotal Phase 3 study in the clinical program and the efficacy of cipaglucosidase alfa/miglustat is mainly based on summaries of the primary and key secondary efficacy results from this study.

A total of 123 subjects were randomized and dosed (95 ERT-experienced and 28 ERT-naive) and 117 completed the study (with 6 discontinuations ERT-experienced Population) at 62 sites across 24 countries. The mean (SD) duration of exposure was similar between subjects treated with cipaglucosidase alfa/miglustat and alglucosidase alfa/placebo (11.8 (1.80) months and 12.0 (0.71) months, respectively) with a maximum duration of 14.8 months and 12.9 months, respectively. There was a very low drop-out rate, and all 117 subjects completing the study subsequently enrolled in the open-label extension, Study ATB200-07.

Baseline demographics were representative of the population and generally similar between the cipaglucosidase alfa/miglustat and alglucosidase alfa/placebo treatment arms. Most subjects (95 [77.2%]) were ERT-experienced, with a mean (SD) ERT treatment duration of 7.4 (3.45) years. Subjects received prior ERT for an average of 7.5 years in the cipaglucosidase alfa/miglustat group and 7.1 years in the alglucosidase alfa/placebo group.

Baseline 6MWD and FVC, as well as MMT and GSGC score were representative of the population and generally similar in the treatment groups. Baseline 6MWD mean (SD) was 357.9 (111.84) meters and 350.1 (119.78) meters, respectively, for subjects in the cipaglucosidase alfa/miglustat and alglucosidase alfa/placebo arms. Within each treatment arm, baseline 6MWD and FVC were both higher in ERT-naïve subjects compared with ERT-experienced subjects (Table 27).

**Table 27: Baseline 6MWD and % Predicted FVC - Study ATB200-03**

| **Endpoint** | **Cipaglucosidase Alfa/Miglustat** | | **Alglucosidase Alfa** | |
|---|---|---|---|---|
| **6MWD, m** | **(N = 85)** | | **(N = 38)** | |
| Mean (SD) | 357.93 (111.843) | | 350.14 (119.776) | |
| Median (min, max) | 359.50 (79.0, 575.0) | | 358.45 (112.5, 623.0) | |

| | **ERT-experienced** | **ERT-naïve** | **ERT-experienced** | **ERT-naïve** |
|---|---|---|---|---|
| n | 65 | 20 | 30 | 8 |
| Mean (SD) | 346.94 (110.213) | 393.64 (112.388) | 334.62 (114.023) | 408.34 (130.628) |
| Median (min, max) | 352.50 (79.0, 557.5) | 375.15 (154.0, 575.0) | 343.50 (112.5, 532.3) | 384.25 (201.0, 623.0) |

| **% Predicted FVC, Sitting** | **N = 85** | | **N = 38** | |
|---|---|---|---|---|
| Mean (SD) | 70.74 (19.573) | | 70.04 (21.301) | |
| Median (min, max) | 70.00 (30.5, 132.5) | | 71.25 (31.5, 122.0) | |

| | **ERT-experienced** | **ERT-naïve** | **ERT-experienced** | **ERT-naïve** |
|---|---|---|---|---|
| **Endpoint** | **Cipaglucosidase Alfa/Miglustat** | | **Alglucosidase Alfa** | |
| **6MWD, m** | **(N = 85)** | | **(N = 38)** | |
| n | 65 | 20 | 30 | 8 |
| Mean (SD) | 67.85 (19.052) | 80.15 (18.689) | 67.48 (20.993) | 79.63 (20.962) |
| Median (min, max) | 68.00 (30.5, 132.5) | 82.25 (48.0, 111.0) | 69.00 (31.5,122.0) | 88.50 (46.5, 98.0) |

| | | | | |
|---|---|---|---|---|
| Abbreviations: 6MWD = 6-minute walk distance; ERT = enzyme replacement therapy; FVC = forced vital capacity; max = maximum; min = minimum; N = number of subjects in each population; n = number of subjects analyzed at baseline; SD = standard deviation | | | | |

The test for the primary endpoint was conducted first at the 1-sided 0.025 significance level, and if significant, the ordered key secondary endpoints were similarly tested at the same significance level. The statistical significance of the key secondary endpoints was interpreted following a hierarchical testing order, each at the 1-sided alpha level of 0.025. If at any point the null hypothesis failed to be rejected, then that comparison and any other comparison below it could not be claimed as statistically significant on superiority, and subsequent analyses would be to assess for nominal significance on superiority. Primary analyses of efficacy were performed using the mixed-effect model for repeated measures (MMRM) for 6MWD in Intent-to-treat Observed Population (ITT) that includes all available, observed data without any missing data imputation (ITT-OBS) and analysis of covariance (ANCOVA) for all other endpoints in the Intent-to-Treat-Last Observation Carried Forward (ITT-LOCF) Population. A nonparametric randomization based ANCOVA was specified as the first sensitivity analysis for 6MWD and FVC. This nonparametric analysis was to be conducted formally if the primary parametric analyses failed to meet assumptions of normality. For the purposes of data presentation in this document, 2-sided p-values are reported hereafter, except as otherwise noted.

Table 28 summarizes results for the primary and 6 key secondary endpoints for the overall ITT Population that includes both ERT-experienced and ERT-naïve subjects. 6MWD showed greater improvement with cipaglucosidase alfa/miglustat versus alglucosidase alfa/placebo but did not reach statistical significance for superiority over approved treatment (p = 0.608). On % predicted FVC, cipaglucosidase alfa/miglustat demonstrated a nominally statistically significant (p = 0.048) and clinically meaningful difference for superiority versus alglucosidase alfa/placebo in the overall ITT Population.

Results for other key secondary endpoints for motor function, pulmonary function, muscle strength, and PROs numerically favored cipaglucosidase alfa/miglustat over alglucosidase alfa/placebo, with GSGC also showing nominal statistical significance on superiority.

**Table 28: Summary of Results on Primary and Key Secondary Endpoints (ITT Population) - Study ATB200-03**

| **Level** | | **Number of Observations Used for Analysis in Cipaglucosidase Alfa/Miglustat** | **Number of Observations Used for Analysis in Alglucosidase Alfa/Placebo** | **LS Mean Treatment Difference (SE)^{a}** | **95% CI of Difference** | **2-sided p-value** |
|---|---|---|---|---|---|---|
| | **Endpoint** | | | | | |
| Primary endpoint^{b} | | | | | | |
| | CHG to Week 52 in 6MWD (m) | 81 | 37 | 5.33 (10.367) | (-15.21, 25.88) | 0.608 |

| First key secondary endpoint^{c} | | | | | | |
|---|---|---|---|---|---|---|
| | CHG to Week 52 in sitting % predicted FVC | 84 | 38 | 2.32 (1.162) | (0.02, 4.62) | 0.048 |

| Additional key secondary endpoints | | | | | | |
|---|---|---|---|---|---|---|
| | CHG to Week 52 in MMT lower extremity score | 80 | 35 | 0.99 (0.716) | (-0.43, 2.41) | 0.168 |
| | CHG to Week 26 in 6MWD (m) | 85 | 38 | 5.46 (6.484) | (-7.39, 18.30) | 0.402 |

**Table 28: Summary of Results on Primary and Key Secondary Endpoints (ITT Population) - Study ATB200-03 (Continued)**

| **Level** | | **Number of Observations Used for Analysis in Cipaglucosidase Alfa/Miglustat** | **Number of Observations Used for Analysis in Alglucosidase Alfa/Placebo** | **LS Mean Treatment Difference (SE)^{a}** | **95% CI of Difference** | **2-sided p-value** |
|---|---|---|---|---|---|---|
| | **Endpoint** | | | | | |
| | CHG to Week 52 in PROMIS-Physical Function total score^{d} | 84 | 38 | 1.37 (1.714) | (-2.03, 4.77) | 0.426 |
| | CHG to Week 52 in PROMIS-Fatigue total score^{d} | 85 | 38 | 0.13 (1.079) | (-2.00, 2.27) | 0.903 |
| | CHG to Week 52 in GSGC total score | 72 | 31 | -1.30 (0.524) | (-2.34, -0.26) | 0.015 |

| | | | | | | |
|---|---|---|---|---|---|---|
| Abbreviations: 6MWD = 6-minute walk distance; ANCOVA = analysis of covariance; CHG = change from baseline; CI = confidence interval; FVC = forced vital capacity; GSGC = Gait, Stairs, Gowers' maneuver, and Chair test; ITT = Intent-to-Treat; ITT-OBS = Intent-to-Treat Population that includes all available, observed data without any missing data imputation at Week 52; LOCF = last observation carried forward; LS = least squares; MMRM = mixed-effect model repeated measures; MMT = manual muscle testing; PROMIS = Patient-reported Outcomes Measurement Information System; SE = standard error ^{a} Cipaglucosidase alfa/miglustat - alglucosidase alfa/placebo. ^{b} MMRM approach was used for the primary analysis of the primary endpoint based on ITT-OBS Population. ^{c} ANCOVA model was used for the primary analysis of the key secondary endpoints based on the ITT-LOCF Population. ^{d} The total score was calculated by summing scores (1 to 5) across all items. | | | | | | |

It should be noted that there were aberrant data points from Subject 4005-2511, an ERT-naive subject, in the comparator arm that turned out to be very influential outliers and distorted the mean change estimates calculated from the raw data. The data point from this 1 subject alone accounted for approximately 56% of the mean change from baseline at Week 52 in the comparator arm (ie, alglucosidase alfa/placebo) and inflated the variance in the comparator arm to approximately 6 times that of the cipaglucosidase alfa/miglustat arm.

A prespecified outlier exclusion analysis of 6MWD in the overall ITT Population that excluded outliers with externally studentized residuals > 3 in magnitude was performed and the subjects treated with cipaglucosidase alfa/miglustat arm walked an average of 14 meters farther than subjects in the alglucosidase alfa/placebo arm (p = 0.100) (Table 29). These results are consistent with the results from the overall ITT Population where Subject 4005-2511 was excluded.

**Table 29: Sensitivity Analysis for Primary Endpoint: ANCOVA for Change from Baseline in 6MWD (meters) at Week 52 (ITT-LOCF Population Excluding Outliers with Externally Studentized Residuals > 3) - Study ATB200-03**

| | **Cipaglucosidase Alfa/Miglustat (N = 85)** | **Alglucosidase Alfa/Placebo (N = 37)** |
|---|---|---|
| Parameter estimation and comparison from ANCOVA^{a} | | |
| n | 85 | 37 |
| LS Mean (SE) | 20.91 (4.519) | 6.95 (6.951) |
| 95% CI of LS Mean | (11.96, 29.86) | (-6.82, 20.72) |
| Difference in LS Mean (SE) (cipaglucosidase alfa/miglustat - alglucosidase alfa/placebo) | | 13.96 (8.421) |
| 95% CI of Difference | | (-2.72, 30.64) |
| 2-sided p-value | | 0.100 |

| | | |
|---|---|---|
| Abbreviations: 6MWD = 6-minute walk distance; ANCOVA = analysis of covariance; CI = confidence interval; ERT = enzyme replacement therapy; ITT-LOCF = Intent-to-Treat-Last Observation Carried Forward; LS = least squares; N = number of subjects in each treatment group; n = number of subjects with available data; SE = standard error Note: ANCOVA was based on ITT-LOCF excluding outliers with externally studentized residuals > 3 in magnitude (absolute value). ^{a} All estimates were obtained from the ANCOVA model including terms for treatment, baseline 6MWD, age, height, weight (all as continuous covariates), ERT-status (ERT-naïve versus ERT-experienced), and gender. | | |

The assumptions for the MMRM analysis of 6MWD including the normality assumption were checked. Based on the examination of the diagnostic plot and the Shapiro-Wilk test, the normality assumption for the MMRM analysis was clearly violated (Shapiro-Wilk test p < 0.01). Therefore, a nonparametric randomization-based covariance analysis was performed as specified to provide a better estimate for the treatment effect. The nonparametric randomization-based covariance analysis had a least squares (LS) mean treatment difference (95% confidence interval [CI]) of 6.61 (-15.12, 28.35), with a p-value of 0.551.

To support the clinical significance and consistency of the treatment effect of cipaglucosidase alfa/miglustat on the primary and key secondary endpoints of 6MWD and % predicted FVC, both important domains of disease in patients with Pompe disease, a post hoc global test was performed (Table 30). This global test was conducted by ranking individual subject response separately for each of these same 2 endpoints from least improvement to greatest improvement, summing the 2 ranks for each subject, and analyzing the summed ranks using the Wilcoxon rank-sum test to assess superiority of cipaglucosidase alfa/miglustat versus alglucosidase alfa/placebo. The global test showed greater improvement in either 6MWD or FVC in patients treated with cipaglucosidase alfa/miglustat versus alglucosidase alfa/placebo (p = 0.022). This global test supports the results observed on each of these endpoints separately in the overall ITT Population studied.

**Table 30: Wilcoxon Rank Sum Test Based on Sum of Ranks for 6MWD and FVC (ITT Population) - Study ATB200-03**

| **Treatment** | **N** | **Sum of Scores** | **Mean Score** | **Statistic** | **Z-statistic** | **2-sided p-value^{a}** |
|---|---|---|---|---|---|---|
| Alglucosidase alfa/placebo | 38 | 1915.5 | 50.4 | 1916 | -2.3 | 0.022 |
| Cipaglucosidase alfa/miglustat | 84^{b} | 5587.5 | 66.5 | | | |

| | | | | | | |
|---|---|---|---|---|---|---|
| Abbreviations: 6MWD = 6-minute walk distance; FVC = forced vital capacity; ITT = Intent-to-Treat; N = number of subjects Note: This global test was conducted by ranking individual subject response (ie, change from baseline to Week 52) separately for each endpoint from least improvement to greatest improvement, summing the 2 ranks for each subject, and analyzing the summed ranks using the Wilcoxon rank sum test. ^{a} P-values are from the Wilcoxon 2-sample test with t-approximation. ^{b} For % predicted FVC (N = 84), Subject 2301-1421 had baseline result of 70.5% but subsequently withdrew from the study due to not wanting to travel to the site and was therefore excluded from analysis. | | | | | | |

After data base lock, discussions with the principal investigator revealed that Subject 4005-2511 deliberately underperformed on the 6MWD and pulmonary function testing at screening to ensure that he would meet inclusion criteria and gain entry into the study. A prespecified sensitivity analysis excluding outlier data points was performed, which excluded this subject's datapoints. Given the subject admitted underperformance on his screening test, his clinically implausible results, and the prespecified outlier sensitivity analysis indicating substantial differences from the ITT analysis that included the subject, all efficacy analyses on the ITT Population were performed with and without this outlier subject. The details of this subject and the results for secondary analyses with this subject included in the overall and ERT-naïve Populations can be found in the Study ATB200-03 CSR. It should be noted that the outlier subject was in the ERT-naïve Population. Therefore, the analysis results in the ERT-experienced Population were not affected.

### Primary Endpoint: 6MWD

In Study ATB200-03, the mean (SD) change in 6MWD (meters) from baseline to Week 52 showed a mean improvement of 20.6 (42.27) meters for the cipaglucosidase alfa/miglustat group compared to 8.0 (40.56) meters for the alglucosidase alfa/placebo group. Therefore, subjects treated with cipaglucosidase alfa demonstrated improvements over time in 6MWD, out to 52 weeks. Similar to the overall ITT Population, even after excluding the outlier, the normality assumption for the MMRM analysis was violated based on the examination of the diagnostic plot and Shapiro-Wilk test (Shapiro-Wilk test p-value < 0.01). Therefore, the nonparametric analysis based ANCOVA was performed as in the overall ITT Population including the outlier.

The nonparametric randomization-based covariance analysis had an LS mean treatment difference (95% CI) of 13.66 (-1.17, 28.48), with a p-value of 0.071.

Table 31 summarizes the mean change in 6MWD (meters) by visit (ITT Population) and MMRM analysis (ITT-OBS Population) for the ERT-experienced Population. Table 31 also summarizes the nonparametric randomization-based covariance analysis for change in 6MWD at Week 52 for subjects in the ITT-LOCF Population. FIG. 23B displays a line plot of the summary statistics by visit.

**Table 31: Summary of Change in 6MWD (meters) by Visit from Baseline to Week 52 (ITT Population) and MMRM Analysis (ITT-OBS Population Excluding Subject 4005-2511) - Study ATB200-03**

| | | **Cipaglucosidase Alfa/Miglustat (N = 85)** | | **Alglucosidase** Alfa/Placebo **(N = 37)** | |
|---|---|---|---|---|---|
| | | **Absolute Value** | **CHG** | **Absolute Value** | **CHG** |
| Baseline^{a} | | | | | |
| | n | 85 | -- | 37 | -- |
| | Mean (SD) | 357.93 (111.843) | -- | 350.95 (121.322) | -- |
| | Median (Q1, Q3) | 359.50 (298.90, 418.50) | -- | 365.50 (285.50, 420.00) | -- |
| | Min, max | 79.0, 575.0 | -- | 112.5, 623.0 | -- |

| Week 52-Observed | | | | | |
|---|---|---|---|---|---|
| | n | 81 | 81 | 36 | 36 |
| | Mean (SD) | 376.41 (122.925) | 20.56 (42.266) | 359.70 (137.361) | 8.02 (40.564) |
| | Median (Q1, Q3) | 380.50 (300.00, 465.00) | 12.50 (-3.75, 43.50) | 371.75 (262.50, 457.50) | 3.15 (-21.75, 22.78) |
| | Min, max | 79.6, 601.5 | -59.5, 173.5 | 67.0, 648.6 | -55.6, 127.0 |

| Week 52-LOCF | | | | | |
|---|---|---|---|---|---|
| | n | 85 | 85 | 37 | 37 |
| | Mean (SD) | 378.72 (123.348) | 20.79 (42.773) | 358.19 (135.750) | 7.24 (40.277) |
| | Median (Q1, Q3) | 380.50 (300.00, 473.00) | 12.50 (-6.90, 43.50) | 370.00 (263.00, 446.50) | 1.40 (-21.00, 20.00) |
| | Min, max | 79.6, 601.5 | -59.5, 173.5 | 67.0, 648.6 | -55.6, 127.0 |

| MMRM parameter estimation and comparison at Week 52^{b} | | | | | |
|---|---|---|---|---|---|
| | | **Cipaglucosidase Alfa/Miglustat (N = 85)** | | **Alglucosidase Alfa/Placebo (N = 37)** | |
| | | **Absolute Value** | **CHG** | **Absolute Value** | **CHG** |
| | n | 81 | | 36 | |
| | LS mean (SE) | 21.31 (4.649) | | 7.10 (7.043) | |
| | 95% CI of LS mean | (12.10, 30.53) | | (-6.86, 21.06) | |
| | Difference of LS means (SE) (cipaglucosidase alfa/miglustat - alglucosidase alfa/placebo) | | | 14.21 (8.481) | |
| | 95% CI of difference | | | (-2.60, 31.02) | |
| | 2-sided p-value | | | 0.097 | |

| Parameter estimation and comparison from nonparametric ANCOVA^{c} | | | | | |
|---|---|---|---|---|---|
| | Difference in LS mean (SE) (cipaglucosidase alfa - alglucosidase alfa/placebo) | | | 13.66 (7.564) | |
| | 95% CI of difference | | | (-1.17, 28.48) | |
| | 2-sided p-value | | | 0.071 | |

| | | | | | |
|---|---|---|---|---|---|
| Abbreviations: 6MWD = 6-minute walk distance; ANCOVA = analysis of covariance; CHG = change from baseline; CI = confidence interval; ERT = enzyme replacement therapy; ITT = Intent-to-Treat; ITT-OBS = Intent-to-Treat Population that includes all available, observed data without any missing data imputation at Week 52; LOCF = last observation carried forward; LS = least squares; max = maximum; min = minimum; MMRM = mixed-effect model for repeated measures; N = number of subjects in each treatment group; n = number of subjects with available data; Q1 = first quartile; Q3 = third quartile; SD = standard deviation; SE = standard error ^{a} Baseline was the average of the last 2 values obtained on or prior to the first dose date. ^{b} The MMRM approach (using restricted maximum likelihood estimation) was used for analysis. The model included terms for treatment, baseline 6MWD, age, height, weight (all as continuous covariates), ERT status (ERT-naive versus ERT-experienced), gender, time, and treatment-by-time interaction. Time was used as a repeated measure, and an unstructured covariance approach was applied. ^{c} Nonparametric ANCOVA compared between the treatment groups, adjusting for baseline 6MWD, age, height, weight (all as continuous covariates), ERT status (ERT-naive versus ERT-experienced) as strata, and gender. | | | | | |

### Secondary Endpoint: Sitting % Predicted FVC

The first key secondary endpoint was sitting % predicted FVC. On FVC, cipaglucosidase alfa/miglustat demonstrated a nominally statistically significant improvement and a clinically meaningful difference for superiority versus alglucosidase alfa/placebo in the overall population (p = 0.023). Cipaglucosidase alfa/miglustat significantly slowed the rate of respiratory decline in subjects treated with cipaglucosidase alfa/miglustat, who showed a 0.9% absolute decline compared with a 4.0% absolute decline in subjects treated with alglucosidase alfa/placebo after 52 weeks.

Table 32 summarizes the mean change in FVC by visit (ITT Population) from baseline to Week 52 and ANCOVA model (for normally distributed data). FIG. 23BError! **Reference source not found.** displays a line plot of the summary statistics by visit.

**Table 32: Summary of Change in Sitting % Predicted FVC by Visit from Baseline to Week 52 (ITT Population) and ANCOVA Model (ITT-LOCF Population) - Excluding Subject 4005-2511 - Study ATB200-03**

| | | **Cipaglucosidase Alfa/Miglustat (N = 85)** | | **Alglucosidase Alfa/Placebo (N = 37)** | |
|---|---|---|---|---|---|
| | | **Absolute Value** | **CHG** | **Absolute Value** | **CHG** |
| Baseline^{a} | | | | | |
| | n | 85 | | 37 | |
| | Mean (SD) | 70.74 (19.573) | | 69.68 (21.475) | |
| | Median (Q1, Q3) | 70.00 (56.00, 84.00) | | 71.00 (50.00, 89.00) | |
| | Min, max | 30.5, 132.5 | | 31.5, 122.0 | |

| Week 52-LOCF | | | | | |
|---|---|---|---|---|---|
| | n | 84^{b} | 84 | 37 | 37 |
| | Mean (SD) | 69.81 (19.861) | -0.93 (6.231) | 65.73 (21.109) | -3.95 (4.892) |
| | Median (Q1, Q3) | 69.00 (57.00, 82.00) | -1.00 (-5.00, 4.00) | 69.00 (49.00, 80.00) | -3.00 (-6.50, 0.00) |
| | Min, max | 30.0, 137.0 | -17.0, 14.0 | 28.0, 108.0 | -19.5, 2.0 |

| **ANCOVA parameter estimation and comparison at Week 52^{c}** | | | | | |
|---|---|---|---|---|---|
| n | | 84 | | 37 | |
| LS means (SE) | | -1.04 (0.624) | | -3.70 (0.953) | |
| 95% CI of LS means | | (-2.28, 0.19) | | (-5.59, -1.81) | |
| Difference of LS means (SE) (cipaglucosidase alfa/miglustat - alglucosidase alfa/placebo) | | | | 2.66 (1.156) | |
| 95% CI of difference | | | | (0.37, 4.95) | |
| 2-sided p-value | | | | 0.023 | |

| | | | | | |
|---|---|---|---|---|---|
| Abbreviations: ANCOVA = analysis of covariance; CHG = change from baseline; CI = confidence interval; ERT = enzyme replacement therapy; FVC = forced vital capacity; ITT = Intent-to-Treat; LOCF = last observation carried forward; LS = least squares; max = maximum; min = minimum; N = number of subjects in each treatment group; n = number of subjects with available data; Q1 = first quartile; Q3 = third quartile; SD = standard deviation; SE = standard error ^{a} Baseline was the average of the last 2 values obtained on or prior to the first dose date. ^{b} For % predicted FVC (N = 84), Subject 2301-1421 had baseline result of 70.5% but subsequently withdrew from the study due to not wanting to travel to the site and was therefore excluded from analysis. ^{c} All estimates were obtained from the ANCOVA model based on the observed data including terms for treatment, baseline sitting % predicted FVC, age, height, weight (all as continuous covariates), ERT status (ERT-naive versus ERT-experienced), and gender | | | | | |

The global test was also performed on the primary and key secondary endpoints of 6MWD and FVC excluding the outlier subject (Table 33). Results from this post hoc test further support the significance and consistency of the treatment effect of cipaglucosidase alfa/miglustat on 6MWD and FVC in the overall ITT Population.

**Table 33: Wilcoxon Rank Sum Test Based on Sum of Ranks for 6MWD and % Predicted FVC (Excluding Subject 4005-2511) - Study ATB200-03**

| **Treatment** | **N** | **Sum of Scores** | **Mean Score** | **Statistic** | **Z-statistic** | **2-sided p-value^{a}** |
|---|---|---|---|---|---|---|
| Alglucosidase alfa/placebo | 37 | 1793.5 | 48.5 | 1794 | -2.6 | 0.010 |
| Cipaglucosidase alfa/miglustat | 84^{b} | 5587.5 | 66.5 | | | |

| | | | | | | |
|---|---|---|---|---|---|---|
| Abbreviations: 6MWD = 6-minute walk distance; FVC = forced vital capacity; N = number of subjects in each treatment group; n = number of subjects with available data Note: This global test was conducted by ranking individual subject response (ie, change from baseline to Week 52) separately for each endpoint from least improvement to greatest improvement, summing the 2 ranks for each subject, and analyzing the summed ranks using the Wilcoxon rank sum test. ^{a} P-values are from the Wilcoxon 2-sample test with t-approximation. ^{b} For % predicted FVC (N = 84), Subject 2301-1421 had baseline result of 70.5% but subsequently withdrew from the study due to not wanting to travel to the site and was therefore excluded from analysis. | | | | | | |

### MMT Lower Extremity Score

In Study ATB200-03, the mean (SD) change in the MMT lower extremity score from baseline to Week 52 showed a numerical improvement of 1.6 (3.78) for the cipaglucosidase alfa/miglustat group compared to 0.9 (2.58) for the alglucosidase alfa/placebo group, although statistical superiority on this assessment of muscle strength was not achieved (p = 0.191).

Table 34 summarizes the change in MMT lower extremity score by visit from baseline to Week 52 (ITT Population) and ANCOVA model (ITT-LOCF Population). FIG. 28 displays a line plot of the summary statistics by visit.

**Table 34: Summary of Change in MMT Lower Extremity Score by Visit from Baseline to Week 52 (ITT Population) and ANCOVA Model (ITT-LOCF Population) - Excluding Subject 4005-2511 - Study ATB200-03**

| | | **Cipaglucosidase Alfa/Miglustat (N = 85)** | | **Alglucosidase Alfa/Placebo (N = 37)** | |
|---|---|---|---|---|---|
| | | **Absolute Value** | **CHG** | **Absolute Value** | **CHG** |
| Baseline^{a} | | | | | |
| | n | 84 | | 34 | |
| | Mean (SD) | 27.96 (5.757) | | 27.65 (6.169) | |
| | Median (Q1, Q3) | 28.00 (24.00, 32.00) | | 28.00 (24.00, 32.00) | |
| | Min, max | 15.0, 39.0 | | 14.0, 40.0 | |

| Week 52-LOCF | | | | | |
|---|---|---|---|---|---|
| | n | 80 | 80 | 34 | 34 |
| | Mean (SD) | 29.73 (6.042) | 1.56 (3.783) | 28.53 (6.999) | 0.88 (2.579) |
| | Median (Q1, Q3) | 30.00 (25.00, 35.00) | 1.00 (0.00, 4.00) | 29.00 (24.00, 34.00) | 1.00 (0.00, 2.00) |
| | Min, max | 14.0, 40.0 | -10.0, 18.0 | 10.0, 40.0 | -5.0, 7.0 |

| Parameter estimation and comparison from ANCOVA^{b} | | | | | |
|---|---|---|---|---|---|
| | n | 80 | | 34 | |
| | LS means (SE) | 1.64 (0.388) | | 0.68 (0.603) | |
| | 95% CI of LS means | (0.87, 2.41) | | (-0.51, 1.88) | |
| | Difference of LS means (SE) (cipaglucosidase alfa/miglustat - alglucosidase alfa/placebo) | | | 0.96 (0.727) | |
| | 95% CI of difference | | | (-0.48, 2.40) | |
| | 2-sided p-value | | | 0.191 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; MMT = manual muscle testing; N = number of subjects in each treatment group; n = number of subjects with available data; Q1 = first quartile; Q3 = third quartile; SD = standard deviation; SE = standard error Note: The total score for the MMT lower extremity strength includes the following 8 body parts: right/left hip flexion, right/left hip abduction, right/left knee flexion, and right/left knee extension. The MMT score ranges from 0 to 40, with lower scores indicating weaker muscle strength. ^{a} Baseline was the last non-missing value prior to the administration of the first dose of study drug. All estimates were obtained from the ANCOVA model including terms for treatment, baseline MMT lower extremity score, age, height, weight (all as continuous covariates), ERT status (ERT-naive versus ERT-experienced), and gender. | | | | | |

### 6MWD at Week 26

Results at Week 26 were in general agreement with results at Week 52. For the ANCOVA model, the LS mean treatment difference (95% CI) was 8.17 (-4.24, 20.57) meters, with a p-value of 0.195.

### PROMIS-Physical Function

In Study ATB200-03, the mean (SD) change in the PROMIS-Physical Function total score from baseline to Week 52 showed a mean improvement of 1.9 (7.50) for the cipaglucosidase alfa/miglustat group compared to 0.2 (10.82) for the alglucosidase alfa/placebo group. PROMIS-Physical Function showed a numerically greater improvement in subjects treated with cipaglucosidase alfa/miglustat compared with subjects treated with alglucosidase alfa/placebo, but these improvements did not achieve statistical superiority (p = 0.276).

Table 35 summarizes the mean change in PROMIS-Physical Function Short Form 20a total score by visit (ITT Population) from baseline to Week 52 and ANCOVA model (ITT-LOCF Population) excluding Subject 4005-2511. FIG. 30 displays a line plot of the summary statistics by visit.

**Table 35: Summary of Change in PROMIS-Physical Function Short Form 20a by Visit from Baseline to Week 52 (ITT Population) and ANCOVA Model (ITT-LOCF Population) - Excluding Subject 4005-2511 - Study ATB200-03**

| | | **Cipaglucosidase Alfa/Miglustat (N = 85)** | | **Alglucosidase Alfa/Placebo (N = 37)** | |
|---|---|---|---|---|---|
| | | **Absolute Value** | **CHG** | **Absolute Value** | **CHG** |
| Baseline^{a} | | | | | |
| | n | 84 | | 37 | |
| | Mean (SD) | 66.86 (12.261) | | 67.97 (13.090) | |
| | Median (Q1, Q3) | 67.00 (60.00, 75.50) | | 67.00 (59.00, 74.00) | |
| | Min, max | 37.0, 96.0 | | 44.0, 97.0 | |

| Week 52-LOCF | | | | | |
|---|---|---|---|---|---|
| | n | 84 | 84 | 37 | 37 |
| | Mean (SD) | 68.80 (12.954) | 1.94 (7.498) | 68.16 (16.269) | 0.19 (10.819) |
| | Median (Q1, Q3) | 68.00 (59.50, 77.00) | 0.00 (-2.50, 6.00) | 69.00 (56.00, 79.00) | -1.00 (-6.00, 4.00) |
| | Min, max | 36.0, 99.0 | -14.0, 28.0 | 37.0, 97.0 | -30.0, 30.0 |

| ANCOVA parameter estimation and comparison at Week 52^{b} | | | | | |
|---|---|---|---|---|---|
| | | **Cipaglucosidase Alfa/Miglustat (N = 85)** | | **Alglucosidase Alfa/Placebo (N = 37)** | |
| | | **Absolute Value** | **CHG** | **Absolute Value** | **CHG** |
| | n | 84 | | 37 | |
| | LS means (SE) | 1.98 (0.921) | | 0.11 (1.406) | |
| | 95% CI of LS means | (0.15, 3.80) | | (-2.68, 2.89) | |
| | Difference of LS means (SE) (cipaglucosidase alfa/miglustat - alglucosidase alfa/placebo) | | | 1.87 (1.706) | |
| | 95% CI of difference | | | (-1.51, 5.25) | |
| | 2-sided p-value | | | 0.276 | |

| | | | | | |
|---|---|---|---|---|---|
| Abbreviations: ANCOVA = analysis of covariance; CI = confidence interval; CHG = change from baseline; ERT = enzyme replacement therapy; ITT = Intent-to-Treat; LOCF = last observation carried forward; LS = least squares; max = maximum; min = minimum; N = number of subjects in each treatment group; n = number of subjects with available data; PROMIS = Patient-reported Outcomes Measurement Information System; Q1 = first quartile; Q3 = third quartile; SD = standard deviation; SE = standard error Note: The total score ranged from 20 to 100, with higher score indicating less impact on physical function. ^{a} Baseline was the last non-missing value prior to the administration of the first dose of study drug. ^{b} All estimates were obtained from the ANCOVA model including terms for treatment, baseline PROMIS-Physical Function total score, age, height, weight (all as continuous covariates), ERT status (ERT-naïve versus ERT-experienced), and gender. | | | | | |

### PROMIS-Fatigue

The mean (SD) change in the PROMIS-Fatigue total score from baseline to Week 52 showed a mean improvement of -1.9 (0.59) for the cipaglucosidase alfa/miglustat group compared to -1.9 (0.90) for the alglucosidase alfa/placebo group. PROMIS-Fatigue showed similar improvement between subjects treated with cipaglucosidase alfa/miglustat and alglucosidase alfa/placebo (p = 0.970).

Table 36 summarizes the mean change in PROMIS-Fatigue total score by visit (ITT Population) and ANCOVA model (ITT-LOCF Population) from baseline to Week 52 for the ITT Population excluding Subject 4005-2511. FIG. 31 displays a line plot of the summary statistics by visit.

**Table 36: Summary of Change in PROMIS-Fatigue Short Form 8a by Visit from Baseline to Week 52 (ITT Population) and ANCOVA Model Total Score (ITT-LOCF Population) - Excluding Subject 4005-2511 - Study ATB200-03**

| | | **Cipaglucosidase Alfa/Miglustat (N = 85)** | | **Alglucosidase Alfa/Placebo (N = 37)** | |
|---|---|---|---|---|---|
| | | **Absolute Value** | **CHG** | **Absolute Value** | **CHG** |
| Baseline^{a} | | | | | |
| | n | 85 | | 37 | |
| | Mean (SD) | 22.26 (8.304) | | 21.08 (6.098) | |
| | Median (Q1, Q3) | 22.00 (16.00, 29.00) | | 21.00 (17.00, 24.00) | |
| | Min, max | 8.0, 40.0 | | 8.0, 34.0 | |

| Week 52-LOCF | | | | | |
|---|---|---|---|---|---|
| | n | 85 | 85 | 37 | 37 |
| | Mean (SD) | 20.24 (7.492) | -2.02 (5.763) | 19.41 (6.735) | -1.67 (6.623) |
| | Median (Q1, Q3) | 19.00 (16.00, 26.00) | -1.00 (-5.00, 2.00) | 18.00 (15.00, 24.00) | -1.00 (-5.00, 3.00) |
| | Min, max | 8.0, 40.0 | -19.0, 12.0 | 8.0, 37.0 | -21.0, 10.0 |

| Parameter estimation and comparison from ANCOVA^{b} | | | | | |
|---|---|---|---|---|---|
| | n | 85 | | 37 | |
| | LS means (SE) | -1.90 (0.585) | | -1.94 (0.901) | |
| | 95% CI of LS means | (-3.06, -0.74) | | (-3.73, -0.15) | |
| | Difference of LS means (SE) (cipaglucosidase alfa/miglustat - alglucosidase alfa/placebo) | | | 0.04 (1.092) | |
| | 95% CI of difference | | | (-2.12, 2.20) | |
| | 2-sided p-value | | | 0.970 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; N = number of subjects in each treatment group; n = number of subjects with available data;; PROMIS = Patient-reported Outcomes Measurement Information System; Q1 = first quartile; Q3 = third quartile; SD = standard deviation; SE = standard error Note: If post-baseline scores were partially missing but ≥ 50% of items were available, the total score was calculated as the average of non-missing items multiplied by the total number of items expected. Note: The total score ranged from 8 to 40, with lower score indicating less impact by fatigue, and it was calculated by summing scores (1 to 5) across all 8 items. ^{a} Baseline was the last non-missing value prior to the administration of the first dose of study drug. ^{b} All estimates were obtained from the ANCOVA model including terms for treatment, baseline PROMIS-Fatigue total score, age, height, weight (all as continuous covariates), ERT status (ERT-naïve versus ERT-experienced), and gender. | | | | | |

### GSGC

In Study ATB200-03, the mean (SD) change in the GSGC total score from baseline to Week 52 showed a mean improvement of -0.53 (2.54) for the cipaglucosidase alfa/miglustat group compared to 0.77 (1.81) for the alglucosidase alfa/placebo group. In the overall population, GSGC showed nominally significant improvements following treatment with cipaglucosidase alfa/miglustat compared with alglucosidase alfa, with a p-value of 0.009.

Table 37 summarizes the mean change in GSGC total score by visit (ITT Population) and ANCOVA model (ITT-LOCF Population) from baseline to Week 52 for the ITT Population excluding Subject 4005-2511. FIG. 29 displays a line plot of the summary statistics by visit.

**Table 37: Summary of Change in GSGC Total Score by Visit from Baseline to Week 52 (ITT Population) and ANCOVA Model (ITT-LOCF Population) - Excluding Subject 4005-2511 - Study ATB200-03**

| | | **Cipaglucosidase Alfa/Miglustat (N = 85)** | | **Alglucosidase Alfa/Placebo (N = 37)** | |
|---|---|---|---|---|---|
| | | **Absolute Value** | **CHG** | **Absolute Value** | **CHG** |
| Baseline^{a} | | | | | |
| | n | 74 | | 32 | |
| | Mean (SD) | 14.51 (5.171) | | 14.50 (4.718) | |
| | Median (Q1, Q3) | 16.00 (12.00, 18.00) | | 16.00 (10.50, 18.00) | |
| | Min, max | 4.0, 24.0 | | 4.0, 22.0 | |

| Week 52-LOCF | | | | | |
|---|---|---|---|---|---|
| | n | 72 | 72 | 30 | 30 |
| | Mean (SD) | 13.74 (5.371) | -0.53 (2.542) | 15.00 (4.488) | 0.77 (1.813) |
| | Median (Q1, Q3) | 15.00 (9.50, 18.00) | 0.00 (-2.00, 1.00) | 16.00 (13.00, 18.00) | 0.50 (0.00, 2.00) |
| | Min, max | 4.0, 24.0 | -8.0, 4.0 | 5.0, 22.0 | -4.0, 5.0 |

| Parameter estimation and comparison from ANCOVA^{b} | | | | | |
|---|---|---|---|---|---|
| | n | 72 | | 30 | |
| | LS means (SE) | -0.567 (0.280) | | 0.847 (0.440) | |
| | 95% CI of LS means | (-1.123, -0.010) | | (-0.026, 1.720) | |
| | Difference of LS means (SE) (cipaglucosidase alfa/miglustat - alglucosidase alfa/placebo) | | | -1.414 (0.528) | |
| | 95% CI of difference | | | (-2.463, -0.364) | |
| | 2-sided p-value | | | 0.009 | |

| | | | | | |
|---|---|---|---|---|---|
| Abbreviations: ANCOVA = analysis of covariance; CHG = change from baseline; CI = confidence interval; ERT = enzyme replacement therapy; GSGC = Gait, Stairs, Gowers' maneuver, and Chair test; ITT = Intent-to-Treat; LOCF = last observation carried forward; LS = least squares; max = maximum; min = minimum; N = number of subjects in each treatment group; n = number of subjects with available data; Q1 = first quartile; Q3 = third quartile; SD = standard deviation; SE = standard error Note: Gait score was based on the 10-m walk test; Stairs score was based on the subject climbing stairs; Gowers' maneuver score was based on the subject lying down on the floor, then rising from the floor to get to a standing position; Chair score was based on the subject arising from a sitting position in a chair to a standing position. Note: GSGC total score was the sum of 4 tests and ranges from a minimum of 4 points (normal performance) to a maximum of 27 points (worst score). Note: LS mean and SE were obtained from the ANCOVA model. ^{a} Baseline was the last non-missing value prior to the administration of the first dose of study drug. ^{b} All estimates were obtained from the ANCOVA model including terms for treatment, baseline GSGC total score, age, height, weight (all as continuous covariates), ERT status (ERT-naïve versus ERT-experienced), and gender. | | | | | |

The absolute values for the timed components of GSGC from baseline to Week 52 in the ITT Population excluding Subject 4005-2511 clearly favored treatment with cipaglucosidase alfa/miglustat compared with alglucosidase alfa/placebo.

### ERT-experienced Population

It should be noted that the outlier subject (Subject 4005-2511) was in the ERT-naïve Population. Therefore, the results in the ERT-experienced Population were not affected.

### Primary Endpoint: 6MWD at Week 52

In the ERT-experienced Population (n = 95), the mean (SD) change in 6MWD (meters) from baseline to Week 52 showed a mean improvement of 16.3 (39.46) meters for the cipaglucosidase alfa/miglustat group compared to 0.7 (39.84) meters for the alglucosidase alfa/placebo group. Therefore, ERT-experienced subjects treated with cipaglucosidase alfa demonstrated improvements over time in 6MWD, out to 52 weeks.

For the MMRM analysis using the restricted maximum likelihood estimation, the LS mean treatment difference (95% CI) was 16.45% (-1.86, 34.77), with a nominal p-value of 0.078 (Table 38).

Similar to the ITT Population excluding the outlier, normality assumption for the MMRM analysis was violated based on the examination of the diagnostic plot and Shapiro-Wilk test (Shapiro-Wilk test p-value < 0.01). Therefore, the nonparametric randomization-based ANCOVA was performed as preplanned, which yielded a nominal p-value of 0.047. FIG. 25 displays a line plot of the summary statistics by visit.

**Table 38: Summary of Change in 6MWD (meters) by Visit from Baseline to Week 52 (ITT Population) and MMRM Analysis (ITT-OBS Population) - ERT-experienced Population - Study ATB200-03**

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| Baseline^{a} | | | | | |
| | n | 65 | | 30 | |
| | Mean (SD) | 346.94 (110.213) | | 334.62 (114.023) | |
| | Median (Q1, Q3) | 352.50 (294.50, 403.00) | | 343.50 (260.60, 420.00) | |
| | Min, max | 79.0, 557.5 | | 112.5, 532.3 | |

| Week 52-Observed | | | | | |
|---|---|---|---|---|---|
| | n | 61 | 61 | 29 | 29 |
| | Mean (SD) | 359.79 (122.494) | 16.34 (39.462) | 335.66 (131.666) | 0.70 (39.841) |
| | Median (Q1, Q3) | 360.50 (296.00, 428.00) | 9.65 (-7.90, 41.00) | 348.00 (250.40, 438.60) | -8.65 (-24.40, 17.35) |
| | Min, max | 79.6, 601.5 | -57.1, 173.5 | 67.0, 576.0 | -55.6, 127.0 |

| Week 52-LOCF | | | | | |
|---|---|---|---|---|---|
| | n | 65 | 65 | 30 | 30 |
| | Mean (SD) | 363.83 (123.522) | 16.89 (40.393) | 334.60 (129.506) | -0.02 (39.345) |
| | Median (Q1, Q3) | 363.50 (296.00, 435.00) | 9.65 (-7.90, 41.00) | 339.03 (250.40, 438.60) | -8.93 (-24.40, 17.35) |
| | Min, max | 79.6, 601.5 | -57.1, 173.5 | 67.0, 576.0 | -55.6, 127.0 |

| Parameter estimation and comparison from MMRM^{b} | | | | | |
|---|---|---|---|---|---|
| | n | 61 | | 29 | |
| | LS means (SE) | 17.04 (5.151) | | 0.59 (7.569) | |
| | 95% CI of LS means | (6.79, 27.29) | | (-14.5, 15.65) | |
| | Difference of LS means (SE) (cipaglucosidase alfa/miglustat - alglucosidase alfa/placebo) | | | 16.45 (9.209) | |
| | 95% CI of difference | | | (-1.86, 34.77) | |
| | 2-sided p-value | | | 0.078 | |

| Parameter estimation and comparison from nonparametric ANCOVA^{c} | | | | | |
|---|---|---|---|---|---|
| | Difference in LS means (SE) (cipaglucosidase alfa - alglucosidase alfa/placebo) | | | 16.76 (8.433) | |
| | 95% CI of difference | | | (0.24, 33.29) | |
| | 2-sided p-value | | | 0.047 | |

| | | | | | |
|---|---|---|---|---|---|
| Abbreviations: 6MWD = 6-minute walk distance; ANCOVA = analysis of covariance; CHG = change from baseline; CI = confidence interval; ERT = enzyme replacement therapy; ITT = Intent-to-Treat; ITT-OBS = Intent-to-Treat Population that includes all available, observed data without any missing data imputation at Week 52; LOCF = last observation carried forward; LS = least squares; max = maximum; min = minimum; MMRM = mixed-effect model for repeated measures; N = number of subjects in each treatment group; n = number of subjects with available data;; Q1 = first quartile; Q3 = third quartile; SD = standard deviation; SE = standard error ^{a} Baseline was the average of the last 2 values obtained on or prior to the first dose date. ^{b} The MMRM approach (using restricted maximum likelihood estimation) was used for analysis. The model includes terms for treatment, baseline 6MWD, age, height, weight (all as continuous covariates), gender, time, and treatment-by-time interaction. Time was used as a repeated measure and an unstructured covariance approach was applied. ^{c} Nonparametric ANCOVA compared between the treatment groups, adjusting for baseline 6MWD, age, height, weight (all as continuous covariates), and gender. | | | | | |

### Sitting % Predicted FVC

In the ERT-experienced subgroup cipaglucosidase alfa/miglustat demonstrated a nominally statistically significant improvement and a clinically meaningful difference for superiority versus alglucosidase alfa/placebo (p = 0.006). Cipaglucosidase alfa/miglustat slightly improved the rate of respiratory decline in subjects treated with cipaglucosidase alfa/miglustat, who showed a 0.1% absolute increase compared with a 4.0% absolute decline in subjects treated with alglucosidase alfa/placebo after 52 weeks.

Table 39summarizes the mean change in % predicted FVC by visit and the LS mean treatment difference (with ANCOVA for normally distributed data) from baseline to Week 52 for ERT-experienced subjects. FIG. 25 displays a line plot of the summary statistics by visit.

**Table 39: Summary of Change in Sitting % Predicted FVC by Visit from Baseline to Week 52 (ITT Population) and ANCOVA Model (ITT-LOCF Population) - ERT-experienced Population - Study ATB200-03**

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| Baseline^{a} | | | | | |
| | n | 65 | | 30 | |
| | Mean (SD) | 67.85 (19.052) | | 67.48 (20.993) | |
| | Median (Q1, Q3) | 68.00 (54.50, 81.00) | | 69.00 (50.00, 81.50) | |
| | Min, max | 30.5, 132.5 | | 31.5, 122.0 | |

| Week 52-LOCF | | | | | |
|---|---|---|---|---|---|
| | n | 64 | 64 | 30 | 30 |
| | Mean (SD) | 67.86 (19.777) | 0.05 (5.843) | 63.47 (20.480) | -4.02 (5.009) |

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| | Median (Q1, Q3) | 67.50 (54.50, 79.50) | 0.50 (-4.50, 4.00) | 62.00 (49.00, 76.00) | -3.25 (-7.00, 0.00) |
| | Min, max | 30.0, 137.0 | -17.0, 14.0 | 28.0, 108.0 | -19.5, 2.0 |

| Parameter estimation and comparison from ANCOVA | | | | | |
|---|---|---|---|---|---|
| | n | 64 | | 30 | |
| | LS means (SE) | -0.12 (0.688) | | -3.64 (1.019) | |
| | 95% CI of LS means | (-1.49, 1.24) | | (-5.66, -1.61) | |
| | Difference of LS means (SE) (cipaglucosidase alfa/miglustat - alglucosidase alfa/placebo) | | | 3.51 (1.250) | |
| | 95% CI of difference | | | (1.03, 5.99) | |
| | 2-sided p-value | | | 0.006 | |

| | | | | | |
|---|---|---|---|---|---|
| Abbreviations: ANCOVA = analysis of covariance; CHG = change from baseline; CI = confidence interval; ERT = enzyme replacement therapy; FVC = forced vital capacity; ITT = Intent-to-Treat; LOCF = last observation carried forward; LS = least squares; max = maximum; min = minimum; N = number of subjects in each treatment group; n = number of subjects with available data; Q1 = first quartile; Q3 = third quartile; SD = standard deviation; SE = standard error Note: All estimates were obtained from the ANCOVA model including terms for treatment, baseline sitting % predicted FVC, age, height, weight (all as continuous covariates), and gender. ^{a} Baseline was the average of the last 2 values obtained on or prior to the first dose date. | | | | | |

### MMT Lower Extremity Score

The mean (SD) change in the MMT lower extremity score from baseline to Week 52 showed an improvement of 1.6 (4.13) for the cipaglucosidase alfa/miglustat group compared to 0.9 (2.81) for the alglucosidase alfa/placebo group. Results for MMT lower extremity scores in both treatment groups showed improvement and directionally favored cipaglucosidase alfa/miglustat over alglucosidase alfa/placebo (p = 0.436).

Table 40 summarizes the mean change in the MMT lower extremity score by visit (ITT Population) from baseline to Week 52 and ANCOVA model (ITT-LOCF Population) for the ERT-experienced Population. FIG. 28 displays a line plot of the summary statistics by visit.

**Table 40: Summary of Change in MMT Lower Extremity Score by Visit from Baseline to Week 52 (ITT Population) and ANCOVA Model (ITT-LOCF Population) - ERT-experienced Population - Study ATB200-03**

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| Baseline^{a} | | | | | |
| | n | 64 | | 27 | |
| | Mean (SD) | 26.38 (5.144) | | 26.11 (5.813) | |
| | Median (Q1, Q3) | 26.00 (23.50, 30.00) | | 26.00 (23.00, 29.00) | |
| | Min, max | 15.0, 39.0 | | 14.0, 40.0 | |

| Week 52-LOCF | | | | | |
|---|---|---|---|---|---|
| | n | 60 | 60 | 27 | 27 |
| | Mean (SD) | 28.18 (5.589) | 1.63 (4.129) | 26.96 (6.802) | 0.85 (2.811) |
| | Median (Q1, Q3) | 28.00 (24.00, 32.50) | 1.00 (0.00, 4.00) | 28.00 (22.00, 32.00) | 1.00 (0.00, 3.00) |
| | Min, max | 14.0, 40.0 | -10.0, 18.0 | 10.0, 40.0 | -5.0, 7.0 |

| Parameter estimation and comparison from ANCOVA^{b} | | | | | |
|---|---|---|---|---|---|
| | n | 60 | | 27 | |
| | LS means (SE) | 1.60 (0.486) | | 0.90 (0.735) | |
| | 95% CI of LS means | (0.64, 2.57) | | (-0.56, 2.36) | |
| | Difference of LS means (SE) (cipaglucosidase alfa/miglustat - alglucosidase alfa/placebo) | | | 0.70 (0.896) | |
| | 95% CI of difference | | | (-1.08, 2.49) | |
| | 2-sided p-value | | | 0.436 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; MMT = manual muscle testing; N = number of subjects in each treatment group; n = number of subjects with available data; Q1 = first quartile; Q3 = third quartile; SD = standard deviation; SE = standard error Note: Total score for the lower extremity included the following 8 body parts: right/left hip flexion, right/left hip abduction, right/left knee flexion, and right/left knee extension. The score ranged from 0 to 40 with lower scores indicating weaker muscle strength. ^{a} Baseline is the non-missing value prior to the administration of the first dose of study drug. ^{b} All estimates were obtained from the ANCOVA model including terms for treatment, baseline MMT lower extremity score, age, height, weight (all as continuous covariates), and gender. | | | | | |

Results for change in 6MWD from baseline to Week 26 for ERT-experienced subjects were in general agreement with results at Week 52. For the ANCOVA model, the LS mean treatment difference (95% CI) was 9.62 meters (-3.82, 23.06), with a p-value of 0.158.

### PROMIS-Physical Function

The mean (SD) change in the PROMIS-Physical Function total score from baseline to Week 52 showed a mean improvement of 1.8 (7.18) for the cipaglucosidase alfa/miglustat group compared to 1.0 (11.20) for the alglucosidase alfa/placebo group. Results for the PROMIS-Physical Function scale showed improvement and directionally favored cipaglucosidase alfa/miglustat over alglucosidase alfa/placebo (p = 0.110).

Table 41 summarizes the mean change in PROMIS-Physical Function total score by visit (ITT Population) from baseline to Week 52 and ANCOVA model (ITT-LOCF Population) for the ERT-experienced Population. FIG. 30 displays a line plot of the summary statistics by visit.

**Table 41: Summary of Change in PROMIS-Physical Function Short Form 20a by Visit from Baseline to Week 52 (ITT Population) and ANCOVA Model (ITT-LOCF Population) - ERT-experienced Population - Study ATB200-03**

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| Baseline^{a} | | | | | |
| | n | 64 | | 30 | |
| | Mean (SD) | 64.43 (11.379) | | 66.87 (12.286) | |
| | Median (Q1, Q3) | 64.50 (58.00, 71.00) | | 67.00 (59.00, 73.00) | |
| | Min, max | 37.0, 94.0 | | 44.0, 97.0 | |

| Week 52-LOCF | | | | | |
|---|---|---|---|---|---|
| | n | 64 | 64 | 30 | 30 |
| | Mean (SD) | 66.19 (12.325) | 1.76 (7.179) | 65.90 (15.790) | -0.97 (11.196) |
| | Median (Q1, Q3) | 66.00 (58.00, 74.00) | 0.50 (-2.00, 6.00) | 66.00 (56.00, 75.00) | -2.00 (-6.00, 3.00) |
| | Min, max | 36.0, 99.0 | -14.0, 26.0 | 37.0, 97.0 | -30.0, 30.0 |

| Parameter estimation and comparison from ANCOVA^{b} | | | | | |
|---|---|---|---|---|---|
| | n | 64 | | 30 | |
| | LS means (SE) | 1.90 (1.071) | | -1.25 (1.587) | |
| | 95% CI of LS means | (-0.23, 4.02) | | (-4.40, 1.91) | |
| | Difference of LS means (SE) (cipaglucosidase alfa/miglustat - alglucosidase alfa/placebo) | | | 3.14 (1.948) | |

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| | 95% CI of difference | | | (-0.73, 7.02) | |
| | 2-sided p-value | | | 0.110 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; N = number of subjects in each treatment group; n = number of subjects with available data; PROMIS = Patient-reported Outcomes Measurement Information System; Q1 = first quartile; Q3 = third quartile; SD = standard deviation; SE = standard error Note: The total score ranges from 20 to 100, with a higher score indicating less impact on physical function. ^{a} Baseline was the last non-missing value prior to the administration of the first dose of study drug. ^{b} All estimates were obtained from the ANCOVA model including terms for treatment, baseline PROMIS-Physical Function total score, age, height, weight (all as continuous covariates), and gender. | | | | | |

### PROMIS-Fatigue

The mean (SD) change in the PROMIS-Fatigue total score from baseline to Week 52 showed a mean improvement of -0.1 (5.99) for the cipaglucosidase alfa/miglustat group compared to -0.5 (5.72) for the alglucosidase alfa/placebo group. The PROMIS-Fatigue scores showed improvement, but they improved similarly between subjects treated with cipaglucosidase alfa/miglustat and alglucosidase alfa/placebo (p = 0.476).

Table 42 summarizes the mean change in PROMIS-Fatigue total score by visit (ITT Population) and ANCOVA model (ITT-LOCF Population) from baseline to Week 52 for the ERT-experienced Population. FIG. 31 displays a line plot of the summary statistics by visit.

**Table 42: Summary of Change in PROMIS-Fatigue Short Form 8a Total Score by Visit from Baseline to Week 52 (ITT Population) and ANCOVA Model (ITT-LOCF Population) - ERT-experienced Population - Study ATB200-03**

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| Baseline^{a} | | | | | |
| | n | 65 | | 30 | |
| | Mean (SD) | 22.00 (7.924) | | 20.37 (5.379) | |
| | Median (Q1, Q3) | 23.00 (16.00, 29.00) | | 20.00 (17.00, 23.00) | |
| | Min, max | 8.0, 39.0 | | 8.0, 32.0 | |

| Week 52-LOCF | | | | | |
|---|---|---|---|---|---|
| | n | 65 | 65 | 30 | 30 |

| | | **Cipaglucosidase Alfa/Miglustat (N = 65)** | | **Alglucosidase Alfa/Placebo (N = 30)** | |
|---|---|---|---|---|---|
| | | **Absolute Value** | **CHG** | **Absolute Value** | **CHG** |
| | Mean (SD) | 21.86 (7.888) | -0.14 (5.986) | 19.87 (6.882) | -0.50 (5.722) |
| | Median (Q1, Q3) | 22.00 (17.00, 28.00) | 0.00 (-4.00, 3.00) | 18.00 (16.00, 24.00) | 0.50 (-5.00, 3.00) |
| | Min, max | 8.0, 39.0 | -16.0, 19.0 | 8.0, 38.0 | -11.0, 11.0 |

| Parameter estimation and comparison from ANCOVA^{b} | | | | | |
|---|---|---|---|---|---|
| | n | 65 | | 30 | |
| | LS means (SE) | -1.63 (0.637) | | -0.79 (0.953) | |
| | 95% CI of LS means | (-2.89, -0.36) | | (-2.68, 1.10) | |
| | Difference of LS means (SE) (cipaglucosidase alfa/miglustat - alglucosidase alfa/placebo) | | | -0.84 (1.169) | |
| | 95% CI of difference | | | (-3.16, 1.49) | |
| | 2-sided p-value | | | 0.476 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; N = number of subjects in each treatment group; n = number of subjects with available data; PROMIS = Patient-reported Outcomes Measurement Information System; Q1 = first quartile; Q3 = third quartile; SD = standard deviation; SE = standard error Note: The total score ranges from 8 to 40, with a lower score indicating less impact by fatigue, and it is calculated by summing up the scores (1 to 5) across all 8 items. ^{a} Baseline is the last non-missing value prior to the administration of the first dose of study drug. ^{b} All estimates were obtained from the ANCOVA model including terms for treatment, baseline PROMIS-Fatigue total score, age, height, weight (all as continuous covariates), and gender. | | | | | |

### GSGC

The mean (SD) change in the GSGC total score from baseline to Week 52 showed a mean improvement of -0.5 (2.53) for the cipaglucosidase alfa/miglustat group compared to 0.6 (1.83) for the alglucosidase alfa/placebo group. Similar to the overall population, GSGC showed nominally significant improvements following treatment with cipaglucosidase alfa/miglustat compared with alglucosidase alfa/placebo (p = 0.050).

Table 43 summarizes the mean change in GSGC total score by visit (ITT Population) and ANCOVA model (ITT-LOCF Population) from baseline to Week 52 for the ERT-experienced Population. FIG. 29 displays a line plot of the summary statistics by visit.

**Table 43: Summary of Change in GSGC Total Score by Visit from Baseline to Week 52 (ITT Population) and ANCOVA Model (ITT-LOCF Population) - ERT-experienced Population - Study ATB200-03**

| | | **Cipaglucosidase Alfa/Miglustat (N = 55)** | | **Alglucosidase Alfa/Placebo (N = 25)** | |
|---|---|---|---|---|---|
| | | **Absolute Value** | **CHG** | **Absolute Value** | **CHG** |
| Baseline^{a} | | | | | |
| | n | 55 | | 25 | |
| | Mean (SD) | 15.61 (4.073) | | 15.52 (4.350) | |
| | Median (Q1, Q3) | 16.00 (13.00, 18.00) | | 17.00 (14.00, 18.00) | |
| | Min, max | 6.0, 24.0 | | 4.0, 22.0 | |

| Week 52-LOCF | | | | | |
|---|---|---|---|---|---|
| | n | 54 | 54 | 23 | 23 |
| | Mean (SD) | 14.96 (4.809) | -0.53 (2.534) | 15.87 (4.170) | 0.61 (1.828) |
| | Median (Q1, Q3) | 17.00 (11.00, 19.00) | 0.00 (-2.00, 1.00) | 17.00 (14.00, 18.00) | 0.00 (0.00, 2.00) |
| | Min, max | 4.0, 24.0 | -7.0, 4.0 | 5.0, 22.0 | -4.0, 5.0 |

| Parameter estimation and comparison from ANCOVA^{b} | | | | | |
|---|---|---|---|---|---|
| | n | 54 | | 23 | |
| | LS means (SE) | -0.54 (0.318) | | 0.65 (0.494) | |
| | 95% CI of LS means | (-1.18, 0.09) | | (-0.34, 1.63) | |
| | Difference of LS means (SE) (cipaglucosidase alfa/miglustat - alglucosidase alfa/placebo) | | | -1.19 (0.596) | |
| | 95% CI of difference | | | (-2.38, -0.00) | |
| | 2-sided p-value | | | 0.050 | |

| | | | | | |
|---|---|---|---|---|---|
| Abbreviations: ANCOVA = analysis of covariance; CHG = change from baseline; CI = confidence interval; ERT = enzyme replacement therapy; GSGC = Gait, Stairs, Gowers' maneuver, and Chair test; ITT = Intent-to-Treat; LOCF = last observation carried forward; LS = least squares; N = number of subjects in each treatment group; n = number of subjects with available data; max = maximum; min = minimum; Q1 = first quartile; Q3 = third quartile; SD = standard deviation; SE = standard error Note: Gait score was based on the 10-m walk test; Stairs score was based on the subject climbing stairs; Gowers' maneuver score was based on the subject lying down on the floor, then rising from the floor to get to a standing position; Chair score was based on the subject arising from a sitting position in a chair to a standing position. GSGC total score was the sum of 4 tests and ranges from a minimum of 4 points (normal performance) to a maximum of 27 points (worst score). ^{a} Baseline was the last non-missing value prior to the administration of the first dose of study drug. ^{b} All estimates were obtained from the ANCOVA model including terms for treatment, baseline GSGC total score, age, height, weight (all as continuous covariates), and gender. | | | | | |

### ERT-Naive Population (Excluding Subject 4005-2511)

### Primary Endpoint: 6MWD at Week 52

The mean (SD) change in 6MWD from baseline to Week 52 showed a mean improvement of 33.4 (48.70) meters for the cipaglucosidase alfa/miglustat group compared to 38.3 (29.32) meters for the alglucosidase alfa/placebo group. In the ERT-naïve Population (n = 27), subjects in both treatment groups showed improvement in 6MWD at Week 52 (p = 0.748).

Table 44 summarizes the mean change in 6MWD by visit (ITT Population) from baseline to Week 52 and MMRM analysis (ITT-OBS Population) for the ERT-naïve Population excluding Subject 4005-2511. FIG. 26B displays a line plot of the summary statistics by visit, including subject 4005-2511.

**Table 44: Summary of Change in 6MWD (meters) by Visit from Baseline to Week 52 (ITT Population) and MMRM Analysis (ITT-OBS Population) - ERT-naïve Population Excluding Subject 4005-2511 - Study ATB200-03**

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| Baseline^{a} | | | | | |
| | n | 20 | | 7 | |
| | Mean (SD) | 393.64 (112.388) | | 420.94 (135.749) | |
| | Median (Q1, Q3) | 375.15 (329.50, 498.75) | | 385.50 (351.40, 522.65) | |
| | Min, max | 154.0, 575.0 | | 201.0, 623.0 | |

| Week 52-Observed | | | | | |
|---|---|---|---|---|---|
| | n | 20 | 20 | 7 | 7 |
| | Mean (SD) | 427.09 (112.473) | 33.44 (48.704) | 459.28 (121.657) | 38.34 (29.315) |
| | Median (Q1, Q3) | 452 (348.75, 505.65) | 24.00 (-0.00, 69.73) | 446.50 (370.00, 570.65) | 33.50 (18.60, 63.50) |
| | Min, max | 166.5, 599.3 | -59.5, 120.0 | 284.5, 648.6 | -4.3, 83.5 |

| Week 52-LOCF | | | | | |
|---|---|---|---|---|---|
| | n | 20 | 20 | 7 | 7 |
| | Mean (SD) | 427.09 (112.473) | 33.44 (48.704) | 459.28 (121.657) | 38.34 (29.315) |
| | Median (Q1, Q3) | 452.58 (348.75, 505.65) | 24.00 (-0.00, 69.73) | 446.50 (370.00, 570.65) | 33.50 (18.60, 63.50) |
| | Min, max | 166.5, 599.3 | -59.5, 120.0 | 284.5, 648.6 | -4.3, 83.5 |

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| Parameter estimation and comparison from MMRM^{b} | | | | | |
| | n | 20 | | 7 | |
| | LS means (SE) | 32.92 (9.912) | | 39.47 (17.15) | |
| | 95% CI of LS means | (12.40, 53.44) | | (4.08, 74.87) | |
| | Difference of LS means (SE) (cipaglucosidase alfa/miglustat - alglucosidase alfa/placebo) | | | -6.55 (20.207) | |
| | 95% CI of difference | | | (-48.19, 35.08) | |
| | 2-sided p-value | | | 0.748 | |

| | | | | | |
|---|---|---|---|---|---|
| Abbreviations: 6MWD = 6-minute walk distance; CHG = change from baseline; CI = confidence interval; ERT = enzyme replacement therapy; ITT = Intent-to-Treat; ITT-OBS = Intent-to-Treat Population that includes all available, observed data without any missing data imputation at Week 52; LOCF = last observation carried forward; LS = least squares; max = maximum; min = minimum; MMRM = mixed-effect model for repeated measures; N = number of subjects in each treatment group; n = number of subjects with available data; Q1 = first quartile; Q3 = third quartile; SD = standard deviation; SE = standard error ^{a} Baseline was the average of the last 2 values obtained on or prior to the first dose date. ^{b} The MMRM approach (using restricted maximum likelihood estimation) was used for analysis. The model includes terms for treatment, baseline 6MWD, age, height, weight (all as continuous covariates), gender, time, and treatment-by-time interaction. Time was used as a repeated measure and an unstructured covariance approach was applied. | | | | | |

For the ERT-naïve Population excluding Subject 4005-2511, the normality assumption for the MMRM analysis was violated based on the examination of the diagnostic plot and Shapiro-Wilk test. Given the small sample size and differences in baseline characteristics (eg, sex) between treatment groups, the nonparametric Wilcoxon rank sum test was performed post hoc.

Table 45 presents results for the nonparametric Wilcoxon Rank Sum Test for change in 6MWD at Week 52 for the ERT-naïve Population excluding Subject 4005-2511, using the ITT-LOCF Population. For this test, the location shift (95% CI of location shift) was -9.0 (-46.50, 34.95), with a p-value of 0.604.

**Table 45: Nonparametric Wilcoxon Rank Sum Test for Change from Baseline in 6MWD (meters) at Week 52 (ITT-LOCF Population) - ERT-naïve Population Excluding Subject 4005-2511 - Study ATB200-03**

| **Parameter Estimation and Comparison from Nonparametric Wilcoxon Rank Sum Test^{a}** | **Cipaglucosidase Alfa/Miglustat (N = 20)** | **Alglucosidase Alfa/Placebo (N = 7)** |
|---|---|---|
| n | 20 | 7 |
| Location shift (asymptotic SE) | | -9.00 (20.778) |
| 95% CI of location shift | | (-46.50, 34.95) |
| 2-sided p-value | | 0.604 |

| | | |
|---|---|---|
| Abbreviations: 6MWD = 6-minute walk distance; CI = confidence interval; ERT = enzyme replacement therapy; ITT = Intent-to- Treat; LOCF = last observation carried forward; N = number of subjects in each treatment group; n = number of subjects with available data; SE = standard error ^{a} Location shift, asymptotic SE, and 95% CI are from Hodges-Lehmann estimation. P-value is from Wilcoxon 2-sample test with t-approximation. | | |

### Sitting % Predicted FVC

The mean (SD) change in sitting % predicted FVC from baseline to Week 52 was of -4.1% (6.53%) for the cipaglucosidase alfa/miglustat group and of -3.6% (4.71%) for the alglucosidase alfa/placebo group. Using an ANCOVA model for normally distributed data, the LS mean treatment difference (95% CI) was -1.95 (-8.93, 5.03), with a p-value of 0.566.

Table 46 summarizes the mean change in sitting % predicted FVC by visit (ITT Population) and the LS mean treatment difference (with ANCOVA for normally distributed data) from baseline to Week 52 (ITT-LOCF Population) for the ERT-naïve Population excluding Subject 4005-2511. FIG. 26B displays a line plot of the summary statistics by visit, including subject 4005-2511.

**Table 46: Summary of Change in Sitting % Predicted FVC by Visit from Baseline to Week 52 (ITT Population) and ANCOVA (ITT-LOCF Population) - ERT-naïve Population Excluding Subject 4005-2511 - Study ATB200-03**

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| Baseline^{a} | | | | | |
| | n | 20 | | 7 | |
| | Mean (SD) | 80.15 (18.689) | | 79.07 (22.578) | |
| | Median (Q1, Q3) | 82.25 (62.00, 94.75) | | 93.50 (49.50, 96.00) | |
| | Min, max | 48.0, 111.0 | | 46.5, 98.0 | |

| Week 52-LOCF | | | | | |
|---|---|---|---|---|---|
| | n | 20 | 20 | 7 | 7 |
| | Mean (SD) | 76.05 (19.300) | -4.10 (6.526) | 75.43 (22.604) | -3.64 (4.706) |
| | Median (Q1, Q3) | 77.00 (61.50, 91.00) | -4.50 (-9.25, -1.00) | 85.00 (45.00, 93.00) | -3.00 (-4.50, -1.00) |
| | Min, max | 36.0, 104.0 | -12.5, 10.0 | 42.0, 94.0 | -13.0, 2.0 |

| Parameter estimation and comparison from ANCOVA^{b} | | | | | |
|---|---|---|---|---|---|
| | n | 20 | | 7 | |
| | LS means (SE) | -4.49 (1.490) | | -2.54 (2.758) | |
| | 95% CI of LS means | (-7.59, -1.38) | | (-8.29, 3.22) | |
| | Difference of LS means (SE) (cipaglucosidase alfa/miglustat - alglucosidase alfa/placebo) | | | -1.95 (3.346) | |
| | 95% CI of difference | | | (-8.93, 5.03) | |
| | 2-sided p-value | | | 0.566 | |

| | | | | | |
|---|---|---|---|---|---|
| Abbreviations: ANCOVA = analysis of covariance; CHG = change from baseline; CI = confidence interval; ERT = enzyme replacement therapy; FVC = forced vital capacity ITT = Intent-to-Treat; LOCF = last observation carried forward; LS = least squares; max = maximum; min = minimum; N = number of subjects in each treatment group; n = number of subjects with available data; Q1 = first quartile; Q3 = third quartile; SD = standard deviation; SE = standard error ^{a} Baseline was the average of the last 2 values obtained on or prior to the first dose date. ^{b} All estimates were obtained from the ANCOVA model including terms for treatment, baseline sitting % predicted FVC, age, height, weight (all as continuous covariates), and gender. | | | | | |

### MMT Lower Extremity Score

The mean (SD) change in the MMT lower extremity score from baseline to Week 52 showed an improvement of 1.4 (2.55) for the cipaglucosidase alfa/miglustat group compared to 1.0 (1.53) for the alglucosidase alfa/placebo group (p = 0.534).

Table 47 summarizes the mean change in the MMT lower extremity score by visit (ITT Population) from baseline to Week 52 and ANCOVA model (ITT-LOCF Population) for the ERT-naïve Population excluding Subject 4005-2511. FIG. 81 displays a line plot of the summary statistics by visit.

**Table 47: Summary of Change in MMT Lower Extremity Score by Visit from Baseline to Week 52 (ITT Population) and ANCOVA Model (ITT-LOCF Population) - ERT-naïve Population Excluding Subject 4005-2511 - Study ATB200-03**

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| Baseline^{a} | | | | | |
| | n | 20 | | 7 | |
| | Mean (SD) | 33.00 (4.686) | | 33.57 (3.359) | |
| | Median (Q1, Q3) | 34.50 (30.00, 36.00) | | 32.00 (30.00, 37.00) | |
| | Min, max | 22.0, 39.0 | | 30.0, 38.0 | |

| Week 52-LOCF | | | | | |
|---|---|---|---|---|---|
| | n | 20 | 20 | 7 | 7 |
| | Mean (SD) | 34.35 (4.977) | 1.36 (2.554) | 34.57 (3.910) | 1.00 (1.528) |
| | Median (Q1, Q3) | 36.00 (30.00, 38.00) | 1.00 (0.00, 2.00) | 34.00 (32.00, 37.00) | 2.00 (0.00, 2.00) |
| | Min, max | 25.0, 40.0 | -2.0, 7.0 | 28.0, 40.0 | -2.0, 2.0 |

| Parameter estimation and comparison from ANCOVA^{b} | | | | | |
|---|---|---|---|---|---|
| | n | 20 | | 7 | |
| | LS means (SE) | 1.46 (0.547) | | 0.69 (1.013) | |
| | 95% CI of LS means | (0.32, 2.61) | | (-1.43, 2.80) | |
| | Difference of LS means (SE) (cipaglucosidase alfa/miglustat - alglucosidase alfa/placebo) | | | 0.78 (1.230) | |
| | 95% CI of difference | | | (-1.79, 3.34) | |
| | 2-sided p-value | | | 0.534 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; MMT = manual muscle testing; N = number of subjects in each treatment group; n = number of subjects with available data;; Q1 = first quartile; Q3 = third quartile; SD = standard deviation; SE = standard error ^{a} Baseline was the last non-missing value prior to the first dose date. ^{b} All estimates were obtained from the ANCOVA model including terms for treatment, baseline MMT lower extremity score, age, height, weight (all as continuous covariates), and gender. | | | | | |

### 6MWD at Week 26

Results for change in 6MWD (meters) from baseline to Week 26 for the ERT-naïve Population excluding Subject 4005-2511 were in general agreement with results at Week 52. For the ANCOVA model, the LS mean treatment difference (95% CI) was -10.38 meters (-49.27, 28.51), with a p-value of 0.584.

### PROMIS-Physical Function

The mean (SD) change in the PROMIS-Physical Function total score from baseline to Week 52 showed a mean improvement of 2.5 (8.62) for the cipaglucosidase alfa/miglustat group compared to 5.1 (7.82) for the alglucosidase alfa/placebo group. Results for PROMIS-Physical Function scores in both treatment groups showed improvement and directionally favored alglucosidase alfa/placebo over cipaglucosidase alfa/miglustat (p = 0.249).

Table 48 summarizes the mean change in PROMIS-Physical Function total score by visit (ITT Population) from baseline to Week 52 and ANCOVA model (ITT-OBS Population) for the ERT-naïve Population excluding Subject 4005-2511. FIG. 82 displays a line plot of the summary statistics by visit.

**Table 48: Summary of Change in PROMIS-Physical Function Short Form 20a by Visit from Baseline to Week 52 (ITT Population) and ANCOVA Model (ITT-LOCF Population) - ERT-naïve Subjects Excluding Subject 4005-2511 - Study ATB200-03**

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| Baseline^{a} | | | | | |
| | n | 20 | | 7 | |
| | Mean (SD) | 74.65 (11.984) | | 72.71 (16.317) | |
| | Median (Q1, Q3) | 76.00 (67.00, 83.00) | | 67.00 (56.00, 91.00) | |
| | Min, max | 50.0, 96.0 | | 53.0, 93.0 | |

| Week 52-LOCF | | | | | |
|---|---|---|---|---|---|
| | n | 20 | 20 | 7 | 7 |
| | Mean (SD) | 77.15 (11.536) | 2.50 (8.618) | 77.86 (15.742) | 5.14 (7.819) |

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| | Median (Q1, Q3) | 77.50 (68.50, 87.50) | 0.00 (-3.00, 7.50) | 80.00 (69.00, 91.00) | 5.00 (-1.00, 13.00) |
| | Min, max | 56.0, 95.0 | -8.0, 28.0 | 50.0, 94.0 | -6.0, 16.0 |

| Parameter estimation and comparison from ANCOVA^{b} | | | | | |
|---|---|---|---|---|---|
| | n | 20 | | 7 | |
| | LS means (SE) | 1.87 (1.917) | | 6.96 (3.539) | |
| | 95% CI of LS means | (-2.13, 5.86) | | (-0.43, 14.34) | |
| | Difference of LS means (SE) (cipaglucosidase alfa/miglustat - alglucosidase alfa/placebo) | | | -5.09 (4.288) | |
| | 95% CI of difference | | | (-14.04, 3.85) | |
| | 2-sided p-value | | | 0.249 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; N = number of subjects in each treatment group; n = number of subjects with available data; PROMIS = Patient-reported Outcomes Measurement Information System; Q1 = first quartile; Q3 = third quartile; SD = standard deviation; SE = standard error Note: The total score ranges from 20 to 100, with a higher score indicating less impact on physical function, and it is calculated by summing the scores (1 to 5) across all 20 items. ^{a} Baseline is the last non-missing value prior to the first dose date. ^{b} All estimates were obtained from the ANCOVA model including terms for treatment, baseline PROMIS-Physical Function total score, age, height, weight (all as continuous covariates), and gender. | | | | | |

### PROMIS-Fatigue

The mean (SD) change in the PROMIS-Fatigue total score from baseline to Week 52 showed a mean improvement of -2.7 (5.17) for the cipaglucosidase alfa/miglustat group and of -5.4 (6.61) for the alglucosidase alfa/placebo group. Results for PROMIS-Fatigue scores in both treatment groups showed an improvement (p = 0.338).

Table 49 summarizes the mean change in PROMIS-Fatigue total score by visit (ITT Population) from baseline to Week 52 and ANCOVA model (ITT-LOCF Population) for the ERT-naïve Population excluding Subject 4005-2511. FIG. 83 displays a line plot of the summary statistics by visit.

**Table 49: Summary of Change in PROMIS-Fatigue Short Form 8a Total Score by Visit from Baseline to Week 52 (ITT Population) and ANCOVA Model (ITT-LOCF Population) - ERT-naïve Population Excluding Subject 4005-2511 - Study ATB200-03**

| | | **Cipaglucosidase Alfa/Miglustat (N = 20)** | | **Alglucosidase Alfa/Placebo (N = 7)** | |
|---|---|---|---|---|---|
| | | **Absolute Value** | **CHG** | **Absolute Value** | **CHG** |
| Baseline^{a} | | | | | |
| | n | 20 | | 7 | |
| | Mean (SD) | 23.10 (9.614) | | 24.13 (8.360) | |
| | Median (Q1, Q3) | 20.95 (15.00, 29.50) | | 24.00 (20.00, 31.00) | |
| | Min, max | 10.0, 40.0 | | 9.0, 34.0 | |

| Week 52-LOCF | | | | | |
|---|---|---|---|---|---|
| | n | 20 | 20 | 7 | 7 |
| | Mean (SD) | 20.60 (9.681) | -2.50 (5.630) | 16.43 (7.231) | -7.70 (8.771) |
| | Median (Q1, Q3) | 16.50 (15.00, 27.50) | -2.00 (-4.50, 0.00) | 15.00 (8.00, 25.00) | -6.90 (-16.00, -1.00) |
| | Min, max | 8.0, 40.0 | -19.0, 9.0 | 8.0, 26.0 | -21.0, 5.0 |

| Parameter estimation and comparison from ANCOVA^{b} | | | | | |
|---|---|---|---|---|---|
| | n | 20 | | 7 | |
| | LS means (SE) | -2.99 (1.487) | | -6.28 (2.757) | |
| | 95% CI of LS means | (-6.09, 0.11) | | (-12.03, -0.53) | |
| | Difference of LS means (SE) (cipaglucosidase alfa/miglustat - alglucosidase alfa/placebo) | | | 3.29 (3.346) | |
| | 95% CI of difference | | | (-3.69, 10.27) | |
| | 2-sided p-value | | | 0.338 | |

| | | | | | |
|---|---|---|---|---|---|
| Abbreviations: ANCOVA = analysis of covariance; CHG = change from baseline; CI = confidence interval; ERT = enzyme replacement therapy; ITT = Intent-to-Treat; LOCF = last observation carried forward; LS = least squares; max = maximum; min = minimum; N = number of subjects in each treatment group; n = number of subjects with available data; PROMIS = Patient-reported Outcomes Measurement Information System; Q1 = first quartile; Q3 = third quartile; SD = standard deviation; SE = standard error Note: The total score ranges from 8 to 40, with a lower score indicating less impact by fatigue, and it is calculated by summing up the scores (1 to 5) across all 8 items. ^{a} Baseline is the last non-missing value prior to the administration of the first dose of study drug. ^{b} All estimates were obtained from the ANCOVA model including terms for treatment, baseline PROMIS-Fatigue total score, age, height, weight (all as continuous covariates), and gender. | | | | | |

### GSSC

The mean (SD) change in the GSGC total score from baseline to Week 52 showed an improvement of -0.6 (2.64) for the cipaglucosidase alfa/miglustat group and of 1.3 (1.80) for the alglucosidase alfa/placebo group. For the ANCOVA model, the LS mean treatment difference (95% CI) was -1.32 (-4.03, 1.39), with a p-value of 0.320.

Table 50 summarizes the mean change in GSGC total score by visit (ITT Population) from baseline to Week 52 and ANCOVA model (ITT-OBS Population) for the ERT-naïve Population excluding Subject 4005-2511. FIG. 84 displays a line plot of the summary statistics by visit.

**Table 50: Summary of Change in GSGC Total Score by Visit from Baseline to Week 52 (ITT Population) and ANCOVA Model (ITT-LOCF Population) - ERT-naïve Subjects Excluding Subject 4005-2511 - Study ATB200-03**

| | | **Cipaglucosidase Alfa/Miglustat (N = 19)** | | **Alglucosidase Alfa/Placebo (N = 7)** | |
|---|---|---|---|---|---|
| | | **Absolute Value** | **CHG** | **Absolute Value** | **CHG** |
| Baseline^{a} | | | | | |
| | n | 19 | | 7 | |
| | Mean (SD) | 11.32 (6.650) | | 10.86 (4.413) | |
| | Median (Q1, Q3) | 12.00 (5.00, 18.00) | | 9.00 (8.00, 16.00) | |
| | Min, max | 4.0, 24.0 | | 6.0, 18.0 | |

| Week 52-LOCF | | | | | |
|---|---|---|---|---|---|
| | n | 18 | 18 | 7 | 7 |
| | Mean (SD) | 10.06 (5.407) | -0.56 (2.640) | 12.14 (4.598) | 1.29 (1.799) |
| | Median (Q1, Q3) | 8.50 (5.00, 13.00) | -0.50 (-1.00, 1.00) | 10.00 (9.00, 16.00) | 1.00 (0.00, 2.00) |
| | Min, max | 4.0, 20.0 | -8.0, 4.0 | 6.0, 19.0 | 0.0, 5.0 |

| Parameter estimation and comparison from ANCOVA^{b} | | | | | |
|---|---|---|---|---|---|
| | n | 18 | | 7 | |
| | LS means (SE) | -0.41 (0.595) | | 0.91 (1.042) | |
| | 95% CI of LS means | (-1.66, 0.84) | | (-1.28, 3.10) | |
| | Difference of LS means (SE) (cipaglucosidase alfa/miglustat - alglucosidase alfa/placebo) | | | -1.32 (1.289) | |
| | 95% CI of difference | | | (-4.03, 1.39) | |
| | 2-sided p-value | | | 0.320 | |

| | | | | | |
|---|---|---|---|---|---|
| Abbreviations: ANCOVA = analysis of covariance; CHG = change from baseline; CI = confidence interval; ERT = enzyme replacement therapy; GSGC = Gait, Stairs, Gowers' maneuver, and Chair test; ITT = Intent-to-Treat; LOCF = last observation carried forward; LS = least squares; max = maximum; min = minimum; N = number of subjects in each treatment group; n = number of subjects with available data; Q1 = first quartile; Q3 = third quartile; SD = standard deviation; SE = standard error Note: Gait score was based on the 10-m walk test; Stairs score was based on the subject climbing stairs; Gowers' maneuver score was based on the subject lying down on the floor, then rising from the floor to get to a standing position; Chair score was based on the subject arising from a sitting position in a chair to a standing position. GSGC total score was the sum of 4 tests and ranges from a minimum of 4 points (normal performance) to a maximum of 27 points (worst score). ^{a} Baseline was the last non-missing value prior to the administration of the first dose of study drug. ^{b} All estimates were obtained from the ANCOVA model including terms for treatment, baseline GSGC total score, age, height, weight (all as continuous covariates), and gender. | | | | | |

### Summary of Results for ATB200-03

Table 51 and FIG. 85 summarize results across the primary, key secondary, and several other secondary (motor and pulmonary function, muscle strength, and PROs) and biomarker endpoints (Hex4 and CK) for the ITT Population excluding the outlier subject, showing standardized effect size on the change from baseline at Week 52 within each treatment group for each endpoint. Results across the vast majority of endpoints improved at Week 52 (designated by bars above 0) and directionally favored cipaglucosidase alfa/miglustat over alglucosidase alfa/placebo (designated by higher left/dark bars versus the right/light bars) in FIG. 85.

Statistically significant improvement from baseline (lower bound of 95% CI >0 for parameters where increase is improvement; upper bound of 95% CI <0 for parameters where decrease is improvement) was observed for cipaglucosidase alfa/miglustat across a number of parameters: 6MWD, % predicted 6MWD, MMT lower extremities, MMT upper extremities, overall MMT, PROMIS-Physical Function, PROMIS-Fatigue, CK, and Hex4, with statistically significant decline observed only for SVC sitting. For comparison, statistically significant improvement from baseline was not observed for any parameters with alglucosidase alfa/placebo, with statistically significant decline observed for GSGC, FVC sitting, FVC supine, SVC sitting, and CK.

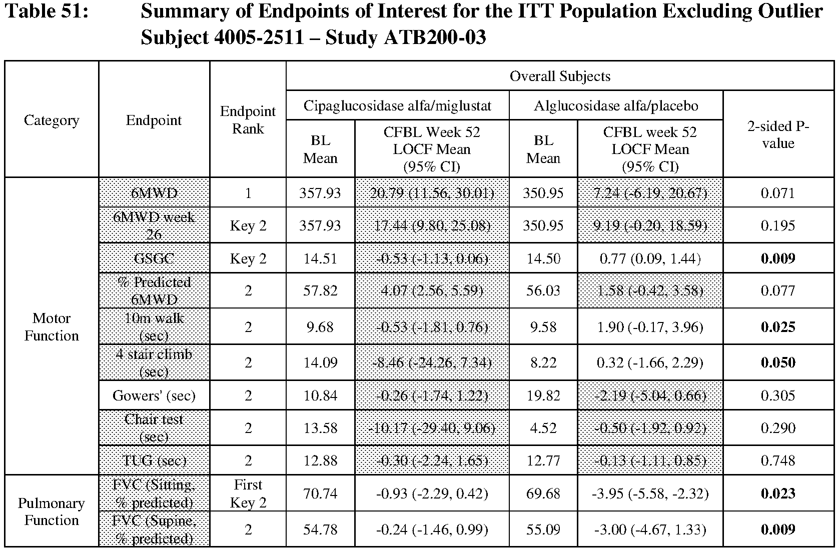

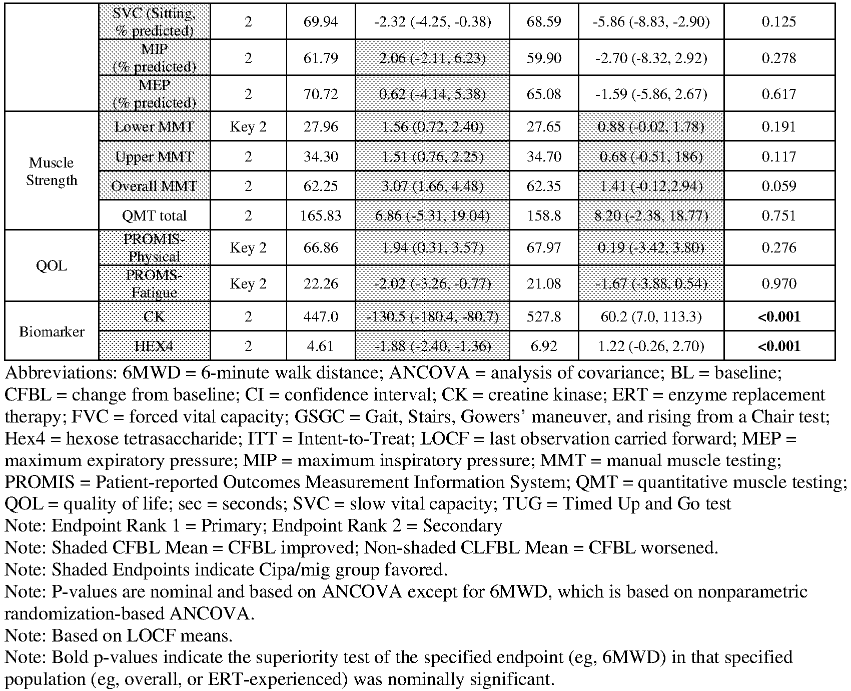

### Analyses of Primary and Secondary Endpoints Using MMRM with Actual Time Point of Assessments - Study ATB200-03

While the prespecified analyses (nonparametric ANCOVA for the primary endpoint of 6MWD and ANCOVA for the key secondary endpoints) are the Amicus core position and are included in the Company Core Data Sheet (CCDS), additional post hoc analyses were performed in response to a request from the Committee for Medicinal Products for Human Use (CHMP) during assessment of the EU Marketing Authorization Application (MAA). These analyses used an MMRM approach with actual time point of assessments and the results of these analyses are presented in the EU summary of product characteristics (SmPC).

### MMRM Analysis in the Overall ITT Population

Analysis of 6MWD was performed using an MMRM model with actual time point of assessments on the ITT-OBS and excluding the outlier Subject 4005-2511. Specifically, the dependent variable is the change from baseline in the assessment. Independent variables include the fixed, categorical effects of treatment, ERT status, and gender, as well as the fixed, continuous covariates of time of assessment (days), baseline 6MWD, baseline age, baseline weight, and baseline height, and the treatment-by-time interaction. A random intercept of subject is also included in the model. Change from baseline at Week 52 for each treatment group and the difference between treatment groups were then estimated with the LS means at Day 364, together with the 95% CIs. The results of this analysis are summarized in Table 52. Six-minute walk distance for cipaglucosidase alfa/miglustat showed significant improvement from baseline and greater improvement versus alglucosidase alfa/placebo but did not demonstrate statistical superiority. At Week 52, the cipaglucosidase alfa/miglustat group had an LS mean improvement (95% CI) of 20.0 m (13.1, 26.9) from baseline compared to 8.3 m (-2.2, 18.8) for the alglucosidase alfa/placebo group. The LS mean treatment difference (95% CI) was 11.7 m (-1.0, 24.4).

**Table 52: Summary of Results on 6MWD Based on MMRM Model, Actual Time Point of Assessments (ITT-OBS Population Excluding Subject 4005-2511) - Study ATB200-03**

| **Endpoint** | **Cipaglucosidase Alfa/Miglustat LS Mean (95 % CI)** | **Alglucosidase Alfa/Placebo LS Mean (95 % CI)** | **LS Mean Treatment Difference^{a}** | **95% CI of Difference^{b}** |
|---|---|---|---|---|
| CHG to week 52 in 6MWD | 20.0 (13.1, 26.9) | 8.3 (-2.2, 18.8) | 11.7 | (-1.0, 24.4) |

| | | | | |
|---|---|---|---|---|
| Abbreviations: 6MWD = 6-minute walk distance; CHG = change from baseline; CI = confidence interval; ERT = enzyme replacement therapy; ITT-OBS = Intent-to-Treat Population that includes all available, observed data without any missing data imputation at Week 52; LS = least squares; MMRM = mixed-effect model for repeated measures ^{a} difference = cipaglucosidase alfa/miglustat - alglucosidase alfa/placebo ^{b} MMRM model is used for the analysis based on the ITT-OBS population. The model includes terms for treatment, assessment time, treatment by assessment time interaction, baseline 6MWD value, age, height, weight (all as continuous covariates), ERT-status (ERT-naïve versus ERT-experienced), and gender. | | | | |

Analyses of the key secondary endpoints of sitting % predicted FVC, MMT lower extremity score, PROMIS-Physical Function, PROMIS-Fatigue, and GSGC total score were also performed using the MMRM model with actual time point of assessments on the ITT-OBS Population. Table 53 summarizes results for the 5 key secondary endpoints for the combined overall ITT-OBS Population (enzyme replacement therapy [ERT]-experienced and ERT-naïve) excluding the outlier subject (4005-2511). For % predicted FVC, cipaglucosidase alfa/miglustat demonstrated nominally significant improvement (95% CI of the difference excluded 0) versus alglucosidase alfa/placebo in the overall ITT-OBS Population. Results for the other key secondary endpoints listed, including assessments of motor function (MMT and GSGC) and PROs (PROMIS-Physical Function and PROMIS-Fatigue), numerically favored cipaglucosidase alfa/miglustat over alglucosidase alfa/placebo, with GSGC also demonstrating nominal superiority. As a change from baseline, mean improvements were observed for cipaglucosidase alfa/miglustat for MMT, PROMIS-Physical Function, PROMIS-Fatigue, and GSGC. Taken together, the consistency of results across several clinical dimensions of Pompe disease provides supportive evidence of the efficacy benefit of cipaglucosidase alfa/miglustat over alglucosidase alfa/placebo in the overall population.

**Table 53: Summary of Results on the Key Secondary Endpoints Based on MMRM Model, Actual Time Point of Assessments (ITT-OBS Population Excluding Subject 4005-2511) - Study ATB200-03**

| **Level Endpoint** | **Cipaglucosidase Alfa/Miglustat LS Mean (95 % CI)** | **Alglucosidase Alfa/Placebo LS Mean (95 % CI)** | **LS Mean Treatment Difference^{a}** | **95% CI of Difference^{b}** |
|---|---|---|---|---|
| CHG to Week 52 in sitting % predicted FVC | -1.4 (-2.5, -0.3) | -3.7 (-5.4, -2.0) | 2.3 | (0.2, 4.4) |
| CHG to Week 52 in MMT lower extremity score | 1.7 (1.1, 2.4) | 0.7 (-0.4, 1.7) | 1.1 | (-0.1, 2.3) |
| CHG to Week 52 in PROMIS-Physical Function total score^{c} | 2.2 (0.5, 3.9) | -0.3 (-2.9, 2.3) | 2.5 | (-0.6, 5.7) |
| CHG to Week 52 in PROMIS-Fatigue total score^{c} | -2.0 (-3.2, -0.9) | -1.7 (-3.4, 0.0) | -0.3 | (-2.4, 1.8) |
| CHG to Week 52 in GSGC total score | -0.7 (-1.2, -0.2) | 0.8 (0.0, 1.5) | -1.5 | (-2.4, -0.6) |

| | | | | |
|---|---|---|---|---|
| Abbreviations: CHG = change from baseline; CI = confidence interval; ERT = enzyme replacement therapy; FVC = forced vital capacity; GSGC = Gait, Stairs, Gowers' maneuver, and Chair test; ITT-OBS = Intent-to-Treat Population that includes all available, observed data without any missing data imputation at Week 52; LS = least squares; MMRM = mixed-effect model for repeated measures; MMT = manual muscle testing; PROMIS = Patient-reported Outcomes Measurement Information System ^{a} difference = cipaglucosidase alfa/miglustat - alglucosidase alfa/placebo. For both PROMIS-Fatigue and GSGC, decrease indicates improvement. ^{b} MMRM model is used for the analysis of the key secondary endpoints based on the ITT-OBS Population. The model includes terms for treatment, assessment time, treatment by assessment time interaction, baseline of response variable, age, height, weight (all as continuous covariates), ERT-status (ERT-naïve versus ERT-experienced), and gender. ^{c} The total score was calculated by summing scores (1 to 5) across all items. | | | | |

### MMRM Analysis by ERT Status

Analyses of the primary and key secondary endpoints were also performed using the same model as above, by enzyme replacement therapy (ERT) status. Table 54 summarizes results for the primary endpoint and 5 key secondary endpoints for the ITT-OBS ERT-experienced Population. For the primary endpoint of change from baseline to Week 52 in 6MWD, cipaglucosidase alfa/miglustat demonstrated significant improvement from baseline and nominally statistically significant (95% CI of the LS mean treatment difference excluded 0) and clinically meaningful improvement versus alglucosidase alfa/placebo. Similarly, for the first key secondary endpoint of % predicted FVC, cipaglucosidase alfa/miglustat demonstrated nominally statistically significant (95% CI of the LS mean treatment difference excluded 0) and clinically meaningful improvement versus alglucosidase alfa/placebo. Results for the other key secondary endpoints listed, including assessments of muscle strength and motor function (MMT lower extremity and GSGC) and PROs (PROMIS-Physical Function and PROMIS-Fatigue), numerically favored cipaglucosidase alfa/miglustat over alglucosidase alfa/placebo, with GSGC also demonstrating nominal superiority. As a change from baseline, mean improvements were observed for cipaglucosidase alfa/miglustat for MMT, PROMIS-Physical Function, PROMIS-Fatigue, and GSGC. Taken together, the consistency of results across several dimensions of clinical manifestations in Pompe disease provides supportive evidence of the efficacy benefit of cipaglucosidase alfa/miglustat over alglucosidase alfa/placebo in the ERT-experienced Population.

**Table 54: Summary of Results on the Primary and Key Secondary Endpoints Based on MMRM Model, Actual Time Point of Assessments (ITT-OBS Population - ERT-experienced Subjects) - Study ATB200-03**

| **Level Endpoint** | **Cipaglucosidase Alfa/Miglustat LS Mean (95% CI)** | **Alglucosidase Alfa/Placebo LS Mean (95 % CI)** | **LS Mean Treatment Difference^{a}** | **95 % CI of Difference^{b}** |
|---|---|---|---|---|
| Primary endpoint | | | | |
| CHG to Week 52 in 6MWD | 15.8 (8.3, 23.4) | 0.9 (-10.2, 12.1) | 14.9 | (1.2, 28.6) |

| Key secondary endpoints | | | | |
|---|---|---|---|---|
| CHG to Week 52 in sitting % predicted FVC | -0.2 (-1.5, 1.1) | -3.8 (-5.7, -1.9) | 3.6 | (1.3, 5.9) |
| CHG to Week 52 in MMT lower extremity score | 1.8 (1.0, 2.6) | 0.9 (-0.3, 2.1) | 0.9 | (-0.6, 2.3) |
| CHG to Week 52 in PROMIS-Physical Function total score^{c} | 2.0 (-0.0, 4.0) | -1.6 (-4.5, 1.4) | 3.5 | (-0.1, 7.1) |
| CHG to Week 52 in PROMIS-Fatigue total score^{c} | -1.9 (-3.2, -0.6) | -0.7 (-2.6, 1.2) | -1.2 | (-3.5, 1.1) |

| **Level Endpoint** | **Cipaglucosidase Alfa/Miglustat LS Mean (95% CI)** | **Alglucosidase Alfa/Placebo LS Mean (95 % CI)** | **LS Mean Treatment Difference^{a}** | **95 % CI of Difference^{b}** |
|---|---|---|---|---|
| CHG to Week 52 in GSGC total score | -0.7 (-1.3, -0.1) | 0.5 (-0.4, 1.4) | -1.2 | (-2.2, -0.1) |

| | | | | |
|---|---|---|---|---|
| Abbreviations: 6MWD = 6-minute walk distance; CHG = change from baseline; CI = confidence interval; ERT = enzyme replacement therapy; FVC = forced vital capacity; GSGC = Gait, Stairs, Gowers' maneuver, and Chair test; ITT-OBS = Intent-to-Treat Population that includes all available, observed data without any missing data imputation at Week 52; LS = least squares; MMRM = mixed-effect model for repeated measures; MMT = manual muscle testing; PROMIS = Patient-reported Outcomes Measurement Information System ^{a} difference = cipaglucosidase alfa/miglustat - alglucosidase alfa/placebo. For both PROMIS-Fatigue and GSGC, decrease indicates improvement. ^{b} MMRM model is used for the analysis of the primary and key secondary endpoints based on the ITT-OBS Population. The model includes terms for treatment, assessment time, treatment by assessment time interaction, baseline of response variable, age, height, weight (all as continuous covariates), and gender. ^{c} The total score was calculated by summing scores (1 to 5) across all items. | | | | |

Conversely, for the ERT-naïve Population, the first key secondary endpoint of % predicted FVC decreased from baseline in both treatment groups, numerically favoring the alglucosidase alfa/placebo group. Results for the key secondary endpoints assessing muscle strength and motor function (MMT lower extremity and GSGC) demonstrated improvement from baseline in the cipaglucosidase alfa/miglustat group and numerically favored cipaglucosidase alfa/miglustat over alglucosidase alfa/placebo. For the PRO key secondary endpoints (PROMIS-Physical Function and PROMIS-Fatigue), both treatment groups demonstrated improvement, numerically favoring the alglucosidase alfa/placebo group. Overall, for the ERT-naïve Population, some endpoints numerically favored cipaglucosidase alfa/miglustat and some endpoints numerically favored alglucosidase alfa/placebo with none of the endpoints achieving nominal statistical significance. These results suggest that efficacy of cipaglucosidase alfa/miglustat is no worse than that of alglucosidase alfa/placebo in the ERT-naive Population.

Table 55 summarizes results for the primary endpoint and 5 key secondary endpoints for the ITT-OBS ERT-naïve Population excluding the outlier subject. For the primary endpoint of change from baseline to Week 52 in 6MWD, clinically meaningful improvement from baseline was observed in both treatment groups, numerically favoring the alglucosidase alfa/placebo group. Interpretability of the MMRM analysis in the ERT-naïve Population is likely impacted by unstable estimates due to imbalance of covariates arising from the small sample size, and thus an analysis that does not involve adjustment for covariates (such as an unadjusted mean change from baseline) should be considered the primary approach for the evaluation of 6MWD in this group.

**Table 55: Summary of Results on the Primary and Key Secondary Endpoints Based on MMRM Model, Actual Time Point of Assessments (ITT-OBS Population Excluding Subject 4005-2511 - ERT-naïve Subjects) - Study ATB200-03**

| **Level Endpoint** | **Cipaglucosidase Alfa/Miglustat LS Mean (95 % CI)** | **Alglucosidase Alfa/Placebo LS Mean (95 % CI)** | **LS Mean Treatment Difference^{a}** | **95 % CI of Difference^{b}** |
|---|---|---|---|---|
| Primary endpoint | | | | |
| CHG to Week 52 in 6MWD | 28.5 (12.4, 44.7) | 52.7 (23.2, 82.3) | -24.2 | (-60.0, 11.7) |

| Key secondary endpoints | | | | |
|---|---|---|---|---|
| CHG to Week 52 in sitting % predicted FVC | -5.2 (-7.5, -2.9) | -2.4 (-6.7, 1.8) | -2.8 | (-7.8, 2.3) |
| CHG to Week 52 in MMT lower extremity score | 1.4 (0.4, 2.5) | -0.0 (-1.9, 1.9) | 1.5 | (-0.8, 3.7) |
| CHG to Week 52 in PROMIS-Physical Function total score^{c} | 2.6 (-1.0, 6.3) | 5.8 (-0.9, 12.4) | -3.1 | (-11.2, 4.9) |
| CHG to Week 52 in PROMIS-Fatigue total score^{c} | -3.0 (-5.7, -0.2) | -4.9 (-9.9, 0.0) | 2.0 | (-4.1, 8.0) |
| CHG to Week 52 in GSGC total score | -0.6 (-1.6, 0.4) | 1.3 (-0.4, 3.1) | -1.9 | (-4.1, 0.2) |

| | | | | |
|---|---|---|---|---|
| Abbreviations: 6MWD = 6-minute walk distance; CHG = change from baseline; CI = confidence interval; ERT = enzyme replacement therapy; FVC = forced vital capacity; GSGC = Gait, Stairs, Gowers' maneuver, and Chair test; ITT-OBS = Intent-to-Treat Population that includes all available, observed data without any missing data imputation at Week 52; LS = least squares; MMRM = mixed-effect model for repeated measures; MMT = manual muscle testing; PROMIS = Patient-reported Outcomes Measurement Information System ^{a} difference = cipaglucosidase alfa/miglustat - alglucosidase alfa/placebo. For both PROMIS-Fatigue and GSGC, decrease indicates improvement. ^{b} MMRM model is used for the analysis of the primary and key secondary endpoints based on ITT-OBS Population. The model includes terms for treatment, assessment time, treatment by assessment time interaction, baseline of response variable, age, height, weight (all as continuous covariates), and gender. ^{c} The total score was calculated by summing scores (1 to 5) across all items. | | | | |

### Patient-reported Outcomes and Physician's Global Impression of Change - Study ATB200-03

Additional PROs included PROMIS-dyspnea, PROMIS-upper extremity, R-PACT, EQ-5D-5L, SGIC. The PGIC was also included. The results for PROMIS, R-PACT, and EQ-5D-5L all showed similar improvements in both treatment groups. SGIC and PGIC showed consistently greater improvement favoring cipaglucosidase alfa/miglustat treatment.

Eight different SGIC endpoints were assessed: overall physical well-being, effort of breathing, muscle strength, muscle function, ability to move around, activities of daily living, energy level, and muscular pain. Across all of these domains a greater percentage of patients in the overall population treated with cipaglucosidase alfa/miglustat reported improvement, and a lower percentage reported worsening, compared with patients treated with alglucosidase alfa. Similar results were observed for the PGIC. Results are shown below for the SGIC overall physical well-domain, which is representative of the benefits reported across these measures (FIG. 86).

### Clinical Relevance of Improvements in 6MWD and FVC - Study ATB200-03

Thresholds for clinically relevant changes for 6MWD and FVC in Pompe specifically are not well established, however a large body of data exists from other neuromuscular and chronic respiratory diseases, especially interstitial pulmonary fibrosis (IPF). There, 6MWD increases greater than 6% (range 3 to 11%) and FVC changes greater than 3% (range 2 to 6%) are considered clinically relevant using both anchor-based and distribution-based methodologies. Accordingly, these thresholds were applied to analyses of Study ATB200-03 data.

The results observed in 6MWD and FVC for cipaglucosidase alfa/miglustat represent clinically meaningful improvements for patients. The mean improvement of 21meters in 6MWD for the cipaglucosidase alfa/miglustat arm represented approximately a 6% increase from baseline (mean 358 meters), which indicates a clinically meaningful group-level improvement, while the mean improvement for the alglucosidase alfa arm, of 7 meters did not reach this threshold. For FVC, the 3% improvement for subjects treated with cipaglucosidase alfa/miglustat relative to alglucosidase alfa/placebo indicates a clinically meaningful group-level improvement, which is of similar magnitude as the clinically meaningful improvement alglucosidase alfa itself demonstrated versus placebo in the LOTS randomized controlled pivotal study.

The clinical relevance of these group level analyses is further supported by a prespecified composite patient-level responder analysis looking at responses in 6MWD, FVC, and MMT lower extremity score using clinically meaningful response thresholds of ± 6%, ± 3%, and ± 7%, respectively based on the medical literature noted above. This prespecified analysis showed a greater proportion of subjects in the overall population treated with cipaglucosidase alfa/miglustat demonstrated clinically meaningful improvement and a smaller proportion demonstrated clinically meaningful worsening across these 3 endpoints, which overall favored cipaglucosidase alfa/miglustat versus alglucosidase alfa/placebo (p = 0.012).

A prespecified comparison of subjects experiencing clinically meaningful improvement for both 6MWD and FVC showed a significantly greater proportion of subjects with improvement on both domains for cipaglucosidase alfa/miglustat (p = 0.041). Additional post hoc patient-level analyses were conducted using these thresholds for 6MWD (± 6%) and FVC (± 3%) separately and combined. These analyses show that a greater proportion of subjects treated with cipaglucosidase alfa/miglustat demonstrated clinically meaningful improvement, and a smaller proportion demonstrated clinically meaningful worsening, than patients treated with alglucosidase alfa/placebo for 6MWD (p = 0.018) (FIG. 87A) and FVC (p = 0.011) (FIG. 87B) individually, and for both 6MWD and FVC (p = 0.002) (FIG. 87C).

Additional sensitivity analyses demonstrate that across a range of response thresholds (eg, 6MWD ± 30m, ± 20m, ± 10m; FVC ± 9%, ± 6%, ± 3%) a greater proportion of subjects treated with cipaglucosidase alfa/miglustat showed clinically meaningful improvement and a smaller proportion showed clinically meaningful worsening versus subjects treated with alglucosidase alfa/placebo.

### Conclusions - Study ATB200-03

The primary endpoint of 6MWD in the overall population was tested for superiority and while numerically superior, statistical significance was not achieved for the cipaglucosidase alfa/miglustat group compared to the alglucosidase alfa/placebo group (p = 0.071).

On the first key secondary endpoint, FVC, cipaglucosidase alfa/miglustat demonstrated a nominally statistically significant (p = 0.023) and clinically meaningful difference for superiority versus alglucosidase alfa/placebo in the overall population. The absolute improvement of 3.0% in FVC versus alglucosidase alfa/placebo is nearly as large as the improvement observed in Phase 3 studies of alglucosidase alfa versus placebo, and is of a magnitude considered clinically meaningful to patients based on medical literature.

Patient-level responder analyses based on clinically relevant thresholds of change indicate that the improvements observed in 6MWD and FVC were clinically meaningful.

In the ERT-experienced subgroup, all primary and key secondary endpoints also showed improvement and directionally favored cipaglucosidase alfa/miglustat, supporting the significant results on 6MWD and % predicted FVC in this population. The analyses for 6MWD and FVC yielded p-values of 0.047 and 0.006, respectively.

In the smaller ERT-naïve Population, patients in both treatment groups showed improvement across the vast majority of primary and key secondary endpoints, with different endpoints numerically favoring different treatment groups.

A post hoc global test using the primary and the first key secondary endpoints 6MWD and FVC showed nominal statistical significance in the overall ITT Population including all subjects (2-sided p = 0.022) and supports the significance of the results observed on each of these endpoints separately in the population studied.

In all 3 populations, including the overall population, the ERT-experienced Population and the ERT-naïve Population, reductions in biomarkers of muscle damage (CK) and disease substrate (Hex4) also were significantly greater with cipaglucosidase alfa/miglustat compared with alglucosidase alfa, with nominal p-value of p < 0.001 for both biomarkers in the cipaglucosidase alfa/miglustat group.

In summary, co-administration of 20 mg/kg IV-infused cipaglucosidase alfa and 260 mg miglustat resulted in clinically significant and relevant improvements in all the important domains of Pompe disease (muscle strength, pulmonary and motor function, and PROs) as a change from baseline. Furthermore, cipaglucosidase alfa/miglustat was directionally favored on outcomes accessed across the totality of important Pompe disease domains over alglucosidase alfa/placebo in the overall population.

### Example 20: Study ATB200-07

Analysis populations presented in this summary are 1) the OLE-ES Population (all subjects who satisfied the eligibility requirements (based on the inclusion and exclusion criteria) and entered Study ATB200-07 and 2) the OLE-FAS Population (all subjects who entered the OLE Study ATB200-07 who had both a valid ATB200-07 baseline and at least 1 post-baseline assessment for at least 1 of the 6 main efficacy endpoints [6MWD, sitting % predicted FVC, MMT-lower extremities, PROMIS-Physical Function, PROMIS-Fatigue, and GSGC]). Data for the 6 main efficacy endpoints are presented 1) for the OLE-ES Population excluding the outlier and 2) for the OLE-FAS Population from the Study ATB200-07 baseline, as portrayed below.

OLE-ES
Population
N = 119

ATB200-03
Baseline

Week 52

Week 104

OLE-FAS
Population
N = 116

ATB200-07
Baseline

Week 52

This is an ongoing open-label extension study to assess the long-term safety and efficacy of cipaglucosidase alfa/miglustat in adult subjects with LOPD who completed Study ATB200-03.

As of the interim data cutoff of 11 January 2022, of the 123 subjects (85 treated with cipaglucosidase alfa/miglustat; 38 treated with alglucosidase alfa) who enrolled in Study ATB200-03, a total of 117 subjects completed the study and then enrolled in StudyATB200-07. Two additional subjects (1107-1681 and 2010-1352) did not complete Study ATB200-03 but enrolled in Study ATB200-07, bringing the total of subjects enrolled in Study ATB200-07 (OLE-ES Population) to 119 (91 ERT-experienced and 28 ERT-naïve).

As noted above, 2 subjects who did not complete Study ATB200-03 enrolled in Study ATB200-07. Subject 1107-1681 discontinued cipaglucosidase alfa/miglustat in Study ATB200-03 due to an adverse event (AE) of coronavirus disease 2019 that is caused by the SARS-Cov-2 virus (COVID-19)-related pneumonia, and subsequently enrolled in Study ATB200-07. Subject 2010-1352 discontinued Study ATB200-03 because of unwillingness to travel to the study site due to concerns about COVID-19, and subsequently enrolled in Study ATB200-07.

Of the 119 subjects who enrolled in Study ATB200-07 (OLE-ES Population), 82 (96.5%) of the 85 subjects previously treated with cipaglucosidase alfa/miglustat in Study ATB200-03 (cipaglucosidase alfa/miglustat group) entered Study ATB200-07 and continued treatment with cipaglucosidase alfa/miglustat (designated as the cipaglucosidase alfa/miglustat - cipaglucosidase alfa/miglustat group), and 37 of the 38 (97.4%) subjects previously treated with alglucosidase alfa in Study ATB200-03 (alglucosidase alfa/placebo group) entered Study ATB200-07 and switched to cipaglucosidase alfa/miglustat (designated as the alglucosidase alfa/placebo - cipaglucosidase alfa/miglustat group or the Treatment Switched Population). One subject in the cipaglucosidase alfa/miglustat group (Subject 2024-1101) withdrew consent due to the COVID-19 pandemic and was never dosed in Study ATB200-07. Therefore, the OLE Safety Population comprised 118 subjects. Finally, 2 subjects did not provide a valid baseline and at least 1 post-baseline assessment for at least 1 of the 6 main efficacy endpoints; therefore, the OLE-FAS Population comprised 116 subjects.

As of the data cutoff (11 January 2022), there was low attrition from the OLE-FAS Population in Study ATB200-07, with a total of 11 (9.2%) dosed subjects prematurely discontinuing from the study. Of the 7 subjects in the cipaglucosidase alfa/miglustat - cipaglucosidase alfa/miglustat group who discontinued early, 5 subjects withdrew consent, 1 subject discontinued due to an AE, and 1 subject was lost to follow-up. Of the 4 subjects in the alglucosidase alfa/placebo - cipaglucosidase alfa/miglustat group who discontinued early, 1 subject withdrew consent, 2 subjects discontinued due to an AE, and 1 subject discontinued due to worsening of condition.

Baseline demographics were generally similar between subjects who stayed on cipaglucosidase alfa/miglustat in Study ATB200-07 (the cipaglucosidase alfa/miglustat - cipaglucosidase alfa/miglustat group) and subjects who switched from alglucosidase alfa/placebo in Study ATB200-03 to cipaglucosidase alfa/miglustat in Study ATB200-07 (the alglucosidase alfa/placebo - cipaglucosidase alfa/miglustat group). The ERT status in Study ATB200-07 for each subject was based on the ERT status recorded at baseline in Study ATB200-03. Of the 116 subjects in the Study ATB200-07 OLE-FAS Population, 23.3% were ERT-naïve (n = 27) and 76.7% subjects were ERT-experienced (n = 89). The percent of subjects who were ERT-experienced or ERT-naïve in each of the treatment groups from Study ATB200-03 was similar. A majority (67.4%) of subjects in both treatment groups had ≥ 5 years of prior treatment with ERT (69.2% of subjects in the Study ATB200-03 cipaglucosidase alfa/miglustat group and 63.3% of subjects in the Study ATB200-03 alglucosidase alfa/placebo group).

Efficacy data are presented in this summary as change from the baseline of Study ATB200-03 (OLE-ES Population). Data from the baseline of Study ATB200-07 (OLE-FAS Population) and in the ERT-experienced and ERT-naïve Populations are consistent with those observed for the overall OLE-ES Population.

### 6MWD and % Predicted 6MWD

As shown in FIG. 88, subjects in the cipaglucosidase alfa/miglustat group (who were treated with cipaglucosidase alfa/miglustat for 52 weeks in Study ATB200-03 and continued cipaglucosidase alfa/miglustat treatment for an additional 52 weeks in Study ATB200-07) showed an improvement through Study ATB200-03, which was then maintained through Week 104. Subjects in the alglucosidase alfa/placebo group (who were treated with alglucosidase alfa/placebo for 52 weeks in Study ATB200-03 and switched to cipaglucosidase alfa/miglustat for 52 weeks in Study ATB200-07) showed smaller improvements than the cipaglucosidase alfa/miglustat group while on alglucosidase alfa to Week 52, then generally stabilized at a lower level than the cipaglucosidase alfa/miglustat group after switching to cipaglucosidase alfa/miglustat through Week 104.

### Sitting % Predicted FVC

As shown in FIG. 89, subjects in the cipaglucosidase alfa/miglustat group (who were treated with cipaglucosidase alfa/miglustat for 52 weeks in Study ATB200-03 and continued on cipaglucosidase alfa/miglustat for an additional 52 weeks in Study ATB200-07) showed a slight decline from baseline through Study ATB200-03, then stabilized through Week 104. Subjects in the alglucosidase alfa/placebo group (who were treated with alglucosidase alfa/placebo for 52 weeks in Study ATB200-03 and switched to cipaglucosidase alfa/miglustat for 52 weeks in Study ATB200-07) showed a greater decline than the cipaglucosidase alfa/miglustat group while on alglucosidase alfa through Week 52, then generally stabilized at a lower level than the cipaglucosidase alfa/miglustat group after switching to cipaglucosidase alfa/miglustat through Week 104, with some visit to visit variability during Study ATB200-07.

### Other Main Secondary Endpoints

Results for the other main endpoints (MMT lower extremity, PROMIS-Physical Function, PROMIS-Fatigue, and GSGC) were generally consistent with those observed for 6MWD. For subjects who were treated with cipaglucosidase alfa/miglustat through Studies ATB200-03 and ATB200-07, MMT lower extremity, PROMIS-Physical Function, PROMIS-Fatigue, and GSGC either improved or remained stable through Study ATB200-03, and then stabilized through Study ATB200-07. For subjects who were treated with alglucosidase alfa/placebo through Study ATB200-03 and switched to cipaglucosidase alfa/miglustat in Study ATB200-07, MMT lower extremity, and PROMIS-Fatigue showed a slight improvement from the Study ATB200-03 baseline, and then either improved further or stabilized through Week 104. The PROMIS-Physical Function and GSGC remained generally stable from the Study ATB200-03 baseline through Study ATB200-07.

### Pharmacodynamics

Analyses of the mean change in the PD markers CK and Hex4 from the Study ATB200-03 baseline to Week 104 support the results presented for the 6 main efficacy endpoints (FIGS. 90A and 90B). For subjects in the cipaglucosidase alfa/miglustat group (who were treated with cipaglucosidase alfa/miglustat for 52 weeks in Study ATB200-03 and continued on cipaglucosidase alfa/miglustat for an additional 52 weeks in Study ATB200-07), CK (normal range for females: 34 to 145 U/L; normal range for males: 46 to 171 U/L) and Hex4 (normal range: 3.0 mmol/mol creatinine) both declined substantially through Week 52, then stabilized from Week 52 through Week 104. For subjects in the alglucosidase alfa/placebo group (who were treated with alglucosidase alfa/placebo for 52 weeks in Study ATB200-03 and switched to cipaglucosidase alfa/miglustat for 52 weeks in Study ATB200-07), CK remained generally stable at or slightly above the Study ATB200-03 baseline values through Week 52, then declined substantially after subjects switched to cipaglucosidase alfa/miglustat at Week 52 through Week 104. Hex4 increased from the ATB200-03 baseline through Week 52 while subjects were treated with alglucosidase alfa/placebo, then decreased after subjects switched to cipaglucosidase alfa/miglustat through Week 104.

### Conclusion

Study ATB200-07, along with efficacy data from Study ATB200-02, provides data supporting the maintenance and durability of the cipaglucosidase alfa/miglustat effect. Table 56 is a summary of changes from the Study ATB200-03 baseline at Week 52 (the Study ATB200-07 baseline) and Week 104 (Week 52 of Study ATB200-07).

The majority of the 6 main endpoints (ie, 6MWD, MMT lower extremity, PROMIS-Physical Function, PROMIS-Fatigue, and GSGC scores) for both treatment groups remained stable or improved through Study ATB200-03. For subjects who stayed on cipaglucosidase alfa/miglustat through Study ATB200-07, the benefits of treatment with cipaglucosidase alfa/miglustat were maintained. Subjects who switched from alglucosidase alfa/placebo to cipaglucosidase alfa/miglustat in Study ATB200-07 showed stability or improvement on the majority of endpoints. For subjects who stayed on cipaglucosidase alfa/miglustat through Study ATB200-07, sitting % predicted FVC showed a slight decline from baseline through Study ATB200-03, then stabilized through Study ATB200-07. For subjects who switched from alglucosidase alfa/placebo to cipaglucosidase alfa/miglustat in Study ATB200-07, sitting % predicted FVC showed a substantial decline in Study ATB200-03, then stabilized at a lower level through Study ATB200-07. Overall, these results support the benefit of cipaglucosidase alfa/miglustat in patients with LOPD.

For subjects in the OLE-ES Population who were treated with cipaglucosidase alfa/miglustat through Studies ATB200-03 and ATB200-07, CK and Hex4 declined substantially through Study ATB200-03, then stabilized through Study ATB200-07. For subjects who switched to cipaglucosidase alfa/miglustat in Study ATB200-07, CK remained generally stable at or slightly above the Study ATB200-03 baseline values through Week 52 while subjects were treated with alglucosidase alfa/placebo, then declined substantially after subjects switched to cipaglucosidase alfa/miglustat through Week 104. Levels of Hex4 increased from the Study ATB200-03 baseline through Week 52 while subjects were treated with alglucosidase alfa/placebo, then decreased substantially after subjects switched to cipaglucosidase alfa/miglustat through Week 104.

The analyses of ERT-experienced and ERT-naïve subjects support the results of the overall population.

**Table 56: Summary of Efficacy Assessments - Change from Study ATB200-03 Baseline at Week 52 and Week 104 in Study ATB200-07 (OLE-ES Population Excluding Subject 4005-2511)**

| **Clinical Response Assessments (Selected Efficacy and Pharmacodynamic)** | **Mean (SD) Baseline and Change from ATB200-03 Baseline at Week 52 and Week 104** | | | | | |
|---|---|---|---|---|---|---|
| | **Cipaglucosidase alfa/Miglustat (n = 82)^{a}** | | | **Alglucosidase alfa/Placebo (n = 36)^{a}** | | |
| | **BSL** | **52-wk CFB** | **104-wk CFB** | **BSL** | **52-wk CFB** | **104-wk CFB** |
| 6MWD (m) | 354.9 (112.39) (n = 82) | 20.6 (42.27) (n = 81) | 20.2 (53.58) (n = 74) | 351.7 (122.96) (n = 36) | 8.0 (40.56) (n = 36) | 3.3 (56.24) (n = 33) |
| Sitting FVC (% predicted) | 70.7 (19.92) (n = 82) | -1.1 (6.32) (n = 74) | -1.6 (7.45) (n = 70) | 69.5 (21.75) (n = 36) | -3.6 (4.70) (n = 32) | -3.6 (6.18) (n = 29) |
| MMT (lower extremity score) | 27.9 (5.85) (n = 81) | 1.6 (3.91) (n = 73) | 1.8 (4.16) (n = 68) | 27.8 (6.23) (n = 33) | 0.9 (2.65) (n = 32) | 1.1 (2.91) (n = 27) |
| PROMIS-Physical Function | 67.0 (12.41) (n = 81) | 2.2 (7.46) (n = 80) | 2.3 (9.11) (n = 75) | 67.9 (13.27) (n = 36) | 0.3 (10.97) (n = 36) | -0.4 (10.52) (n = 33) |
| PROMIS-Fatigue | 22.1 (8.40) (n = 82) | -2.3 (5.79) (n = 80) | -1.6 (5.88) (n = 74) | 21.2 (6.12) (n = 36) | -1.7 (6.72) (n = 36) | -0.1 (7.94) (n = 33) |
| GSGC | 14.4 (5.25) (n = 71) | -0.6 (2.65) (n = 59) | -1.0 (2.72) (n = 58) | 14.3 (4.69) (n = 31) | 0.9 (1.93) (n = 25) | 0.3 (2.75) (n = 25) |
| CK | 440.6 (403.15) (n = 82) | -129.1 (234.12) (n = 77) | -154.9 (224.89) (n = 67) | 527.4 (432.62) (n = 36) | 41.0 (140.15) (n = 35) | -173.6 (276.06) (n = 32) |
| Hex4 | 4.6 (3.36) (n = 81) | -2.0 (2.52) (n = 72) | -2.1 (3.07) (n = 73) | 7.0 (7.01) (n = 36) | 1.6 (4.50) (n = 33) | -2.7 (3.48) (n = 32) |

| | | | | | | |
|---|---|---|---|---|---|---|
| Abbreviations: 6MWD = 6-minute walk distance; BSL = baseline; CFB = change from baseline; CK = creatine kinase; FVC = forced vital capacity; GSGC = Gait, Stair, Gowers' maneuver, and Chair test; Hex4 = hexose tetrasaccharide; MMT = manual muscle testing; n = number of subjects; PROMIS = Patient-reported Outcomes Measurement Information System; wk = week Note: Week 52 = Study ATB200-07 baseline. Note: For both PROMIS-Fatigue and GSGC, decrease indicates improvement. ^{a} n at Study ATB200-03 baseline. Any differences in the n at baseline are indicated in parentheses. | | | | | | |

### Example 21: Comparison of Efficacy Results Across Studies

Detailed efficacy results included with this submission are presented for the three individual studies above. Due to the inherent differences in the study designs and methodologies, a comparison of efficacy has been limited to 6MWD and % predicted FVC across Studies ATB200-03/07 and ATB200-02 in Table 57 and Table 58. Data for Study ATB200-03/07 are excluding the outlier. Note that data for the alglucosidase alfa/cipaglucosidase alfa group represents 12 months treated with alglucosidase alfa/placebo followed by 12 months treated with cipaglucosidase alfa/miglustat. The benefits observed in 6MWD and % predicted FVC in Study ATB200-03 and maintained in Study ATB200-07 are supported by the directionally consistent results of Study ATB200-02. Note that the improvements in % predicted FVC observed in Study ATB200-02 occurred in subjects with lower baselines than those in Study ATB200-03.

**Table 57: Comparison of 6MWD Results Across Studies ATB200-03/07 and ATB200-02**

| **Population** | **Study** | **Treatment** | **N (at Baseline)** | **Baseline Mean (SD)** | **CFBL M12 (W52) Mean (SD)** | **CFBL M24 (W104) Mean (SD)** |
|---|---|---|---|---|---|---|
| ERT-experienced | ATB200-03/07 | Alglucosidase alfa/ Cipaglucosidase alfa | 29 | 335.0 (116.03) | 0.7 (39.84) | -8.8 (46.19) |
| | | Cipaglucosidase alfa | 62 | 342.4 (110.40) | 16.3 (39.46) | 14.2 (53.44) |
| | ATB200-02 | Cipaglucosidase alfa | 16^{a} | 393.5 (119.66) | 33.5 (49.62) | 25.2 (63.30) |
| ERT-naïve | ATB200-03/07 | Alglucosidase alfa/ Cipaglucosidase alfa | 7 | 420.9 (135.75) | 38.3 (29.32) | 48.3 (70.56) |
| | | Cipaglucosidase alfa | 20 | 393.6 (112.39) | 33.4 (48.70) | 38.8 (51.03) |
| | ATB200-02 | Cipaglucosidase alfa | 6^{b} | 396.0 (75.20) | 57.0 (29.96) | 54.4 (36.18) |

| | | | | | | |
|---|---|---|---|---|---|---|
| Abbreviations: 6MWD = 6-minute walk distance; CFBL = change from baseline; ERT = enzyme replacement therapy; M = month; N = number of subjects in each treatment group; SD = standard deviation; W = week ^{a} Cohort 1 and 4 combined ^{b} Cohort 3 | | | | | | |

**Table 58: Comparison of % Predicted FVC Results Across Studies ATB200-03/07 and ATB200-02**

| **Population** | **Study** | **Treatment** | **N (at Baseline)** | **Baseline Mean (SD)** | **CFBL M12 (W52) Mean (SD)** | **CFBL M24 (W104) Mean (SD)** |
|---|---|---|---|---|---|---|
| ERT-experienced | ATB200-03/07 | Alglucosidase alfa/ Cipaglucosidase alfa | 29 | 67.2 (21.29 | -3.5 (4.72) | -3.8 (6.23) |
| | | Cipaglucosidase alfa | 62 | 67.7 (19.48) | 0.1 (5.94) | -0.6 (7.50) |
| | ATB200-02 | Cipaglucosidase alfa | 16^{a} | 57.4 (17.42) | -1.2 (5.95) | 1.0 (7.96) |
| ERT-naive | ATB200-03/07 | Alglucosidase alfa | 7 | 79.1 (22.58) | -4.0 (5.05) | -3.1 (6.66) |
| | | Cipaglucosidase alfa | 20 | 80.2 (18.69) | -4.7 (6.20) | -4.8 (6.48) |
| | ATB200-02 | Cipaglucosidase alfa | 6^{b} | 57.2 (20.84) | 3.2 (8.42) | 4.7 (5.09) |

| | | | | | | |
|---|---|---|---|---|---|---|
| Abbreviations: CFBL = change from baseline; ERT = enzyme replacement therapy; FVC = forced vital capacity; M = month; N = number of subjects in treatment group; SD = standard deviation; W = week ^{a} Cohort 1 and 4 combined ^{b} Cohort 3 | | | | | | |

### Integrated Efficacy Including the Outlier Subject

The pooled efficacy analyses presented within this section include the outlier (Pool 2). The results of these pooled analyses are consistent with results from Study ATB200-03. Pooled efficacy analyses excluding the outlier subject (Pool 2A) were also performed. These analyses showed that improvements in 6MWD and stabilization of FVC were maintained beyond 24 months in the cipaglucosidase alfa/miglustat arm.

### 6MWT

The data demonstrate an initial improvement in 6MWD that was maintained greater than baseline out to > 24 months in subjects receiving cipaglucosidase alfa/miglustat. The mean (SD) improvement in 6MWD for the cipaglucosidase alfa/miglustat group was 17.1 (44.92) meters at ≤ 15 months (n = 143), 17.7 (55.61) meters at > 15 to ≤ 24 months (n = 108), and 9.7 (67.13) meters at > 24 months (n = 70). No long-term data for the alglucosidase alfa/placebo group are available, as all subjects switched to cipaglucosidase alfa/miglustat in Study ATB200-07.

The data for % predicted 6MWD demonstrate a similar pattern in subjects receiving cipaglucosidase alfa/miglustat after at least 24 months of treatment administration. The mean (SD) improvement in % predicted 6MWD for the cipaglucosidase alfa/miglustat group was 3.3 (10.84) (n = 70).

The improvements in 6MWD and % predicted 6MWD above baseline were maintained beyond 24 months with the outlier included or excluded. A summary of 6MWD by exposure interval of pooled efficacy data is presented including the outlier in Table 59.

**Table 59: Summary of 6MWD (meters) by Exposure Interval (Pool 2: Studies ATB200-02/03/07 - Efficacy Population Including the Outlier)**

| | | **Cipaglucosidase Alfa/Miglustat (N = 143)** | | | **Alglucosidase Alfa/Placebo (N = 38)** |
|---|---|---|---|---|---|
| | | **≤ 15 months (N = 143)** | **> 15 to ≤ 24 months (N = 121)** | **> 24 months (N = 100)** | **≤ 15 months (N = 38)** |
| Baseline^{a} | | | | | |
| | n | 143 | 108 | 70 | 38 |
| | Mean (SD) | 367.10 (120.278) | 366.33 (105.788) | 367.77 (105.001) | 350.14 (119.776) |
| | Median (min, max) | 373.75 (67.0, 675.4) | 369.25 (79.0, 575.0) | 376.43 (105.0, 575.0) | 358.45 (112.5, 623.0) |
| Change from baseline^{b} | | | | | |
| | n | 143 | 108 | 70 | 38 |
| | Mean (SD) | 17.13 (44.916) | 17.67 (55.609) | 9.67 (67.129) | 16.40 (69.027) |
| | Median (min, max) | 12.00 (-78.0, 179.5) | 11.75 (-173.1, 185.5) | 5.23 (-260.0, 179.5) | 3.15 (-55.6, 355.2) |

| | | | | | |
|---|---|---|---|---|---|
| Abbreviations: 6MWD = 6-minute walk distance; max = maximum; min = minimum; N = number of subjects in each treatment group; n = number of subjects with available data; SAP = statistical analysis plan; SCE = Summary of Clinical Efficacy; SD = standard deviation ^{a} For Studies ATB200-02 and ATB200-03, baseline was the same as the study baseline. For Study ATB200-07, the extension to Study ATB200-03, baseline depended on the treatment group the subject was randomized to in Study ATB200-03. Refer to the SCE SAP for detailed definition. ^{b} Change from baseline = the last available post-baseline value - baseline value, with no imputation for missing value | | | | | |

### Pulmonary Function

The data demonstrate small decreases in % predicted FVC that are slightly greater at > 24 months in subjects receiving cipaglucosidase alfa/miglustat. The mean (SD) change from baseline in FVC for the cipaglucosidase alfa/miglustat group was -0.74 (6.121) at ≤ 15 months (n = 139), -0.88 (7.103) at > 15 to ≤ 24 months (n = 97), and -2.55 (8.393) at > 24 months (n = 68). No long-term data for the alglucosidase alfa/placebo group are available, as all subjects switched to cipaglucosidase alfa/miglustat in Study ATB200-07. These improvements in FVC were maintained beyond 24 months with the outlier included or excluded. A summary of sitting % predicted FVC by exposure interval of pooled efficacy data including the outlier is presented in Table 60.

**Table 60: Summary of Sitting % Predicted FVC by Exposure Interval (Pool 2: Studies ATB200-02/03/07 - Efficacy Population Including the Outlier)**

| | | **Cipaglucosidase Alfa/Miglustat (N = 143)** | | | **Alglucosidase Alfa/Placebo (N = 38)** |
|---|---|---|---|---|---|
| | | **≤ 15 months (N = 143)** | **> 15 to ≤ 24 months (N = 121)** | **> 24 months (N = 100)** | **≤ 15 months (N = 38)** |
| Baseline^{a} | | | | | |
| | n | 139 | 97 | 68 | 38 |
| | Mean (SD) | 67.51 (19.836) | 66.89 (20.174) | 67.15 (19.174) | 70.04 (21.301) |
| | Median (min, max) | 68.00 (30.5, 132.5) | 67.50 (30.5, 132.5) | 69.00 (31.0, 107.0) | 71.25 (31.5, 122.0) |
| Change from baseline^{b} | | | | | |
| | n | 139 | 97 | 68 | 38 |
| | Mean (SD) | -0.74 (6.121) | -0.88 (7.103) | -2.55 (8.393) | -3.64 (5.170) |
| | Median (min, max) | -1.00 (-16.0, 22.5) | -1.00 (-24.0, 17.0) | -2.25 - (-25.0, 17.0) | -3.00 (-19.5, 7.5) |

| | | | | | |
|---|---|---|---|---|---|
| Abbreviations: FVC = forced vital capacity; max = maximum; min = minimum; N = number of subjects in each treatment group; n = number of subjects with available data; SAP = statistical analysis plan; SCE = Summary of Clinical Efficacy; SD = standard deviation ^{a} For Studies ATB200-02 and ATB200-03, baseline is the same as the study baseline. For Study ATB200-07, the extension to Study ATB200-03, baseline depends on the treatment group the subject was randomized to in Study ATB200-03. Refer to the SCE SAP for detailed definition. ^{b} Change from baseline = the last available post-baseline value - baseline value, with no imputation for missing value | | | | | |

### MMT

The data demonstrate that the initial improvement in the MMT lower extremity score from baseline was maintained out to > 24 months in subjects receiving cipaglucosidase alfa/miglustat. The mean (SD) change from baseline in MMT lower extremity score for the cipaglucosidase alfa/miglustat group was 1.36 (3.807) in subjects exposed to cipaglucosidase alfa/miglustat at ≤ 15 months (n = 135), 1.73 (3.675) at > 15 to ≤ 24 months (n = 101), and 1.98 (4.527) at > 24 months (n = 65). These improvements in MMT were maintained beyond 24 months with the outlier included or excluded.

### GSGC

The data demonstrate that the initial improvement in the GSGC total score from baseline was maintained and improved further out to > 24 months in subjects receiving cipaglucosidase alfa/miglustat. The mean (SD) change from baseline in GSGC total score for the cipaglucosidase alfa/miglustat group was -0.51 (2.947) in subjects exposed to cipaglucosidase alfa/miglustat at ≤ 15 months (n = 123), -0.86 (3.086) at > 15 to ≤ 24 months (n = 87), and -1.12 (3.586) at > 24 months (n = 56). Note that a negative score indicates an improvement. These improvements in GSGC were maintained beyond 24 months with the outlier included or excluded.

### Subgroup Analyses

Analyses of the pooled efficacy data were performed by the following subgroups and by exposure interval (≤ 15 months, > 15 to ≤ 24 months, > 24 months):
- Gender: Male or Female
- ERT status (categorized as ERT-experienced versus ERT-naïve)
- Race: White, Japanese, Asian (excluding Japanese), Black or African American, and Other (or White, Non-white)
- Baseline 6MWD (categorized as 75 to < 150 meters, 150 to < 400 meters, and ≥ 400 meters)
- Age: ≥ 18 to < 35 years, ≥ 35 to < 50 years, ≥ 50 to < 65 years, and ≥ 65 years
- Prior ERT duration: ≥ 2 to < 3 years, ≥ 3 to < 5 years, ≥ 5 to < 8 years, and ≥ 8 years
- Prior infusion-associated reaction: yes and no

Efficacy results by subgroups were generally consistent with the results in the overall population. Although sample size was limited in many subgroups and results should be interpreted carefully, a few differences across subgroups for change from baseline in 6MWD are noted below. For race, there were too few non-white subjects to interpret these subgroup analyses.

For change from baseline in 6MWD, there was no notable difference between the results in males and females, or among ERT-experienced subjects by duration of prior ERT exposure or based on history of IARs. While CIs were overlapping, there was a consistent observation of greater mean change from baseline in 6MWD in ERT-naïve subjects compared to ERT-experienced subjects, with notable differences between the ERT subgroups for subjects with less than 15 months exposure (on either cipaglucosidase alfa/miglustat or alglucosidase alfa/placebo). Similarly, while the number of subjects with very low baseline 6MWDs was quite small, there was a consistent observation of greater improvement from baseline among those with higher baseline 6MWDs than among those with the lowest baseline 6MWD. The observations across age categories were also notable for an observation of lower improvement with increasing age, with the most notable differences in the oldest age category, though the number of subjects ≥ 65 years was small.

For change from baseline in % predicted FVC, there was no notable difference between the results in males and females, ERT-experienced versus ERT-naïve, baseline 6MWD, age categories, or among ERT-experienced subjects by duration of prior ERT exposure or based on history of IARs.

### Example 23: Efficacy and/or Tolerance

Long-term data across Studies ATB200-02, ATB200-03, and ATB200-07 support the durability and persistence of efficacy of cipaglucosidase alfa/miglustat. Data from the controlled Study ATB200-03 showed continued improvement from initiation through Week 52, and data from integrated analyses across studies showed that the improvements were maintained or continued > 24 months. Study ATB200-02 showed improvements that were maintained or continued up to 48 months.

Efficacy data from the 3 adult Pompe disease clinical studies were pooled and analyzed to further support the long-term efficacy of cipaglucosidase alfa/miglustat in adult subjects. Data from non-ambulatory subjects (Study ATB200-02 Cohort 2) were not included in the pool as there was little overlap of efficacy endpoints for this group with the various ambulatory groups, such as 6MWD and lower MMT.

The 3 adult Pompe disease clinical studies were pooled as follows:
- The cipaglucosidase alfa/miglustat treatment group consists of:
   o All ambulatory subjects in Study ATB200-02 (Cohorts 1, 3, and 4),
   o All subjects randomized to the cipaglucosidase alfa/miglustat treatment group in Study ATB200-03, and
   o All subjects enrolled in Study ATB200-07.
- The alglucosidase alfa/placebo treatment group consists of:
   o All subjects randomized to the ERT treatment group in Study ATB200-03.

A total of 151 subjects with LOPD have been exposed to cipaglucosidase alfa/miglustat in Studies ATB200-02, ATB200-03, and ATB200-07. The mean (SD) duration of exposure was 28.0 (14.27) months, and 105 subjects have been exposed for more than 24 months. The longest exposure to cipaglucosidase alfa/miglustat for any subject was 64.9 months. Note that all subjects who completed Study ATB200-03 enrolled in Study ATB200-07 and continued or initiated treatment with cipaglucosidase alfa/miglustat.

The long-term data from Study ATB200-02 also support that the effects of cipaglucosidase alfa/miglustat were durable and maintained through 48 months. As of the data cutoff of 13 December 2021, a total of 29 subjects were enrolled and treated in Study ATB200-02 and the majority of subjects had ≥ 48 months of exposure. A summary of the efficacy results across the main Pompe disease domains in Study ATB200-02 is presented in Table 26. Briefly, improvements in motor function observed over the first year were maintained in ambulatory subjects across cohorts regardless of ERT experience. Treatment also resulted in improved PFTs for ERT-naïve subjects and stable or improved PFTs for ERT-experienced subjects, regardless of ambulatory status, as measured by % predicted FVC, MIP, MEP, and SNIP, as well as stable or improved muscle strength in all tested body parts of both ambulatory and non-ambulatory subjects, as measured by MMTs. Improvements were sustained up to 48 months of exposure.

The long-term data from the open-label extension of Study ATB200-03, Study ATB200-07, support that the effects of cipaglucosidase alfa/miglustat were maintained for at least 24 months. As of the data cutoff of 11 January 2022, a total of 118 subjects were enrolled and treated in Study ATB200-07, and 79 subjects had > 24 months of exposure. A summary of the efficacy results across the main Pompe disease domains in Study ATB200-07 is presented in Table 56. Briefly, the majority of the 6 main endpoints (ie, 6MWD, MMT lower extremity, PROMIS-Physical Function, PROMIS-Fatigue, and GSGC scores) for both treatment groups remained stable or improved through Study ATB200-03. For subjects who stayed on cipaglucosidase alfa/miglustat through Study ATB200-07, the benefits of treatment with cipaglucosidase alfa/miglustat were maintained. Subjects who switched from alglucosidase alfa/placebo to cipaglucosidase alfa/miglustat in Study ATB200-07 showed stability or improvement on the majority of endpoints. For sitting % predicted FVC, subjects who stayed on cipaglucosidase alfa/miglustat through Study ATB200-07 showed a slight decline from baseline through Study ATB200-03, then stabilized through Study ATB200-07. Subjects who switched from alglucosidase alfa/placebo to cipaglucosidase alfa/miglustat in Study ATB200-07 showed a substantial decline in Study ATB200-03, then stabilized at a lower level through Study ATB200-07.

For subjects in the OLE-ES Population who were treated with cipaglucosidase alfa/miglustat through Studies ATB200-03 and ATB200-07, CK and Hex4 declined substantially through Study ATB200-03, then stabilized through Study ATB200-07. For subjects who switched to cipaglucosidase alfa/miglustat in Study ATB200-07, CK remained generally stable at or slightly above the Study ATB200-03 baseline values through Week 52 while subjects were treated with alglucosidase alfa/placebo, then declined substantially after subjects switched to cipaglucosidase alfa/miglustat through Week 104. Levels of Hex4 increased from the Study ATB200-03 baseline through Week 52 while subjects were treated with alglucosidase alfa/placebo, then decreased substantially after subjects switched to cipaglucosidase alfa/miglustat through Week 104.

Integrated analyses of efficacy across Studies ATB200-02, ATB200-03, and ATB200-07 show that the effects of cipaglucosidase alfa/miglustat were durable and maintained through 24 months.

### Conclusions

Pompe is a monogenetic disease directly attributable to mutation in the gene that encodes GAA and a resulting deficiency of GAA, primarily in heart, skeletal muscle, and liver, leading to accumulation of lysosomal glycogen and related disease pathophysiology. Alglucosidase alfa has demonstrated the clinical benefit of IV ERT with rhGAA for patients with Pompe disease, showing initial improvement in FVC and other parameters. However as described above, over the last decade clinical evidence has demonstrated that the burden of disease remains high and substantial morbidity persists even with ERT treatment. After an initial improvement, many patients treated with alglucosidase alfa continue to decline such that they require ambulatory and ventilator support. Cipaglucosidase alfa/miglustat addresses important mechanistic and clinical unmet need for a more effective therapy to treat Pompe disease.

In the pivotal Study ATB200-03, in the overall population, the key study endpoints assessing motor function, pulmonary function, and muscle strength improved from baseline and favored cipaglucosidase alfa/miglustat over alglucosidase alfa/placebo. Subjects treated with cipaglucosidase alfa/miglustat walked on average 21 meters farther than baseline compared to 7 meters for the alglucosidase alfa/placebo patients, a difference of 14 meters (p = 0.071).

On the first key secondary endpoint of sitting % predicted FVC, subjects treated with cipaglucosidase alfa/miglustat showed a 0.9% absolute decline compared with a 4.0% absolute decline in the alglucosidase alfa/placebo arm, a difference of 3.0%, which was nominally statistically significant and represents a clinically meaningful stabilization of FVC (p = 0.023). This relative benefit in FVC versus alglucosidase alfa/placebo is of similar magnitude as the benefit observed in Phase 3 studies of alglucosidase alfa versus placebo and is considered clinically meaningful to patients based on Minimal clinically important differences (MCIDs) reported in the medical literature.

To further support the significance and consistency of the treatment effect of cipaglucosidase alfa/miglustat on the primary and key secondary endpoints of 6MWD and % predicted FVC, a post hoc global test was performed to assess superiority of cipaglucosidase alfa/miglustat versus alglucosidase alfa/placebo. This global test showed greater improvement in subjects treated with cipaglucosidase alfa/miglustat versus alglucosidase alfa/placebo (p = 0.010) and supports the results observed on each of these endpoints separately in the various populations studied.

The results observed in 6MWD and FVC for cipaglucosidase alfa/miglustat represent clinically meaningful improvements over alglucosidase alfa/placebo. For FVC, the 3% mean improvement for subjects treated with cipaglucosidase alfa/miglustat relative to alglucosidase alfa/placebo indicates a clinically meaningful group-level improvement and is of similar magnitude as the clinically meaningful improvement alglucosidase alfa itself demonstrated versus placebo. Additional patient-level responder analyses show that a greater proportion of subjects treated with cipaglucosidase alfa/miglustat had clinically meaningful improvement, and a smaller proportion had clinically meaningful worsening, than patients treated with alglucosidase alfa/placebo for 6MWD (p = 0.018) and FVC (p = 0.011) individually, and for both 6MWD and FVC (p = 0.002). Additional sensitivity analyses demonstrate that across a range of 6MWD and FVC response thresholds, a greater proportion of subjects treated with cipaglucosidase alfa/miglustat showed clinically meaningful improvement and a smaller proportion showed clinically meaningful worsening versus subjects treated with alglucosidase alfa/placebo. The improvements observed for 6MWD and % predicted FVC were supported by results of the additional key secondary endpoints that spanned motor function and muscle strength, pulmonary function, and PROs, which all numerically favored cipaglucosidase alfa/miglustat. GSGC, a composite measure of functional independence, showed nominally significant improvement for superiority of cipaglucosidase alfa/miglustat over alglucosidase alfa/placebo (p = 0.009).

In the ERT-experienced Population with high unmet need, cipaglucosidase alfa/miglustat showed clinically meaningful and nominally statistically significant improvements for both 6MWD (difference of 17 meters; p = 0.047) and % predicted FVC (difference of 4.1%; p = 0.006) compared to alglucosidase alfa. Subjects remaining on alglucosidase alfa demonstrated no change in 6MWD and experienced significant decline in FVC, whereas subjects switching to cipaglucosidase alfa/miglustat demonstrated an improvement in 6MWD and stabilization of FVC. For the ERT-naïve subgroup, subjects in both treatment groups demonstrated similar clinically meaningful improvements in 6MWD and changes in FVC.

Across musculoskeletal and several other secondary endpoints assessing motor function, muscle strength, and PROs, the vast majority showed improvement and directionally favored cipaglucosidase alfa/miglustat over alglucosidase alfa/placebo, supporting the observations in the primary and key secondary endpoints. In short, cipaglucosidase alfa/miglustat improved important Pompe disease domains over the approved therapy alglucosidase alfa. Additionally, nominally statistically significant (p < 0.001) reductions were observed versus alglucosidase alfa/placebo on key biomarkers of glycogen reduction (Hex4) and muscle damage (CK), confirming the strong mechanistic nonclinical evidence. Data from Studies ATB200-02 and ATB200-07 are consistent with Study ATB200-03 and provide strong evidence of durability of effect with improvements and stability lasting beyond 24 months. Altogether, the results of the clinical studies demonstrate that cipaglucosidase alfa/miglustat addresses the significant need for a more effective, next-generation therapy in the treatment of Pompe disease.

### ITEMS

1. A method for improving and/or stabilizing for more than 24 months one or more measure of muscle strength, motor function, pulmonary function or pharmacodynamic measures relative to baseline in a subject having Pompe disease, the method comprising administering to the subject a population of recombinant human acid α-glucosidase (rhGAA) molecules, concurrently or sequentially, with an enzyme stabilizer,
   wherein each rhGAA molecule comprises seven potential N-glycosylation sites, and the rhGAA molecules comprise at least 0.5 mol bis-mannose-6-phosphate (bis-M6P) per mol of rhGAA at the first potential N-glycosylation site.
2. The method of item 1, wherein the measure is improved and/or stabilized for more than 36 months.
3. The method of item 1 or 2, wherein the measure is improved and/or stabilized for at least 48 months.
4. The method of any one of items 1-3, wherein the quantity of bis-M6P at the first potential glycosylation site is determined by LC MS/MS.
5. The method of any one of items 1-4, wherein the measure is improved relative to baseline for a first period and stabilized for a second period.
6. The method of any one of items 1-5, wherein the subject is an enzyme replacement therapy (ERT)-experienced subject.
7. The method of any one of items 1-5, wherein the subject is an enzyme replacement therapy (ERT)-naïve subject.
8. The method of any one of items 1-7, wherein muscle strength is measured by a manual muscle test (MMT).
9. The method of any one of items 1-8, wherein muscle strength score improves relative to baseline by at least 1.5, 2.0, or 2.5 after more than 24 months of treatment.
10. The method of any one of items 1-9, wherein motor function is measured by a 6-minute walk test.
11. The method of any one of items 1-10, wherein the subject is ERT-experienced and motor function improves relative to baseline by at least 15, 20, or 25 meters after more than 24 months of treatment.
12. The method of any one of items 1-11, wherein the subject is ERT-naïve and motor function improves relative to baseline by at least 30, 40, or 50 meters after more than 24 months of treatment.
13. The method of any one of items 1-12, wherein pulmonary function is measured by a sitting forced vital capacity (FVC) test.
14. The method of any one of items 1-13, wherein the subject's percent-predicted FVC is stable compared to baseline after more than 24 months of treatment.
15. The method of any one of items 1-14, wherein the ERT-experienced subject had been previously treated with alglucosidase alfa.
16. The method of any one of items 1-15, wherein the ERT-experienced subject had been previously treated with alglucosidase alfa for from about 2 years to about 6 years.
17. The method of any one of items 1-15, wherein the ERT-experienced subject had been previously treated with alglucosidase alfa for at least about 7 years.
18. The method of any one of items 1-17, wherein the subject is non-ambulatory.
19. The method of any one of items 1-17, wherein the subject is ambulatory.
20. The method of any one of items 1-19, wherein the pharmacodynamic measure is creatine kinase (CK) or urine hexose tetrasaccharide (Hex4).
21. The method of any one of items 1-20, wherein the population of rhGAA molecules is administered at a dose of 5 mg/kg to 40 mg/kg.
22. The method of any one of items 1-21, wherein the population of rhGAA molecules is administered at a dose of 20 mg/kg.
23. The method of any one of items 1-22, wherein the population of rhGAA molecules is administered bi-weekly.
24. The method of any one of items 1-23, wherein the population of rhGAA molecules is administered intravenously.
25. The method of any one of items 1-24, wherein the enzyme stabilizer is miglustat or a pharmaceutically acceptable salt thereof.
26. The method of item 25, wherein the miglustat or pharmaceutically acceptable salt thereof is administered orally.
27. The method of item 25 or 26, wherein the miglustat or pharmaceutically acceptable salt thereof is administered at a dose of 195 mg or 260 mg.
28. The method of any one of items 25-27, wherein the miglustat or pharmaceutically acceptable salt thereof is administered prior to administration of the population of rhGAA molecules.
29. The method of any one of items 25-28, wherein the miglustat or pharmaceutically acceptable salt thereof is administered one hour prior to administration of the population of rhGAA molecules.
30. The method of any one of items 25-29, wherein the subject fasts for at least two hours before and at least two hours after the administration of miglustat or a pharmaceutically acceptable salt thereof.
31. The method of any one of items 1-30, wherein the rhGAA molecules comprise an amino acid sequence at least 90% identical to SEQ ID NO: 4.
32. The method of any one of items 1-31, wherein the rhGAA molecules comprise an amino acid sequence at least 98% identical to SEQ ID NO: 6.
33. The method of any one of items 1-32, wherein the rhGAA molecules comprise the amino acid sequence of SEQ ID NO: 4 or SEQ ID NO: 6.
34. The method of any one of items 1-33, wherein 40%-60% of the N-glycans on the rhGAA molecules are complex type N-glycans.
35. The method of any one of items 1-34, wherein at least 30% of the rhGAA molecules comprise one or more N-glycan units bearing one mannose-6-phosphate residue (mono-M6P) or bis-M6P.
36. The method of any one of items 1-35, wherein the rhGAA molecules comprise on average from 0.5 mol to 7.0 mol of mono-M6P or bis-M6P per mol of rhGAA.
37. The method of any one of items 1-36, wherein the rhGAA molecules comprise on average from 2.0 to 8.0 mol of sialic acid per mol of rhGAA.
38. The method of any one of items 1-37, wherein the rhGAA molecules comprise on average at least 2.5 mol M6P per mol of rhGAA and at least 4 mol sialic acid per mol of rhGAA.
39. The method of any one of items 1-38, wherein, per mol of rhGAA, the rhGAA molecules comprise on average:
   (a) 0.4 to 0.6 mol mono-M6P at the second potential N-glycosylation site;
   (b) 0.4 to 0.6 mol bis-M6P at the fourth potential N-glycosylation site; or
   (c) 0.3 to 0.4 mol mono-M6P at the fourth potential N-glycosylation site.
40. The method of item 39, wherein, per mol of rhGAA, the rhGAA molecules further comprise 4 mol to 7.3 mol sialic acid,
   wherein, per mol of rhGAA, the rhGAA molecules comprise on average:
   (a) 0.9 to 1.2 mol sialic acid at the third potential N-glycosylation site;
   (b) 0.8 to 0.9 mol sialic acid at the fifth potential N-glycosylation site; or
   (c) 1.5 to 4.2 mol sialic acid at the sixth potential N-glycosylation site.
41. The method of any one of items 1-40, wherein the population of rhGAA molecules is formulated in a pharmaceutical composition further comprising at least one pharmaceutically acceptable buffer, excipient, or carrier.
42. The method of items 41, wherein the pharmaceutical composition further comprises at least one buffer selected from the group consisting of a citrate, a phosphate, and a combination thereof, and at least one excipient selected from the group consisting of mannitol, polysorbate 80, and a combination thereof; wherein the pharmaceutical composition has a pH of 5.0 to 7.0.
43. The method of item 41 or 42, wherein the pharmaceutical composition has a pH of 5.0 to 6.0.
44. The method of any one of items 41-43, wherein the pharmaceutical composition further comprises water, an acidifying agent, an alkalizing agent, or a combination thereof.
45. The method of any one of items 41-44, wherein, in the pharmaceutical composition, the population of rhGAA molecules is present at a concentration of 5-50 mg/mL, the at least one buffer is a sodium citrate buffer present at a concentration of 10-100 mM, the at least one excipient is mannitol present at a concentration of 10-50 mg/mL and polysorbate 80 present at a concentration of 0.1-1 mg/mL, and the pharmaceutical composition further comprises water and optionally comprises an acidifying agent and/or alkalizing agent; wherein the pharmaceutical composition has a pH of 6.0.
46. The method of any one of items 41-45, wherein, in the pharmaceutical composition, the population of rhGAA molecules is present at a concentration of 15 mg/mL, the sodium citrate buffer is present at a concentration of 25 mM, the mannitol is present at a concentration of 20 mg/mL, and the polysorbate 80 is present at a concentration of 0.5 mg/mL.
47. The method of any one of items 1-46, wherein the rhGAA is produced from Chinese hamster ovary (CHO) cells.
48. The method of any one of items 1-47, wherein at least 6% of the total N-glycan units on the rhGAA molecules are glycans bearing mono-M6P residues.
49. The method of any one of items 1-48, wherein at least 3% of the total N-glycan units on the rhGAA molecules are glycan bearing bis-M6P residues.
50. The method of any one of items 1-49, wherein up to 55% of the total N-glycans on the rhGAA molecules are sialylated complex glycans.

## Claims

1. A pharmaceutical composition comprising miglustat, or a pharmaceutically acceptable salt thereof, and one or more of microcrystalline cellulose, pregelatinized starch, sucralose, magnesium stearate, or colloidal silicon dioxide.

2. The pharmaceutical composition of claim 1, wherein the pharmaceutical composition comprises miglustat, or a pharsmaceutically acceptable salt thereof, microcrystalline cellulose, pregelatinized starch, sucralose, magnesium stearate, and colloidal silicon dioxide.

3. The pharmaceutical composition of claim 1 or claim 2, wherein the pharmaceutical composition is a hard gelatin capsule for oral administration, comprising 65 mg miglustat, or a pharmaceutically acceptable salt thereof.

4. The pharmaceutical composition of any of claims 1-3, wherein the pharmaceutical composition comprises 20%-40% by weight miglustat, or a pharmaceutically acceptable salt thereof, 40%-60% by weight microcrystalline cellulose, 5%-25% by weight pregelatinized starch, 0.1%-5% by weight sucralose, 0.1%-5% by weight magnesium stearate, and 0.1%-5% by weight colloidal silicon dioxide.

5. The pharmaceutical composition of any of claims 1-4, wherein the pharmaceutical composition comprises 30-35% by weight miglustat, or a pharmaceutically acceptable salt thereof, 45-55% by weight microcrystalline cellulose, 10-20% by weight pregelatinized starch, 0.2-1% by weight sucralose, 0.2-1% by weight magnesium stearate, and 0.2-1% by weight colloidal silicon dioxide.

6. The pharmaceutical composition of any of claims 1-5, wherein the pharmaceutical composition comprises about 65 mg miglustat or a pharmaceutically acceptable salt thereof, about 50 to about 150 mg microcrystalline cellulose, about 20 to about 50 mg pregelatinized starch, about 0.1 to about 5 mg sucralose, about 0.1 to about 5 mg magnesium stearate, and about 0.1 to about 5 mg colloidal silicon dioxide.

7. The pharmaceutical composition of any of claims 1-6, wherein the pharmaceutical composition comprises about 65 mg miglustat or a pharmaceutically acceptable salt thereof, about 75 to about 125 mg microcrystalline cellulose, about 30 to about 40 mg pregelatinized starch, about 0.5 to about 2 mg sucralose, about 0.5 to about 2 mg magnesium stearate, and about 0.2 to about 1 mg colloidal silicon dioxide.

8. The pharmaceutical composition of any of claims 1-7, wherein the pharmaceutical composition is provided in a hard gelatin capsule for oral administration and comprises about 65 mg miglustat or an amount of a pharmaceutically acceptable salt of miglustat that is equivalent to about 65 mg of miglustat, about 100 mg microcrystalline cellulose, about 32.6 mg pregelatinized starch, about 1 mg sucralose powder, about 1 mg magnesium stearate, and about 0.4 mg colloidal silicon dioxide.

9. The pharmaceutical composition of any of claims 1-8 for use in a method of treating Pompe disease in a subject, wherein the method comprises administering to the subject a population of recombinant human acid alpha-glucosidase (rhGAA) molecules, concurrently or sequentially with the pharmaceutical composition of any of claims 1-8.

10. The pharmaceutical composition for use of claim 9, wherein the pharmaceutical composition comprising miglustat or a pharmaceutically acceptable salt thereof is administered prior to administration of the population of rhGAA molecules.

11. The pharmaceutical composition for use of claim 9 or claim 10, wherein the pharmaceutical composition comprising miglustat or a pharmaceutically acceptable salt thereof is administered one hour prior to administration of the population of rhGAA molecules.

12. The pharmaceutical composition for use of any of claims 9-11, wherein the subject fasts for at least two hours before and at least two hours after administration of the pharmaceutical composition comprising miglustat or a pharmaceutically acceptable salt thereof.

13. The pharmaceutical composition for use of any of claims 9-12, wherein the population of rhGAA molecules and the pharmaceutical composition comprising miglustat or a pharmaceutically acceptable salt thereof are administered every two weeks.

14. The pharmaceutical composition for use of any of claims 9-13, wherein:
(i) for patients weighing 50 kg or more, four 65 mg capsules are administered; or
(ii) for patients weighing between 40 and 50 kg, a dosage of three 65 mg capsules is administered.

15. The pharmaceutical composition for use of any of claims 9-14, wherein the pharmaceutical composition comprising miglustat or a pharmaceutically acceptable salt thereof is administered orally while the population of rhGAA molecules is administered intravenously.
